(19) Europäisches Patentamt
European Patent Office
Office européen des brevets

(11) **EP 3 891 167 B1**

(12) **EUROPEAN PATENT SPECIFICATION**

(45) Date of publication and mention of the grant of the patent:
**13.11.2024 Bulletin 2024/46**

(21) Application number: **19827769.1**

(22) Date of filing: **05.12.2019**

(51) International Patent Classification (IPC):
***C07J 41/00** (2006.01)* ***C07J 43/00** (2006.01)*
***C07J 71/00** (2006.01)* ***A61K 31/58** (2006.01)*
***A61P 35/00** (2006.01)*

(52) Cooperative Patent Classification (CPC):
**C07J 41/0072; C07J 43/003; C07J 71/0052**

(86) International application number:
**PCT/FI2019/050874**

(87) International publication number:
**WO 2020/115371 (11.06.2020 Gazette 2020/24)**

(54) **ESTRA-1,3,5(10)-TRIENE COMPOUNDS CONDENSED IN POSITION 16(17) WITH A PYRAZOLE RING AS INHIBITORS OF 17-HSD1**

ESTRA-1,3,5 (10)-TRIEN-VERBINDUNGEN, KONDENSIERT IN POSITION 16 (17) MIT EINEM PYRAZOLRING ALS INHIBITOREN VON 17-HSD1

COMPOSÉS ESTRA-1,3,5(10)-TRIÈNE CONDENSÉS EN POSITION 16(17) AVEC UN CYCLE PYRAZOLE UTILISÉS COMME INHIBITEURS DE LA 17-HSD1

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.12.2018 FI 20186056**

(43) Date of publication of application:
**13.10.2021 Bulletin 2021/41**

(73) Proprietor: **Organon R&D Finland Oy**
**20520 Turku (FI)**

(72) Inventors:
- **HIRVELÄ, Leena**
  **20520 Turku (FI)**
- **HAKOLA, Marjo**
  **20520 Turku (FI)**
- **LINNANEN, Tero**
  **21180 Naantali (FI)**
- **KOSKIMIES, Pasi**
  **20520 Turku (FI)**
- **STJERNSCHANTZ, Camilla**
  **20520 Turku (FI)**

(74) Representative: **Kolster Oy Ab**
**Salmisaarenaukio 1**
**P.O. Box 204**
**00181 Helsinki (FI)**

(56) References cited:
**WO-A1-2006/125800** **WO-A1-2014/207310**
**WO-A2-2005/047303** **WO-A2-2018/224736**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

FIELD OF THE INVENTION

The present invention relates to novel steroidal C-15 derivatives, to their pharmaceutically acceptable salts, and their use in therapy. The invention further relates to pharmaceutical compositions comprising these compounds as active ingredients and to methods for their preparation.

BACKGROUND OF THE INVENTION

17β-hydroxysteroid dehydrogenases (17β-HSDs), also known as 17-ketosteroid reductases (17-KSR) are NAD(H)- and/or NAPD(H)-dependent alcohol oxidoreductase enzymes which catalyse the last and key step in formation of all estrogens and androgens. More specifically 17β-HSDs catalyse the dehydrogenation (oxidation) of 17-hydroxysteroids into corresponding 17-ketosteroids or hydrogenation (reduction) of inactive 17-ketosteroids into corresponding active 17-hydroxysteroids.

As both estrogens and androgens have the highest affinity for their receptors in the 17β-hydroxy form, the 17β-HSD/KSRs regulate the biological activity of the sex hormones. At present, 15 human members of 17β-HSDs have been described (type 1 - 15). Different types of 17β-HSD/KSRs differ in their substrate and cofactor specificities. The 17KSR activities convert low-activity precursors to more potent forms while 17β-HSD activities decrease the potency of estrogens and androgens and consequently may protect tissues from excessive hormone action.

Each type of 17β-HSD has a selective substrate affinity and a distinctive, although in some cases overlapping, tissue distribution.

Type 1 17β-hydroxysteroid dehydrogenase (17β-HSD1) is most abundantly expressed in the ovarian granulosa cells of the developing follicles in ovaries and in human placenta, both being estrogen biosynthetic tissues. In addition, 17β-HSD1 is expressed in estrogen target tissues, including breast, endometrium and bone. The human 17P-HSD1 is specific to estrogenic substrates and *in vivo* catalyzes the reduction of estrone to estradiol.

Type 2 17β-hydroxysteroid dehydrogenase (17β-HSD2) on the other hand converts estradiol, testosterone and 5a-dihydrotestrosterone to their less active forms estrone, androstenedione and 5a-androstanedione, respectively. Due to its wide and abundant expression in number of various estrogen and androgen target tissues, such as uterus, placenta, liver and the gastrointestinal and urinary tracts, it has been suggested that type 2 enzyme protects tissues from excessive steroid actions.

Estradiol (E2) is about 10 times as potent as estrone (E1) and about 80 times as potent as estratriol (E3) in its estrogenic effect. In contrast to certain other estrogens, estradiol binds well to both estrogen receptors ERα and ERβ, and thus regulates the expression of a variety of genes.

Although both 17P-HSD1 and 17β-HSD2 are present in healthy pre-menopausal humans, increased ratio of 17P-HSD1 to 17-HSD2 in the tumors of postmenopausal patients with hormone-dependent breast cancer has been shown in several studies. 17HSD1 gene amplification and loss of heterozygosity of 17HSD2 allele are potential mechanisms involved to increased reductive estrogen synthesis pathway in breast tumors. Increased ratio of type 1 enzyme to type 2 enzyme results in an increased level of estradiol that then promotes the proliferation of the cancerous tissue via the estrogen receptors (ER). High levels of estrogen thus support certain cancers such as breast cancer and cancer of the uterine lining i.e. endometrial cancer and uterine cancer.

Similarly it has been suggested that 17β-HSD2 is down-regulated in endometriosis while both aromatase and 17P-HSD1 are expressed or up-regulated in comparison with normal endometrium. This again results in the presence of high concentration of estradiol (E2) which drives the proliferation of the tissue. Similar mechanism has been elucidated in uterine leiomyoma (uterine fibroids) and endometrial hyperplasia.

Reduction of the endogenous estradiol concentration in affected tissues will result in reduced or impaired proliferation of 17β-estradiol cells in said tissues and may thus be utilized in prevention and treatment of malign and benign estradiol dependent pathologies. Due to the proposed involvement of 17β-estradiol in a number of malign and benign pathologies, inhibitors of 17β-hydroxysteroid dehydrogenases, that can be used to impair endogenous production of estradiol from estrone, can have therapeutic value in the prevention or the treatment of such disorders or diseases are in great demand.

Some small-molecule inhibitors of 17P-HSD1 enzyme have been identified and reviewed in Poirier D. (2003) Curr Med Chem 10: 453-77 and Poirier D. (2010) Expert Opin. Ther. Patents 20(9): 1123-1145. Further, small molecule inhibitors of 17β-HSD's have been disclosed in WO 2001/42181, WO 2003/022835, WO 2003/033487, WO 2004/046111, WO 2004/060488, WO 2004/110459, WO 2005/032527, and WO 2005/084295.

WO2004/085457 discloses steroidal compounds capable of inhibiting 17β-hydroxysteroid dehydrogenase. WO2006/003012 discloses 2-substituted D-homo-estriene derivatives suitable for the treatment of estrogen-dependent diseases that can be influenced by the inhibition of the 17β-hydroxysteroid dehydrogenase type 1. Similarly

WO2006/003013 presents 2-substituted estratrienones usable for preventing and treating estrogen-dependent diseases influenced by inhibiting 17β-hydroxysteroid dehydrogenase type 1.

is-substituted estradiol analogues acting as locally active estrogens are presented in WO2004/085345. WO2006/027347 discloses 15 β-substituted estradiol derivatives having selective estrogenic activity for the treatment or prevention of estrogen receptor-related diseases and physiological conditions. Further, WO2005/047303 discloses 3, 15 substituted estrone derivatives capable of inhibiting the 17β-hydroxysteroid dehydrogenase type 1.

International application WO2008/034796 relates to estratrien triazoles suitable for use in treatment and prevention of steroid hormone dependent diseases or disorders requiring the inhibition of a 17β-hydroxysteroid dehydrogenases such as 17β-HSD type 1, type 2 or type 3 enzyme. Inhibitors of 17β-HSD type 3 enzyme have been disclosed in WO99/46279.

International applications WO2014/207309, WO2014/207310 and WO2014/207311 relate to estrone C-15 thiazole derivatives, estrone C-17 ketimine C-15 thiazole derivatives and estradiol C-15 thiazole derivatives, respectively, as well as their use in therapy.

BRIEF DESCRIPTION OF THE INVENTION

An object of the present invention is to provide compounds useful in treating disorders and diseases associated with increased level of estradiol and/or treatable by inhibition of 17P-HSD1 enzyme. It is further an object of the present invention to provide compounds that show little or no inhibitory effect on 17β-HSD2 enzyme.

One of the problems associated with the known 17P-HSD1 inhibitors is the disposition, in particular the metabolic stability, of the compounds. It is therefore yet a further object of the present invention to provide compounds with improved metabolic stability.

Some further problems associated with the known 17P-HSD1 inhibitors are the formation of conjugative metabolites and species selectivity of the compounds. It is therefore yet a further object of the present invention to provide compounds with improved properties in these parameters.

One further problem associated with the known 17P-HSD1 inhibitors is that while some inhibitors may show 17β-HSD1 inhibition, said inhibitors may not exhibit properties of low 17β-HSD2 inhibition, metabolic stability and/or inhibition in other species. It is therefore yet a further object of the present invention to provide compounds with improved one or more of said property (properties), including inhibition of 17β-HSD1.

The present invention provides novel compounds of formula (I)

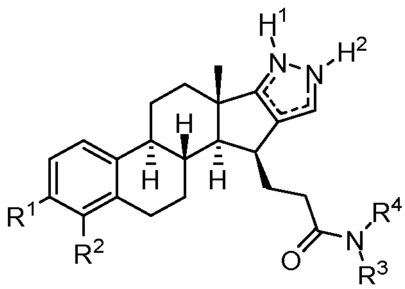

(I)

wherein

R1 and R2 are each independently selected from the group consisting of H and halogen;
(i) R3 is selected from the group consisting of H and C1-4-alkyl and
R4 is selected from the group consisting of

- phenyl, 6 membered unsaturated, or aromatic heterocycle comprising 1 to 3 heteroatom(s) independently selected from the group consisting of nitrogen, sulfur, and oxygen, and being optionally substituted with one to five substituent(s) independently selected from the group consisting of halogen, CN, C1-4 alkyl, C1-3-(per)haloalkyl, OH, oxo, C1-3-alkoxy, morpholino, C(O)N(C1-3-alkyl)$_2$ and a 6 membered saturated heterocycle with 1 to 3 heteroatom(s) selected from the group consisting of nitrogen, oxygen and sulfur, optionally substituted with C1-4 alkyl; and
- 6 membered saturated heterocycle comprising 1 to 3 heteroatom(s) independently selected from the group consisting of nitrogen, oxygen and sulfur and being optionally substituted with one to three substituent(s) independently selected from the group consisting of halogen, CN, C1-4-alkyl, C1-3-(per)haloalkyl, OH, oxo, and C1-3-alkoxy, or two adjacent substituents may form a 5 or 6 membered saturated fused ring;

provided that only one of hydrogens $H^1$ and $H^2$ is present at the same time, and the position of the double bonds in the pyrazole ring to which the hydrogens $H^1$ and $H^2$ are attached is determined based on which of the hydrogen $H^1$ and $H^2$ is present
or a pharmaceutically acceptable salt thereof.

The invention also relates to pharmaceutical composition comprising an effective amount on one or more compound(s) of formula (I).

Further the invention relates to a compound of formula (I) or a pharmaceutical acceptable salt thereof for use as a medicament.

Still further the invention relates to a compound of formula (I) or pharmaceutically acceptable salt thereof for use in the treatment of estradiol dependent malign or benign diseases or disorders.

Finally the invention provides a method for the preparation of compounds according to formula (I) and its intermediate compound according to formula (II).

DETAILED DESCRIPTION OF THE INVENTION

Compounds of the present disclosure contain steroidal core structure having a defined stereochemistry that is the natural configuration of estrogens.

Compounds of the present disclosure bear a side chain at C15, which, together with the specific substitution pattern of the A ring and C16-C17 fused pyrazole ring provides the inventive properties of the compounds of the present disclosure. These three modifications of native steroidal enhance the metabolic and/or inhibitory properties of the compounds of the present disclosure. Furthermore, metabolic and/or inhibitory properties are enhanced on other species, like in rabbit. The rabbit is by far the most common non-rodent species used for evaluation of reprotoxicity of small molecules. Target inhibition in the rabbit can therefore be considered an important and/or desirable feature for new compounds.

Compounds of the present disclosure show inhibition selectivity between 17β-HSD1 and 17β-HSD2. It is to be understood that compounds of the present disclosure show a higher inhibition of 17P-HSD1 than 17β-HSD2.

Compounds of the present disclosure show a 17P-HSD1 inhibition of at least 40%, preferably at least 60%, more preferably at least 70%, even more preferably at least 80%, most preferably at least 90%. The term "17β-HSD1 inhibition" as used herein and hereafter refers to inhibition of 17P-HSD1 by a compound (with a concentration of 100 nM) of the present disclosure determined with a method disclosed in chapter "Pharmacological tests" of the present disclosure.

In addition, or alternatively, compounds of the present disclosure show a 17β-HSD2 inhibition equal to or less than 40%, preferably equal to or less than 20%, more preferably equal to or less than 10%. The term "17β-HSD2 inhibition" as used herein and hereafter refers to inhibition of 17β-HSD2 by a compound (with a concentration of 1 μM) of the present disclosure determined with a method disclosed in chapter "Pharmacological tests" of the present disclosure.

In addition, or alternatively, compounds of the present disclosure show a metabolic stability corresponding to a T1/2 of at least 5 min, preferably at least 10 min, more preferably at least 20 min, even more preferably at least 40 min, still even more preferably at least 80 min, even more preferably at least 100 min, most preferably at least 140 min. The term "metabolic stability" as used herein and hereafter refers to susceptibility of compounds of the present disclosure to biotransformation. Example of metabolic stability include, but is not limited to, in vitro metabolic stability determined by using human hepatocyte incubation of a compound (with a concentration of 1 μM) of the present disclosure and expressed by the half life (T1/2, min), determined with a method disclosed in chapter "Pharmacological tests" of the present disclosure.

In addition, or alternatively, compounds of the present disclosure show inhibition in other species, wherein the inhibition is at least 10%, more preferably at least 20%, even more preferably at least 40%, most preferably at least 50%. The term "inhibition in other species" as used herein and hereafter refers to 17P-HSD1 inhibition in other species than human by a compound of the present disclosure. Examples of other species include, but is not limited to, rabbit, rat, mouse, pig, and dog. Example of inhibition in other species include, but is not limited to, the inhibition of E1 to E2 conversion in rabbit placenta tissue by a compound (with a concentration of 100 nM) of the present disclosure determined with a method disclosed in chapter "Pharmacological tests" of the present disclosure.

It is to be understood that the combination of the features of 17P-HSD1 inhibition, 17β-HSD2 inhibition, metabolic stability and/or inhibition in other species may be desirable. Therefore, the novel and inventive compounds of the present disclosure may exhibit a superior combination of said features.

The term "halogen" as used herein and hereafter by itself or as part of other groups refers to the Group VIIa elements and includes F, Cl, Br and I groups.

The term "alkyl" as used herein and hereafter is an aliphatic linear, branched or cyclic, especially linear or branched, hydrocarbon group having the indicated number of carbon atoms, for example $C_{1-6}$-alkyl has 1 to 6 carbon

atoms in the alkyl moiety and thus, for example, $C_{1-4}$-alkyl includes methyl, ethyl, n-propyl, isopropyl, n-butyl, sec-butyl, isobutyl, tert-butyl and $C_{1-6}$-alkyl additionally includes branched and straight chain pentyl and hexyl.

The term "alicycle" as used herein and hereafter refers to a cyclic aliphatic hydrocarbon, which is a hydrocarbon group with a ring structure with only carbon atoms forming the ring structure. The alicycle can be saturated, partially unsaturated or unsaturated.

The term "(per)haloalkyl" as used herein and hereafter refers to any of the above alkyl groups where one or more hydrogen atoms are replaced by halogenes): in particular I, Br, F or Cl. Examples of haloalkyl groups include without limitation chloromethyl, fluoromethyl and $-CH_2CF_3$. The term "perhaloalkyl" is understood to refer to an alkyl group, in which all the hydrogen atoms are replaced by halogen atoms. Preferred examples include trifluoromethyl ($-CF_3$) and trichloromethyl ($-CCl_3$).

The term "$C_{1-3}$-alkoxy" as used herein and hereafter refers to a -O-($C_{1-3}$-alkyl) group where the "$C_{1-3}$-alkyl" has the above-defined meaning. Examples of preferred alkoxy groups include, but are not limited to, methoxy, ethoxy, and iso-propyloxy.

The term "sulfonyl" as used herein and hereafter refers to a sulfonyl group having the general structure $-S(=O)_2-$ or -SOz- where the sulfur (S) is attached to two separate carbon atoms and the sulfur is substituted with two oxo-groups. The sulfonyl group can also be part of a ring structure with the carbon atoms to which it is attached. The ring structure can include only carbon atoms in addition to the sulfonyl group or other heteroatoms such as but not limited to nitrogen, oxygen and sulfur.

The term "6 membered saturated heterocycle containing 1 to 3 heteroatom(s) independently selected from the group consisting of nitrogen, oxygen and sulfur", refers to a monocyclic ring, which is saturated and has 4 to 6 ring atoms, and comprises 1 heteroatom selected from N, S and O while the remaining ring atoms are carbon atoms. It may be substituted with one or two substituent(s) as denoted, in particular one, at any suitable ring atom, including N. Preferred substituent groups include, but are not limited to halogen, in particular fluoro, CN, methoxy, and methyl.

The term "4 to 6 membered unsubstituted saturated heterocycle containing 1 heteroatom selected from the group consisting of nitrogen, sulfur, and oxygen", refers to a monocyclic ring, which is saturated and has 4 to 6 ring atoms, and comprises 1 heteroatom selected from N, S and O while the remaining ring atoms are carbon atoms. Representing groups include oxetanyl, pyrrolidinyl, piperidinyl, and tetrahydropyranyl, in particular oxetanyl and tetrahydropyranyl.

The term "5 membered partially unsaturated heterocycle comprising 1 to 3 heteroatom(s) selected from the group consisting of nitrogen, sulfur, and oxygen" refers to a monocyclic ring which is partially unsaturated with 5 ring atoms comprising at least one double bond between the ring atoms and contains 1 to 3 heteroatom(s) selected from the group consisting of N, S and O, while the remaining ring atoms are carbon atoms. It may be substituted with one or two substituents as denoted, in particular one, at any suitable ring atom, including N. Preferred substituent groups include, but are not limited to halogen, in particular fluoro, CN, methoxy, and methyl.

The term "5 membered unsubstituted unsaturated or aromatic heterocycle containing 1 to 3 heteroatom(s) independently selected from the group consisting of nitrogen, sulfur, and oxygen" refers to a monocyclic ring with 5 ring atoms and which may be aromatic or unsaturated and which contains 1 to 3 heteroatom(s) independently selected from N, S and O, while the remaining ring atoms are carbon atoms.

The term "5 membered unsaturated or aromatic heterocycle" refers to a monocyclic ring with 5 ring atoms and which may be aromatic or unsaturated and comprises 1 to 3 heteroatom(s) independently selected from the group consisting of N, and O, while the remaining ring atoms are carbon atoms. It may be substituted with one or two substituents as denoted, in particular one, at any suitable ring atom, including N. Preferred substituent groups include, but are not limited to halogen, in particular fluoro, CN, methoxy, and methyl. Representing groups include oxazolyl and methyloxazolyl.

The term "6 membered unsaturated or aromatic heterocycle comprising 1 to 3 further heteroatom(s) independently selected from the group consisting of nitrogen, and oxygen" refers to a monocyclic ring with 6 ring atoms and which may be aromatic or unsaturated containing 1 to 3 heteroatom(s) independently selected from the group consisting of N, S, and O, while the remaining ring atoms are carbon atoms. It may be substituted with one or two, preferably one, substituents as denoted, in particular one, at any suitable ring atom, including N. Preferred substituent groups include, but are not limited to halogen, in particular fluoro, CN, methoxy, and methyl. Advantageously the substituent is at the para- and meta positions of the ring. Representing groups include pyridinyl, fluoropyridinyl, cyano-pyridinyl, methylpyridinyl, dimethylpyridinyl, isopropylpyridinyl, hydroxypyridinyl, methoxypyridinyl, morpholinopyridinyl, methylpiperazinylpyridinyl, pyrazinyl, methylpyridazinyl, and methoxypyridazinyl; in particular fluoropyridinyl, methoxypyridinyl, methylpyridazinyl, and methoxypyridazinyl.

The term "a 5 to 6 membered saturated heterocycle comprising nitrogen atom", refers to a saturated monocyclic ring with 6 ring atoms and contains 1 nitrogen atom while the remaining ring atoms are carbon atoms. It may be substituted with one or two substituent(s) as denoted, in particular one, at any suitable ring atom, including N. Preferred substituent groups include, but are not limited to halogen, in particular fluoro, CN, methoxy, and methyl. Representing groups include

pyrrolidinyl, and methoxymethylpyrrolidinyl.

The term "an unsubstituted bicyclic spirocyclic or fused heterocycle comprising said nitrogen atom and optionally 1 or 2 further heteroatom(s) selected from a group consisting of nitrogen, oxygen and sulfur" refers to a bicyclic ring system where the rings may be joined together as a spirocyclic system or as a fused system, preferably as a spirocyclic system, and contains a nitrogen atom and optionally 1 or 2 further heteroatom(s) selected from N, O and S as indicated while the remaining ring atoms are carbon atoms. Representing groups include oxaazaspiro [4.5]decanyl.

The term "a 5 or 6 membered saturated fused ring" refers to a fused ring, which is saturated or partly unsaturated and adds 3 to 4, accordingly, additional ring atoms to the original ring into which is fused and optionally comprises 1 to 3 heteroatoms each independently selected from N, S and O while the remaining ring atoms are carbon atoms.

The term "optionally substituted" as used herein and hereafter in context of a phenyl group denotes phenyl that is either unsubstituted or substituted independently with one or more, in particular 1, 2, or 3, substituent(s) attached at any available atom to produce a stable compound, e.g. pyridinyl may be substituted once with a denoted substituent attached to any suitably position of the pyridinyl ring. In general "substituted" refers to a substituent group as defined herein in which one or more bonds to a hydrogen atom contained therein are replaced by a bond to a non-hydrogen atom unless otherwise denoted. In particular the substituent groups are each independently selected from the group consisting of halogen, in particular F; $C_{1-4}$-alkyl, in particular methyl; OH; $C_{1-4}$-alkoxy, in particular methoxy; and CN.

"Optional" or "optionally" denotes that the subsequently described event or circumstance may but need not occur, and that the description includes instances where the event or circumstance occurs and instances in which it does not. "Comprises" or "comprising" denotes that the subsequently described set may but need not include other elements.

The expression "pharmaceutically acceptable" represents being useful in the preparation a pharmaceutical composition that is generally safe, non-toxic, and neither biologically nor otherwise undesirable, and includes being useful for both veterinary use as well as human pharmaceutical use.

The expression "acid addition salt" includes any non-toxic organic and inorganic acid addition salts that compounds of formula (I) can form. Illustrative inorganic acids, which form suitable salts, include, but are not limited to, hydrogen chloride, hydrogen bromide, sulphuric and phosphoric acids. Illustrative organic acids, which form suitable salts, include, but are not limited to, acetic acid, lactic acid, malonic acid, succinic acid, glutaric acid, fumaric acid, malic acid, tartaric acid, citric acid, ascorbic acid, maleic acid, benzoic acid, phenylacetic acid, cinnamic acid, methane sulfonic acid, salicylic acid, and the like. The term "acid addition salt" as used herein also comprises solvates which the compounds and salts thereof are able to form, such as, for example, hydrates, alcoholates, and the like. These salts also include salts useful for the chiral resolution of racemates.

The expression "base addition salt" includes any non-toxic base addition salts that the compound of formula (I) can form. Suitable base salts include, but are not limited to, those derived from inorganic bases such as aluminum, ammonium, calcium, copper, iron, lithium, magnesium, manganese, potassium, sodium, and zinc salts, in particular sodium and ammonium salts. Further examples of organic base addition salt include salts of trialkylamines, such as triethyl amine and trimethyl amine, and choline salts.

The present invention relates to novel compounds of formula (I)

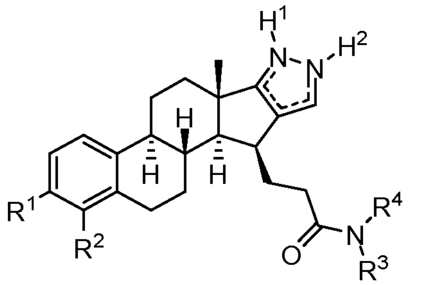

(I)

wherein

R1 and R2 are each independently selected from the group consisting of H and halogen;
(i) R3 is selected from the group consisting of H and C1-4 alkyl and
R4 is selected from the group consisting of

- phenyl, 6 membered unsaturated, or aromatic heterocycle comprising 1 to 3 heteroatom(s) independently selected from the group consisting of nitrogen, sulfur, and oxygen, and being optionally substituted with one to five substituent(s) independently selected from the group consisting of halogen, CN, C1-4 alkyl,

C1-3-(per)haloalkyl, OH, oxo, C1-3-alkoxy, morpholino, C(O)N(C1-3-alkyl)$_2$ and a 6 membered saturated heterocycle with 1 to 3 heteroatom(s) selected from the group consisting of nitrogen, oxygen and sulfur, optionally substituted with C1-4 alkyl; and

- 6 membered saturated heterocycle comprising 1 to 3 heteroatom(s) independently selected from the group consisting of nitrogen, oxygen and sulfur and being optionally substituted with one to three substituent(s) independently selected from the group consisting of halogen, CN, C1-4-alkyl, C1-3-(per)haloalkyl, OH, oxo, and C1-3-alkoxy, or two adjacent substituents may form a 5 or 6 membered saturated fused ring;

provided that only one of hydrogens $H^1$ and $H^2$ is present at the same time, and the position of the double bonds in the pyrazole ring to which the hydrogens $H^1$ and $H^2$ are attached is determined based on which of the hydrogen $H^1$ and $H^2$ is present
or a pharmaceutically acceptable salt thereof.

In one embodiment of the invention the pyrazole ring of the compound is in the form shown in formula (Ia),

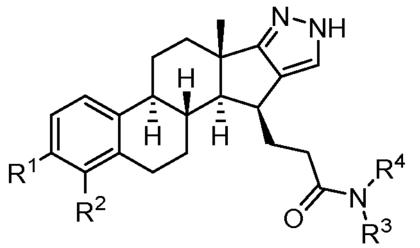

(Ia)

in which formula (Ia) R1, R2, R3 and R4 are as defined above.

In one embodiment of the invention substituent R1 is selected from the group consisting of H, F and Cl, substituent R2 is selected from the group consisting of H, F and Cl, and substituents R3 and R4 are as defined above.

In one embodiment of the invention substituent R1 is selected from the group consisting of H, F and Cl, substituent R2 is H or F, and substituents R3 and R4 are as defined above.

In one embodiment of the invention substituent R1 is H, substituent R2 is F and substituents R3 and R4 are as defined above.

In one embodiment of the invention substituent R3 is H and substituent R4 is a 6 membered unsaturated or aromatic heterocycle with 1 to 3 heteroatoms selected from the group consisting of nitrogen, oxygen and sulfur, optionally substituted with one or two substituent(s) selected from the group consisting of CN, C1-4 alkyl, C1-3-alkoxy, halogen and C(O)N(C1-3-alkyl)$_2$ or alternatively R4 is a substituent with formula:

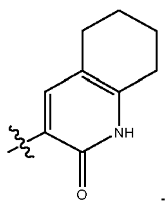

In one embodiment of the invention substituent R1 is selected from the group consisting of H, Cl or F, substituent R2 is selected from the group consisting of H, Cl or F, substituent R3 is H, and substituent R4 is a 6 membered aromatic heterocycle with 1 to 3 heteroatoms selected from the group consisting of nitrogen, oxygen and sulfur, optionally substituted with one or two substituent(s) selected from the group consisting of CN, C1-4 alkyl, C1-3-alkoxy, halogen and C(O)N(C1-3-alkyl)$_2$, or alternatively substituent R4 is a substituent with formula:

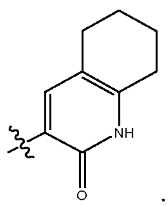

or a pharmaceutically acceptable salt thereof.

In one embodiment of the invention substituent R1 is H or F, substituent R2 is H or F, substituent R3 is H, and substituent R4 is a 6 membered aromatic heterocycle with 1 to 3 heteroatoms selected from the group consisting of nitrogen, oxygen and sulfur, optionally substituted with one or two substituent(s) selected from the group consisting of CN, C1-4 alkyl, C1-3-alkoxy, halogen and C(O)N(C1-3-alkyl)$_2$, or alternatively substituent R4 is a substituent with formula:

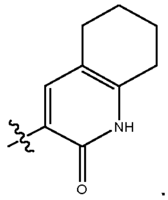

.

or a pharmaceutically acceptable salt thereof.

In one embodiment of the invention substituent R1 is H, substituent R2 is H or F, substituent R3 is H, substituent R4 is a 6 membered aromatic heterocycle with 1 to 3 heteroatoms selected from the group consisting of nitrogen, oxygen and sulfur, optionally substituted with one or two substituent(s) selected from the group consisting of CN, C1-4-alkyl, C1-3-alkoxy, halogen and C(O)N(C1-3-alkyl)$_2$, or a pharmaceutically acceptable salt thereof.

In one embodiment of the invention substituent R1 is H, substituent R2 is F, substituent R3 is H, substituent R4 is a 6 membered aromatic heterocycle with 1 to 3 heteroatoms selected from the group consisting of nitrogen, oxygen and sulfur, optionally substituted with one or two substituent(s) selected from the group consisting of CN, methyl, methoxy, F and C(O)N(methyl)$_2$, or alternatively substituent R4 is a substituent with formula:

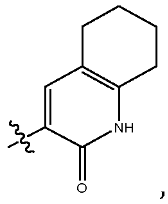

,

or a pharmaceutically acceptable salt thereof.

In one embodiment of the invention the compound of formula (I) is a compound selected from the list:

-3-((8a*S*,12*S*)-3-fluoro-8a-methyl-1,2,6b,7,8,8a,10,12,12a,12b-decahydronaphtho[2',1':4,5]indeno[1,2-c]pyrazol-12-yl)-*N*-(6-methoxypyridazin-3-yl)-propanamide,
-*N*-(5-Cyanopyridin-2-yl)-3-((8a*S*,12*S*)-3-fluoro-8a-methyl-1,2,6b,7,8,8a,10,12,12a,12b-decahydronaphtho[2',1':4,5]indeno[1,2-c]pyrazol-12-yl)propanamide,
-3-((8a*S*,12*S*)-3-fluoro-8a-methyl-1,2,6b,7,8,8a,10,12,12a,12b-decahydronaphtho[2',1':4,5]indeno[1,2-c]pyrazol-12-yl)-*N*-(4-fluoropyridin-2-yl)propanamide,
-3-((8a*S*,12*S*)-3-fluoro-8a-methyl-1,2,6b,7,8,8a,10,12,12a,12b-decahydronaphtho[2',1':4,5]indeno[1,2-c]pyrazol-12-yl)-*N*-(5-methoxypyridin-2-yl)propanamide,
-3-((8a*S*,12*S*)-3-fluoro-8a-methyl-1,2,6b,7,8,8a,10,12,12a,12b-decahydronaphtho [2',1':4,5]indeno [1,2-c]pyrazol-12-yl)-N-(4-methylpyridin-2-yl)propanamide,
-3-((8aS,12S)-3-fluoro-8a-methyl-1,2,6b,7,8,8a,10,12,12a,12b-decahydronaphtho [2',1':4,5]indeno [1,2-c]pyrazol-12-yl)-N-(2-oxo-1,2,5,6,7,8-hexahydroquinolin-3-yl)propanamide,
-6-(3-((8aS,12S)-3-fluoro-8a-methyl-1,2,6b,7,8,8a,10,12,12a,12b-decahydronaphtho[2',1':4,5]indeno[1,2-c]pyrazol-12-yl)propanamido)-N,N-dimethylnicotinamide,
-N-(6-methoxypyridazin-3-yl)-3-((8aS,12S)-8a-methyl-1,2,6b,7,8,8a,10, 12,12a, 12b-decahydronaphtho [2',1':4,5] indeno [1,2-c]pyrazol-12-yl)propanamide,
- N,N -dimethyl-6-(3-((8aS,12S)-8a-methyl-1,2,6b, 7,8,8a,10, 12,12a, 12b-decahydronaphtho[2',1':4,5]indeno[1,2-c]pyrazol-12-yl)propanamido)nicotinamide,
-N-(5-fluoropyridin-2-yl)-3-((8aS,12S)-8a-methyl-1,2,6b,7,8,8a,10,12,12a,12b-decahydronaphtho[2',1':4,5]indeno[1,2-c]pyrazol-12-yl)propanamide, and
-N-(4-fluoropyridin-2-yl)-3-((8aS,12S)-8a-methyl-1,2,6b,7,8,8a,10,12,12a,12b-decahydronaphtho[2',1':4,5]indeno[1,2-c]pyrazol-12-yl)propanamide,

or a pharmaceutically acceptable salt thereof.

The invention also relates to a method for the preparation of a compound according to formula (I) in which method a compound of formula (II) is reacted with hydrazine hydrate to form a compound according to formula (I).

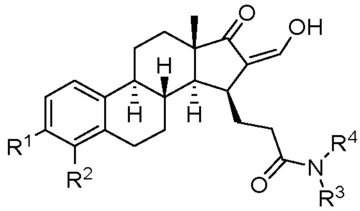

(II)

The method for preparing the compound according to the invention having formula (I) involves more specifically a method where the compound of formula (II) is dissolved in methanol (1.5 mL). Hydrazine hydrate (200 mol%) is added and stirred at +50°C under nitrogen 30 minutes. The solvent is evaporated. Evaporation residue is dissolved in ethyl acetate, washed trice with 1N hydrochloric acid. The aqueous layers are combined and then is washed with ethyl acetate, finally the aqueous layer is neutralized (pH ~ 8) and the product is extracted with ethyl acetate. The product is purified by chromatography or by crystallization. More details of the various methods of preparation for the compound according to the invention can be found in the Examples.

The current invention also relates to the intermediate according to formula (II).

Further the invention relates to compounds of formula (I) for use as a medicament. Specifically, the medicament can be for use in treatment or prevention of a disease selected from a group consisting of breast cancer, prostate carcinoma, ovarian cancer, uterine cancer, endometrial cancer, endometrial hyperplasia, endometriosis, uterine fibroids, adenomyosis, polycystic ovarian syndrome, dysmenorrhea, menorrhagia, metrorrhagia, contraception, prostadynia, benign prostatic hyperplasia, urinary dysfunction, lower urinary tract symptoms, chronic prostatitis/chronic pelvic pain syndrome (CP/CPPS), systemic lupus erythematosus (SLE), multiple sclerosis, obesity, rheumatoid arthritis, chronic obstructive pulmonary disease (COPD), lung cancer, colon cancer, tissue wounds, skin wrinkles and cataracts.

Still further the invention relates to a pharmaceutical composition comprising an effective amount of one or more compound(s) of formula (I), together with one or more pharmaceutically acceptable excipient(s). The pharmaceutical composition can also comprise one or more other active ingredients.

Representative examples of compounds of formula (I) are shown in Table 1, of the compounds in this table, compounds 24, 54, 57, 60, 63, 66, 72, 75, 78, 81, 102, 117, 144, 147, 174, 177, 180, 186, 192, 201, 210, 213, 216, 219, 222, 225, 234, 243, 246 and 249 are not according to the present invention and are included for the purposes of comparison.

**Table 1**

| | | |
|---|---|---|
| 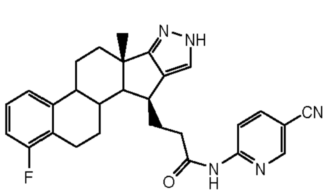 **3** | 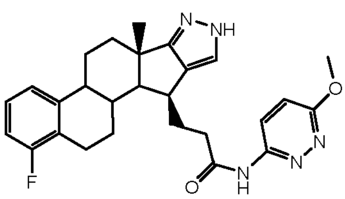 **6** | 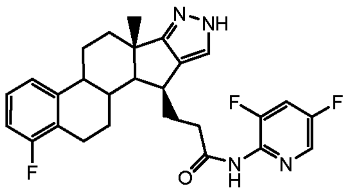 **9** |
| 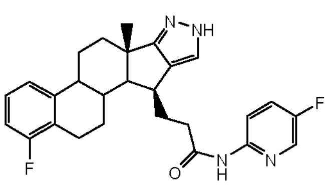 **12** | 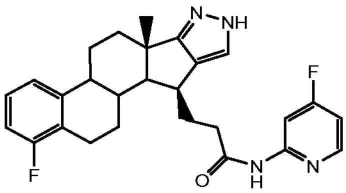 **15** | 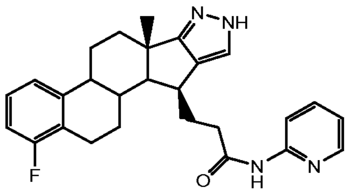 **18** |
| 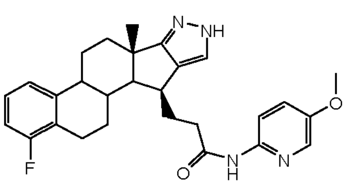 **21** | 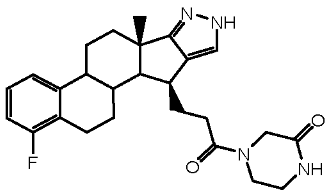 **24** | 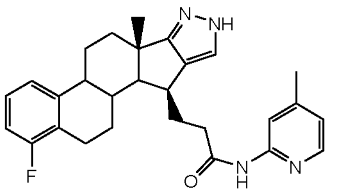 **27** |
| 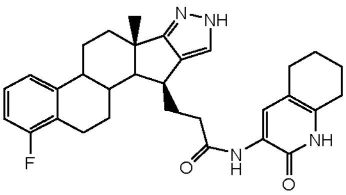 **30** | 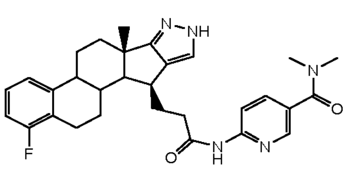 **33** | 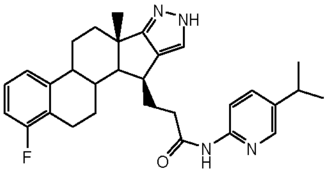 **36** |
| 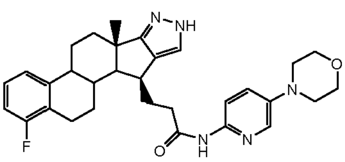 **39** | 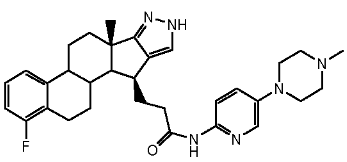 **42** | 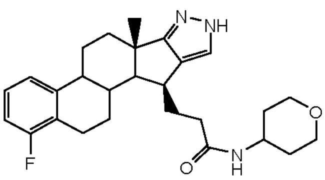 **45** |

| | | |
|---|---|---|
| 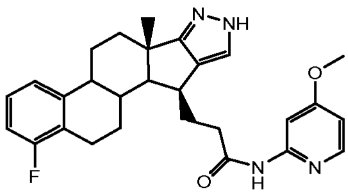 48 | 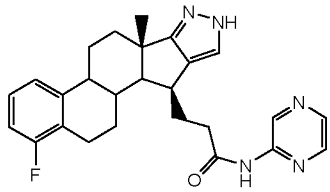 51 | 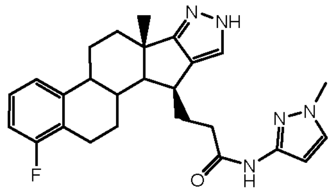 54 |
| 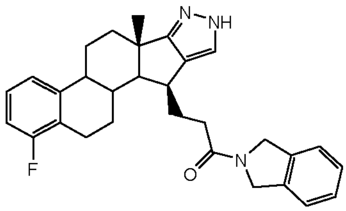 57 | 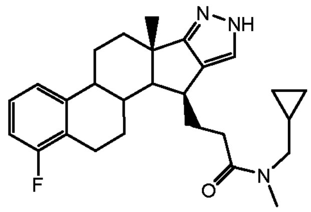 60 | 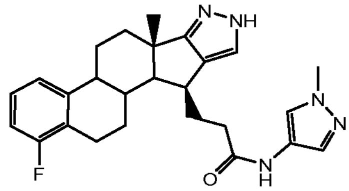 63 |
| 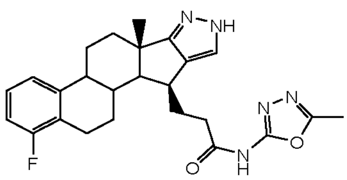 66 | 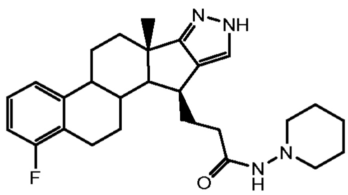 69 | 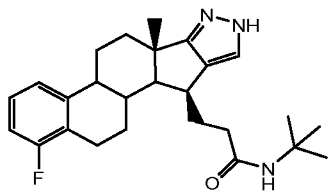 72 |
| 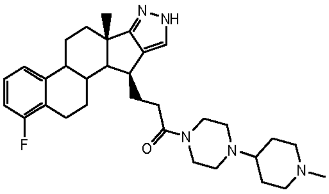 75 | 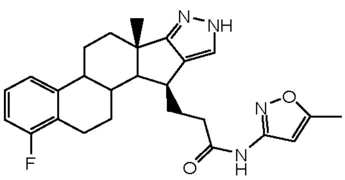 78 | 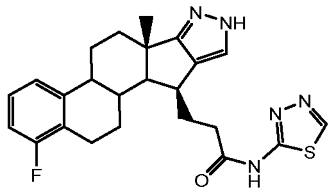 81 |
| 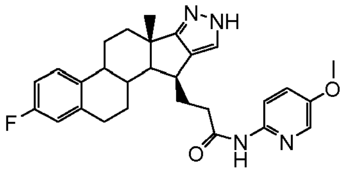 84 | 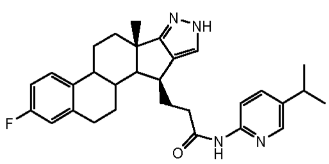 87 | 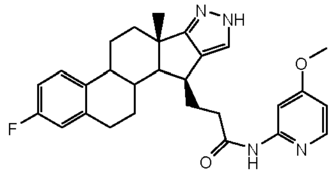 90 |

| | | |
|---|---|---|
| 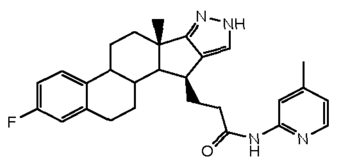<br>**93** | 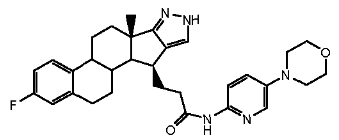<br>**96** | 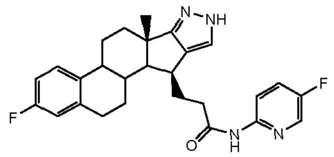<br>**99** |
| 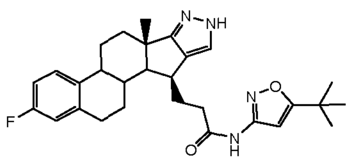<br>**102** | 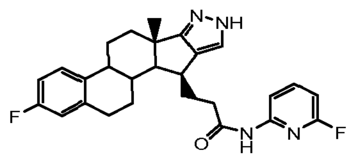<br>**105** | 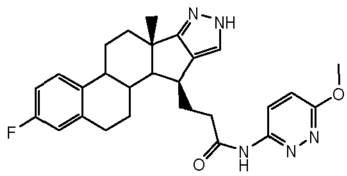<br>**108** |
| 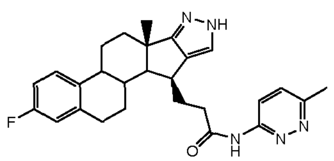<br>**111** | 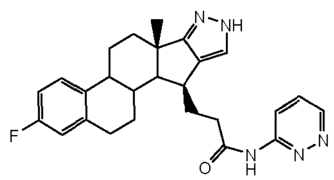<br>**114** | 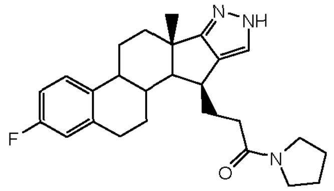<br>**117** |
| 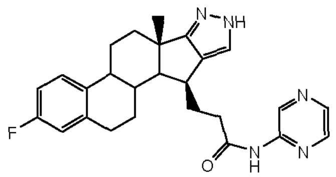<br>**120** | 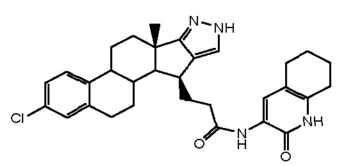<br>**123** | 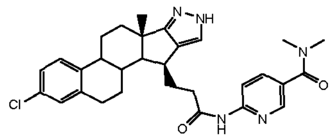<br>**126** |
| 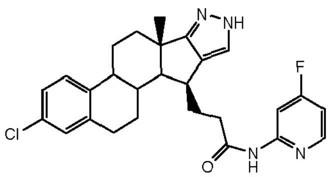<br>**129** | 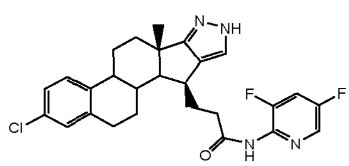<br>**132** | 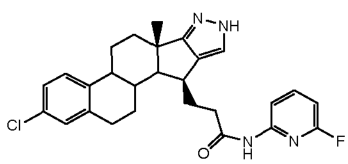<br>**135** |

| | | |
|---|---|---|
| 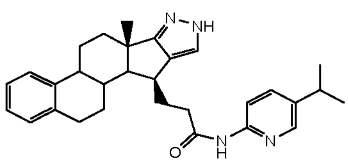 138 | 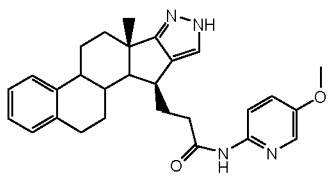 141 | 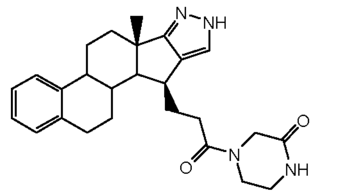 144 |
| 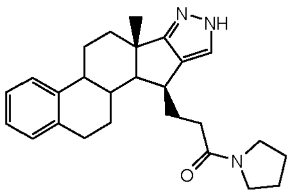 147 | 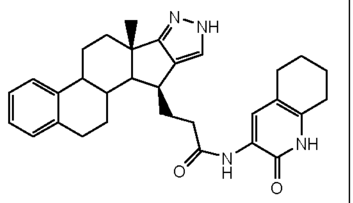 150 | 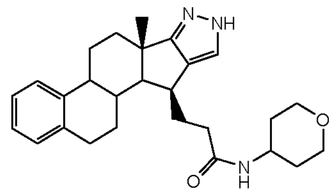 153 |
| 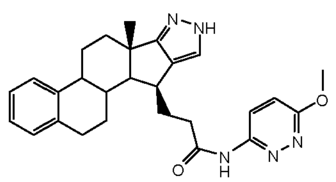 156 | 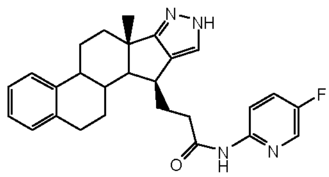 159 | 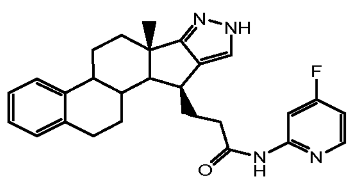 162 |
| 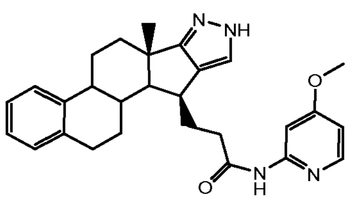 165 | 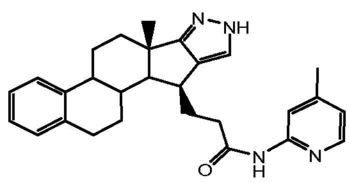 168 | 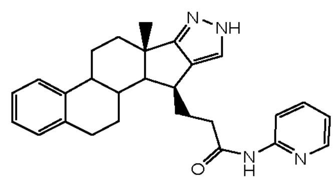 171 |
| 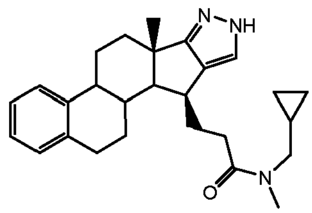 174 | 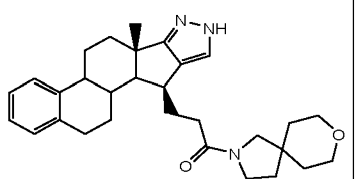 177 | 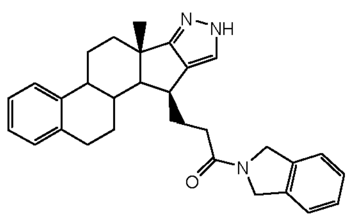 180 |

| | | |
|---|---|---|
| 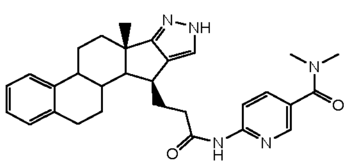 183 | 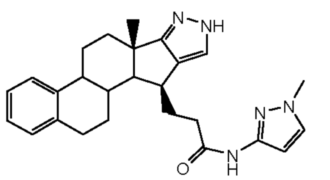 186 | 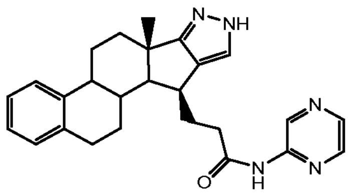 189 |
| 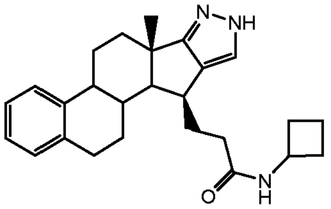 192 | 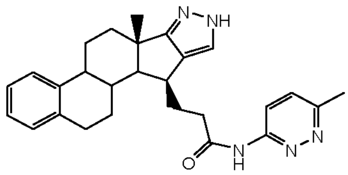 195 | 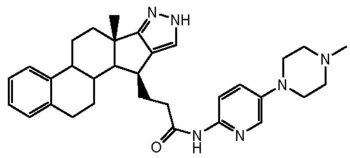 198 |
| 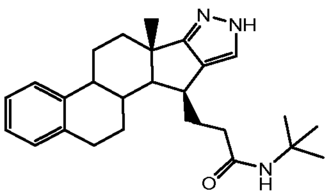 201 | 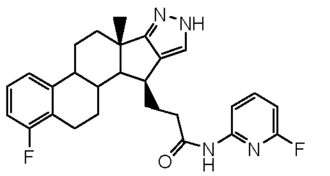 204 | 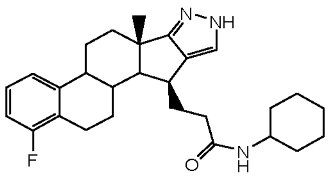 207 |
| 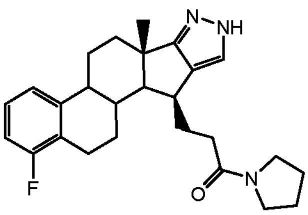 210 | 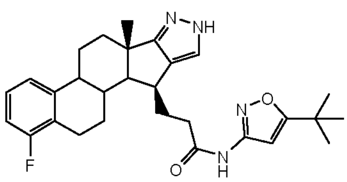 213 | 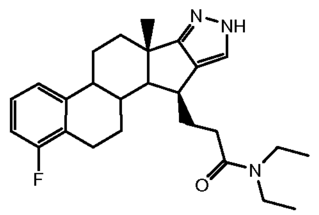 216 |
| 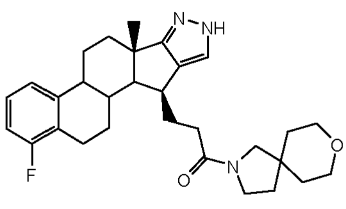 219 | 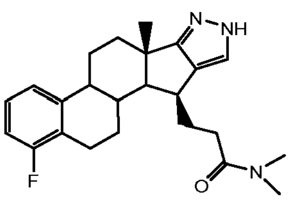 222 | 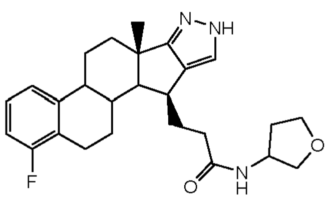 225 |

| | | |
|---|---|---|
| 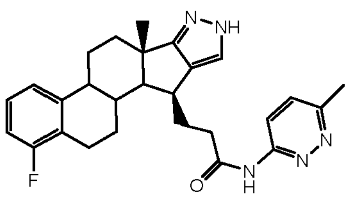 **228** | 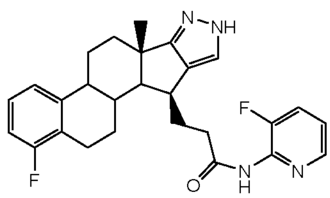 **231** | 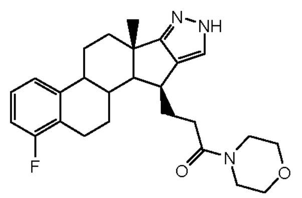 **234** |
| 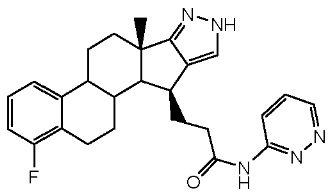 **237** | 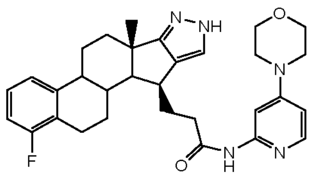 **240** | 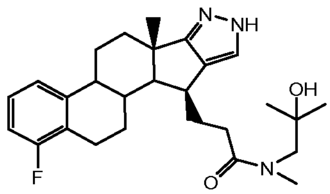 **243** |
| 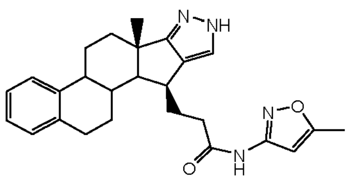 **246** | 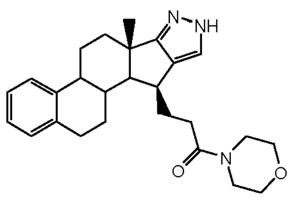 **249** | 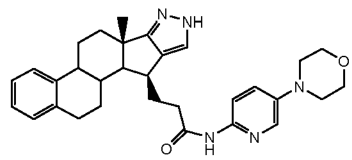 **252** |
| 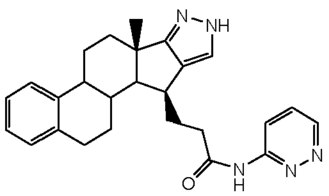 **255** | 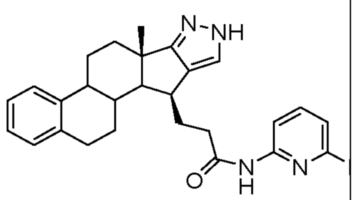 **258** | 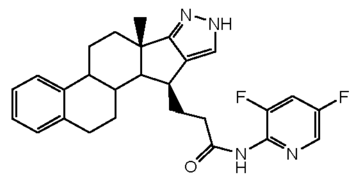 **261** |
| 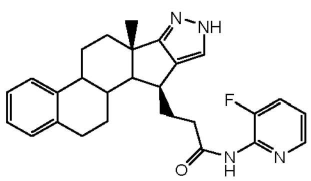 **264** | 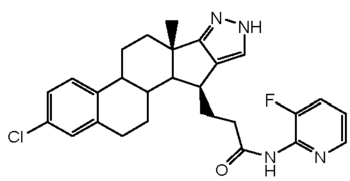 **267** | |

EXAMPLES OF THE INVENTION

In the following examples, compounds 2, 3, 5, 6, 8, 9, 11, 12, 14, 15, 17, 18, 20, 21, 26, 27, 29, 30, 32, 33, 35, 36, 38, 39, 41, 42, 44, 45, 47, 48, 50, 51, 68, 69, 83, 84, 86, 87, 89, 90, 92, 93, 95, 96, 98, 99, 104, 105, 107, 108, 110, 111, 113, 114, 119, 120, 122, 123, 125, 126, 128, 129, 131, 132, 134, 135, 137, 138, 140, 141, 149, 150, 152, 153, 155, 156, 158, 159, 161, 162, 164, 165, 167. 168, 170, 171, 182, 183, 188, 189, 194, 195, 197, 198, 203, 204, 206, 207, 227, 228, 230, 231, 236, 237, 239, 240, 251, 252, 254, 255, 257, 258, 260, 261, 263, 264, 266 and 267 are compounds according to the present invention. Other compounds of the following examples are not part of the present invention

because these are either unclaimed intermediates for the preparation of the compounds of the invention (in particular 17-ketone compounds) or are compounds not falling within the scope of formula (I) of claim 1 or of formula (II) of claim 13, because the N-substituent corresponding to group R4 is not in line with the definition of this group in claim 1 as well as 17-ketone intermediates for the preparation of such compounds.

**Preparation of synthesis starting materials and precursors**

**Preparation of the starting material Acid IX**

Compound **SM-IX** was synthesized from Estrone (Scheme 1.). Methods of Horwitz et al (J. Med. Chem., 1986, 29 (5), 692-698) yielded amine **SM-III** which was fluorinated using conditions of Labrie et al. WO2008124922. The fluoride **SM-IV** was converted to enone **SM-VI** by silylation/oxidation method of Kobayashi et (Tetrahedron, 71(35), 5918-5924; 2015). The allylation, hydroboration and oxidation of **SM-VI** to **SM-IX** was performed as in patents WO2005/047303 and WO2006/125800.

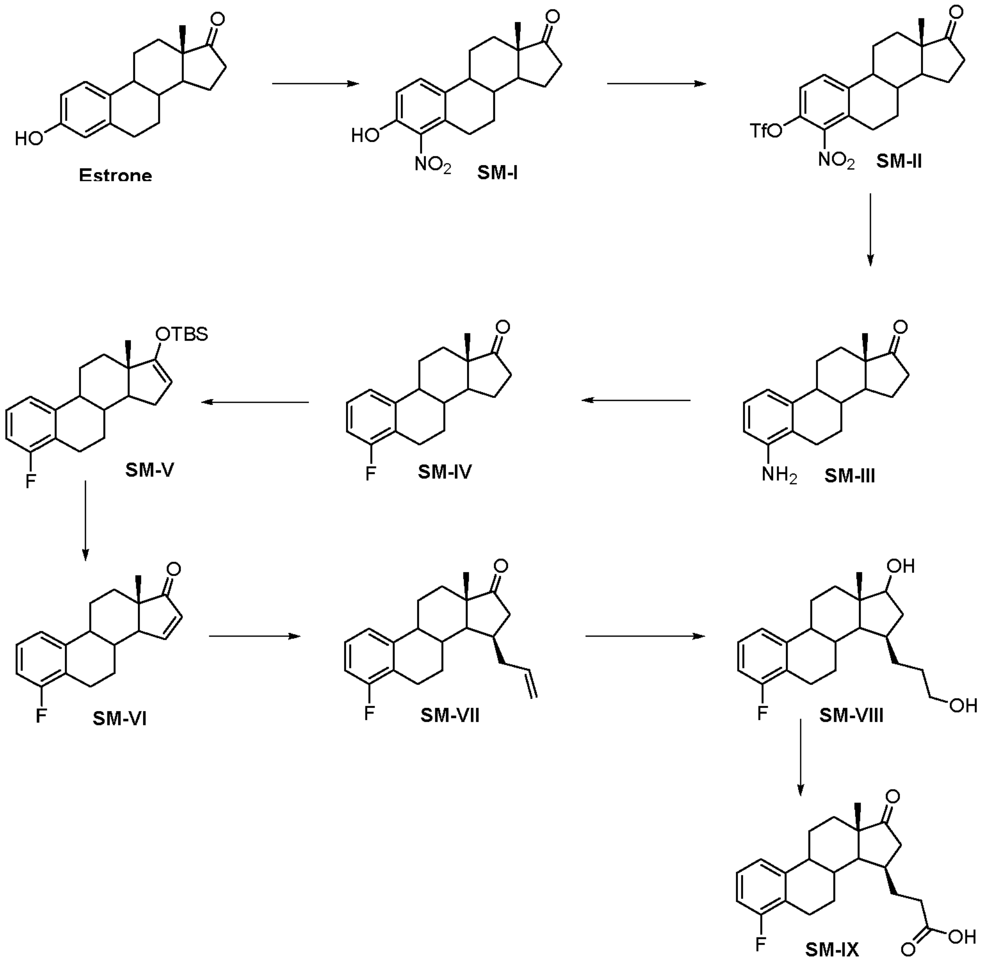
Estrone
SM-I
SM-II
SM-III
SM-IV
SM-V
SM-VI
SM-VII
SM-VIII
SM-IX

## Compound SM-IV:

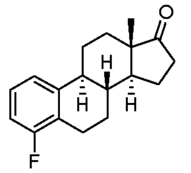

A solution of **Compound SM-III** (11.00 g, 40.8 mmol, 100 mol-%) in dichloromethane (430 mL) was added to neat boron trifluoride diethyl etherate (7.9 mL, 64.20 mmol, 157 mol-%) while stirring at -15°C under nitrogen (approx 10-15 mins addition time). The reaction mixture was stirred for 15 min. at -15°C before a solution of *tert*-butyl nitrite (5.9

mL, 49.80 mmol, 122 mol-%) in dichloromethane (50 mL) was added to it dropwise over a period of 10 min. The reaction mixture was stirred for another 15 min. at -15°C, and afterwards at 0-5°C for 30 min.

The solution was added to n-pentane (2.25 L) on order to give a beige precipitate. The liquors were decanted and the residue was washed with more n-pentane (400 mL). The beige solid (12.00 g) was dried *in vacuo* at room temperature overnight.

The crude material was purified by flash column chromatography using n-hexanes and ethyl acetate (10-30%) as solvent system. The yield of **Compound SM-IV** as a cream solid was 70% (7.82 g).

$^1$H NMR (400 MHz, $CDCl_3$) δ ppm 0.91 (s, 3H, $-CH_3$), 1.34-1.70 (m, 6H), 1.93-1.99 (m, 1H), 2.04-2.21 (m, 3H), 2.27-2.46 (m, 2H), 2.48-2.56 (m, 1H), 2.66-2.77 (m, 1H), 2.95-3.03 (m, 1H), 6.84-6.90 (m, 1H, -ArH), 7.06-7.16 (m, 2H, 2x-ArH).

## Compound SM-V:

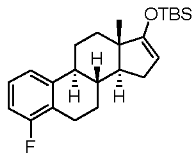

*tert*-Butyldimethylsilyl triflate (7.1 mL, 31.10 mmol, 110 mol-%) was added dropwise, over a period of 20 min., to a stirred solution of **Compound SM-IV** (7.70 g, 28.27 mmol, 100 mol-%) and triethylamine (6.0 mL, 42.72 mmol, 151 mol-%) in dichloromethane (75 mL) at room temperature under nitrogen and stirred for 2h.

The reaction mixture was diluted with dichloromethane (95 mL) and it was washed with a saturated aqueous solution of sodium bicarbonate (2x70 mL) and brine (70 mL). The organic layer was dried over sodium sulfate, filtered and concentrated. The yield of **Compound SM-V** as a cream solid was quantitative (11.42 g) and used in the next reaction without further purification.

$^1$H NMR (400 MHz, $CDCl_3$) δ ppm 0.14-0.19 (m, 6H, $2x-CH_3$), 0.86 (s, 3H, $-CH_3$), 0.94 (s, 9H, $3x-CH_3$), 1.21-1.62 (m, 5H), 1.78-2.06 (m, 3H), 2.08-2.16 (m, 1H), 2.25-2.38 (m, 2H), 2.64-2.88 (m, 1H), 2.90-2.99 (m, 1H), 4.48 (dd, 1H, J= 3.1, 1.5 Hz), 6.82-6.88 (m, 1H, -ArH), 7.05-7.13 (m, 2H, 2x-ArH).

## Compound SM-VI:

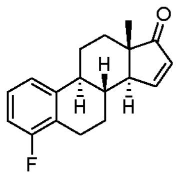

A mixture of **Compound SM-V** (11.42 g, 28.27 mmol, 100 mol-%) and palladium acetate (0.63 g, 2.83 mmol, 10 mol-%) in dimethylsulfoxide (75 mL) and dichloromethane (50 mL) was stirred at 35°C under an oxygen atmosphere (balloon) for 16h, palladium acetate (126 mg, 0.56 mmol, 2 mol-%) was added to the mixture and it was stirred for another 7h at 35°C.

The reaction mixture was cooled to room temperature and it was poured into a saturated aqueous solution of sodium bicarbonate (300 mL). The mixture was extracted with ethyl acetate (400 mL). The organic layer was washed with water (300 mL) and brine (200 mL) and dried over sodium sulfate, filtered and concentrated to afford an orange/brown solid.

The crude material was purified by flash column chromatography using n-hexanes and ethyl acetate (0-30%) as solvent system. The yield of **Compound SM-VI** as a pinkish/white solid was 72% (5.50 g).

$^1$H NMR (400 MHz, $CDCl_3$) δ ppm 1.11 (s, 3H, $-CH_3$), 1.46-1.58 (m, 1H), 1.66-1.88 (m, 3H), 1.97-2.07 (m, 1H), 2.23-2.31 (m, 1H), 2.35-2.54 (m, 3H), 2.72-2.84 (m, 1H), 3.03 (dd, 1H, J=17.9, 6.4 Hz), 6.11 (dd, 1H, J=6.0, 3.2 Hz), 6.83-6.92 (m, 1H, -ArH), 7.05-7.18 (m, 2H, 2x-ArH), 7.63-7.66 (m, 1H).

MS m/z (ES$^+$): 271 (M + H).

## Compound SM-VII:

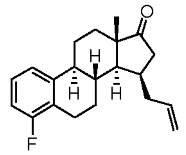

A dry three-neck flask was charged under a nitrogen atmosphere with copper iodide (7.90 g, 41.48 mmol, 350 mol-%), lithium chloride (1.76 g, 41.48 mmol, 350 mol-%) and anhydrous tetrahydrofuran (60 mL). The mixture was stirred for 20 min. at room temperature and it was cooled to -70°C. Allyl magnesium bromide (41.5 mL, 41.48 mmol, 350 mol-%) was then added dropwise, keeping the temperature under -70°C. Chlorotrimethylsilane (5.3 mL, 41.48 mmol, 350 mol-%) was added dropwise to the reaction mixture, keeping the temperature at -70°C, followed by the addition of a solution of **Compound SM-VI** (3.20 g, 11.85 mmol, 350 mol-%) in anhydrous tetrahydrofuran (60 mL), which was added dropwise keeping the temperature bellow -65°C. The reaction mixture was allowed to warm slowly to room temperature and stirred overnight.

The mixture was poured into a saturated aqueous solution of ammonium chloride (75 mL) and extracted with ethyl acetate (3 x 70 mL). The combined extracts were washed with 1M HCl (2x50 mL), water (2 x 50 mL) and diluted aqueous ammonia solution (5 x 25 mL) (until the solution was colourless). The organic layer was dried over sodium sulfate, filtered and concentrated. The crude material was purified by flash column chromatography using n-hexanes and ethyl acetate (10%) as solvent system. The yield of **Compound SM-VII** was 77% (2.85 g).

$^1H$ NMR (400 MHz, $CDCl_3$) δ ppm 1.05 (s, 3H, $-CH_3$), 1.40-1.57 (m, 3H), 1.71-1.82 (m, 2H), 1.89-1.96 (m, 1H), 2.04-2.20 (m, 2H), 2.31-2.50 (m, 6H), 2.72-2.84 (m, 1H), 2.94-3.03 (m, 1H), 5.02-5.08 (m, 2H, $CH=CH_2$), 5.69-5.81 (m, 1H, $C\underline{H}=CH_2$), 6.88 (t, 1H, ArH, J=8.7Hz), 7.05-7.16 (m, 2H, 2xArH).

## Compound SM-VIII:

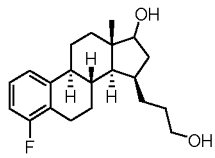

A dry, nitrogen flushed, flask was charged with **Compound SM-VII** (2.85 g, 9.13 mmol, 100 mol-%) and anhydrous tetrahydrofuran (70 mL). A 1 M solution of borane THF complex (18.3 mL, 18.30 mmol, 200 mol-%) was added dropwise to the previous solution. The resulting reaction mixture was refluxed for 1h, cooled in an ice-bath to -5°C and a 3M aqueous solution of sodium hydroxide (28 mL) was added to it very cautiously. After the addition was complete and the effervescence ceased, hydrogen peroxide 30% (28 mL) was added and the mixture was gently refluxed for 2h.

The reaction mixture was cooled to room temperature and was extracted with ethyl acetate (3 x 70 mL). The combined extracts were washed with water (2 x 50 mL) and brine (50 mL), dried over sodium sulfate, filtered and concentrated. The yield of **Compound SM-VIII** was quantitative (3.09 g).

$^1H$ NMR (400 MHz, $CDCl_3$) δ ppm 0.82 (s, 3H, $-CH_3$), 1.13-1.64 (m, 9H), 1.81-1.88 (m, 1H), 1.91-2.06 (m, 2H), 2.16-2.27 (m, 2H), 2.30-2.39 (m, 1H), 2.63-2.74 (m, 1H), 2.81-2.89 (m, 1H), 3.54-3.69 (m, 3H), 6.76-6.82 (m, 1H, -ArH), 6.98-7.08 (m, 2H, 2x-ArH).

**Acid SM-IX: [3-((13*S*,15*R*)-4-fluoro-13-methyl-17-oxo-7,8,9,11,12,13,14,15,16, 17-decahydro-6H-cyclopenta[a] phenanthren-15-yl)propanoic acid]**

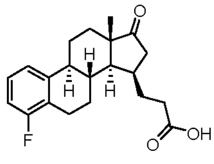

Periodic acid (5.15 g, 22.60 mmol, 500 mol-%) and chromium trioxide (23 mg, 0.23 mmol, 5.0 mol-%) were dissolved in a mixture of acetonitrile (36 mL) and water (12 mL). The solution was cooled to 0°C in an ice/salt bath. A

slurry of **Compound SM-VIII** (1.5 g, 4.52 mmol, 100 mol-%) in acetonitrile (30 mL) was added to the previous solution over a period of 40 min. maintaining the temperature at or below 0°C. The reaction mixture was stirred for 1h at 0°C, then the mixture was slowly warmed to room temperature and stirred for 3.5 h.

The reaction mixture was poured into aqueous sodium phosphate dibasic (~5 g in 100 mL) and extracted with ethyl acetate (3 x 60 mL). The organic extracts were combined and washed with a 5% aqueous solution of sodium bisulfite (2 x 40 mL), water (50 mL) and brine (50 mL), dried over sodium sulfate, filtered and concentrated. The crude material was purified by flash column chromatography using n-hexanes, ethyl acetate (10-30%) and acetic acid (1%) as solvent system. The product was dissolved in toluene (50 mL) and stirred for 15 min. Solvent was removed *in vacuo* and the solid was dried under vacuum at 50°C. The crude yield of **Acid SM-IX** as a white solid was 71% (1.11 g).

$^1$H NMR (400 MHz, $CDCl_3$) δ ppm 0.99 (s, 3H, $-CH_3$), 1.31-1.53 (m, 3H), 1.55-1.78 (m, 3H), 1.83-2.00 (m, 2H), 2.09-2.17 (m, 1H), 2.23-2.47 (m, 7H), 2.68-2.80 (m, 1H), 2.88-2.97 (m, 1H), 6.81 (t, 1H, -ArH, J=8.6 Hz), 6.98-7.10 (m, 2H, 2x-ArH).

MS m/z (ES-): 343 (M - H).

**Preparation of the starting material Acid SM-XV**

C-3 Fluoro SM-**XV** was synthesized from Estrone (Scheme 2.) via the Compound SM-**X**, which may be synthesized as disclosed in Messinger et al. Mol Cell Endocrinol. 2009 (301) 216-224. The detailed synthesis of compound **X** starting from estrone has been described in WO2008065100, WO2005/047303 and WO2006/125800. The acid **SM-X** was methylated by heating in methanol in the presence of sulphuric acid followed by triflation. Bistributyltin derivative **SM-XIII** was prepared from the corresponding triflate **SM-XII** followed by fluorination to XIV in 75% yield, (ref. WO 2010059943 and Furuya et al., JACS 2009, 13 (15),1662). Several estrone deoxyfluorination methods are available (Labrie, Fernand et al. PCT Int. Appl., 9946279, 16 Sep 1999; Labrie, Fernand et al. PCT Int. AppL, 2004089971, 21 Oct 2004).

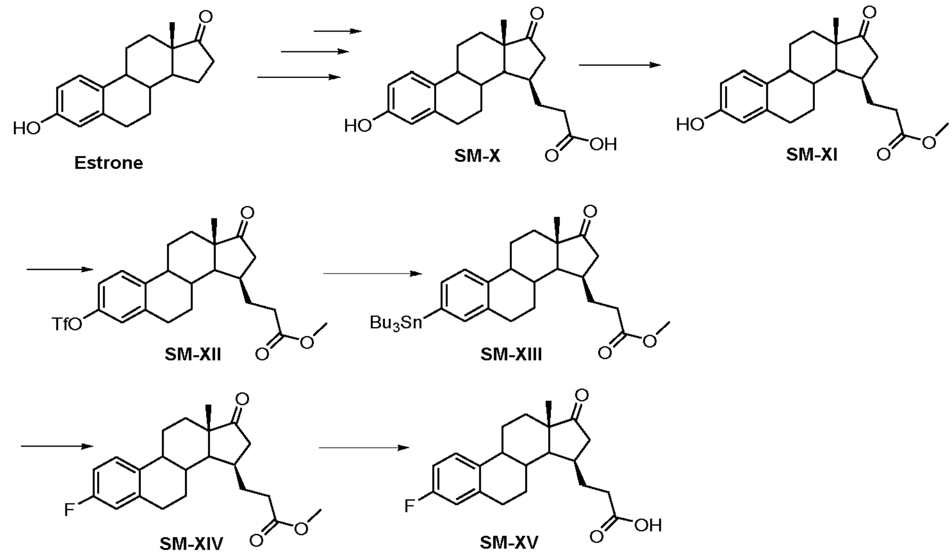
Estrone
SM-X
SM-XI
SM-XII
SM-XIII
SM-XIV
SM-XV

## Compound XIII:

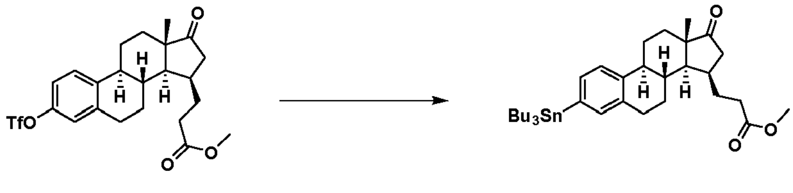

To a screw-cap sealed tube was added **Compound SM-XII** (10.0 g, 20.47 mmol, 100 mol-%) and 1,4- dioxane (120 mL). Bistributyltin (230.7 mL, 40.99 mmol, 200 mol-%) and LiCl (4.2 g, 102.3 mmol, 500 mol-%) were added to reaction mixture. The reaction mixture was degassed with argon gas for 10 min then added $Pd(PPh_3)4$ (1.41 g, 1.22 mmol, 6 mol-%) to it. The tube was sealed under nitrogen and the mixture was stirred and heated at 100°C in a preheated oil bath for 4 hours. The mixture was cooled to room temperature and quenched with water (100 mL), extracted with

ethyl acetate (2x 200 mL), then filtered through celite, washing well with ethyl acetate. The solvents were concentrated to brown viscous oil. The crude product was purified by flash chromatography eluting with a gradient of 0 to 10% ethyl acetate in hexanes to give the **Compound SM-XIII.**

$^1H$ NMR (400 MHz, CDCl3) δ ppm: 7.29-7.19 (m, 3H), 3.69 (s, 3H), 2.95 (bs, 2H), 2.42-0.87 (m, 46H). MS m/z (ES+): poor ionization.

**Compound SM-XIV:**

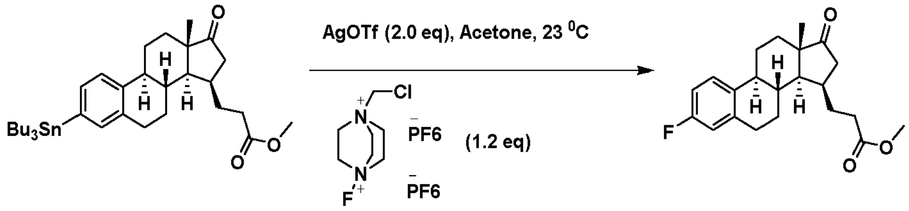


To a stirred solution of **Compound SM-XIII** (14.0 g, 22.2 mmol, 1.0 eq) in acetone (140 mL) was added AgOTf (11.41 g, 44.4 mmol, 2.0 eq) at room temperature. The reaction mixture was cooled to 0°C and added 1-Chloromethyl-4-fluoro- 1,4-diazoniabicyclo [2.2.2] octane bis(hexafluorophosphate) (12.53 g, 26.6 mmol, 1.2 eq) and the reaction mixture was stirred for 40 min. The reaction was quenched with water (100 mL) and extracted with ethyl acetate (2 x 150 mL). The organic layer was dried over sodium sulphate, filtered and concentrated. The crude compound was purified by flash chromatography eluted with 0-20% ethyl acetate in hexane. **Compound SM-XIV** (6.0 g, 75.9%) was afforded as a white solid.

$^1H$ NMR (400 MHz, DMSO-d6) δ ppm: 7.30-7.27 (m,1H), 7.10-7.08 (d, 1H, J=8 Hz), 6.94-6.89 (m, 1H), 3.59 (s, 3H), 2.87 (bs, 2H), 2.45-2.07 (m, 8H), 1.86-1.32 (m, 8 H), 0.95 (s, 3H). MS m/z (ES+): poor ionization.

**Acid SM-XV:**

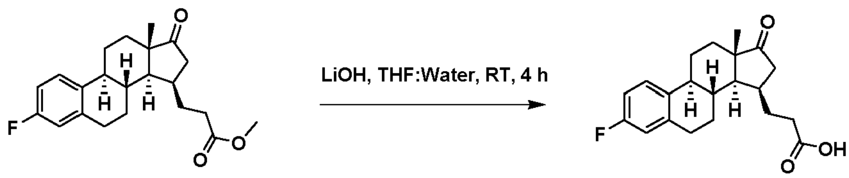


To a stirred solution of compound **SM-XIV** (6.0 g, 16.7 mmol, 1.0 eq) in THF (60 mL), water (10.5 mL) and was added LiOH.HzO (1.41 g, 33.5, 2.0 eq) and stirred for 4 h at RT. The reaction mixture was cooled to 10°C, and neutralized with 1 N HCl (pH= 6) and extracted with ethyl acetate (2 x 50 mL). The organic layer was dried over sodium sulphate, filtered and concentrated. The crude compound was triturated with n-pentane (2 x 10 mL) followed by prep HPLC purification to afford **Acid SM-XV** (2.2 g, 38.19%) as a white solid.

$^1H$ NMR (400 MHz, DMSO-d6) δ ppm: 12.06 (s, 1H), 7.29-7.27 (d, 1H, J=8 Hz), 7.16-7.14 (d, 2H, J=8 Hz), 2.87 (bs, 2H), 2.37-2.12 (m, 8H), 1.82-1.67 (m, 4H), 1.55-1.38 (m, 4H), 0.84 (s, 3H). MS m/z (ES+): 343.23 (M - H).

**Acid SM-XVII**

The Triflate **SM-XII** in scheme 3 was prepared followed by methods of Messinger et al, WO2008065100. **SM-XII** was converted to chloro derivative SM-**XVI** by using *t*-BuBrettPhos in the presence of tris(dibenzylidene-acetone)dipalladium(0) (Pan et al., Organic Letters, 13(18), 4974-4976; 2011) followed by LiOH treatment in THF:water affording the desired **Acid SM-XVII**.

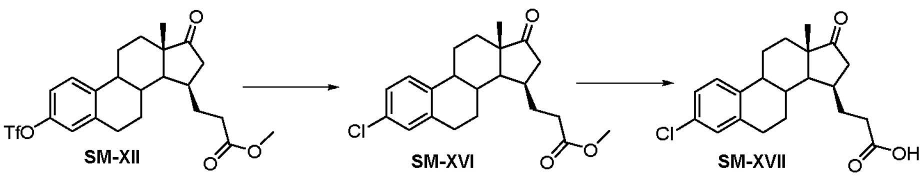

SM-XII SM-XVI SM-XVII

## Compound SM-XVI:

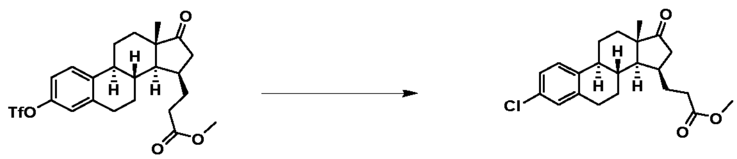

To a screw-cap sealed tube was added tris(dibenzylideneacetone)dipalladium(0) (0.084 g, 0.092 mmol, 3 mol-%) and t-BuBrettPhos (0.133 g, 0.27 mmol, 9 mol-%) and 1,4-dioxane (10 mL) and the tube was sealed under nitrogen. The mixture was stirred and heated at 130°C in a preheated oil bath for 3 minutes. The catalyst mixture was cooled to room temperature and this mixture was added to a solution of the **Compound SM-XII** (1.5 g, 3.04 mmol, 100 mol-%) in 1,4-dioxane (11 mL), potassium chloride (0.908 g, 12.28 mmol, 400 mol-%) and potassium fluoride (0.178 g, 3.0 mmol, 100 mol-%). The mixture was stirred and heated at 130°C in a preheated oil bath for 3 hours. The mixture was cooled to room temperature and then filtered through celite, washing with ethyl acetate. The solvents were concentrated to leave brown viscous oil. The crude product was purified by flash chromatography eluting with a gradient of 0 to 20% the **SM-Compound XVI.**

$^1$H NMR (400 MHz, DMSO-d6) δ ppm: 7.29-7.27 (d, 1H, J=8 Hz), 7.16-7.14 (d, 2H, J=8 Hz), 3.59 (s, 3H), 2.87 (bs, 2H), 2.41-2.07 (m, 8H), 1.85-1.38 (m, 8 H), 0.95 (s, 3H). MS m/z (ES+): poor ionization.

**Acid SM-XVII:**

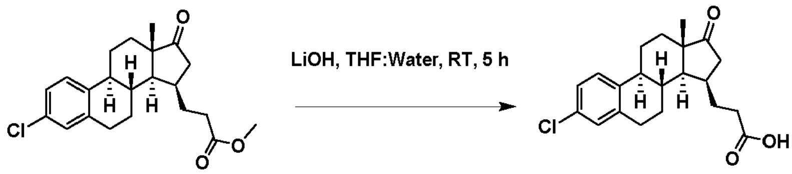


To a stirred solution of **Compound SM-XVI** (1.7 g, 4.54 mmol, 1.0 eq) in THF:MeOH:Water (12.5 mL, 2:2:1) and was added LiOH.HzO (0.572 g, 13.6, 3.0 eq) at RT. The reaction mixture was heated at 80°C for 1.5 h. The reaction progress was monitored by TLC and LC-MS. The reaction mixture was cooled to RT, diluted with water 10 mL and washed with ethyl acetate 3 x 3 mL. The aqueous layer was neutralized with 1 N HCl (pH= 6) and extracted with ethyl acetate (2 x 50 mL). The organic layer was dried over sodium sulphate, filtered and concentrated. The crude product was triturated with n-pentane (2x 10 mL) to afford **Acid SM-XVII** (1.3 g, 79%) as a white solid.

$^1$H NMR (400 MHz, DMSO-d6) δ ppm: 12.06 (s, 1H), 7.29-7.27 (d, 1H, J=8 Hz), 7.16-7.14 (d, 2H, J=8 Hz), 2.87 (bs, 2H), 2.37-2.12 (m, 8H), 1.82-1.67 (m, 4H), 1.55-1.38 (m, 4H), 0.84 (s, 3H). MS m/z (ES+): 358.9 (M - H).

**Preparation of the starting material Acid SM-XXVI:**

Compound **SM-XXVI** was synthesized from Estrone via the triflate **SM-XVIII,** which was prepared by methods of Messinger et al, WO2008065100. The C15-C16 **SM-XXIII** was prepared according to methods described in WO2008065100. The allylation, hydroboration and oxidation of **SM-XXIII** to **SM-XXVI** was performed as in patents WO2005/047303 and WO2006/125800.

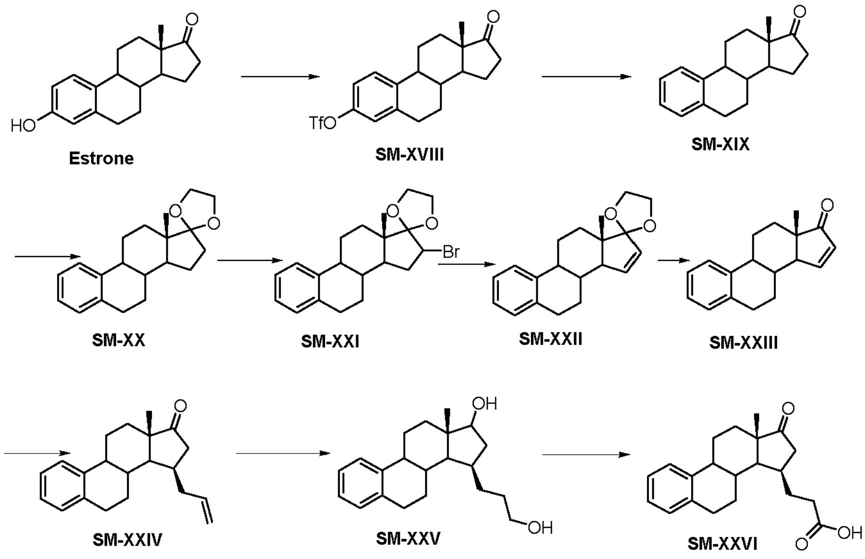

Estrone SM-XVIII SM-XIX

SM-XX SM-XXI SM-XXII SM-XXIII

SM-XXIV SM-XXV SM-XXVI

**Acid SM-XXVI:**

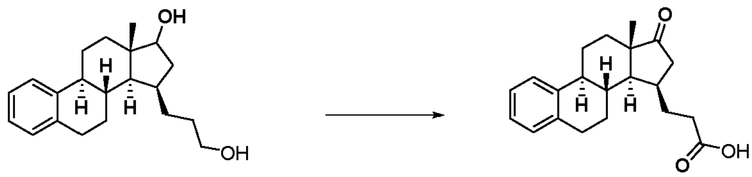

A stirred solution of (8R,9S,13S,14S,15R)-15-(3-hydroxypropyl)-13-methyl-7,8,9,11,12,13,14,15,16,17-decahydro-6H-cyclopenta[a]phenanthren-17-ol (44.0 g 0.140 mol) in acetone (875 mL) was cooled to 0°C. In another RBF, Jones reagent was prepared by dissolving the chromic acid (35 g, 0.350 mol) in water (350 mL) and con. Sulphuric acid (41.14 g, 0.420 mol). The Jones reagent prepared was added in 45 minutes to the starting material solution maintaining the temperature at 0-2°C for 2-3 h. The reaction mass was quenched using ice cold water (875 mL), the sticky material was filtered and dissolved in 3N NaOH solution (200 mL). The mixture was extracted with ethyl acetate (3 x 200 mL). The aqueous layer was neutralized with aqueous 2N HCl (pH=6) and extracted with ethyl acetate (3x 200 ml). The combined organic layer was washed with brine (200 mL), dried over anhydrous sodium sulphate and solvent was evaporated to obtain solid **Acid SM-XXVI** 3-((8R,9S,13S,14S,15R)-13-methyl-17-oxo-7,8,9,11,12,13,14,15,16,17-decahydro-6H-cyclopenta[a]phenanthren-15-yl)propanoic acid (24g, 52%) as a white solid.

$^1$H NMR (400 MHz, DMSO-d6) $\delta$ ppm: 12.0 (s, 1H), 7.27-7.25 (d, 1H, J=8 Hz), 7.13-7.05 (m, 3H,), 2.87 (bs, 2H), 2.41-2.10 (m, 8H), 1.87-1.36 (m, 8H), 0.95 (s, 3H).

MS m/z (ES+): 325.23 (M - H).

**General information**

Commercial grade reagents and solvents were used without further purification. Thin-layer chromatography (TLC) was performed on Merck-plates; precoated aluminium sheets. Visualization of plates was done the following techniques: 1) ultraviolet illumination (254 nm), 2) dipping the plate into anisaldehyde or vanilline solution followed by heating. 1H-NMR spectra were measured with a Bruker DPX (200 MHz) or Avance III 400 (400 MHz) spectrometer with the solvent as indicated.

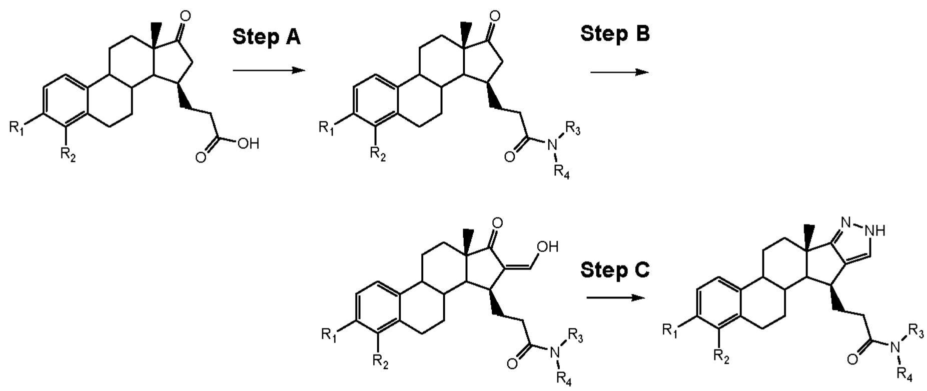


**Synthesis methods for Step A:**

**Method A1:** General procedure for amide preparation by the T3P-method

The acid (100 mg, 100 mol%) was dissolved in dry THF (3 mL). The corresponding amine (200 mol%) and pyridine (300 mol%) were added. T3P (200 mol%) was added dropwise to the reaction mixture. Stirred at room temperature or at +50°C until the reaction was completed. The evaporation residue was dissolved in EtOAc and 10% $NaHCO_3$ was carefully added. The aqueous layer was extracted with ethyl acetate. The organic layers were combined, washed with dilute HCl, water and brine, and dried with sodium sulphate. The crude product was generally purified by chromatography.

**Method A2:** General procedure for amide preparation by the EDCI-method

The acid (150 mg, 100 mol%) was dissolved in dry DMF or DCM (4 mL). HOBt (220 mol%) and EDCI (220 mol%) and the amine (200 mol%) were added to the reaction mixture and stirring was continued at +50°C until the reaction was completed. Water (4 ml) was added to the reaction mixture when the product precipitates by water addition, followed by washing with water several times.

**Method A3:** Modified procedure for amide preparation by the EDCI-method

The acid (200 mg, 100 mol%) was dissolved in dry dichloromethane (4 mL). The amine (150 mol%), *N*-methylmorpholine (300 mol%) and 1-hydroxy-1H-benzotriazole (220 mol-%) was added to the reaction mixture. After stirring for 5 minutes, the reaction mixture was cooled to 0-5°C. EDCI (220 mol%) was added to the reaction mixture. Stirred at room temperature until the reaction was completed. The reaction mixture was diluted with DCM (~ 5 ml), washed with 0.5 N HCl solution (2 x 10 ml), water (3 x 10 ml) and brine (2 x 10 ml). The organic layer was dried over sodium sulfate. The crude product was purified if needed.

**Synthesis method for Step B:**

**Method B:** General procedure for preparation of the hydroxymethylenes by the ethylformate/NaH -method

The steroidal C-17 carbonyl intermediate containing suitable amide unit at the C-15 position (95 mg, 100 mol%) was co-evaporated with toluene (3 x 10 mL), then dissolved in dry THF (400 μl). To the reaction mixture was added under nitrogen dry toluene (1000 μl), ethyl formate (600 mol%) and NaH (450 mol%) and then stirred at room temperature until the reaction was completed. The solvent was evaporated, and the residue was dissolved in EtOAc and washed with dilute hydrochloric acid, water and brine, and dried with sodium sulfate.

**Synthesis method for Step C:**

**Method C:** General procedure for pyrazole preparation by the hydrazine hydrate - method

The hydroxymethylene derivative (90 - 100 mg, 100 mol%) was dissolved in methanol (1.5 mL). Hydrazine hydrate (200 mol%) was added and stirred at +50°C under nitrogen 30 minutes. The solvent was evaporated. Evaporation residue was dissolved in ethyl acetate, washed trice with 1N hydrochloric acid. The aqueous layers were combined and

then was washed with ethyl acetate, finally the aqueous layer was neutralized (pH -8) and the product was extracted with ethyl acetate. The product was purified by chromatography or by crystallization or trituration.

**Compound 1**

*N*-(5-Cyanopyridin-2-yl)-3-((13*S*,15*R*)-4-fluoro-13-methyl-17-oxo-7,8,9,11,12,13,14,15,16,17-decahydro-6*H*-cyclopenta[*a*]phenanthren-15-yl)propanamide

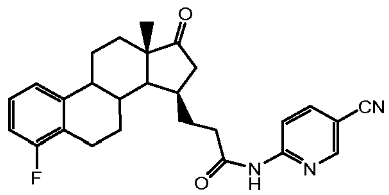

The compound **1** was prepared Method A1 from Acid **SM-IX** and 5-cyano-2-aminopyridine by stirring overnight at room temperature. The yield was 83%. $^1$H-NMR (400 MHz, DMSO-$d_6$): 0.98 (s, 3H), 1.33-2.48 (m, 15H), 2.57 (m, 1H), 2.68-2.90 (m, 2H), 6.97 (dd, 1H), 7.14 (m, 2H), 8.25 (s, 2H), 8.78 (s, 1H), 11.04 (s, 1H).

**Compound 2**

*N*-(5-Cyanopyridin-2-yl)-3-((13*S*,15*S*,*Z*)-4-fluoro-16-(hydroxymethylene)-13-methyl-17-oxo-7,8,9,11,12,13,14,15,16,17-decahydro-6*H*-cyclopenta[*a*]phenanthren-15-yl)propanamide

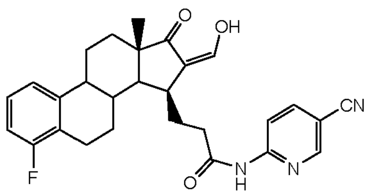

The compound **2** was prepared from the compound **1** by Method B stirring overnight at room temperature in 51% yield.

$^1$H NMR (400 MHz, DMSO-$d_6$): 1.00 (s, 3H), 1.38-2.37 (m, 12H), 2.64 (m, 2H), 2.74-2.96 (m, 3H), 6.96 (dd, 1H), 7.14 (m, 2H), 7.57 (s, 1H), 8.23 (m, 2H), 8.76 (s, 1H), 11.03 (s, 1H).

**Compound 3**

*N*-(5-Cyanopyridin-2-yl)-3-((8a*S*,12*S*)-3-fluoro-8a-methyl-1,2,6b, 7,8,8a, 1 0, 12, 12a, 12b-decahydronaphtho [2',1':4,5] indeno [1,2-c] pyrazol-12-yl)propanamide

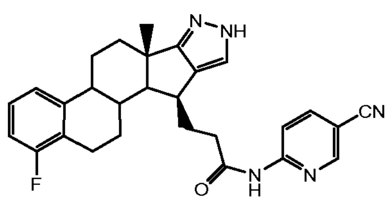

The compound **3** was prepared in 80% yield from the compound **2** by the Method C and purified by chroma-

tography.

$^1H$ NMR (400 MHz, DMSO-$d_6$): 1.11(s, 3H), 1.43-2.45 (m, 11H), 2.56 (m, 2H), 2.76-2.93 (m, 3H), 6.97 (dd, 1H), 7.16 (m, 2H), 7.39 (s, 1H), 8.25 (m, 2H), 8.79 (d, 1H), 11.08 (s, 1H), 12.13 (br s, 1H).

**Compound 4**

3-((13*S*,15*R*)-4-fluoro-13-methyl-17-oxo-7,8,9,11,12,13,14,15,16,17-decahydro-6*H*-cyclopenta[*a*]phenanthren-15-yl)-*N*-(6-methoxypyridazin-3-yl)propanamide

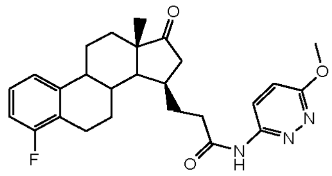

The compound **4** was prepared Method A2 from Acid **SM-IX** and 3-amino-6-methoxypyridazine stirring four hours at room temperature. The yield was 95%.

$^1H$-NMR (200 MHz, DMSO-$d_6$): 0.98 (s, 3H), 1.20-2.47 (m, 16H), 2.60-2.97 (m, 2H), 3.98 (s, 3H), 6.89-7.06 (m, 1H), 7.08-7.21 (m, 2H), 7.25 (d,1H), 8.26 (d, 1H), 10.94 (br s, 1H).

**Compound 5**

3-((13*S*,15*S*,*Z*)-4-fluoro-16-(hydroxymethylene)-13-methyl-17-oxo-7,8,9,11,12,13,14,15,16,17-decahydro-6*H*-cyclopenta[*a*]phenanthren-15-yl)-*N*-(6-methoxypyridazin-3-yl) propanamide

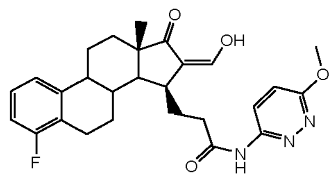

The compound **5** was prepared from the compound **4** by Method B in 89% yield. $^1H$ NMR (400 MHz, DMSO-$d_6$): 1.00 (s, 3H), 1.38-2.37 (m, 11H), 2.68-2.98 (m, 5H), 3.98 (s, 3H), 6.97 (dd, 1H), 7.14 (m, 2H), 7.23 (d, 1H), 7.55 (s, 1H), 8.24 (d, 2H), 10.87 (s, 1H).

**Compound 6**

3-((8a*S*,12*S*)-3-fluoro-8a-methyl-1,2,6b,7,8,8a,10,12,12a,12b-decahydronaphtho[2',1':4,5]indeno[1,2-c]pyrazol-12-yl)-*N*-(6-methoxypyridazin-3-yl)propanamide

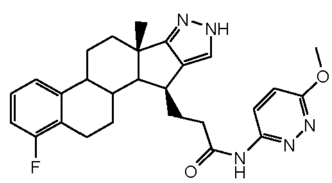

The compound **6** was prepared in 55% yield from compound **5** by Method C purifying the crude product by trituration with heptane-ethanol 1:1 mixture.

$^1H$ NMR (400 MHz, DMSO-$d_6$): 1.11(s, 3H), 1.41-2.44 (m, 11H), 2.53-2.92 (m, 5H), 3.99 (s, 3H), 6.97 (dd, 1H),

7.15 (m, 2H), 7.24 (d, 1H), 7.42 (s, 1H), 8.26 (d, 1H), 10.97 (s, 1H), 12.13 (br s, 1H).

**Compound 7**

*N*-(3,5-difluoropyridin-2-yl)-3-((13S,15R)-4-fluoro-13-methyl-17-oxo-7,8,9,11,12,13,14,15,16,17-decahydro-6*H*-cyclopenta[*a*]phenanthren-15-yl)propanamide

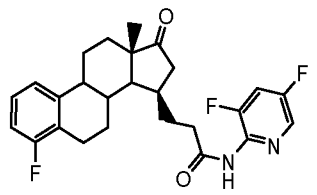

The compound **7** was prepared by Method A1 from Acid **SM-IX** using 2-amino-3,5-difluoropyridine as an amine stirring two hours at room temperature. The yield was 91%.

$^1$H-NMR (400 MHz, DMSO-$d_6$): 0.98 (s, 3H), 1.34-2.55 (m, 16H), 2.69-2.90 (m, 2H), 6.97 (dd, 1H), 7.14 (m, 2H), 8.01 (dd, 1H), 8.34 (d, 1H), 10.31 (s, 1H).

**Compound 8**

*N*-(3,5-difluoropyridin-2-yl)-3-((13S,15S,Z)-4-fluoro-16-(hydroxylmethylene)-13-methyl-17-oxo-7,8,9,11,12,13,14,15,16,17-decahydro-6*H*-cyclopenta[*a*]phenanthren-15-yl)propanamide

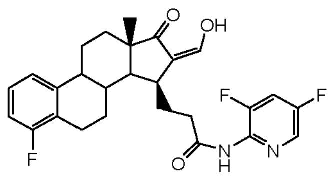

The compound **8** was prepared from the compound **7** by Method B stirring three hours at room temperature in 96% yield.

$^1$H NMR (400 MHz, DMSO-$d_6$): 0.99 (s, 3H), 1.39-2.45 (m, 12H), 2.67-2.96 (m, 5H), 6.97 (dd, 1H), 7.14 (m, 2H), 7.57 (s, 1H), 8.00 (m, 1H), 8.33 (d, 1H), 10.31 (s, 1H).

**Compound 9**

*N*-(3,5-difluoropyridin-2-yl)-3-((8a*S*,12*S*)-3-fluoro-8a-methyl-1,2,6b, 7,8,8a, 1 0, 12, 12a, 12b-decahydronaphtho [2',1':4,5] indeno [1,2-c] pyrazol-12-yl)propanamide

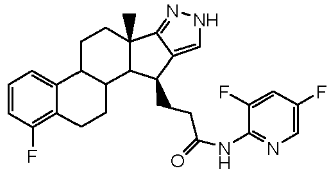

The compound **9** was prepared from the compound **8** by Method C in 84% yield. $^1$H NMR (400 MHz, DMSO-$d_6$): 1.11 (s, 3H), 1.37-2.48 (m, 13H), 2.71-2.92 (m, 3H), 6.97 (dd, 1H), 7.16 (m, 2H), 7.44 (s, 1H), 8.00 (m, 1H), 8.34 (d, 1H), 10.36 (s, 1H), 12.14 (br s, 1H).

**Compound 10**

3-((13*S*,15*R*)-4-fluoro-13-methyl-17-oxo-7,8,9,11,12,13,14,15,16,17-decahydro-6*H*-cyclopenta[a]phenanthren-15-yl)-N-(5-fluoropyridin-2-yl)propanamide

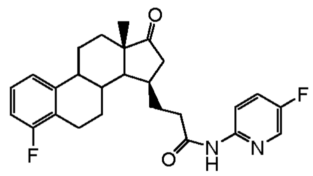

The compound **10** was prepared in 99% yield by Method A1 from Acid **SM-IX** using 2-amino-5-fluoropyridine as an amine stirring 3 hours at room temperature.

$^1$H NMR (200 MHz, DMSO-$d_6$): 0.98 (s, 3 H), 1.24 - 2.46 (m, 16 H), 2.59 - 3.03 (m, 2 H), 6.90 - 7.05 (m, 1 H), 7.06 - 7.22 (m, 2 H), 7.73 (td, 1 H), 8.15 (dd, 1 H), 8.32 (d, 1 H), 10.63 (s, 1 H). MS m/z (TOF ES$^+$): 439 (M+1)

**Compound 11**

3-((13*S*,15*S*,*Z*)-4-fluoro-16-(hydroxymethylene)-13-methyl-17-oxo-7,8,9,11,12,13,14,15,16,17-decahydro-6*H*-cyclopenta[*a*]phenanthren-15-yl)-*N*-(5-fluoropyridin-2-yl)propanamide

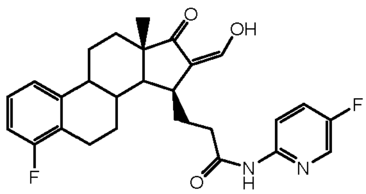

The compound **11** was prepared from the compound **10** by Method B in 99% yield. $^1$H NMR (400 MHz, DMSO-$d_6$): 0.99 (s, 3H), 1.39-2.37 (m, 11H), 2.58 (m, 2H), 2.70-2.97 (m, 3H), 6.96 (dd, 1H), 7.14 (m, 2H), 7.54 (s, 1H), 7.71 (m, 1H), 8.14 (dd, 1H), 8.30 (d, 1H), 10.55 (s, 1H), 10.99 (br s, 1H).

**Compound 12**

3-((8a*S*,12*S*)-3-fluoro-8a-methyl-1,2,6b,7,8,8a,10,12,12a,12b-decahydronaph-tho[2',1':4,5]indeno[1,2-c]pyrazol-12-yl)-*N*-(5-fluoropyridin-2-yl)propanamide

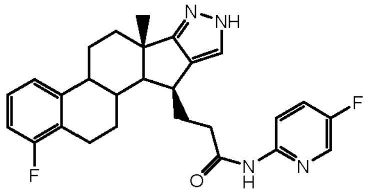

The compound **12** was prepared from the compound **11** by Method C in 90% yield. $^1$H NMR (400 MHz, DMSO-$d_6$): 1.11(s, 3H), 1.48-2.42 (m, 13H), 2.72-2.93 (m, 3H), 6.97 (dd, 1H), 7.16 (m, 2H), 7.40 (s, 1H), 7.72 (m, 1H), 8.15 (m, 1H), 8.31 (d, 1H), 10.66 (s, 1H), 12.15 (br s, 1H).

**Compound 13**

3-((13*S*,15*R*)-4-fluoro-13-methyl-17-oxo-7,8,9,11,12,13,14,15,16,17-decahydro-6*H*-cyclopenta[*a*]phenanthren-15-yl)-*N*-(4-fluoropyridin-2-yl)propanamide

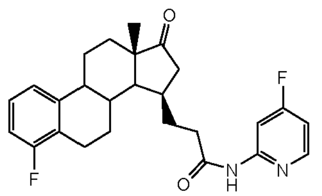

The compound **13** was synthesized in 83% yield by the Method A1 in THF by using acid **SM-IX** and 2-amino-4-fluoropyridine as starting materials in overnight reaction time.

$^{1}$H-NMR (400 MHz, DMSO-$d_6$): 0.98 (s, 3H), 1.36-1.46 (m, 3H), 1.58-1.74 (m, 4H), 1.89-1.94 (m, 1H), 2.16-2.43 (m, 7H), 2.68-2.91 (m, 3H), 6.95-7.04 (m, 2H), 7.05-7.20 (m, 2H), 7.93 (dd, 1H), 8.34 (dd, 1H), 10.83 (s, 1H).

**Compound 14**

3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)-N-(4-fluoropyridin-2-yl)propanamide

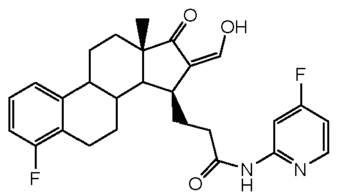

The compound **14** was prepared from the compound **13** by the Method B in 44% yield.

$^{1}$H NMR (400 MHz, DMSO-$d_6$): 1.00 (s, 3H), 1.32-2.30 (m, 12H), 2.55-2.96 (m, 5H), 6.96 (dd, 1H), 7.02 (m, 1H), 7.14 (m, 2H), 7.56 (s, 1H), 7.92 (dd, 1H), 8.34 (m, 1H), 10.80 (s, 1H).

**Compound 15**

3-((8a*S*,12*S*)-3-fluoro-8a-methyl-1,2,6b,7,8,8a,10,12,12a,12b-decahydronaphtho[2',1':4,5]indeno[1,2-c]pyrazol-12-yl)-*N*-(4-fluoropyridin-2-yl)propanamide

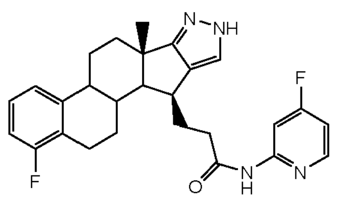

The compound **15** was prepared from the compound **14** by Method C in 57% yield.

$^{1}$H NMR (400 MHz, DMSO-$d_6$): 1.11(s, 3H), 1.40-2.42 (m, 13H), 2.76-2.93 (m, 3H), 6.97 (dd, 1H), 7.03 (m, 1H), 7.16 (m, 2H), 7.40 (s, 1H), 7.93 (dd, 1H), 8.36 (dd, 1H), 10.87 (s, 1H), 12.16 (br s, 1H).

**Compound 16**

3-((13*S*,15*R*)-4-fluoro-13-methyl-17-oxo-7,8,9,11,12,13,14,15,16,17-decahydro-6*H*-cyclopenta[*a*]phenanthren-15-yl)-*N*-(pyridin-2-yl)propanamide

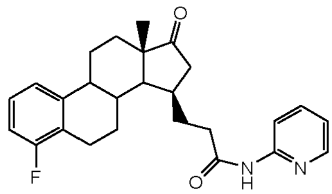

The compound **16** was synthesized in 51% yield by the Method A2 in DMF by using acid **SM-IX** and 2-aminopyridine as starting materials in overnight reaction time. $^1$H-NMR (400 MHz, DMSO-$d_6$): 0.98 (s, 3H), 1.34-1.47 (m, 3H), 1.59-1.68 (m, 4H), 1.78-1.90 (m, 1H), 2.17- 2.46 (m, 8H), 2.68-2.82 (m, 2H), 6.95-6.99 (m, 1H), 7.07-7.13 (m, 1H), 7.14-7.20 (m, 2H), 7.76 (dd, 1H), 8.10 (d, 1H), 8.30 (dd, 1H), 10.50 (s, 1H).

**Compound 17**

3-((13*S*,15*S*,*Z*)-4-fluoro-16-(hydroxymethylene)-13-methyl-17-oxo-7,8,9,11,12,13,14,15,16,17-decahydro-6*H*-cyclopenta[a]phenanthren-15-yl)-N-(pyridin-2-yl)propanamide

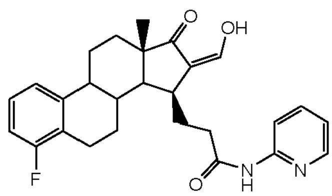

The compound **17** was prepared from the compound **16** by the Method B in 97% yield.

$^1$H NMR (400 MHz, DMSO-*$d_6$*): 0.99 (s, 3H), 1.35-2.23 (m, 12H), 2.68-2.98 (m, 5H), 6.96 (dd, 1H), 7.05 (m, 1H), 7.14 (m, 2H), 7.70 (s, 1H), 7.75 (dd, 1H), 8.09 (d, 1H), 8.30 (m, 1H), 10.70 (s, 1H).

**Compound 18**

3-((8a*S*,12*S*)-3-fluoro-8a-methyl-1,2,6b,7,8,8a,10,12,12a,12b-decahydronaphtho[2',1':4,5]indeno[1,2-c]pyrazol-12-yl)-*N*-(pyridin-2-yl)propanamide

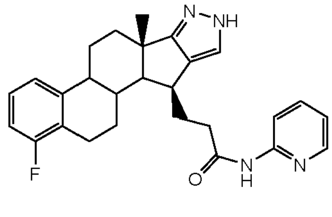

The compound **18** was prepared from the compound **17** by the Method C in 91% yield.

$^1$H NMR (400 MHz, DMSO-*$d_6$*): 1.11(s, 3H), 1.40-2.45 (m, 13H), 2.67-2.99 (m, 3H), 6.97 (dd, 1H), 7.07 (m, 1H), 7.16 (m, 2H), 7.41 (s, 1H), 7.76 (dd, 1H), 8.10 (d, 1H), 8.30 (d, 1H), 10.54 (s, 1H), 12.13 (br s, 1H).

**Compound 19**

3-((13*S*,15*R*)-4-fluoro-13-methyl-17-oxo-7,8,9,11,12,13,14,15,16,17-decahydro-6*H*-cyclopenta[*a*]phenanthren-15-yl)-*N*-(5-methoxypyridin-2-yl)propanamide

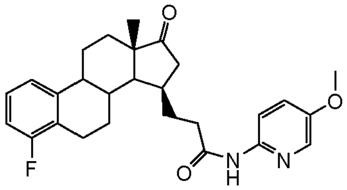

The compound **19** was synthesized in 80% yield by the Method A2 in DMF stirring at + 50°C for two hours by using acid **SM-IX** and 5-methoxy-2-aminopyridine as starting materials in overnight reaction time.

$^1$H-NMR (200 MHz, $CDCl_3$): 1.07 (s, 3H), 1.35-2.53 (m, 16H), 2.72-3.03 (m, 2H), 3.85 (s, 3H), 6.83-6.92 (m, 1H), 7.05-7.18 (m, 2H), 7.24-7.30 (m, 1H), 7.92-8.01 (m, 2H), 8.15 (d, 1H).

**Compound 20**

3-((13*S*,15*S,Z*)-4-fluoro-16-(hydroxymethylene)-13-methyl-17-oxo-7,8,9,11,12,13,14,15,16,17-decahydro-6*H*-cyclopenta[*a*]phenanthren-15-yl)-*N*-(5-methoxypyridin-2-yl)propanamide

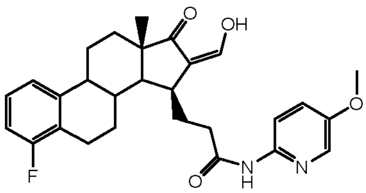

The compound **20** was prepared from the compound **19** by the Method B in quantitative yield.

$^1$H NMR (400 MHz, DMSO-$d_6$): 0.99 (s, 3H), 1.41-2.35 (m, 12H), 2.68-2.96 (m, 4H), 3.80 (s, 3H), 6.96 (m, 1H), 7.14 (m, 2H), 7.40 (d, 1H), 7.55 (s, 1H), 8.01 (s, 2H), 10.32 (s, 1H), 11.04 (br s, 1H).

**Compound 21**

3-((8a*S*,12*S*)-3-fluoro-8a-methyl-1,2,6b,7,8,8a,10,12,12a,12b-decahydronaphtho[2',1':4,5]indeno[1,2-c]pyrazol-12-yl)-*N*-(5-methoxypyridin-2-yl)propanamide

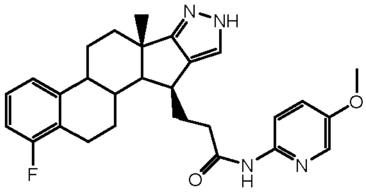

The compound **21** was prepared from the compound **20** by the Method C in 57% yield.

$^1$H NMR (400 MHz, DMSO-$d_6$): 1.10 (s, 3H), 1.40-2.47 (m, 13H), 2.70-2.99 (m, 3H), 3.80 (s, 3H), 6.97 (m, 1H), 7.16 (m, 2H), 7.41(s, 2H), 8.03 (m, 2H), 10.41 (s, 1H), 12.12 (br s, 1H).

**Compound 22**

4-(3-((13*S*,15R*)*-4-fluoro-13-methyl-17-oxo-7,8,9,11,12,13,14,15,16,17-decahydro-6*H*-cyclopenta[*a*]phenanthren-15-yl)propanoyl)piperazin-2-one

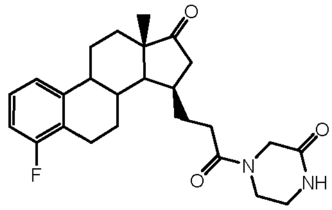

The compound **22** was synthesized in 81% yield by the Method A1 in THF using acid **SM-IX** and piperazin-2-one as starting materials in three hours reaction time. $^1$H-NMR (400 MHz, DMSO-$d_6$): 0.97 (s, 3H), 1.37-2.45 (m, 17H), 2.68-2.90 (m, 2H), 3.57-3.64 (m, 4H), 3.93 (dd, 1H), 6.97 (m, 1H), 7.13-7.20 (m, 2H), 8.09 (d, 1H).

**Compound 23**

4-(3-((13*S*,15*S*,*Z*)-4-fluoro-16-(hydroxymethylene)-13-methyl-17-oxo-7,8,9,11,12,13,14,15,16,17-decahydro-6*H*-cyclopenta[*a*]phenanthren-15-yl)propanoyl)piperazin-2-one

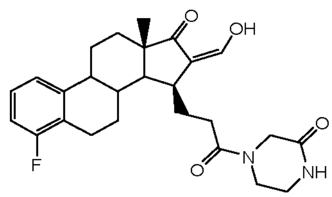

The compound **23** was prepared from the compound **22** by the Method B in 71% yield.

$^1$H NMR (400 MHz, DMSO-$d_6$): 0.97 (s, 3H), 1.35-2.37 (m, 14H), 2.67-2.92 (m, 3H), 3.16-3.25 (m, 3H), 3.58-3.67 (m, 2H), 3.93 (d, 1H), 6.97 (dd, 1H), 7.14 (m, 2H), 7.53 (s, 1H), 8.11 (d, 1H).

**Compound 24**

4-(3-((8a*S*,12*S*)-3-fluoro-8a-methyl-1,2,6b,7,8,8a,10,12,12a,12b-decahydronaphtho[2',1':4,5]indeno[1,2-c]pyrazol-12-yl)propanoyl)piperazin-2-one

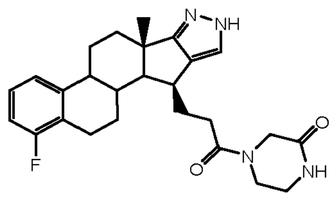

The compound **24** was prepared from the compound **23** by the Method C in 36% yield.

$^1$H NMR (400 MHz, DMSO-$d_6$): 1.09 (s, 3H), 1.30-2.47 (m, 13H), 2.67-2.92 (m, 3H), 3.15-3.25 (m, 2H), 3.51-3.68 (m, 2H), 3.92 (d, 1H), 4.02-4.08 (dd, 1H), 6.98 (m, 1H), 7.16 (m, 2H), 7.45 (d, 1H), 8.10 (d, 1H), 12.12 (br s, 1H).

**Compound 25**

3-((13S,15R)-4-fluoro-13-methyl-17-oxo-7,8,9,11,12,13,14,15,16,17-decahydro-6H-cyclopenta[a]phenanthren-15-yl)-N-(4-methylpyridin-2-yl)propanamide

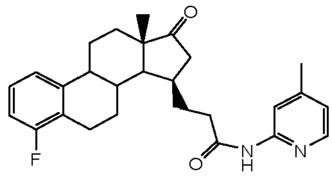

The compound **25** was synthesized in 37% yield after chromatographic purification by the Method A2 in THF by using 200 mol-% of EDCI and HOBT from acid **SM-IX** and 2-amino-4-methylpyridine as starting materials in 4.5 hours reaction time.

$^1$H-NMR (400 MHz, DMSO-$d_6$): 0.98 (s, 3H), 1.30-2.48 (m, 16H), 2.30 (s, 3H), 2.65-2.78 (m, 1H), 2.80-2.92 (m, 1H), 6.90-6.93 (m, 1H), 6.94-7.00 (m, 1H), 7.10-7.21 (m, 2H), 7.95 (s, 1H), 8.13-8.17 (m, 1H), 10.42 (s, 1H).

**Compound 26**

3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)-N-(4-methylpyridin-2-yl)propanamide

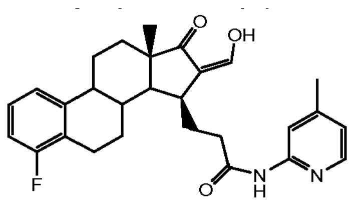

The compound **26** was synthesized in quantitative yield from the compound **25** by the Method B in 5 hours reaction time by using 500 mol-% of ethyl formate and 300 mol-% of NaH.

$^1$H-NMR (400 MHz, DMSO-$d_6$): 0.99 (s, 3H), 1.33-2.60 (m, 13H), 2.30 (s, 3H), 2.65-3.00 (m, 3H), 6.92 (d, 1H), 6.94-7.00 (m, 1H), 7.10-7.21 (m, 2H), 7.55 (s, 1H), 7.94 (s, 1H), 8.14 (d, 1H), 10.35 (s, 1H), 11.00 (br s, 1H).

**Compound 27**

3-((8aS,12S)-3-fluoro-8a-methyl-1,2,6b,7,8,8a,10,12,12a,12b-decahydronaph-tho[2',1':4,5]indeno[1,2-c]pyrazol-12-yl)-N-(4-methylpyridin-2-yl)propanamide

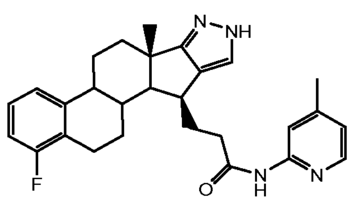

The compound **27** was synthesized in 51% yield from the compound **26** by the Method C in 0.5 hours reaction time.

$^1$H-NMR (400 MHz, DMSO-$d_6$): 1.10 (s, 3H), 1.33-2.60 (m, 13H), 2.30 (s, 3H), 2.69-2.95 (m, 3H), 6.92 (d, 1H),

6.94-7.00 (m, 1H), 7.10-7.21 (m, 2H), 7.42 (s, 1H), 7.96 (s, 1H), 8.16 (d, 1H), 10.47 (s, 1H), 12.16 (br s, 1H).

**Compound 28**

3-((13S,15R)-4-fluoro-13-methyl-17-oxo-7,8,9,11,12,13,14,15,16,17-decahydro-6H-cyclopenta[a]phenanthren-15-yl)-N-(2-oxo-1,2,5,6,7,8-hexahydroquinolin-3-yl)propanamide

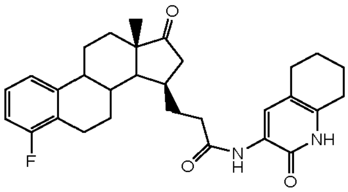

The compound **28** was synthesized in 86% yield by the Method A1 in THF by using acid **SM-IX** and 3-amino-1,2,5,6,7,8-hexahydroquinolin-2-one as starting materials in overnight reaction time.

$^1$H-NMR (400 MHz, DMSO-$d_6$): 0.97 (s, 3H), 1.30-2.47 (m, 24H), 2.65-2.93 (m, 2H), 6.94-7.00 (m, 1H), 7.12-7.21 (m, 2H), 8.01 (s, 1H), 9.15 (s, 1H), 11.68 (br s, 1H).

**Compound 29**

3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)-N-(2-oxo-1,2,5,6,7,8-hexahydroquinolin-3-yl)propanamide

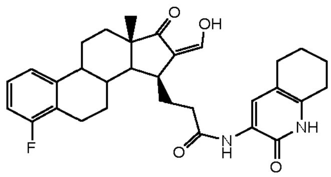

The compound **29** was synthesized in 73% yield from the compound **28** by the Method B in overnight reaction time.

$^1$H-NMR (400 MHz, DMSO-$d_6$): 0.98 (s, 3H), 1.30-2.65 (m, 21H), 2.65-2.97 (m, 3H), 6.94-7.00 (m, 1H), 7.11-7.21 (m, 2H), 7.55 (s, 1H), 8.00 (s, 1H), 9.00 (s, 1H), 11.20 (br s, 1H), 11.68 (s, 1H).

**Compound 30**

3-((8aS,12S)-3-fluoro-8a-methyl-1,2,6b,7,8,8a,10,12,12a,12b-decahydronaph-tho[2',1':4,5]indeno[1,2-c]pyrazol-12-yl)-N-(2-oxo-1,2,5,6,7,8-hexahydroquinolin-3-yl)propanamide

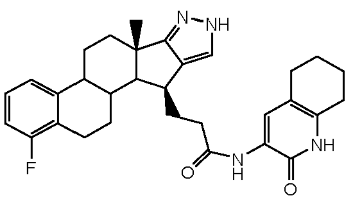

The compound **30** was synthesized in 72% yield from the compound **29** by the Method C in 2 hours reaction time.

$^1$H-NMR (400 MHz, DMSO-$d_6$): 1.10 (s, 3H), 1.30-2.65 (m, 21H), 2.69-2.97 (m, 3H), 6.94-7.00 (m, 1H), 7.11-7.21

(m, 2H), 7.42 (s, 1H), 8.00 (s, 1H), 9.15 (s, 1H), 11.68 (s, 1H), 12.12 (br s, 1H).

**Compound 31**

6-(3-((13S,15R)-4-fluoro-13-methyl-17-oxo-7,8,9,11,12,13,14,15,16,17-decahydro-6H-cyclopenta[a]phenanthren-15-yl)propanamido)-N,N-dimethylnicotinamide

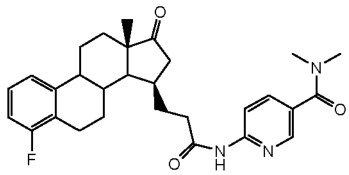

The compound **31** was synthesized in quantitative yield by the Method A1 in THF by using acid **SM-IX** and 6-amino-N,N-dimethylpyridine-3-carboxamide as starting materials in overnight reaction time.

$^1$H-NMR (400 MHz, DMSO-d$_6$): 0.98 (s, 3H), 1.30-2.47 (m, 16H), 2.66-2.94 (m, 2H), 2.98 (s, 6H), 6.94-7.00 (m, 1H), 7.12-7.21 (m, 2H), 7.85 (dd, 1H), 8.14 (d, 1H), 8.38 (d, 1H), 10.72 (s, 1H).

**Compound 32**

6-(3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)propanamido)-N,N-dimethylnicotinamide

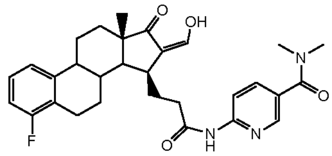

The compound **32** was synthesized in 33% yield from the compound **31** by the Method B in overnight reaction time.

$^1$H-NMR (400 MHz, DMSO-d$_6$): 1.00 (s, 3H), 1.30-2.65 (m, 13H), 2.66-2.94 (m, 3H), 2.97 (s, 6H), 6.93-7.00 (m, 1H), 7.11-7.21 (m, 2H), 7.55 (s, 1H), 7.84 (dd, 1H), 8.14 (d, 1H), 8.37 (d, 1H), 10.66 (s, 1H), 10.98 (br s, 1H).

**Compound 33**

6-(3-((8aS,12S)-3-fluoro-8a-methyl-1,2,6b,7,8,8a,10,12,12a,12b-decahydronaphtho[2',1':4,5]indeno[1,2-c]pyrazol-12-yl)propanamido)-N,N-dimethylnicotinamide

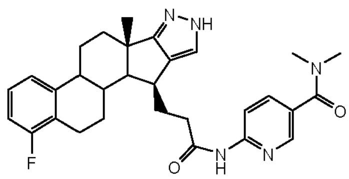

The compound **33** was synthesized in 59% yield from the compound **32** by the Method C in one hour reaction time.

$^1$H-NMR (400 MHz, DMSO-$d_6$): 1.11 (s, 3H), 1.35-2.60 (m, 13H), 2.69-2.94 (m, 3H), 2.98 (s, 6H), 6.93-7.02 (m, 1H), 7.11-7.21 (m, 2H), 7.41 (s, 1H), 7.85 (dd, 1H), 8.15 (d, 1H), 8.39 (d, 1H), 10.76 (s, 1H), 12.14 (br s, 1H).

**Compound 34**

3-((13S,15R)-4-fluoro-13-methyl-17-oxo-7,8,9,11,12,13,14,15,16,17-decahydro-6H-cyclopenta[a]phenanthren-15-yl)-N-(5-isopropylpyridin-2-yl)propanamide

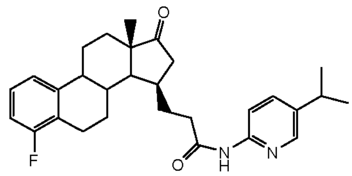

The compound **34** was synthesized in 20% yield after chromatographic purification by the Method A1 in THF by using acid **SM-IX** and 2-amino-5-isopropylpyri-dine as starting materials in 4 hours reaction time.
$^1$H-NMR (200 MHz, $CDCl_3$): 1.07 (s, 3H), 1.25 (s, 3H), 1.28 (s, 3H), 1.34-2.60 (m, 17H), 2.72-3.05 (m, 2H), 6.83-6.92 (m, 1H), 7.05-7.18 (m, 2H), 7.57-7.63 (m, 1H), 8.09-8.17 (m, 2H), 8.49 (br s, 1H).

**Compound 35**

3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)-N-(5-isopropylpyridin-2-yl)propanamide

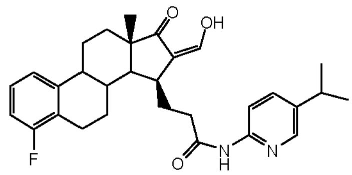

The compound **35** was synthesized in 51% yield after chromatographic purification from the compound **34** by the Method B in overnight reaction time by using 1000 mol-% of ethyl formate and 600 mol-% of NaH.
$^1$H-NMR (400 MHz, DMSO-$d_6$): 0.99 (s, 3H), 1.21 (s, 6H), 1.30-2.63 (m, 14H), 2.64-3.05 (m, 3H), 6.93-7.00 (m, 1H), 7.11-7.21 (m, 2H), 7.56 (s, 1H), 7.63-7.67 (m, 1H), 7.99-8.02 (m, 1H), 8.18 (s, 1H), 10.41 (s, 1H), 11.03 (br s, 1H).

**Compound 36**

3-((8aS,12S)-3-fluoro-8a-methyl-1,2,6b,7,8,8a,10,12,12a,12b-decahydronaphtho[2',1':4,5]indeno[1,2-c]pyrazol-12-yl)-N-(5-isopropylpyridin-2-yl)propanamide

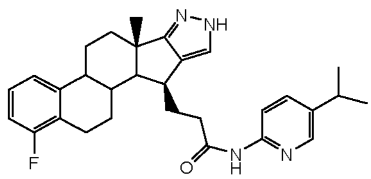

The compound **36** was synthesized in 88% yield from the compound **35** by the Method C at 60 °C in one-hour reaction time.
$^1$H-NMR (400 MHz, DMSO-$d_6$): 1.10 (s, 3H), 1.20 (s, 3H), 1.21 (s, 3H), 1.35-2.49 (m, 14H), 2.64-2.95 (m, 3H), 6.93-7.00 (m, 1H), 7.11-7.21 (m, 2H), 7.41 (s, 1H), 7.66 (dd, 1H), 8.03 (d, 1H), 8.19 (d, 1H), 10.48 (s, 1H), 12.13 (br s, 1H).

**Compound 37**

3-((13S,15R)-4-fluoro-13-methyl-17-oxo-7,8,9,11,12,13,14,15,16,17-decahydro-6H-cyclopenta[a]phenanthren-15-yl)-N-(5-morpholinopyridin-2-yl)propanamide

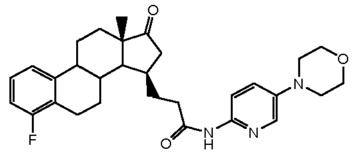

The compound **37** was synthesized in 82% yield after chromatographic purification by the Method A1 in DCM by using acid **SM-IX** and 5-morpholinopyridin-2-amine as starting materials and triethylamine as base in 2 hours reaction time.

$^1$H-NMR (400 MHz, DMSO-$d_6$): 0.98 (s, 3H), 1.30-2.46 (m, 16H), 2.63-2.80 (m, 1H), 2.81-2.96 (m, 1H), 3.03-3.15 (m, 4H), 3.68-3.80 (m, 4H), 6.90-7.03 (m, 1H), 7.10-7.22 (m, 2H), 7.40 (dd, 1H), 7.95-8.01 (m, 2H), 10.29 (s, 1H).

**Compound 38**

3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)-N-(5-morpholinopyridin-2-yl)propanamide

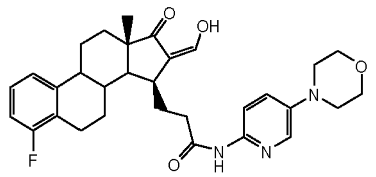

The compound **38** was synthesized in 57% yield after chromatographic purification from the compound **37** by the Method B in 2 days reaction time by using 1500 mol-% of ethyl formate and 1050 mol-% of NaH.

$^1$H-NMR (400 MHz, DMSO-$d_6$): 0.99 (s, 3H), 1.30-2.60 (m, 13H), 2.63-2.99 (m, 3H), 3.03-3.15 (m, 4H), 3.68-3.80 (m, 4H), 6.90-7.03 (m, 1H), 7.10-7.22 (m, 2H), 7.39 (dd, 1H), 7.56 (s, 1H), 7.95 (d, 1H), 7.99 (d, 1H), 10.28 (s, 1H), 11.04 (br s, 1H).

**Compound 39**

3-((8aS,12S)-3-fluoro-8a-methyl-1,2,6b,7,8,8a,10,12,12a,12b-decahydronaphtho[2',1':4,5]indeno[1,2-c]pyrazol-12-yl)-N-(5-morpholinopyridin-2-yl)propanamide

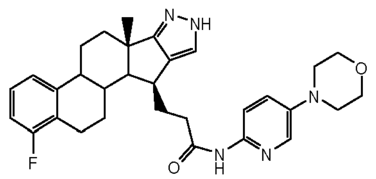

The compound **39** was synthesized in 71% yield after chromatographic purification from the compound **38** by the Method C in one-hour reaction time.

$^1$H-NMR (400 MHz, DMSO-$d_6$): 1.10 (s, 3H), 1.35-2.49 (m, 13H), 2.67-2.95 (m, 3H), 3.05-3.14 (m, 4H), 3.69-3.78 (m, 4H), 6.92-7.03 (m, 1H), 7.12-7.22 (m, 2H), 7.39 (dd, 1H), 7.41 (s, 1H), 7.97 (d, 1H), 8.00 (d, 1H), 10.32 (s, 1H), 12.11 (br s, 1H).

**Compound 40**

3-((13S,15R)-4-fluoro-13-methyl-17-oxo-7,8,9,11,12,13,14,15,16,17-decahydro-6H-cyclopenta[a]phenanthren-15-yl)-N-(5-(4-methylpiperazin-1-yl)pyridin-2-yl)propanamide

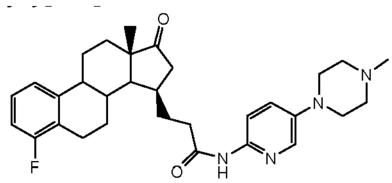

The compound **40** was synthesized in 83% yield after chromatographic purification by the Method A1 in DCM by using acid **SM-IX** and 1-methyl-4-(6-aminopyridin-3-yl)piperazine as starting materials and triethylamine as base in 2 hours reaction time.

$^1$H-NMR (400 MHz, DMSO-$d_6$): 0.98 (s, 3H), 1.30-2.42 (m, 16H), 2.21 (s, 3H), 2.43-2.48 (m, 4H), 2.63-2.80 (m, 1H), 2.81-2.96 (m, 1H), 3.05-3.15 (m, 4H), 6.93-7.03 (m, 1H), 7.10-7.22 (m, 2H), 7.39 (dd, 1H), 7.92-8.00 (m, 2H), 10.27 (s, 1H).

**Compound 41**

3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)-N-(5-(4-methylpiperazin-1-yl)pyridin-2-yl)propanamide

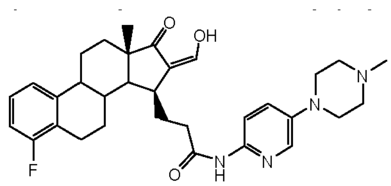

The compound **41** was synthesized in 57% yield from the compound **40** by the Method B in overnight reaction time.

$^1$H-NMR (400 MHz, DMSO-$d_6$): 0.99 (s, 3H), 1.30-2.49 (m, 13H), 2.28 (s, 3H), 2.51-2.60 (m, 4H), 2.63-2.96 (m, 3H), 3.10-3.20 (m, 4H), 6.93-7.03 (m, 1H), 7.09-7.22 (m, 2H), 7.39 (dd, 1H), 7.57 (s, 1H), 7.93 (d, 1H), 7.99 (d, 1H), 10.28 (s, 1H).

**Compound 42**

3-((8aS,12S)-3-fluoro-8a-methyl-1,2,6b,7,8,8a,10,12,12a,12b-decahydronaphtho[2',1':4,5]indeno[1,2-c]pyrazol-12-yl)-N-(5-(4-methylpiperazin-1-yl)pyridin-2-yl)propanamide

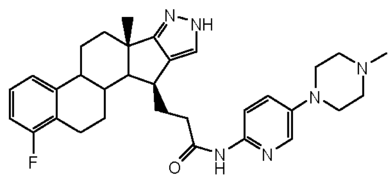

The compound **42** was synthesized in 53% yield from the compound **41** by the Method C in 2.5 hours reaction time.

$^1$H-NMR (400 MHz, DMSO-$d_6$): 1.10 (s, 3H), 1.30-2.49 (m, 13H), 2.21 (s, 3H), 2.42-2.59 (m, 4H), 2.65-2.96

(m, 3H), 3.05-3.20 (m, 4H), 6.93-7.03 (m, 1H), 7.09-7.22 (m, 2H), 7.36-7.39 (m, 1H), 7.40 (s, 1H), 7.95 (d, 1H), 7.99 (d, 1H), 10.31 (s, 1H), 12.12 (br s, 1H).

**Compound 43**

3-((13S,15R)-4-fluoro-13-methyl-17-oxo-7,8,9,11,12,13,14,15,16,17-decahydro-6H-cyclopenta[a]phenanthren-15-yl)-N-(tetrahydro-2H-pyran-4-yl)propanamide

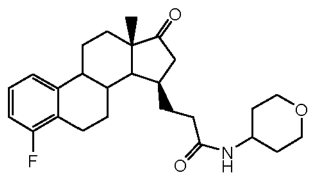

The compound **43** was synthesized in 56% yield after chromatographic purification by the Method A3 in DCM by using acid **SM-IX** and 4-aminotetrahydropyran as starting materials in 5 hours reaction time.

$^1$H-NMR (400 MHz, DMSO-$d_6$): 0.96 (s, 3H), 1.30-2.41 (m, 20 H), 2.67-2.76 (m, 1H), 2.85-2.90 (m, 1H), 3.29-3.30 (m, 2H), 3.70-3.77 (m, 1H), 3.80-3.83 (m, 2H), 6.94-7.00 (m, 1H), 7.10-7.22 (m, 2H), 7.84 (d, 1H).

**Compound 44**

3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)-N-(tetrahydro-2H-pyran-4-yl)propanamide

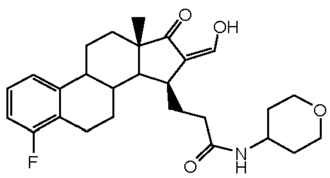

The compound **44** was synthesized in quantitative yield from the compound **43** by the Method B in 6 hours reaction time by using 1000 mol-% of ethyl formate and 600 mol-% of NaH.

$^1$H-NMR (400 MHz, DMSO-$d_6$): 0.97 (s, 3H), 1.30-2.45 (m, 17 H), 2.65-2.95 (m, 3H), 3.25-3.45 (m, 2H), 3.70-3.95 (m, 3H), 6.94-7.00 (m, 1H), 7.10-7.22 (m, 2H), 7.49 (s, 1H), 8.14 (d, 1H), 11.59 (br s, 1H).

**Compound 45**

3-((8aS,12S)-3-fluoro-8a-methyl-1,2,6b,7,8,8a,10,12,12a,12b-decahydronaphtho[2',1':4,5]indeno[1,2-c]pyrazol-12-yl)-N-(tetrahydro-2H-pyran-4-yl)propanamide

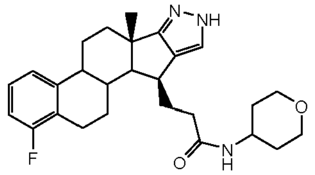

The compound **45** was synthesized in 76% yield from the compound **44** by the Method C in 1.5 hours reaction time.

$^1$H-NMR (400 MHz, DMSO-$d_6$): 1.09 (s, 3H), 1.30-2.49 (m, 17 H), 2.65-2.95 (m, 3H), 3.25-3.45 (m, 2H), 3.70-3.90

(m, 3H), 6.94-7.01 (m, 1H), 7.10-7.22 (m, 2H), 7.40 (s, 1H), 7.86 (d, 1H), 12.12 (br s, 1H).

**Compound 46**

3-((13S,15R)-4-fluoro-13-methyl-17-oxo-7,8,9,11,12,13,14,15,16,17-decahydro-6H-cyclopenta[a]phenanthren-15-yl)-N-(4-methoxypyridin-2-yl)propanamide

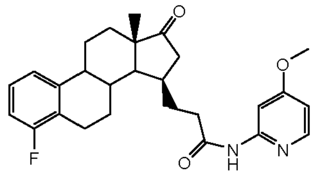

The compound **46** was synthesized in 47% yield after chromatographic purification by the Method A2 in THF by using acid **SM-IX** and 2-amino-4-methoxypyridine as starting materials in 10 hours and overnight at room temperature. Reaction needed 250 mol-% of amine, EDCI and HOBT.

$^1$H-NMR (400 MHz, DMSO-$d_6$): 0.98 (s, 3H), 1.30-2.48 (m, 16H), 2.65-2.78 (m, 1H), 2.80-2.92 (m, 1H), 3.81 (s, 3H), 6.68-6.72 (m, 1H), 6.94-7.00 (m, 1H), 7.10-7.21 (m, 2H), 7.73 (s, 1H), 8.10-8.13 (m, 1H), 10.47 (s, 1H).

**Compound 47**

3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)-N-(4-methoxypyridin-2-yl)propanamide

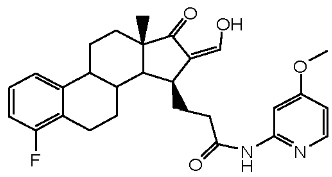

The compound **47** was synthesized in 86% yield from the compound **46** by the Method B in 2.5 hours reaction time.

$^1$H-NMR (400 MHz, DMSO-$d_6$): 0.99 (s, 3H), 1.30-2.65 (m, 13H), 2.62-2.97 (m, 3H), 3.80 (s, 3H), 6.65-6.70 (dd, 1H), 6.93-7.00 (m, 1H), 7.10-7.21 (m, 2H), 7.58 (s, 1H), 7.73 (d, 1H), 8.10-8.13 (d, 1H), 10.46 (s, 1H), 11.04 (br s).

**Compound 48**

3-((8aS,12S)-3-fluoro-8a-methyl-1,2,6b,7,8,8a,10,12,12a,12b-decahydronaphtho[2',1':4,5]indeno[1,2-c]pyrazol-12-yl)-N-(4-methoxypyridin-2-yl)propanamide

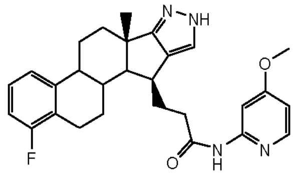

The compound **48** was synthesized in 47% yield from the compound **47** by the Method C in 3 hours reaction time.

$^1$H-NMR (400 MHz, DMSO-$d_6$): 1.11 (s, 3H), 1.30-2.49 (m, 13H), 2.70-2.97 (m, 3H), 3.80 (s, 3H), 6.68-6.72 (dd, 1H), 6.93-7.01 (m, 1H), 7.10-7.21 (m, 2H), 7.39 (s, 1H), 7.73 (d, 1H), 8.10-8.14 (d, 1H), 10.50 (s, 1H), 12.12 (br s).

**Compound 49**

3-((13S,15R)-4-fluoro-13-methyl-17-oxo-7,8,9,11,12,13,14,15,16,17-decahydro-6H-cyclopenta[a]phenanthren-15-yl)-N-(pyrazin-2-yl)propanamide

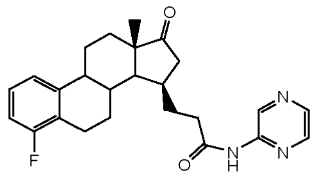

The compound **49** was synthesized in 53% yield after chromatographic purification by the Method A1 in DCM by using acid **SM-IX** and aminopyrazine as starting materials in 5 hours reaction time.

$^1$H-NMR (400 MHz, DMSO-$d_6$): 0.99 (s, 3H), 1.30-2.60 (m, 16H), 2.69-2.78 (m, 1H), 2.84-2.92 (m, 1H), 6.94-7.00 (m, 1H), 7.12-7.20 (m, 2H), 8.33-8.40 (m, 2H), 9.35 (s, 1H), 10.81 (s, 1H).

**Compound 50**

3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)-N-(pyrazin-2-yl)propanamide

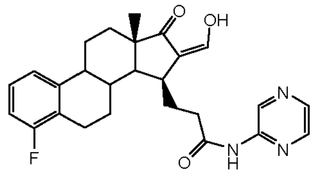

The compound **50** was synthesized in quantitative yield from the compound **49** by the Method B in overnight reaction time by using 750 mol-% of ethyl formate and 450 mol-% of NaH.

$^1$H-NMR (400 MHz, DMSO-$d_6$): 1.00 (s, 3H), 1.30-2.49 (m, 13H), 2.55-2.99 (m, 3H), 6.94-7.00 (m, 1H), 7.11-7.20 (m, 2H), 7.54 (s, 1H), 8.33-8.40 (m, 2H), 9.34 (s, 1H), 10.75 (s, 1H), 10.98 (br s, 1H).

**Compound 51**

3-((8aS,12S)-3-fluoro-8a-methyl-1,2,6b,7,8,8a,10,12,12a,12b-decahydronaphtho[2',1':4,5]indeno[1,2-c]pyrazol-12-yl)-N-(pyrazin-2-yl)propanamide

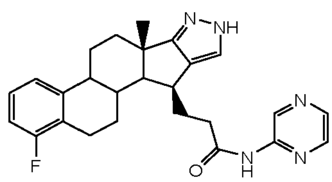

The compound **51** was synthesized in 30% yield after chromatographic purification from the compound **50** by the Method C in one-hour reaction time.

$^1$H-NMR (400 MHz, DMSO-$d_6$): 1.11 (s, 3H), 1.35-2.60 (m, 13H), 2.65-2.99 (m, 3H), 6.94-7.01 (m, 1H), 7.11-7.20 (m, 2H), 7.40 (s, 1H), 8.33-8.41 (m, 2H), 9.35 (s, 1H), 10.85 (s, 1H), 12.16 (br s, 1H).

**Compound 52**

3-((13S,15R)-4-fluoro-13-methyl-17-oxo-7,8,9,11,12,13,14,15,16,17-decahydro-6H-cyclopenta[a]phenanthren-15-yl)-N-(1-methyl-1H-pyrazol-3-yl)propanamide

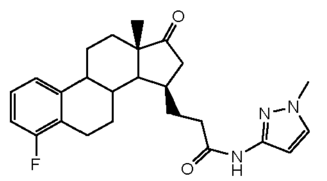

The compound **52** was synthesized in 80% yield by the Method A1 in THF by using acid **SM-IX** and 1-methyl-1H-pyrazol-3-amine as starting materials and 300 mol-% of T3P in overnight reaction time.

$^1$H-NMR (400 MHz, DMSO-$d_6$): 0.97 (s, 3H), 1.30-2.49 (m, 16H), 2.65-2.92 (m, 2H), 3.72 (s, 3H), 6.43 (d, 1H), 6.94-7.00 (m, 1H), 7.12-7.20 (m, 2H), 7.52 (d, 1H), 10.36 (s, 1H).

**Compound 53**

3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)-N-(1-methyl-1H-pyrazol-3-yl)propanamide

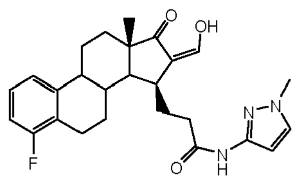

The compound **53** was synthesized in quantitative yield from the compound **52** by the Method B in 4 hours reaction time.

$^1$H-NMR (400 MHz, DMSO-$d_6$): 0.99 (s, 3H), 1.30-2.49 (m, 13H), 2.65-2.96 (m, 3H), 3.72 (s, 3H), 6.42 (d, 1H), 6.93-7.02 (m, 1H), 7.11-7.23 (m, 2H), 7.51 (d, 1H), 7.54 (s, 1H), 10.35 (s, 1H), 11.02 (br s, 1H).

**Compound 54**

3-((8aS,12S)-3-fluoro-8a-methyl-1,2,6b,7,8,8a,10,12,12a,12b-decahydronaphtho[2',1':4,5]indeno[1,2-c]pyrazol-12-yl)-N-(1-methyl-1H-pyrazol-3-yl)propanamide

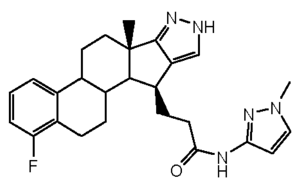

The compound **54** was synthesized in 69% yield from the compound **53** by the Method C in one-hour reaction time.

$^1$H-NMR (400 MHz, DMSO-$d_6$): 1.10 (s, 3H), 1.30-2.49 (m, 13H), 2.65-2.96 (m, 3H), 3.73 (s, 3H), 6.44 (d, 1H), 6.93-7.02 (m, 1H), 7.11-7.23 (m, 2H), 7.40 (s, 1H), 7.52 (d, 1H), 10.41 (s, 1H), 12.12 (br s, 1H).

**Compound 55**

(13S,15R)-4-fluoro-15-(3-(isoindolin-2-yl)-3-oxopropyl)-13-methyl-6,7,8,9,11,12,13,14,15,16-decahdro-17H-cyclopenta[a]phenanthren-17-one

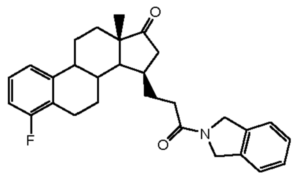

The compound **55** was synthesized in quantitative yield by the Method A1 in THF by using acid **SM-IX** and isoindoline as starting materials in overnight reaction time.
$^1$H-NMR (400 MHz, $CDCl_3$): 1.09 (s, 3H), 1.34-2.60 (m, 16H), 2.72-3.05 (m, 2H), 4.82 (s, 4H), 6.83-6.92 (m, 1H), 7.05-7.18 (m, 2H), 7.25-7.34 (m, 4H).

**Compound 56**

(13S,15S,Z)-4-fluoro-16-(hydroxymethylene)-15-(3-(isoindolin-2-yl)-3-oxopropyl)-13-methyl-6,7,8,9,11,12,13,14,15,16-decahydro-17H-cyclopenta[a]phenanthren-17-one

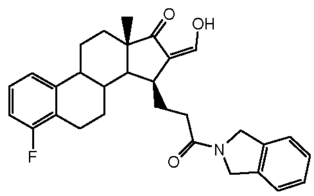

The compound **56** was synthesized in 34% yield from the compound **55** by the Method B in overnight reaction time.
$^1$H-NMR (400 MHz, DMSO-$d_6$): 1.00 (s, 3H), 1.30-2.62 (m, 13H), 2.63-3.05 (m, 3H), 4.65 (s, 2H), 4.75-4.88 (m, 2H), 6.93-7.02 (m, 1H), 7.11-7.23 (m, 2H), 7.25-7.40 (m, 4H), 7.55 (s, 1H), 11.09 (br s, 1H).

**Compound 57**

3-((8aS,12S)-3-fluoro-8a-methyl-1,2,6b,7,8,8a,10,12,12a,12b-decahydronaphtho[2',1':4,5]indeno[1,2-c]pyrazol-12-yl)-1-(isoindolin-2-yl)propan-1-one

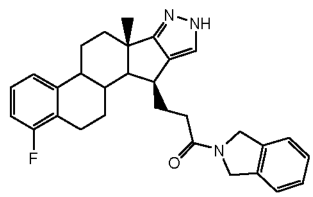

The compound **57** was synthesized in 93% yield from the compound **56** by the Method C in one-hour reaction time.
$^1$H-NMR (400 MHz, DMSO-*d*$_6$): 1.12 (s, 3H), 1.30-2.49 (m, 13H), 2.63-3.00 (m, 3H), 4.64 (s, 2H), 4.80-4.99 (m, 2H), 6.91-7.05 (m, 1H), 7.11-7.23 (m, 2H), 7.25-7.40 (m, 4H), 7.48 (s, 1H), 12.13 (br s, 1H).

**Compound 58**

N-(cyclopropylmethyl)-3-((13S,15R)-4-fluoro-13-methyl-17-oxo-7,8,9,11,12,13,14,15,16,17-decahydro-6H-cyclopenta[a]phenanthren-15-yl)-N-methylpropanamide

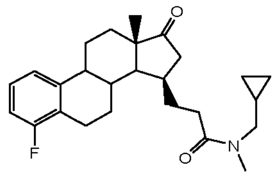

The compound **58** was synthesized in 34% yield by the Method A1 in THF by using acid **SM-IX** and 400 mol-% of (cyclopropylmethyl) (methyl)amine, 600 mol-% of pyridine and 400 mol-% of T3P in overnight reaction time.

$^1$H-NMR (400 MHz, $CDCl_3$): 0.21-0.27 (m, 2H), 0.45-0.55 (m, 1H), 0.55-0.65 (m, 1H), 0.87-1.02 (m, 1H), 1.08 (s, 3H), 1.30-2.65 (m, 16H), 2.75-3.00 (m, 2H), 3.01/3.07 (2 x s, 3H, isomers), 3.13-3.36 (m, 2H), 6.83-6.92 (m, 1H), 7.04-7.18 (m, 2H).

**Compound 59**

N-(cyclopropylmethyl)-3-((13S,15S,Z)-4-fluoro-16-(hydroxymethylene)-13-methyl-17-oxo-7,8,9,11,12,13,14,15,16,17-decahydro-6H-cyclopenta[a]phenan-thren-15-yl)-N-methylpropanamide

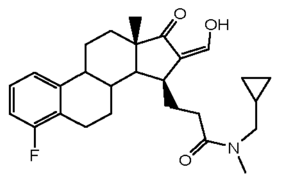

The compound **59** was synthesized in quantitative yield from the compound **58** by the Method B in overnight reaction time by using 1200 mol-% of ethyl formate and 800 mol-% of NaH.

$^1$H-NMR (400 MHz, DMSO-$d_6$): 0.15-0.27 (m, 2H), 0.35-0.55 (m, 2H), 0.90-1.02 (m, 1H), 1.17 (s, 3H), 1.30-2.49 (m, 13H), 2.59-3.00 (m, 3H), 2.88/3.04 (2 x s, 3H, isomers), 3.05-3.30 (m, 2H), 6.93-7.02 (m, 1H), 7.11-7.23 (m, 2H), 7.48-7.51 (br s, 1H), 11.33/11.50 (2 x br s, 1H, isomers).

**Compound 60**

N-(cyclopropylmethyl)-3-((8aS,12S)-3-fluoro-8a-methyl-1,2,6b,7,8,8a,10,12,12a,12b-decahydronaphtho[2',1':4,5]indeno[1,2-c]pyrazol-12-yl)-N-methylpropanamide

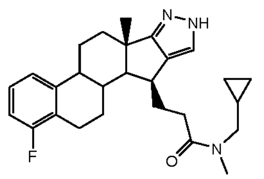

The compound **60** was synthesized in 30% yield after chromatographic purification from the compound **59** by the Method C in one-hour reaction time.

$^1$H-NMR (400 MHz, DMSO-$d_6$): 0.15-0.27 (m, 2H), 0.35-0.55 (m, 2H), 0.90-1.02 (m, 1H), 1.10 (s, 3H), 1.30-2.49 (m, 13H), 2.68-3.00 (m, 3H), 2.86/3.04 (2 x s, 3H, isomers), 3.05-3.30 (m, 2H), 6.93-7.02 (m, 1H), 7.11-7.23 (m, 2H),

7.38-7.45 (2 x br s, 1H, isomers), 12.12 (br s, 1H).

**Compound 61**

3-((13S,15R)-4-fluoro-13-methyl-17-oxo-7,8,9,11,12,13,14,15,16,17-decahydro-6H-cyclopenta[a]phenanthren-15-yl)-N-(1-methyl-1H-pyrazol-4-yl)propanamide

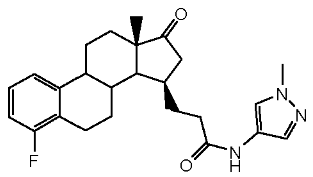

The compound **61** was synthesized in 70% yield by the Method A2 in DMF by using acid **SM-IX** and 1-methyl-1H-pyrazol-4-amine as starting materials.
$^1$H-NMR (400 MHz, $CDCl_3$): 1.07 (s, 3H), 1.39-2.48 (m, 16H), 2.76-3.02 (m, 2H), 3.49 (s, 3H), 6.88 (dd, 1H), 7.06-7.15 (m, 2H), 7.36 (m, 2H), 7.91 (s, 1H).

**Compound 62**

3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)-N-(1-methyl-1H-pyrazol-4-yl)propanamide

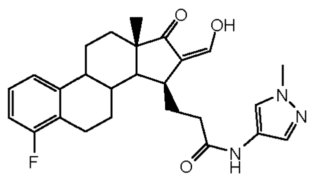

The compound **62** was synthesized in 76% yield from the compound **61** by the Method B in overnight reaction time.
$^1$H-NMR (400 MHz, DMSO-$d_6$): 0.99 (s, 3H), 1.36-2.45 (m, 13H), 2.69-2.93 (m, 3H), 3.77 (s, 3H), 6.97 (m, 1H), 7.09-7.22 (m, 2H), 7.36 (s, 1H), 7.55 (s, 1H), 7.84 (s, 1H), 9.98 (s, 1H), 11.10 (br s, 1H).

**Compound 63**

3-((8aS,12S)-3-fluoro-8a-methyl-1,2,6b,7,8,8a,10,12,12a,12b-decahydronaph-tho[2',1':4,5]indeno[1,2-c]pyrazol-12-yl)-N-(1-methyl-1H-pyrazol-4-yl)propanamide

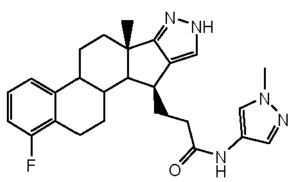

The compound **63** was synthesized in 84% yield from the compound **62** by the Method C in one-hour reaction time.
$^1$H-NMR (400 MHz, DMSO-$d_6$): 1.10 (s, 3H), 1.47-2.29 (m, 13H), 2.67-2.92 (m, 3H), 3.77 (s, 3H), 6.98 (m, 1H), 7.16 (m, 2H), 7.37 (d, 2H), 7.87 (s, 1H), 9.95 (s, 1H), 12.15 (br s, 1H).

**Compound 64**

3-((13S,15R)-4-fluoro-13-methyl-17-oxo-7,8,9,11,12,13,14,15,16,17-decahydro-6H-cyclopenta[a]phenanthren-15-yl)-N-(5-methyl-1,3,4-oxadiazol-2-yl)propanamide

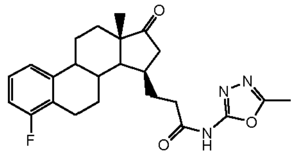

The compound **64** was synthesized in 45% yield by the Method A2 in DMF by using acid **SM-IX** and 5-methyl-1,3,4-oxadiazol-2-ylamine as starting materials in 5 hours reaction time.

$^1$H-NMR (400 MHz, DMSO-$d_6$): 0.97 (s, 3H), 1.30-2.43 (m, 16H), 2.44 (s, 3H), 2.66-2.98 (m, 2H), 6.92-7.03 (m, 1H), 7.10-7.22 (m, 2H), 11.50 (br s, 1H).

**Compound 65**

3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)-N-(5-methyl-1,3,4-oxadiazol-2-yl)propanamide

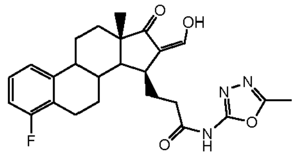

The compound **65** was synthesized in 97% yield from the compound **64** by the Method B in overnight reaction time by using 900 mol-% of ethyl formate and 600 mol-% of NaH.

$^1$H-NMR (400 MHz, DMSO-$d_6$): 0.99 (s, 3H), 1.30-2.65 (m, 13H), 2.43 (s, 3H), 2.66-2.99 (m, 3H), 6.92-7.03 (m, 1H), 7.10-7.22 (m, 2H), 7.56 (s, 1H), 10.96 (br s, 1H), 11.52 (br s, 1H).

**Compound 66**

3-((8aS,12S)-3-fluoro-8a-methyl-1,2,6b,7,8,8a,10,12,12a,12b-decahydronaph-tho[2',1':4,5]indeno[1,2-c]pyrazol-12-yl]-N-(5-methyl-1,3,4-oxadiazol-2-yl)propanamide

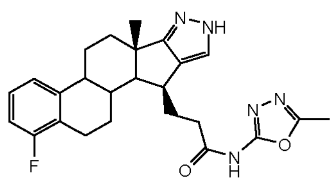

The compound **66** was synthesized in 3% yield from the compound **65** by the Method C in 2.5 hours reaction time.

$^1$H-NMR (400 MHz, $CDCl_3$): 1.18 (s, 3H), 1.40-2.50 (m, 13H), 2.57 (s, 3H), 2.65-3.05 (m, 3H), 6.84-6.95 (m, 1H), 7.03-7.17 (m, 2H).

**Compound 67**

3-((13S,15R)-4-fluoro-13-methyl-17-oxo-7,8,9,11,12,13,14,15,16,17-decahydro-6H-cyclopenta[a]phenanthren-15-yl)-N-(piperidin-1-yl)propanamide

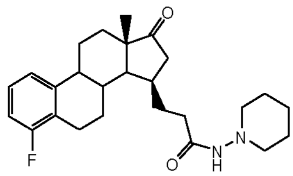

The compound **67** was synthesized in 45% yield after chromatographic purification by the Method A3 in DCM by using acid **SM-IX** and piperidin-1-amine as starting materials in 4 hours reaction time.

$^1$H-NMR (400 MHz, DMSO-$d_6$): 0.95/0.96 (2 x s, 3H, isomers), 1.30-2.45 (m, 23H), 2.60-2.98 (m, 5H), 6.92-7.03 (m, 1H), 7.10-7.22 (m, 2H), 8.33/8.75 (2 x s, 1H, isomers).

**Compound 68**

3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)-N-(piperidin-1-yl)propanamide

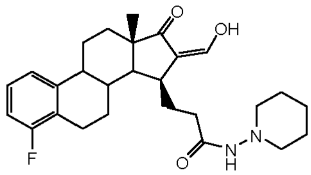

The compound **68** was synthesized in 77% yield from the compound **67** by the Method B in overnight reaction time by using 1200 mol-% of ethyl formate and 800 mol-% of NaH.

$^1$H-NMR (400 MHz, DMSO-$d_6$): 0.97 (s, 3H), 1.30-2.45 (m, 20H), 2.56-2.95 (m, 6H), 6.92-7.03 (m, 1H), 7.10-7.22 (m, 2H), 7.50/7.53 (2 x s, 1H, isomers), 8.49/9.05 (2 x s, 1H, isomers), 11.37 (br s, 1H).

**Compound 69**

3-((8aS,12S)-3-fluoro-8a-methyl-1,2,6b,7,8,8a,10,12,12a,12b-decahydronaph-tho[2',1':4,5]indeno[1,2-c]pyrazol-12-yl)-N-(piperidin-1-yl)propanamide

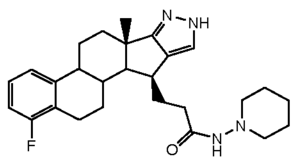

The compound **69** was synthesized in 72% yield from the compound **68** by the Method C in one hour reaction time.

$^1$H-NMR (400 MHz, DMSO-$d_6$): 1.08/1.09 (2 x s, 3H, isomers), 1.29-2.49 (m, 20H), 2.59-2.95 (m, 6H), 6.92-7.03 (m, 1H), 7.10-7.22 (m, 2H), 7.32/7.41 (2 x s, 1H, isomers), 8.31/8.79 (2 x s, 1H, isomers), 12.11 (br s, 1H).

**Compound 70**

N-(tert-butyl)-3-((13S,15R)-4-fluoro-13-methyl-17-oxo-7,8,9,11,12,13,14,15,16,17-decahydro-6H-cyclopenta[a]phenanthren-15-yl)propanamide

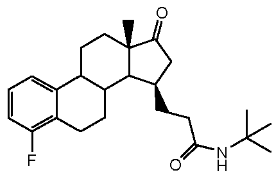

The compound **70** was synthesized in 81% yield by the Method A1 in DCM by using acid **SM-IX**, 300 mol-% of tert-butylamine, 450 mol-% of pyridine and 300 mol-% of T3P in overnight reaction time and then warming at 40 °C for 5 hours. $^1$H-NMR (400 MHz, DMSO-$d_6$): 0.95 (s, 3H), 1.25 (s, 9H), 1.30-2.43 (m, 16H), 2.66-2.95 (m, 2H), 6.92-7.02 (m, 1H), 7.10-7.22 (m, 2H), 7.46 (s, 1H).

**Compound 71**

N-(tert-butyl)-3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)propanamide

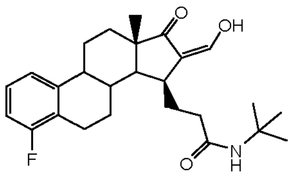

The compound **71** was synthesized in 77% yield from the compound **70** by the Method B in 2.5 hours reaction time.

$^1$H-NMR (400 MHz, DMSO-$d_6$): 0.98 (s, 3H), 1.28 (s, 9H), 1.30-2.43 (m, 13H), 2.66-2.95 (m, 3H), 6.92-7.02 (m, 1H), 7.10-7.22 (m, 2H), 7.50 (s, 1H), 7.93 (s, 1H), 11.91 (br s, 1H).

**Compound 72**

N-(tert-butyl)-3-((8aS,12S)-3-fluoro-8a-methyl-1,2,6b,7,8,8a,10,12,12a,12b-decahydronaphtho[2',1':4,5]indeno[1,2-c]pyrazol-12-yl)propanamide

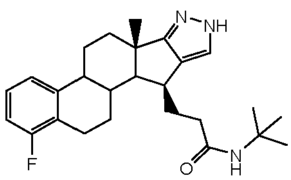

The compound **72** was synthesized in 89% yield from the compound **71** by the Method C in 30 minutes reaction time.

$^1$H-NMR (400 MHz, DMSO-$d_6$): 1.09 (s, 3H), 1.25 (s, 9H), 1.30-2.45 (m, 13H), 2.66-2.95 (m, 3H), 6.92-7.02 (m, 1H), 7.10-7.22 (m, 2H), 7.38 (s, 1H), 7.45 (s, 1H), 12.12 (br s, 1H).

**Compound 73**

(13S,15R)-4-fluoro-13-methyl-15-(3-(4-(1-methylpiperidin-4-yl)piperazin-1-yl)-3-oxopropyl)-6,7,8,9,11,12,13,14,15,16-decahydro-17H-cyclopenta[a]phenanthren-17-one

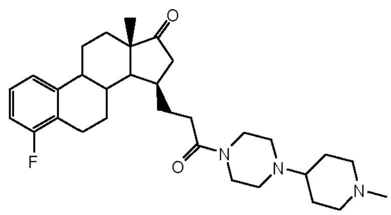

The compound **73** was synthesized in 70% yield after chromatographic purification by the Method A3 in DCM by using acid **SM-IX** and 1-(1-Methyl-4-piperidinyl)piperazine as starting materials in 5 hours reaction time.
$^1$H-NMR (400 MHz, DMSO-$d_6$): 0.96 (s, 3H), 1.30-2.49 (m, 28H), 2.12 (s, 3H), 2.65-2.95 (m, 3H), 3.35-3.50 (m, 4H), 6.92-7.02 (m, 1H), 7.10-7.22 (m, 2H).

**Compound 74**

(13S,15S,Z)-4-fluoro-16-(hydroxymethylene)-13-methyl-15-(3-(4-(1-methylpiperidin-4-yl)piperazin-1-yl)-3-oxopropyl)-6,7,8,9,11,12,13,14,15,16-decahydro-17H-cyclopenta[a]phenanthren-17-one

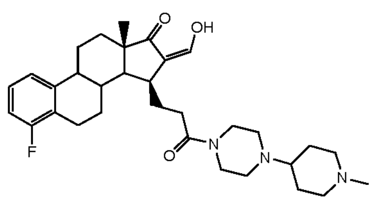

The compound **74** was synthesized in 89% yield from the compound **73** by the Method B in 6 hours reaction time.
$^1$H-NMR (400 MHz, $CDCl_3$): 1.08 (s, 3H), 1.35-3.10 (m, 29H), 2.29 (s, 3H), 3.40-3.70 (m, 4H), 6.83-6.92 (m, 1H), 7.02-7.22 (m, 2H), 7.66 (s, 1H).

**Compound 75**

3-((8aS,12S)-3-fluoro-8a-methyl-1,2,6b,7,8,8a,10,12,12a,12b-decahydronaphtho[2',1':4,5]indeno[1,2-c]pyrazol-12-yl)-1-(4-(1-methylpiperidin-4-yl)piperazin-1-yl)propan-1-one

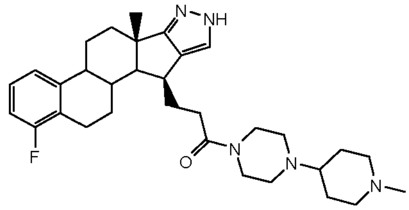

The compound **75** was synthesized in 85% yield from the compound **74** by the Method C in one hour reaction time.
$^1$H-NMR (400 MHz, DMSO-$d_6$): 1.09 (s, 3H), 1.30-2.49 (m, 24H), 2.12 (s, 3H), 2.65-2.95 (m, 5H), 3.35-3.50 (m, 4H), 6.92-7.02 (m, 1H), 7.10-7.22 (m, 2H), 7.41 (s, 1H), 12.11 (br s, 1H).

**Compound 76**

3-((13S,15R)-4-fluoro-13-methyl-17-oxo-7,8,9,11,12,13,14,15,16,17-decahydro-6H-cyclopenta[a]phenanthren-15-yl)-N-(5-methylisoxazol-3-yl)propanamide

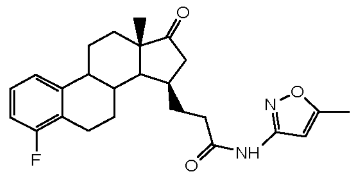

The compound **76** was synthesized in 95% yield by the Method A1 in DCM by using acid **SM-IX** and 3-amino-5-methylisoxazole as starting materials in 4 hours reaction time.

$^1$H-NMR (200 MHz, DMSO-$d_6$): 0.97 (s, 3H), 1.24-2.46 (m, 16H), 2.37 (s, 3H), 2.58-3.01 (m, 2H), 6.64 (s, 1H), 6.88-7.06 (m, 1H), 7.07-7.25 (m, 2H), 10.88 (s, 1H).

**Compound 77**

3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)-N-(5-methylisoxazol-3-yl)propanamide

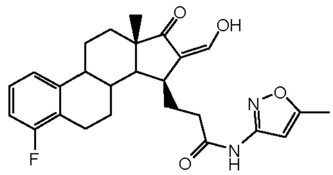

The compound **77** was synthesized in quantitative yield from the compound **76** by the Method B in one hour reaction time.

$^1$H-NMR (400 MHz, DMSO-$d_6$): 0.99 (s, 3H), 1.30-2.60 (m, 13H), 2.36 (s, 3H), 2.65-3.10 (m, 3H), 6.63 (s, 1H), 6.91-7.06 (m, 1H), 7.07-7.25 (m, 2H), 7.53 (s, 1H), 10.82 (s, 1H), 11.00 (br s, 1H).

**Compound 78**

3-((8aS,12S)-3-fluoro-8a-methyl-1,2,6b,7,8,8a,10,12,12a,12b-decahydronaphtho[2',1':4,5]indeno[1,2-c]pyrazol-12-yl)-N-(5-methylisoxazol-3-yl)propanamide

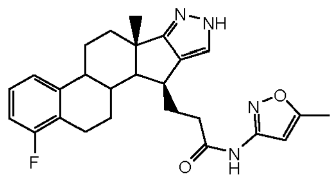

The compound **78** was synthesized in 59% yield from the compound **77** by the Method C in 0.5 hours reaction time.

$^1$H-NMR (400 MHz, DMSO-$d_6$): 1.10 (s, 3H), 1.32-2.49 (m, 13H), 2.36 (s, 3H), 2.65-3.10 (m, 3H), 6.65 (s, 1H), 6.93-7.04 (m, 1H), 7.09-7.25 (m, 2H), 7.38 (s, 1H), 10.92 (s, 1H), 12.14 (br s, 1H).

**Compound 79**

3-((13S,15R)-4-fluoro-13-methyl-17-oxo-7,8,9,11,12,13,14,15,16,17-decahydro-6H-cyclopenta[a]phenanthren-15-yl')-N-(1,3,4-thiadiazol-2-yl')propanamide

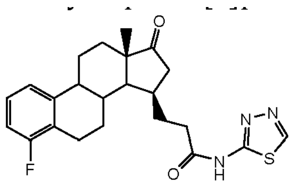

The compound **79** was synthesized in 61% yield after chromatographic purification by the Method A3 in DCM by using acid **SM-IX** and 2-amino-1,3,4-thiadiazole as starting materials in 5.5 hours reaction time.

$^1$H-NMR (200 MHz, $CDCl_3$): 1.03 (s, 3H), 1.20-3.05 (m, 18H), 6.80-6.95 (m, 1H), 7.03-7.18 (m, 2H), 8.82 (s,1H), 13.67 (br s, 1H).

**Compound 80**

3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)-N-(1,3,4-thiadiazol-2-yl)propanamide

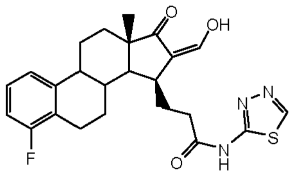

The compound **80** was synthesized in 98% yield from the compound **81** by the Method B in overnight reaction time.

$^1$H-NMR (400 MHz, DMSO-$d_6$): 1.00 (s, 3H), 1.30-2.49 (m, 13H), 2.65-3.05 (m, 3H), 6.91-7.04 (m, 1H), 7.10-7.25 (m, 2H), 7.57 (s, 1H), 9.13 (s, 1H), 10.98 (br s, 1H), 12.63 (br s, 1H).

**Compound 81**

3-((8aS,12S)-3-fluoro-8a-methyl-1,2,6b,7,8,8a,10,12,12a,12b-decahydronaphtho[2',1':4,5]indeno[1,2-c]pyrazol-12-yl)-N-(1,3,4-thiadiazol-2-yl)propanamide

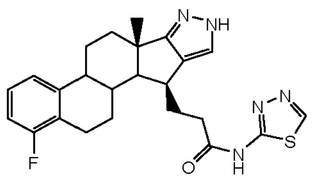

The compound **81** was synthesized in 98% yield from the compound **80** by the Method C in 30 minutes reaction time.

$^1$H-NMR (400 MHz, DMSO-$d_6$): 1.11 (s, 3H), 1.35-2.49 (m, 11H), 2.51-3.01 (m, 5H), 6.91-7.04 (m, 1H), 7.10-7.25 (m, 2H), 7.37 (s, 1H), 9.15 (s, 1H), 12.15 (br s, 1H), 12.61 (br s, 1H).

**Compound 82**

3-((13S,15R)-3-fluoro-13-methyl-17-oxo-7,8,9,11,12,13,14,15,16,17-decahydro-6H-cyclopenta[a]phenanthren-15-yl)-N-(5-methoxypyridin-2-yl)propanamide

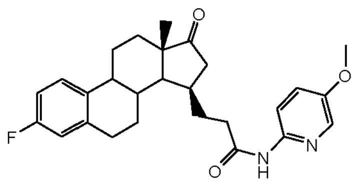

The compound **82** was synthesized in 62% yield by the Method A2 in DMF by using acid **SM-IX** and 5-methoxypyridine-2-amine as starting materials in 2 hours reaction time.
$^1$H-NMR (200 MHz, $CDCl_3$): 1.07 (s, 3H), 1.39-2.50 (m, 16H), 2.94 (m, 2H), 3.85 (s, 3H), 6.79-6.88 (m, 2H), 7.19-7.30 (m, 2H), 7.90 (br s, 1H), 7.95 (d, 1H), 8.14 (d, 1H).

**Compound 83**

3-((13S,15S,Z)-3-fluoro-16-(hydroxymethylene)-13-methyl-17-oxo-7,8,9,11,12,13,14,15,16,17-decahydro-6H-cyclopenta[a]phenanthren-15-yl)-N-(5-methoxypyridin-2-yl)propanamide

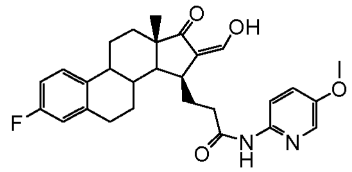

The compound **83** was prepared from the compound **82** by the Method B in quantitative yield.
$^1$H NMR (400 MHz, DMSO-$d_6$): 0.99 (s, 3H), 1.48-2.46 (m, 12H), 2.83-2.97 (m, 3H), 3.79 (s, 3H), 6.89-6.95 (m, 2H), 7.27 (m, 1H), 7.40 (dd, 1H), 7.54 (s, 1H), 8.01 (dd, 2H), 10.31 (s, 1H), 11.05 (br s, 1H).

**Compound 84**

3-((8aS,12S)-4-fluoro-8a-methyl-1,2,6b,7,8,8a,10,12,12a,12b-decahydronaphtho[2',1':4,5]indeno[1,2-c]pyrazol-12-yl)-N-(5-methoxypyridin-2-yl)propanamide

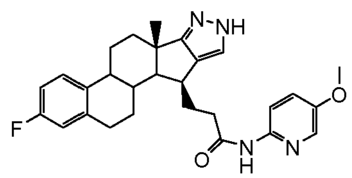

The compound **84** was prepared from the compound **83** by the Method C in 71% yield.
$^1$H NMR (400 MHz, DMSO-$d_6$): 1.10 (s, 3H), 1.40-1.74 (m, 5H), 2.02-2.48 (m, 8H), 2.78-2.98 (m, 3H), 3.80 (s, 3H), 6.90-7.09 (m, 2H), 7.30 (m, 1H), 7.41 (dd, 2H), 8.02 (d, 1H), 8.05 (d, 1H), 10.41 (s, 1H), 12.13 (br s, 1H).

**Compound 85**

3-((13*S*,15*R*)-3-fluoro-13-methyl-17-oxo-7,8,9,11,12,13,14,15,16,17-decahydro-6H-cyclopenta[a]phenanthren-15-yl)-*N*-(5-isopropylpyridin-2-yl)propanamide

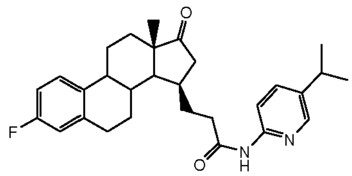

The compound **85** was synthesized in 60% yield by the Method A1 using Acid **SM-XV** and 2-amino-5-isopropylpyridine as starting materials in overnight reaction time.

$^1$H NMR (200 MHz, DMSO-$d_6$): 0.98 (s, 3 H), 1.20 (d, 6H), 1.28 - 2.49 (m, 16 H), 2.74 - 3.02 (m, 3 H), 6.79 - 7.03 (m, 2 H), 7.19 - 7.39 (m, 1 H), 7.66 (d, 1H), 8.02 (d, 1H), 8.19 (s, 1H), 10.43 (s, 1H).

**Compound 86**

3-((13S,15*S,Z*)-3-fluoro-16-(hydroxymethylene)-13-methyl-17-oxo-7,8,9,11,12,13,14,15,16,17-decahydro-6*H*-cyclopenta[*a*]phenanthren-15-yl)-*N*-(5-isopropylpyridin-2-yl)-propanamide

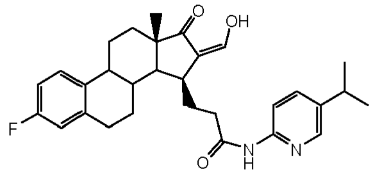

The compound **86** was prepared from the compound **85** by the Method B in 98% yield.

$^1$H NMR (400 MHz, DMSO-$d_6$): 0.99 (s, 3H), 1.19 &1.21 (2 x s, 6H), 1.38-2.35 (m, 12H), 2.55 (m, 2H), 2.84-2.97 (m, 4H), 6.89-6.95 (m, 2H), 7.28 (m, 1H), 7.55 (s, 1H), 7.65 (dd, 1H), 8.00 (d, 1H), 8.17 (d, 1H), 10.37 (s, 1H).

**Compound 87**

3-((8aS,12S)-4-fluoro-8a-methyl-1,2,6b,7,8,8a,10,12,12a,12b-decahydronaphtho[2',1':4,5]indeno[1,2-c]pyrazol-12-yl)-N-(5-isopropylpyridin-2-yl)propanamide

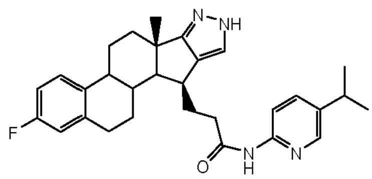

The compound **87** was prepared from the compound **86** by the Method C in 71% yield.

$^1$H NMR (400 MHz, DMSO-$d_6$): 1.18 (s, 3H), 1.21 & 1.23 (2 x s, 6H), 1.46-2.31 (m, 11H), 2.58-2.63 (m, 3H), 2.91-3.02 (m, 4H), 6.91-6.96 (m, 2H), 7.31 (m, 1H), 7.91 (dd, 2H), 8.05 (d, 1H), 8.22 (s, 1H), 11.67 (s, 1H).

**Compound 88**

3-((13*S*,15*R*)-3-fluoro-13-methyl-17-oxo-7,8,9,11,12,13,14,15,16,17-decahydro-6H-cyclopenta[*a*]phenanthren-15-yl)-*N*-(4-methoxypyridin-2-yl)propanamide

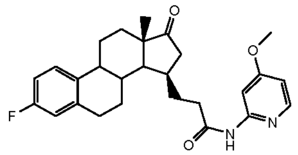

The compound **88** was prepared in 84% yield by the Method A2 from Acid **SM-XV** and 4-methoxy-2-aminopyridine stirring first at +50 °C for five hours and then overnight at room temperature.

$^1$H-NMR (400 MHz, DMSO-$d_6$): 0.98 (s, 3H), 1.37-2.45 (m, 16H), 2.94 (m, 2H), 3.80 (s, 3H), 6.69 (dd, 1H), 6.92 (m, 2H), 7.29 (dd, 1H), 7.73 (d, 1H), 8.11 (d, 1H), 10.46 (s, 1H).

**Compound 89**

3-((13*S*,15*S*,*Z*)-3-fluoro-16-(hydroxymethylene)-13-methyl-17-oxo-7,8,9,11,12,13,14,15,16,17-decahydro-6*H*-cyclopenta[a]phenanthren-15-yl)-*N*-(4-methoxypyridin-2-yl)-propanamide

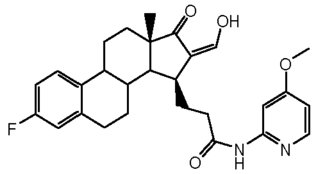

The compound **89** was prepared from the compound **88** by the Method B in quantitative yield.

$^1$H NMR (400 MHz, DMSO-$d_6$): 0.99 (s, 3H), 1.45-2.32 (m, 12H), 2.55 (m, 1H), 2.83-2.97 (m, 3H), 3.80 (s, 3H), 6.68 (dd, 1H), 6.92 (m, 2H), 7.28 (dd, 1H), 7.53 (s, 1H), 7.72 (s, 1H), 8.10 (d, 1H), 10.38 (s, 1H), 10.98 (br s, 1H).

**Compound 90**

3-((8a*S*,12*S*)-4-fluoro-8a-methyl-1,2,6b,7,8,8a,10,12,12a,12b-decahydronaphtho[2',1':4,5]indeno[1,2-c]pyrazol-12-yl)-N-(4-methoxypyridin-2-yl)propanamide

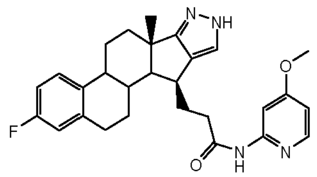

The compound **90** was prepared from the compound **89** by the Method C in 71% yield.

$^1$H NMR (400 MHz, DMSO-$d_6$): 1.10 (s, 3H), 1.39-2.46 (m, 13H), 2.81 (m, 1H), 2.92 (m, 2H), 3.80 (s, 3H), 6.70 (dd, 1H), 6.93 (m, 2H), 7.30 (dd, 1H), 7.39 (s, 1H), 7.74 (d, 1H), 8.12 (d, 1H), 10.50 (s, 1H), 12.13 (br s, 1H). MS m/z 475 (M +1)

**Compound 91**

3-((13*S*,15*R*)-3-fluoro-13-methyl-17-oxo-7,8,9,11,12,13,14,15,16,17-decahydro-6H-cyclopenta[a]phenanthren-15-yl)-*N*-(4-methylpyridin-2-yl)propanamide

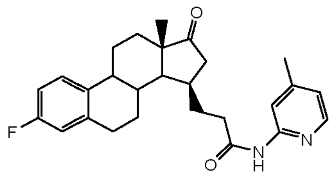

The compound **91** was prepared in 41% yield by the Method A2 from Acid **SM-XV** and 4-methyl-2-aminopyridine stirring at +50 °C for seven hours and then at room temperature for overnight.

$^1$H-NMR (400 MHz, DMSO-$d_6$): 0.98 (s, 3H), 1.33-2.48 (m, 18H), 2.89 (m, 2H), 6.92 (m, 3H), 7.29 (dd, 2H), 7.95 (s, 1H), 8.15 (d, 1H), 10.41 (s, 1H).

**Compound 92**

3-((13*S*,15*S*,*Z*)-3-fluoro-16-(hydroxymethylene)-13-methyl-17-oxo-7,8,9,11,12,13,14,15,16,17-decahydro-6*H*-cyclopenta[a]phenanthren-15-yl)-*N-(*4-methylpyridin-2-yl)-propanamide

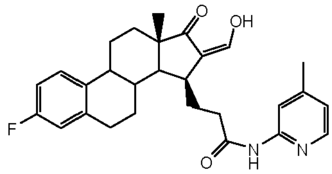

The compound **92** was prepared from the compound **91** by the Method B in quantitative yield.

$^1$H NMR (400 MHz, DMSO-$d_6$): 0.99 (s, 3H), 1.35-2.45 (m, 12H), 2.55 (m, 1H), 2.88 (m, 3H), 3.80 (s, 3H), 6.92 (m, 3H), 7.28 (m, 1H), 7.54 (s, 1H), 7.94 (s, 1H), 8.14 (s, 1H), 10.33 (s, 1H), 11.00 (br s, 1H).

**Compound 93**

3-((8a*S*,12*S*)-4-fluoro-8a-methyl-1,2,6b,7,8,8a,10,12,12a,12b-decahydronaph-tho[2',1':4,5]indeno[1,2-c]pyrazol-12-yl)-*N*-(4-methylpyridin-2-yl)-propanamide

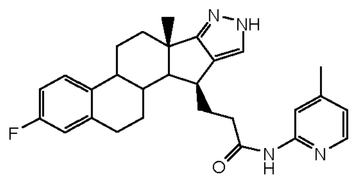

The compound **93** was prepared from the compound **92** by the Method C in 55% yield.

$^1$H NMR (400 MHz, DMSO-$d_6$): 1.10 (s, 3H), 1.41-2.38 (m, 16H), 2.81 (m, 1H), 2.92 (m, 2H), 6.93 (m, 3H), 7.30 (m, 1H), 7.40 (s, 1H), 7.96 (s, 1H), 8.16 (d, 1H), 10.45 (s, 1H), 12.12 (br s, 1H). MS m/z 459 (M +1)

**Compound 94**

3-((13S,15R)-3-fluoro-13-methyl-17-oxo-7,8,9,11,12,13,14,15,16,17-decahydro-6H-cyclopenta[a]phenanthren-15-yl)-N-(5-morpholinopyridin-2-yl)propanamide

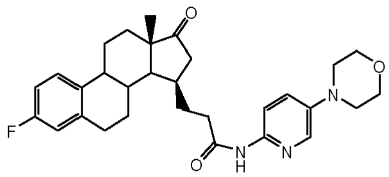

The compound **94** was synthesized in 63% yield after chromatographic purification by the Method A1 in DCM by using acid **SM-XV** and 5-morpholinopyridin-2-amine as starting materials and triethylamine as base in 2 hours reaction time.

$^1$H-NMR (400 MHz, DMSO-$d_6$): 0.98 (s, 3H), 1.30-2.47 (m, 16H), 2.81-2.96 (m, 2H), 3.06-3.12 (m, 4H), 3.70-3.78 (m, 4H), 6.90-6.95 (m, 2H), 7.25-7.32 (t, 1H), 7.40 (dd, 1H), 7.95-8.01 (m, 2H), 10.28 (s, 1H).

**Compound 95**

3-((13S,15S,Z)-3-fluoro-16-(hydroxymethylene)-13-methyl-17-oxo-7,8,9,11,12,13,14,15,16,17-decahydro-6H-cyclopenta[a]phenanthren-15-yl)-N-(5-morpholinopyridin-2-yl)propanamide

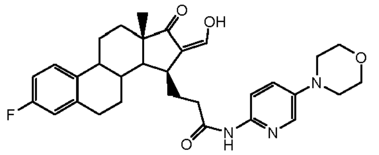

The compound **95** was synthesized in 94% yield from the compound **94** by the Method B in overnight reaction time.

$^1$H-NMR (400 MHz, DMSO-$d_6$): 0.99 (s, 3H), 1.30-2.60 (m, 13H), 2.81-3.00 (m, 3H), 3.06-3.12 (m, 4H), 3.70-3.78 (m, 4H), 6.88-6.95 (m, 2H), 7.25-7.32 (t, 1H), 7.40 (dd, 1H), 7.54 (s, 1H), 7.92-8.01 (m, 2H), 10.24 (s, 1H), 11.02 (br s, 1H).

**Compound 96**

3-((8aS,12S)-4-fluoro-8a-methyl-1,2,6b,7,8,8a,10,12,12a,12b-decahydronaphtho[2',1':4,5]indeno[1,2-c]pyrazol-12-yl)-N-(5-morpholinopyridin-2-yl)propanamide

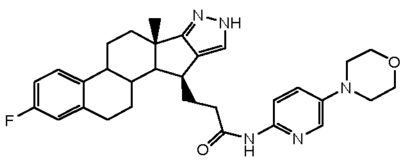

The compound **96** was synthesized in 73% yield from the compound **95** by the Method C in one hour reaction time.

$^1$H-NMR (400 MHz, DMSO-$d_6$): 1.10 (s, 3H), 1.30-2.50 (m, 13H), 2.75-2.95 (m, 3H), 3.06-3.12 (m, 4H), 3.70-3.78 (m, 4H), 6.88-6.96 (m, 2H), 7.26-7.33 (t, 1H), 7.38-7.43 (m, 2H), 7.94-8.01 (m, 2H), 10.32 (s, 1H), 12.11 (br s, 1H).

**Compound 97**

3-((13S,15R)-3-fluoro-13-methyl-17-oxo-7,8,9,11,12,13,14,15,16,17-decahydro-6H-cyclopenta[a]phenanthren-15-yl)-N-(5-fluoropyridin-2-yl)propanamide

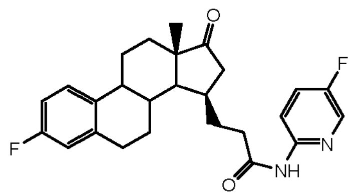

The compound **97** was synthesized in 93% yield by the Method A1 in DCM by using acid **SM-XV** and 2-amino-5-fluoropyridine as starting materials in 2 hours reaction time.
$^1$H-NMR (200 MHz, DMSO-$d_6$): 0.98 (s, 3H), 1.22-2.45 (m, 16H), 2.80-2.95 (m, 2H), 6.83-7.03 (m, 2H), 7.20-7.39 (m, 1H), 7.73 (td, 1H), 8.14 (dd, 1H), 8.31 (d, 1H), 10.62 (s, 1H).

**Compound 98**

3-((13S,15S,Z)-3-fluoro-16-(hydroxymethylene)-13-methyl-17-oxo-7,8,9,11,12,13,14,15,16,17-decahydro-6H-cyclopenta[a]phenanthren-15-yl)-N-(5-fluoropyridin-2-yl)propanamide

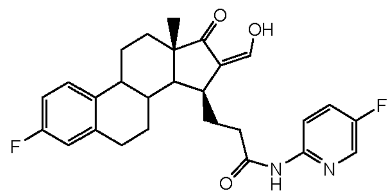

The compound **98** was synthesized in 81% yield from the compound **97** by the Method B in overnight reaction time by using 500 mol-% of ethyl formate and 300 mol-% of NaH.
$^1$H-NMR (400 MHz, DMSO-$d_6$): 0.99 (s, 3H), 1.30-2.60 (m, 13H), 2.80-2.99 (m, 3H), 6.85-7.03 (m, 2H), 7.20-7.39 (m, 1H), 7.58 (s, 1H), 7.71 (td, 1H), 8.13 (dd, 1H), 8.30 (d, 1H), 10.62 (s, 1H).

**Compound 99**

3-((8aS,12S)-4-fluoro-8a-methyl-1,2,6b,7,8,8a,10,12,12a,12b-decahydronaphtho[2',1':4,5]indeno[1,2-c]pyrazol-12-yl)-N-(5-fluoropyridin-2-yl)propanamide

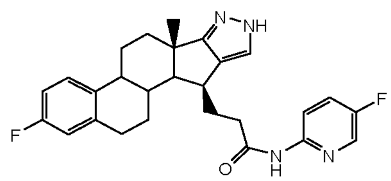

The compound **99** was synthesized in 45% yield from the compound **98** by the Method C in one hour reaction time.
$^1$H-NMR (400 MHz, DMSO-$d_6$): 1.10 (s, 3H), 1.35-2.49 (m, 13H), 2.79-2.99 (m, 3H), 6.85-7.00 (m, 2H), 7.25-7.35 (m, 1H), 7.40 (s, 1H), 7.73 (td, 1H), 8.14 (dd, 1H), 8.31 (d, 1H), 10.66 (s, 1H), 12.12 (s, 1H).

**Compound 100**

*N*-(5-(*tert*-butyl)isoxazol-3-yl)-3-((13S,15R)-3-fluoro-13-methyl-17-oxo-7,8,9,11,12,13,14,15,16,17-decahydro-6H-cyclopenta[a]phenanthren-15-yl)propanamide

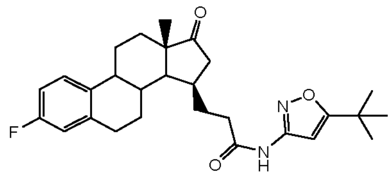

The compound **100** was prepared in 39% yield by the Method A1 from Acid **SM-XV** and 3-amino-5-*tert*-butylisoxazole stirring at room temperature for five hours. The product was used directly to the next step.

**Compound 101**

N-(5-(*tert*-butyl)isoxazol-3-yl)-3-((13S,15S,Z)-3-fluoro-16-(hydroxymethylene)-13-methyl-17-oxo-7,8,9,11,12,13,14,15,16,17-decahydro-6H-cyclopenta[a]phenanthren-15-yl)propanamide

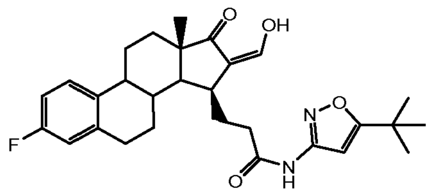

The compound **101** was prepared from the compound **100** by the Method B in quantitative yield.
$^1H$ NMR (400 MHz, $CDCl_3$): 1.16 (s, 3H), 1.26 (s, 9H), 1.32-2.84 (m, 17H), 6.68-6.85 (m, 3H), 7.19-7.23 (m, 1H), 7.31 (s, 1H), 9.47 (s, 1H)

**Compound 102**

N-(5-(*tert*-butyl)isoxazol-3-yl)-3-((8aS,12S)-4-fluoro-8a-methyl-1,2,6b,7,8,8a,10,12,12a,12b-decahydronaphtho[2',1':4,5]indeno[1,2-c]pyrazol-12-yl)propanamide

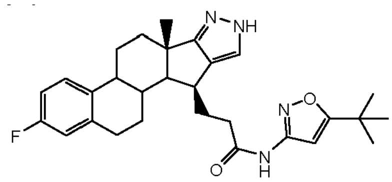

The compound **102** was prepared from the compound **101** by the Method C in 46% yield.
$^1H$ NMR (400 MHz, DMSO-$d_6$): 1.10 (s, 3H), 1.29 (s, 9H), 1.41-2.43 (m, 13H), 2.80-2.92 (m, 3H), 6.61 (m, 1H), 6.93 (m, 2H), 7.30 (m, 1H), 7.39 (s, 1H), 10.97 (s, 1H), 12.13 (s, 1H).

**Compound 103**

3-((13S,15R)-3-fluoro-13-methyl-17-oxo-7,8,9,11,12,13,14,15,16,17-decahydro-6H-cyclopenta[a]phenanthren-15-yl)-N-(6-fluoropyridin-2-yl)propanamide

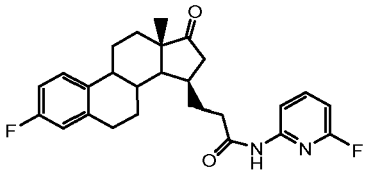

The compound **103** was synthesized in 82% yield by the Method A1 in DCM by using acid **SM-XV** and 2-amino-6-fluoropyridine as starting materials in 3 hours reaction time.

$^1$H-NMR (200 MHz, DMSO-$d_6$): 0.98 (s, 3H), 1.33-2.47 (m, 16H), 2.89 (m, 2H), 6.84 (dd, 1H), 6.92 (m, 2H), 7.29 (m, 1H), 7.94 (dd, 1H), 8.01 (m, 1H), 10.67 (s, 1H).

**Compound 104**

3-((13S,15S,Z)-3-fluoro-16-(hydroxymethylene)-13-methyl-17-oxo-7,8,9,11,12,13,14,15,16,17-decahydro-6H-cyclopenta[a]phenanthren-15-yl)-N-(6-fluoropyridin-2-yl)propanamide

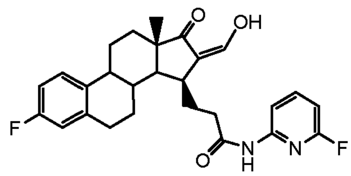

The compound **104** was synthesized in 92% yield from the compound **103** by the Method B in two hours reaction time.

$^1$H-NMR (400 MHz, DMSO-$d_6$): 0.99 (s, 3H), 1.36-2.33 (m, 12H), 2.57 (m, 2H), 2.89 (m, 3H), 6.81 (dd, 1H), 6.92 (m, 2H), 7.28 (m, 1H), 7.57 (s, 1H), 7.93 (dd, 1H), 8.00 (m, 1H), 10.66 (s, 1H).

**Compound 105**

3-((8aS,12S)-4-fluoro-8a-methyl-1,2,6b,7,8,8a,10,12,12a,12b-decahydronaphtho[2',1':4,5]indeno[1,2-c]pyrazol-12-yl)-N-(6-fluoropyridin-2-yl)propanamide

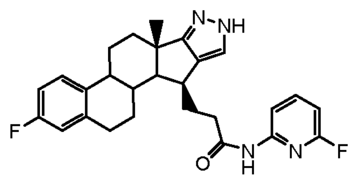

The compound **105** was synthesized in 50% yield from the compound **104** by the Method C in 30 minutes reaction time.

$^1$H-NMR (400 MHz, DMSO-$d_6$): 1.10 (s, 3H), 1.42-2.38 (m, 14H), 2.78-2.95 (m, 3H), 6.83 (m, 1H), 6.93 (m, 2H), 7.30 (m, 1H), 7.39 (s, 1H), 7.94 (m, 1H), 8.00 (m, 1H), 10.72 (s, 1H).

**Compound 106**

3-((13S,15R)-3-fluoro-13-methyl-17-oxo-7,8,9,11,12,13,14,15,16,17-decahydro-6H-cyclopenta[a]phenanthren-15-yl)-N-(6-methoxypyridazin-3-yl)propanamide

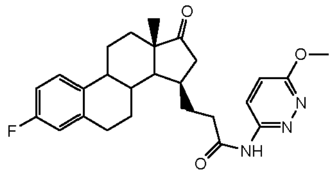

The compound **106** was synthesized in 79% yield by the Method A2 in THF by using acid **SM-XV** and 3-amino-6-methoxypyridazine as starting materials in 3 hours reaction time.

$^1$H-NMR (400 MHz, DMSO-$d_6$): 0.98 (s, 3H), 1.30-2.47 (m, 16H), 2.79-3.02 (m, 2H), 3.98 (s, 3H), 6.85-7.03 (m, 2H), 7.24 (d, 1H), 7.26-7.35 (m, 1H), 8.25 (d, 1H), 10.93 (br s, 1H).

**Compound 107**

3-((13S,15S,Z)-3-fluoro-16-(hydroxymethylene)-13-methyl-17-oxo-7,8,9,11,12,13,14,15,16,17-decahydro-6H-cyclopenta[a]phenanthren-15-yl)-N-(6-methoxypyridazin-3-yl) propanamide

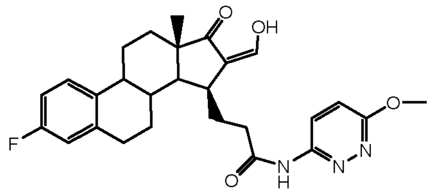

The compound **107** was synthesized in quantitative yield from the compound **106** by the Method B in overnight reaction time.

$^1$H-NMR (400 MHz, DMSO-$d_6$): 0.99 (s, 3H), 1.30-2.47 (m, 13H), 2.55-3.00 (m, 3H), 3.98 (s, 3H), 6.85-7.03 (m, 2H), 7.23 (d, 1H), 7.25-7.33 (m, 1H), 7.55 (s, 1H), 8.23 (d, 1H), 10.88 (s, 1H), 10.96 (br s, 1H).

**Compound 108**

3-((8aS,12S)-4-fluoro-8a-methyl-1,2,6b,7,8,8a,10,12,12a,12b-decahydronaphtho[2',1':4,5]indeno[1,2-c]pyrazol-12-yl)-N-(6-methoxypyridazin-3-yl)propanamide

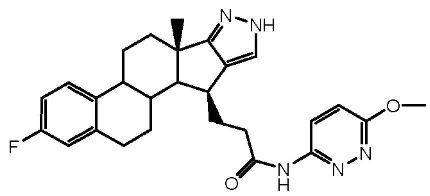

The compound **108** was synthesized in 92% yield from the compound **107** by the Method C in one hour reaction time.

$^1$H-NMR (400 MHz, DMSO-$d_6$): 1.11 (s, 3H), 1.30-2.47 (m, 13H), 2.75-3.00 (m, 3H), 3.98 (s, 3H), 6.85-7.03 (m, 2H), 7.23 (d, 1H), 7.25-7.33 (m, 1H), 7.41 (s, 1H), 8.25 (d, 1H), 10.96 (s, 1H), 12.12 (br s, 1H).

**Compound 109**

3-((13S,15R)-3-fluoro-13-methyl-17-oxo-7,8,9,11,12,13,14,15,16,17-decahydro-6H-cyclopenta[a]phenanthren-15-yl)-N-(6-methylpyridazin-3-yl)propanamide

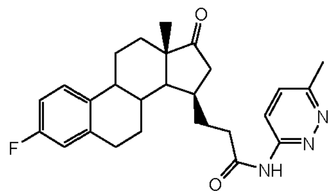

The compound **109** was synthesized in 79% yield by the Method A2 in THF by using acid **SM-XV** and 3-amino-6-methylpyridazine as starting materials in 3 hours reaction time.

$^1$H-NMR (400 MHz, DMSO-$d_6$): 0.98 (s, 3H), 1.30-2.46 (m, 16H), 2.55 (s, 3H), 2.80-3.00 (m, 2H), 6.85-7.02 (m, 2H), 7.23-7.35 (m, 1H), 7.54 (d, 1H), 8.22 (d, 1H), 11.03 (br s, 1H).

**Compound 110**

3-((13S,15S,Z)-3-fluoro-16-(hydroxymethylene)-13-methyl-17-oxo-7,8,9,11,12,13,14,15,16,17-decahydro-6H-cyclopenta[a]phenanthren-15-yl)-N-(6-methylpyridazin-3-yl)propanamide

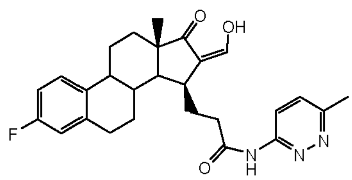

The compound **110** was synthesized in quantitative yield from the compound **109** by the Method B in overnight reaction time.

$^1$H-NMR (400 MHz, DMSO-$d_6$): 0.99 (s, 3H), 1.30-2.70 (m, 13H), 2.55 (s, 3H), 2.80-3.00 (m, 3H), 6.85-7.02 (m, 2H), 7.21-7.33 (m, 1H), 7.52 (d, 1H), 7.53 (s, 1H), 8.21 (d, 1H), 10.96 (br s, 2 x 1H).

**Compound 111**

3-((8aS,12S)-4-fluoro-8a-methyl-1,2,6b,7,8,8a,10,12,12a,12b-decahydronaphtho[2',1':4,5]indeno[1,2-c]pyrazol-12-yl)-N-(6-methylpyridazin-3-yl)propanamide

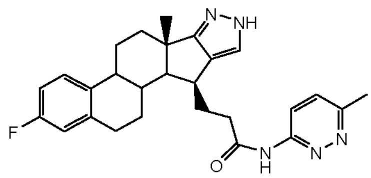

The compound **111** was synthesized in 24% yield after chromatographic purification from the compound **110** by the Method C in one hour reaction time.

$^1$H-NMR (400 MHz, DMSO-$d_6$): 1.11 (s, 3H), 1.30-2.59 (m, 13H), 2.55 (s, 3H), 2.77-3.00 (m, 3H), 6.85-7.02 (m, 2H), 7.23-7.33 (m, 1H), 7.41 (s, 1H), 7.53 (d, 1H), 8.22 (d, 1H), 11.07 (s, 1H), 12.13 (br s, 1H).

**Compound 112**

3-((13S,15R)-3-fluoro-13-methyl-17-oxo-7,8,9,11,12,13,14,15,16,17-decahydro-6H-cyclopenta[a]phenanthren-15-yl)-N-(pyridazin-3-yl)propanamide

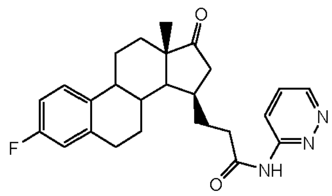

The compound **112** was synthesized in 79% yield by the Method A2 in THF by using acid **SM-XV** and 3-aminopyridazine as starting materials in 3.5 hours reaction time.

$^1$H-NMR (400 MHz, DMSO-$d_6$): 0.98 (s, 3H), 1.30-2.62 (m, 16H), 2.80-2.95 (m, 2H), 6.85-7.03 (m, 2H), 7.20-7.31 (m, 1H), 7.67 (dd, 1H), 8.32 (d, 1H), 8.95 (d, 1H), 11.13 (s, 1H).

**Compound 113**

3-((13S,15S,Z)-3-fluoro-16-(hydroxymethylene)-13-methyl-17-oxo-7,8,9,11,12,13,14,15,16,17-decahydro-6H-cyclopenta[a]phenanthren-15-yl)-N-(pyridazin-3-yl)propanamide

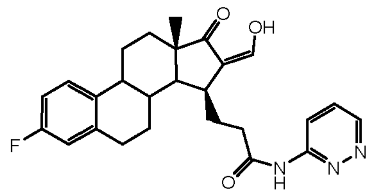

The compound **113** was synthesized in quantitative yield from the compound **112** by the Method B in overnight reaction time.

$^1$H-NMR (400 MHz, DMSO-$d_6$): 1.00 (s, 3H), 1.30-2.70 (m, 13H), 2.80-2.99 (m, 3H), 6.85-7.03 (m, 2H), 7.20-7.33 (m, 1H), 7.55 (s, 1H), 7.65 (dd, 1H), 8.31 (d, 1H), 8.93 (d, 1H), 11.06 (s, 1H), 11.07 (br s, 1H).

**Compound 114**

3-((8aS,12S)-4-fluoro-8a-methyl-1,2,6b,7,8,8a,10,12,12a,12b-decahydronaphtho[2',1':4,5]indeno[1,2-c]pyrazol-12-yl)-N-(pyridazin-3-yl)propanamide

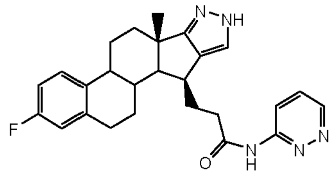

The compound **114** was synthesized in 45% yield after chromatographic purification from the compound **113** by the Method C in one hour reaction time. $^1$H-NMR (400 MHz, DMSO-$d_6$): 1.11 (s, 3H), 1.30-2.65 (m, 13H), 2.80-2.99 (m, 3H), 6.85-7.02 (m, 2H), 7.25-7.35 (m, 1H), 7.42 (s, 1H), 7.66 (dd, 1H), 8.33 (d, 1H), 8.95 (d, 1H), 11.17 (s, 1H), 12.14 (br s, 1H).

**Compound 115**

(13S,15R)-3-fluoro-13-methyl-15-(3-oxo-3-(pyrrolidin-1-yl)propyl)-6,7,8,9,11,12,13,14,15,16-decahydro-17H-cyclopenta[a]phenanthren-17-one

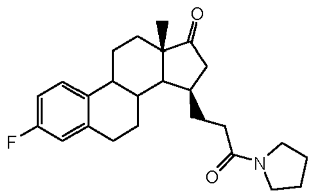

The compound **115** was synthesized in 46% yield after chromatographic purification by the Method A1 in DCM by using acid **SM-XV** and pyrrolidine as starting materials in 4 hours reaction time.
$^1$H-NMR (200 MHz, $CDCl_3$): 1.07 (s, 3H), 1.33-2.50 (m, 20H), 2.79-3.09 (m, 2H), 3.35-3.55 (m, 4H), 6.70-6.90 (m, 2H), 7.17-7.26 (m, 1H).

**Compound 116**

(13S,15S,Z)-3-fluoro-16-(hydroxymethylene)-13-methyl-15-(3-oxo-3-(pyrrolidin-1-yl)propyl)-6,7,8,9,11,12,13,14,15,16-decahydro-17H-cyclopenta[a]phenanthren-17-one

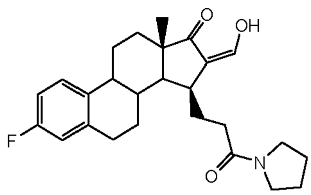

The compound **116** was synthesized in 98% yield from the compound **115** by the Method B in 2 hours reaction time.
$^1$H-NMR (400 MHz, DMSO-$d_6$): 0.97 (s, 3H), 1.30-2.46 (m, 17H), 2.80-3.00 (m, 3H), 3.25-3.45 (m, 4H), 6.86-6.98 (m, 2H), 7.25-7.35 (m, 1H), 7.52 (s, 1H).

**Compound 117**

3-((8aS,12S)-4-fluoro-8a-methyl-1,2,6b,7,8,8a,10,12,12a,12b-decahydronaphtho[2',1':4,5]indeno[1,2-c]pyrazol-12-yl)-1-(pyrrolidin-1-yl)propan-1-one

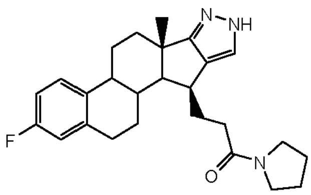

The compound **117** was synthesized in 88% yield from the compound **116** by the Method C in 0.5 hours reaction time.
$^1$H-NMR (400 MHz, DMSO-$d_6$): 1.09 (s, 3H), 1.30-2.44 (m, 17H), 2.80-3.00 (m, 3H), 3.21-3.49 (m, 4H), 6.87-6.98 (m, 2H), 7.27-7.35 (m, 1H), 7.40 (s, 1H), 12.10 (br s, 1H).

**Compound 118**

3-((13S,15R)-3-fluoro-13-methyl-17-oxo-7,8,9,11,12,13,14,15,16,17-decahydro-6H-cyclopenta[a]phenanthren-15-yl)-N-(pyrazin-2-yl)propanamide

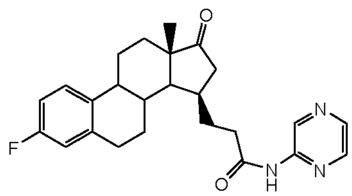

The compound **118** was synthesized in 44% yield by the Method A1 in DCM by using acid **SM-XV** and aminopyrazine as starting materials in 3 hours reaction time. $^1$H-NMR (400 MHz, $CDCl_3$): 1.08 (s, 3H), 1.30-2.65 (m, 16H), 2.85-3.08 (m, 2H), 6.74-6.95 (m, 2H), 7.20-7.26 (m, 1H), 7.80 (s, 1H), 8.25 (d, 1H), 8.37 (d, 1H), 9.53 (s, 1H).

**Compound 119**

3-((13S,15S,Z)-3-fluoro-16-(hydroxymethylene)-13-methyl-17-oxo-7,8,9,11,12,13,14,15,16,17-decahydro-6H-cyclopenta[a]phenanthren-15-yl)-N-(pyrazin-2-yl)propanamide

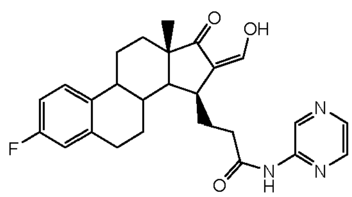

The compound **119** was synthesized in 99% yield from the compound **118** by the Method B in overnight reaction time.

$^1$H-NMR (400 MHz, DMSO-$d_6$): 1.00 (s, 3H), 1.30-2.68 (m, 13H), 2.80-3.08 (m, 3H), 6.80-6.96 (m, 2H), 7.25-7.35 (m, 1H), 7.60 (s, 1H), 8.32 (d, 1H), 8.37 (d, 1H), 9.33 (s, 1H), 10.86 (br s, 1H), 10.96 (br s, 1H).

**Compound 120**

3-((8aS,12S)-4-fluoro-8a-methyl-1,2,6b,7,8,8a,10,12,12a,12b-decahydronaphtho[2',1':4,5]indeno[1,2-c]pyrazol-12-yl)-N-(pyrazin-2-yl)propanamide

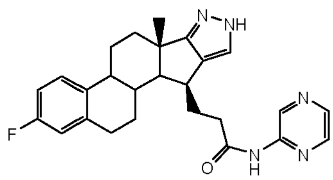

The compound **120** was synthesized in quantitative yield from the compound **119** by the Method C in one hour reaction time.

$^1$H-NMR (400 MHz, DMSO-$d_6$): 1.11 (s, 3H), 1.30-2.60 (m, 13H), 2.79-3.01 (m, 3H), 6.85-6.99 (m, 2H), 7.25-7.35 (m, 1H), 7.39 (s, 1H), 8.34 (d, 1H), 8.39 (d, 1H), 9.35 (s, 1H), 10.84 (s, 1H), 12.13 (br s, 1H).

**Compound 121**

3-((13S,15R)-3-chloro-13-methyl-17-oxo-7,8,9,11,12,13,14,15,16,17-decahydro-6H-cyclopenta[a]phenanthren-15-yl)-N-(2-oxo-1,2,5,6,7,8-hexahydroquinolin-3-yl)propanamide

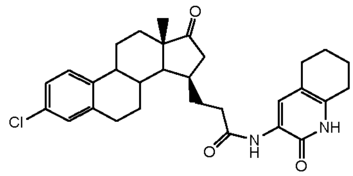

The compound **121** was synthesized in 79% yield by the Method A1 in THF by using acid **SM-XVII** and 3-amino-1,2,5,6,7,8-hexahydroquinolin-2-one as starting materials in overnight reaction time.

$^1$H-NMR (400 MHz, DMSO-$d_6$): 0.97 (s, 3H), 1.30-2.47 (m, 24H), 2.80-2.95 (m, 2H), 7.14-7.17 (m, 2H), 7.28-7.31 (m, 1H), 8.01 (s, 1H), 9.14 (s, 1H), 11.68 (s, 1H).

**Compound 122**

3-((13S,15S,Z)-3-chloro-16-(hydroxymethylene)-13-methyl-17-oxo-7,8,9,11,12,13,14,15,16,17-decahydro-6H-cyclopenta[a]phenanthren-15-yl)-N-(2-oxo-1,2,5,6,7,8-hexahydroquinolin-3-yl)propanamide

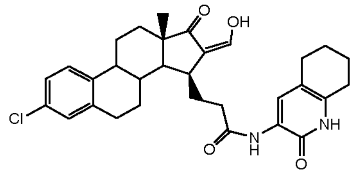

The compound **122** was synthesized in 76% yield from the compound **121** by the Method B in overnight reaction time.

$^1$H-NMR (400 MHz, DMSO-$d_6$): 0.98 (s, 3H), 1.30-2.60 (m, 22H), 2.80-2.95 (m, 2H), 7.14-7.17 (m, 2H), 7.28-7.31 (m, 1H), 7.54 (s, 1H), 7.99 (s, 1H), 8.99 (s, 1H), 11.09 (br s, 1H), 11.66 (s, 1H).

**Compound 123**

3-((8aS,12S)-4-chloro-8a-methyl-1,2,6b,7,8,8a,10,12,12a,12b-decahydronaphtho[2',1':4,5]indeno[1,2-c]pyrazol-12-yl)-N-(2-oxo-1,2,5,6,7,8-hexahydroquinolin-3-yl)propanamide

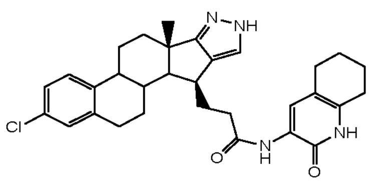

The compound **123** was synthesized in 46% yield from the compound **122** by the Method C in one hour reaction time.

$^1$H-NMR (400 MHz, DMSO-$d_6$): 1.09 (s, 3H), 1.30-2.59 (m, 21H), 2.75-2.96 (m, 3H), 7.14-7.19 (m, 2H), 7.28-7.33 (m, 1H), 7.41 (s, 1H), 8.00 (s, 1H), 9.14 (s, 1H), 11.68 (s, 1H), 12.11 (br s, 1H).

**Compound 124**

6-(3-((13S,15R)-3-chloro-13-methyl-17-oxo-7,8,9,11,12,13,14,15,16,17-decahydro-6H-cyclopenta[a]phenanthren-15-yl)propanamido)-N,N-dimethylnicotinamide

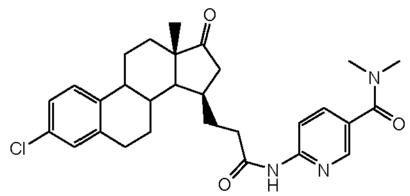

The compound **124** was synthesized in 85% yield by the Method A1 in THF by using acid **SM-XVII** and 6-amino-N,N-dimethylpyridine-3-carboxamide as starting materials in overnight reaction time.

$^1$H-NMR (400 MHz, DMSO-$d_6$): 0.98 (s, 3H), 1.30-2.47 (m, 16H), 2.80-2.95 (m, 2H), 2.97 (s, 6H), 7.14-7.17 (m, 2H), 7.28-7.31 (m, 1H), 7.85 (dd, 1H), 8.14 (d, 1H), 8.38 (d, 1H), 10.71 (s, 1H).

**Compound 125**

6-(3-((13S,15S,Z)-3-chloro-16-(hydroxymethylene)-13-methyl-17-oxo-7,8,9,11,12,13,14,15,16,17-decahydro-6H-cyclopenta[a]phenanthren-15-yl)propanamido)-N,N-dimethylnicotinamide

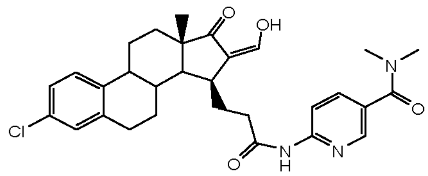

The compound **125** was synthesized in quantitative yield from the compound **124** by the Method B in overnight reaction time.

$^1$H-NMR (400 MHz, DMSO-$d_6$): 0.99 (s, 3H), 1.30-2.40 (m, 12H), 2.50-2.65 (m, 2H), 2.80-2.95 (m, 2H), 2.97 (s, 6H), 7.13-7.17 (m, 2H), 7.26-7.30 (m, 1H), 7.54 (s, 1H), 7.84 (dd, 1H), 8.13 (d, 1H), 8.37 (d, 1H), 10.64 (s, 1H), 10.98 (br s, 1H).

**Compound 126**

6-(3-((8aS,12S)-4-chloro-8a-methyl-1,2,6b,7,8,8a,10,12,12a,12b-decahydronaphtho[2',1':4,5]indeno[1,2-c]pyrazol-12-yl)propanamido)-N,N-dimethylnicotinamide

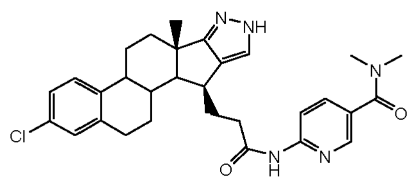

The compound **126** was synthesized in 63% yield from the compound **125** by the Method C in one hour reaction time.

$^1$H-NMR (400 MHz, DMSO-$d_6$): 1.10 (s, 3H), 1.30-2.60 (m, 13H), 2.70-2.97 (m, 3H), 2.98 (s, 6H), 7.14-7.19 (m, 2H), 7.28-7.32 (m, 1H), 7.41 (s, 1H), 7.85 (dd, 1H), 8.15 (d, 1H), 8.39 (d, 1H), 10.76 (s, 1H), 12.12 (br s, 1H).

**Compound 127**

3-((13S,15R)-3-chloro-13-methyl-17-oxo-7,8,9,11,12,13,14,15,16,17-decahydro-6H-cyclopenta[a]phenanthren-15-yl)-N-(4-fluoropyridin-2-yl)propanamide

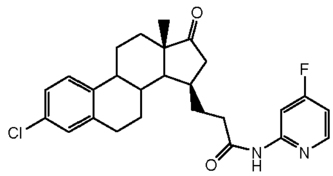

The compound **127** was synthesized in 90% yield by the Method A1 in THF by using acid **SM-XVII** and 2-amino-4-fluoropyridine as starting materials in 4 hours reaction time.

$^1$H-NMR (400 MHz, DMSO-$d_6$): 0.98 (s, 3H), 1.30-2.47 (m, 16H), 2.81-2.96 (m, 2H), 7.00-7.06 (m, 1H), 7.14-7.17 (m, 2H), 7.27-7.31 (m, 1H), 7.92 (dd, 1H), 8.30-8.37 (m, 1H), 10.82 (s, 1H).

**Compound 128**

3-((13S,15S,Z)-3-chloro-16-(hydroxymethylene)-13-methyl-17-oxo-7,8,9,11,12,13,14,15,16,17-decahydro-6H-cyclopenta[a]phenanthren-15-yl)-N-(4-fluoropyridin-2-yl)propanamide

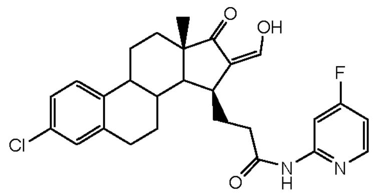

The compound **128** was synthesized in quantitative yield from the compound **127** by the Method B in 4 hours reaction time.

$^1$H-NMR (400 MHz, DMSO-$d_6$): 0.99 (s, 3H), 1.30-2.65 (m, 13H), 2.81-2.99 (m, 3H), 6.98-7.06 (m, 1H), 7.09-7.20 (m, 2H), 7.26-7.31 (m, 1H), 7.55 (s, 1H), 7.92 (dd, 1H), 8.30-8.37 (m, 1H), 10.76 (s, 1H), 10.96 (br s, 1H).

**Compound 129**

3-((8aS,12S)-4-chloro-8a-methyl-1,2,6b,7,8,8a,10,12,12a,12b-decahydronaphtho[2',1':4,5]indeno[1,2-c]pyrazol-12-yl)-N-(4-fluoropyridin-2-yl)propanamide

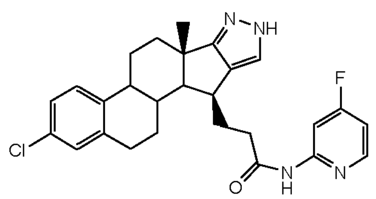

The compound **129** was synthesized in 72% yield from the compound **128** by the Method C in one hour reaction time.

$^1$H-NMR (400 MHz, DMSO-$d_6$): 1.10 (s, 3H), 1.30-2.53 (m, 13H), 2.76-2.99 (m, 3H), 6.99-7.06 (m, 1H), 7.10-7.20 (m, 2H), 7.27-7.32 (m, 1H), 7.40 (s, 1H), 7.93 (dd, 1H), 8.30-8.38 (m, 1H), 10.86 (s, 1H), 12.12 (br s, 1H).

**Compound 130**

3-((13S,15R)-3-chloro-13-methyl-17-oxo-7,8,9,11,12,13,14,15,16,17-decahydro-6H-cyclopenta[a]phenanthren-15-yl)-N-(3,5-difluoropyridin-2-yl)propanamide

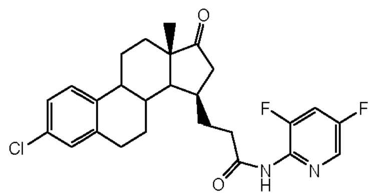

The compound **130** was synthesized in 90% yield by the Method A1 in THF by using acid **SM-XVII** and 2-amino-3,5-difluoropyridine as starting materials in 5 hours reaction time.

$^1$H-NMR (400 MHz, DMSO-$d_6$): 0.98 (s, 3H), 1.30-2.47 (m, 16H), 2.80-2.94 (m, 2H), 7.15-7.16 (m, 2H), 7.28-7.30 (m, 1H), 7.98-8.03 (m, 1H), 8.34-8.35 (m, 1H), 10.31 (s, 1H).

**Compound 131**

3-((13S,15S,Z)-3-chloro-16-(hydroxymethylene)-13-methyl-17-oxo-7,8,9,11,12,13,14,15,16,17-decahydro-6H-cyclopenta[a]phenanthren-15-yl)-N-(3,5-difluoropyridin-2-yl)propanamide

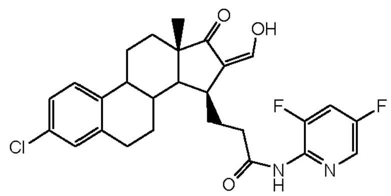

The compound **131** was synthesized in quantitative yield from the compound **130** by the Method B in 3 hours reaction time.

$^1$H-NMR (400 MHz, DMSO-$d_6$): 0.99 (s, 3H), 1.30-2.62 (m, 13H), 2.80-3.00 (m, 3H), 7.13-7.19 (m, 2H), 7.25-7.31 (m, 1H), 7.59 (s, 1H), 7.93-8.03 (m, 1H), 8.31-8.34 (m, 1H), 10.37 (s, 1H), 10.99 (br s, 1H).

**Compound 132**

3-((8aS,12S)-4-chloro-8a-methyl-1,2,6b,7,8,8a,10,12,12a,12b-decahydronaphtho[2',1':4,5]indeno[1,2-c]pyrazol-12-yl)-N-(3,5-difluoropyridin-2-yl)propanamide

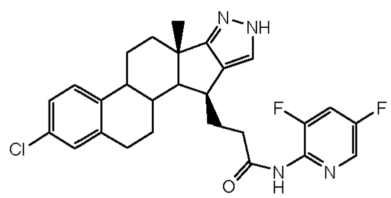

The compound **132** was synthesized in 67% yield from the compound **131** by the Method C in 1.5 hours reaction time.

$^1$H-NMR (400 MHz, DMSO-$d_6$): 1.10 (s, 3H), 1.30-2.49 (m, 13H), 2.80-3.00 (m, 3H), 7.13-7.19 (m, 2H), 7.27-7.31

(m, 1H), 7.43 (s, 1H), 7.96-8.04 (m, 1H), 8.34 (s, 1H), 10.34 (s, 1H), 12.13 (br s, 1H).

**Compound 133**

3-((13S,15R)-3-chloro-13-methyl-17-oxo-7,8,9,11,12,13,14,15,16,17-decahydro-6H-cyclopenta[a]phenanthren-15-yl)-N-(6-fluoropyridin-2-yl)propanamide

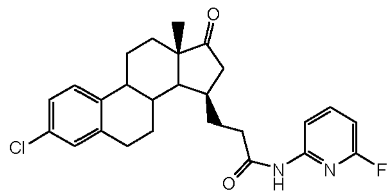

The compound **133** was synthesized in 71% yield by the Method A1 in THF by using acid **SM-XVII** and 2-amino-6-fluoropyridine as starting materials in overnight reaction time.

$^1$H-NMR (400 MHz, DMSO-$d_6$): 0.97 (s, 3H), 1.30-2.47 (m, 16H), 2.80-2.95 (m, 2H), 6.83 (dd, 1H), 7.14-7.17 (m, 2H), 7.28-7.31 (m, 1H), 7.91-7.97 (m, 1H), 8.00-8.03 (m, 1H), 10.68 (s, 1H).

**Compound 134**

3-((13S,15S,Z)-3-chloro-16-(hydroxymethylene)-13-methyl-17-oxo-7,8,9,11,12,13,14,15,16,17-decahydro-6H-cyclopenta[a]phenanthren-15-yl)-N-(6-fluoropyridin-2-yl)propanamide

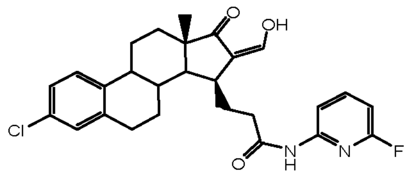

The compound **134** was synthesized in 69% yield from the compound **133** by the Method B in 6 hours reaction time.

$^1$H-NMR (400 MHz, DMSO-$d_6$): 0.99 (s, 3H), 1.30-2.60 (m, 13H), 2.80-2.99 (m, 3H), 6.81 (dd, 1H), 7.12-7.20 (m, 2H), 7.25-7.31 (m, 1H), 7.55 (s, 1H), 7.88-7.97 (m, 1H), 7.98-8.02 (m, 1H), 10.66 (br s, 1H), 10.95 (br s, 1H).

**Compound 135**

3-((8aS,12S)-4-chloro-8a-methyl-1,2,6b,7,8,8a,10,12,12a,12b-decahydronaphtho[2',1':4,5]indeno[1,2-c]pyrazol-12-yl)-N-(6-fluoropyridin-2-yl)propanamide

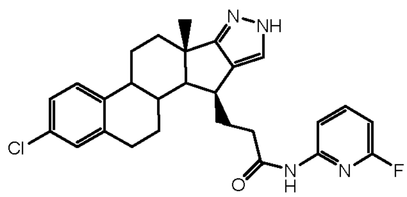

The compound **135** was synthesized in 49% yield from the compound **134** by the Method C in one hour reaction time.

$^1$H-NMR (400 MHz, DMSO-$d_6$): 1.10 (s, 3H), 1.30-2.55 (m, 13H), 2.75-2.99 (m, 3H), 6.83 (dd, 1H), 7.11-7.20 (m, 2H), 7.27-7.33 (m, 1H), 7.39 (s, 1H), 7.89-7.98 (m, 1H), 7.99-8.04 (m, 1H), 10.72 (s, 1H), 12.12 (br s, 1H).

**Compound 136**

*N*-(5-isopropylpyridin-2-yl)-3-((13S,15R)-13-methyl-17-oxo-7,8,9,11,12,13,14,15,16,17-decahydro-6*H*-cyclopenta[*a*] phenanthren-15-yl)propanamide

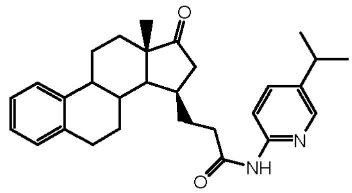

The compound **136** was prepared by the Method A1 from Acid **SM-XXVI** and 5-(1-Methyl-ethyl)-2-pyridinamine stirring overnight at room temperature. The yield after chromatographic purification was 75%.

$^1$H-NMR (200 MHz, $CDCl_3$): 1.07 (s, 3H), 1.24 & 1.28 (2 x s, 6H), 1.45-2.58 (m, 16H), 2.95 (m, 3H), 7.13 - 7.18 (m, 3H), 7.29 - 7.31 (m, 1H), 7.58 (dd, 1H), 8.07 (d, 1H), 8.14 (br s, 2H).

**Compound 137**

3-((13*S*,15*S*,*Z*)-16-(hydroxymethylene)-13-methyl-17-oxo-7,8,9,11,12,13,14,15,16,17-decahydro-6*H*-cyclopenta[*a*] phenanthren-15-yl)-*N*-(5-isopropylpyridin-2-yl)-propanamide

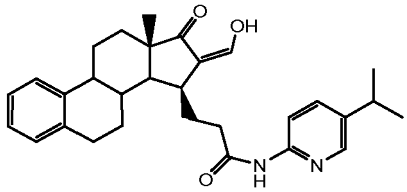

The compound **137** was prepared from the compound 136 by the Method B in 97% yield.

$^1$H NMR (400 MHz, DMSO-$d_6$): 1.00 (s, 3H), 1.19 & 1.21 (2 x s, 6H), 1.38-1.78 (m, 8H), 1.90-1-97 (m, 1H), 2.15 (m, 1H), 2.33 (m, 2H), 2.55 (m, 1H), 2.80-3.00 (m, 4H), 7.05-7.12 (m, 3H), 7.26 (m, 1H), 7.56 (s, 1H), 7.65 (dd, 1H), 8.00 (d, 1H), 8.17 (d, 1H), 10.39 (s, 1H), 11.0 (br s, 1H).

**Compound 138**

N-(5-isopropylpyridin-2-yl)-3-((8aS,12S)-8a-methyl-1,2,6b,7,8,8a,10,12,12a,12b-decahydronaphtho[2',1':4,5]indeno[1,2-c]pyrazol-12-yl)propanamide

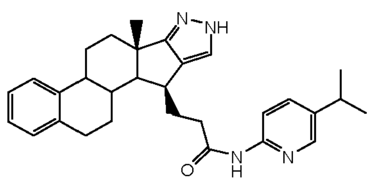

The compound **138** was prepared from the compound 137 by the Method C in 93% yield.

$^1$H NMR (400 MHz, DMSO-$d_6$): 1.11 (s, 3H), 1.20 & 1.21 (2 x s, 6H), 1.37-2.45 (m, 13H), 2.79-2.94 (m, 4H), 7.06-7.14 (m, 3H), 7.28 (m, 1H), 7.40 (s, 1H), 7.66 (dd, 1H), 8.02 (d, 1H), 8.19 (d, 1H), 10.46 (s, 1H), 12.11 (br s, 1H).

**Compound 139**

*N*-(5-methoxypyridin-2-yl)-3-((13*S*,15*R*)-13-methyl-17-oxo-7,8,9,11,12,13,14,15,16,17-decahydro-6*H*-cyclopenta[*a*] phenanthren-15-yl)propanamide

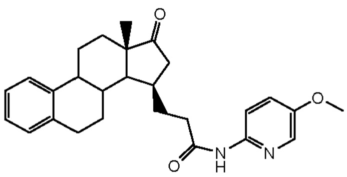

The compound **139** was synthesized in 99% yield from acid Acid **SM-XXVI** and 5-triethoxy-2-airiitiopyriditie by the Method A1 refluxing for two hours.
$^1$H-NMR (200 MHz, $CDCl_3$): 1.07 (s, 3H), 1.40-2.57 (m, 17H), 2.96 (m, 2H), 3.85 (s, 3H), 7.13 - 7.18 (m, 3H), 7.24 - 7.30 (m, 2H), 7.96 (d, 1H), 8.15 (d, 1H).

**Compound 140**

3-((13*S*,15*S*,*Z*)-16-(hydroxymethylene)-13-methyl-17-oxo-7,8,9,11,12,13,14,15,16,17-decahydro-6*H*-cyclopenta[a] phenanthren-15-yl)-*N*-(5-methoxypyridin-2-yl)-propanamide

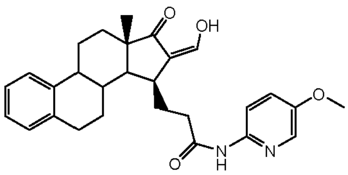

The compound **140** was prepared from the compound **139** by the Method B in quantitative yield.
$^1$H NMR (400 MHz, DMSO-$d_6$): 1.00 (s, 3H), 1.36-2.55 (m, 13H), 2.81-2.95 (m, 3H), 3.82 (s, 3H), 7.05-7.13 (m, 3H), 7.27 (m, 1H), 7.40 (dd, 1H), 7.55 (s, 1H), 8.01 (dd, 2H), 10.33 (s, 1H), 11.01 (br s, 1H).

**Compound 141**

*N*-(5-methoxypyridin-2-yl)-3-((8a*S*,12*S*)-8a-methyl-1,2,6b,7,8,8a,10,12,12a,12b-decahydronaphtho[2',1':4,5]indeno[1,2-c]pyrazol-12-yl)propanamide

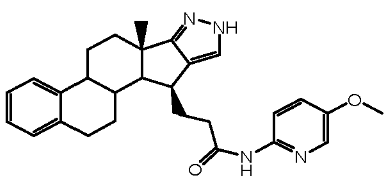

The compound **141** was prepared from the compound **140** by the Method C in 86% yield.
$^1$H NMR (400 MHz, DMSO-$d_6$): 1.11 (s, 3H), 1.41-1.81 (m, 5H), 2.02-2.67 (m, 8H), 2.79-2.98 (m, 3H), 3.80 (s, 3H), 7.07-7.14 (m, 3H), 7.28 (m, 1H), 7.40 (dd, 2H), 8.02 (d, 1H), 8.05 (d, 1H), 10.39 (s, 1H), 12.09 (br s, 1H).

**Compound 142**

4-(3-((13S,15R)-13-methyl-17-oxo-7,8,9,11,12,13,14,15,16,17-decahydro-6H-cyclopenta[a]phenanthren-15-yl)propanoyl)piperazin-2-one

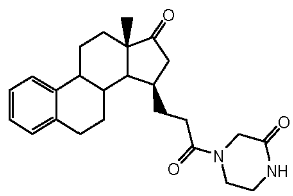

The compound **142** was synthesized in 86% yield from acid Acid **SM-XXVI** and piperazin-2-one by the Method A1 stirring at room temperature for two hours. $^{1}$H-NMR (400 MHz, DMSO-$d_6$): 0.97 (s, 3H), 1.37-2.45 (m, 14H), 2.87 (m, 2H), 3.16-3.24 (m, 3H), 3.63 (m, 3H), 3.93 (s, 1H), 4.04 (s, 1H), 7.10 (m, 3H), 7.27 (d, 1H), 8.08 (d, 1H).

**Compound 143**

4-(3-((13S,15S,Z)-16-(hydroxymethylene)-13-methyl-17-oxo-7,8,9,11,12,13,14,15,16,17-decahydro-6H-cyclopenta[a]phenanthren-15-yl)propanoyl)piperazin-2-one

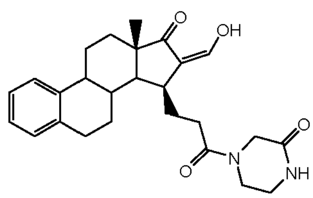

The compound **143** was prepared in 80% yield from the compound **142** by the Method B stirring overnight at room temperature.

$^{1}$H NMR (400 MHz, DMSO-$d_6$): 0.98 (s, 3H), 1.38-2.35 (m, 12H), 2.56 (m, 1H), 2.88 (m, 3H), 3.16 (m, 3H), 3.54-3.66 (m, 2H), 3.93 (d, 1H), 3.99 (s, 1H), 7.09 (m, 3H), 7.26 (m, 1H), 7.53 (s, 1H), 8.08 (d, 1H).

**Compound 144**

4-(3-((8aS,12S)-8a-methyl-1,2,6b,7,8,8a,10,12,12a,12b-decahydronaphtho[2',1':4,5]indeno[1,2-c]pyrazol-12-yl)propanoyl)piperazin-2-one

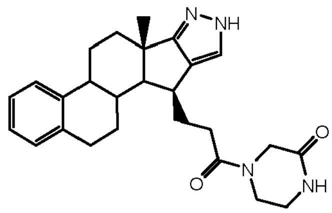

The compound **144** was prepared from the compound **143** by the Method C in 36% yield.

$^{1}$H NMR (400 MHz, DMSO-$d_6$): 1.10 (s, 3H), 1.35-2.46 (m, 15H), 2.90 (m, 2H), 3.15-3.25 (m, 2H), 3.51-3.65 (m, 2H), 3.92 (d, 1H), 7.11 (m, 3H), 7.28 (m, 1H), 7.44 (d, 1H), 8.10 (d, 1H), 12.11 (br s, 1H).

**Compound 145**

(13S,15R)-13-methyl-15-(3-oxo-3-(pyrrolidin-1-yl)propyl)-6,7,8,9,11,12,13,14,15,16-decahydro-17H-cyclopenta[a]phenanthren-17-one

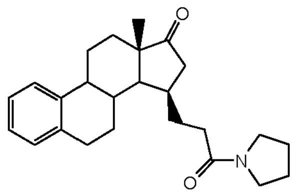

The compound **145** was synthesized in 93% yield from acid Acid **SM-XXVI** and pyrrolidine by the Method A1 in two hours reaction time.

$^1$H-NMR (400 MHz, DMSO-$d_6$): 0.97 (s, 3H), 1.37-2.43 (m, 20H), 2.87 (m, 2H), 3.27 (t, 2H), 3.40 (t, 2H), 7.09 (m, 3H), 7.27 (m, 1H).

**Compound 146**

(13S,15S,Z)-16-(hydroxymethylene)-13-methyl-15-(3-oxo-3-(pyrrolidin-1-yl)propyl)-6,7,8,9,11,12,13,14,15,16-decahydro-17H-cyclopenta[a]phenanthren-17-one

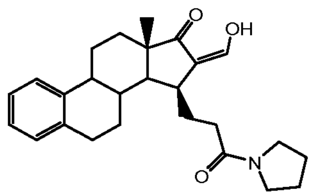

The compound **146** was prepared from the compound **145** by the Method B in 58% yield.

$^1$H NMR (400 MHz, DMSO-$d_6$): 0.97 (s, 3H), 1.35-2.40 (m, 16H), 2.88 (m, 3H), 3.26-3.45 (m, 4H), 7.09 (m, 3H), 7.27 (m, 1H), 7.50 (s, 1H), 11.42 (br s, 1H).

**Compound 147**

3-((8aS,12S)-8a-methyl-1,2,6b,7,8,8a,10,12,12a,12b-decahydronaphtho[2',1':4,5]indeno[1,2-c]pyrazol-12-yl)-1-(pyrrolidin-1-yl)propan-1-one

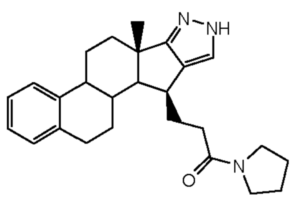

The compound **147** was prepared from the compound **146** by the Method C in 86% yield.

$^1$H NMR (400 MHz, DMSO-$d_6$): 1.10 (s, 3H), 1.39-2.43 (m, 17H), 2.81-2.97 (m, 3H), 3.25-3.30 (m, 2H), 3.38-3.48 (m, 2H), 7.10 (m, 3H), 7.29 (m, 1H), 7.41 (s, 1H), 12.11 (br s, 1H).

**Compound 148**

3-((13S,15R)-13-methyl-17-oxo-7,8,9,11,12,13,14,15,16,17-decahydro-6H-cyclopenta[a]phenanthren-15-yl)-N-(2-oxo-1,2,5,6,7,8-hexahydroquinolin-3-yl)propanamide

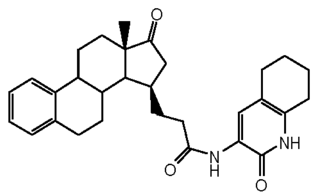

Compound **148** was synthesized in 59% yield by the Method A1 in THF by using acid SM-XXVI and 3-amino-1,2,5,6,7,8-hexahydroquinolin-2-one as starting materials in overnight reaction time.
$^1$H-NMR (400 MHz, DMSO-$d_6$): 0.97 (s, 3H), 1.30-2.60 (m, 24H), 2.79-2.99 (m, 2H), 7.00-7.15 (m, 3H), 7.23-7.30 (m, 1H), 8.01 (s, 1H), 9.13 (s, 1H), 11.67 (s, 1H).

**Compound 149**

3-((13S,15S,Z)-16-(hydroxymethylene)-13-methyl-17-oxo-7,8,9,11,12,13,14,15,16,17-decahydro-6H-cyclopenta[a] phenanthren-15-yl)-N-(2-oxo-1,2,5,6,7,8-hexahydroquinolin-3-yl)propanamide

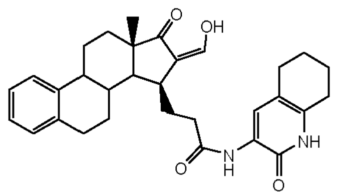

Compound **149** was synthesized in quantitative yield from the compound **148** by the Method B in 2 days reaction time by using 900 mol-% of ethyl formate and 800 mol-% of NaH.
$^1$H-NMR (400 MHz, DMSO-$d_6$): 0.98 (s, 3H), 1.30-2.60 (m, 21H), 2.70-2.99 (m, 3H), 7.00-7.15 (m, 3H), 7.23-7.30 (m, 1H), 7.52 (s, 1H), 8.00 (s, 1H), 8.97 (s, 1H), 11.08 (br s, 1H), 11.66 (s, 1H).

**Compound 150**

3-((8aS,12S)-8a-methyl-1,2,6b,7,8,8a,10,12,12a,12b-decahydronaphtho[2',1':4,5]indeno[1,2-c]pyrazol-12-yl)-N-(2-oxo-1,2,5,6,7,8-hexahydroquinolin-3-yl)propanamide

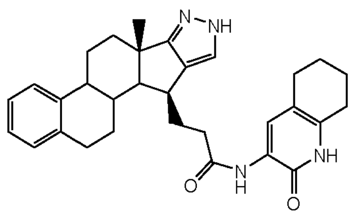

Compound **150** was synthesized in 47% yield after chromatographic purification from the compound **149** by the Method C in one hour reaction time.
$^1$H-NMR (400 MHz, DMSO-$d_6$): 1.10 (s, 3H), 1.30-2.60 (m, 21H), 2.75-2.99 (m, 3H), 7.00-7.15 (m, 3H), 7.23-7.30

(m, 1H), 7.41 (s, 1H), 8.00 (s, 1H), 9.15 (s, 1H), 11.68 (br s, 1H), 12.10 (br s, 1H).

**Compound 151**

3-((13S,15R)-13-methyl-17-oxo-7,8,9,11,12,13,14,15,16,17-decahydro-6H-cyclopenta[a]phenanthren-15-yl)-N-(tetrahydro-2H-pyran-4-yl)propanamide

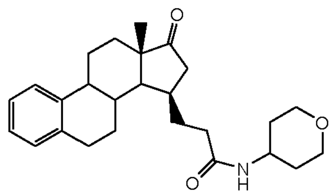

The compound **151** was synthesized in 99% yield from acid Acid **SM-XXVI** and 4-aminotetrahydropyran by the Method A1 in THF stirring at room temperature for two hours.

$^1$H-NMR (400 MHz, DMSO-$d_6$): 0.96 (s, 3H), 1.33-2.40 (m, 20H), 2.87 (m, 2H), 3.31 (m, 1H), 3.36 (m, 1H), 3.70-3.83 (m, 3H), 7.08 (m, 3H), 7.27 (d, 1H), 7.82 (d, 1H).

**Compound 152**

3-((13S,15S,Z)-16-(hydroxymethylene)-13-methyl-17-oxo-7,8,9,11,12,13,14,15,16,17-decahydro-6H-cyclopenta[a]phenanthren-15-yl)-N-(tetrahydro-2H-pyran-4-yl)propanamide

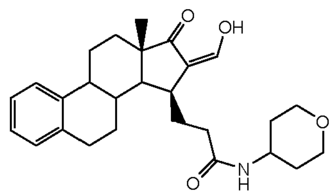

The compound **152** was prepared from the compound **151** by the Method B in 89% yield.

$^1$H NMR (400 MHz, DMSO-$d_6$): 0.98 (s, 3H), 1.13-2.40 (m, 20H), 2.88 (m, 3H), 3.74-3.84 (m, 2H), 7.09 (m, 3H), 7.27 (m, 1H), 7.48 (br s, 1H), 8.14 (d, 1H), 11.57 (br s, 1H).

**Compound 153**

3-((8aS,12S)-8a-methyl-1,2,6b,7,8,8a,10,12,12a,12b-decahydronaphtho[2',1':4,5]indeno[1,2-c]pyrazol-12-yl)-N-(tetrahydro-2H-pyran-4-yl)propanamide

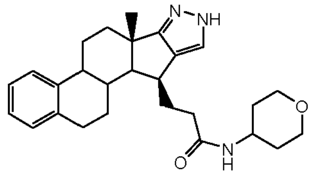

The compound **153** was prepared from the compound **152** by the Method C in 59% yield.

$^1$H NMR (400 MHz, DMSO-$d_6$): 1.09 (s, 3H), 1.30-2.39 (m, 17H), 2.77 (m, 1H), 2.89 (m, 2H), 3.36 (m, 2H), 3.80 (m, 3H), 7.09 (m, 3H), 7.28 (m, 1H), 7.38 (br s, 1H), 7.85 (d, 1H), 12.10 (br s, 1H).

**Compound 154**

N-(6-methoxypyridazin-3-yl)-3-((13S,15R)-13-methyl-17-oxo-7,8,9,11,12,13,14,15,16,17-decahydro-6H-cyclopenta[a]phenanthren-15-yl)propanamide

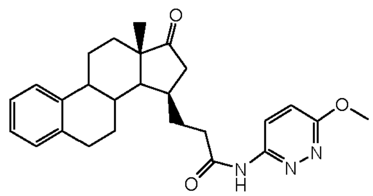

Compound **154** was synthesized in 91% yield by the Method A1 in THF by using acid **SM-XXVI** and 3-amino-6-methoxypyridazine as starting materials in overnight reaction time.

$^1$H-NMR (400 MHz, DMSO-$d_6$): 0.98 (s, 3H), 1.30-2.55 (m, 16H), 2.81-2.96 (m, 2H), 3.98 (s, 3H), 7.00-7.15 (m, 3H), 7.22-7.30 (m, 2H), 8.25 (d, 1H), 10.93 (s, 1H).

**Compound 155**

3-((13S,15S,Z)-16-(hydroxymethylene)-13-methyl-17-oxo-7,8,9,11,12,13,14,15,16,17-decahydro-6H-cyclopenta[a]phenanthren-15-yl)-N-(6-methoxypyridazin-3-yl) propanamide

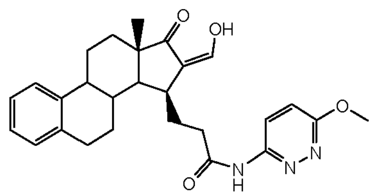

Compound **155** was synthesized in 96% yield from the compound **154** by the Method B in 3 hours reaction time.

$^1$H-NMR (400 MHz, DMSO-$d_6$): 1.00 (s, 3H), 1.30-2.65 (m, 13H), 2.81-3.05 (m, 3H), 3.98 (s, 3H), 7.00-7.15 (m, 3H), 7.20-7.30 (m, 2H), 7.60 (s, 1H), 8.24 (d, 1H), 10.98 (s, 1H).

**Compound 156**

N-(6-methoxypyridazin-3-yl)-3-((8aS,12S)-8a-methyl-1,2,6b,7,8,8a,10,12,12a,12b-decahydronaphtho[2',1':4,5]indeno[1,2-c]pyrazol-12-yl)propanamide

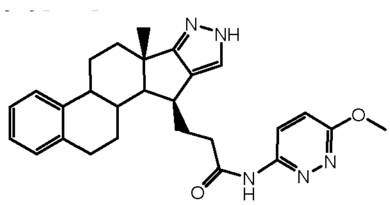

Compound **156** was synthesized in 74% yield from the compound **155** by the Method C in one hour reaction time.

$^1$H-NMR (400 MHz, DMSO-$d_6$): 1.11 (s, 3H), 1.40-2.60 (m, 13H), 2.80-3.02 (m, 3H), 3.98 (s, 3H), 7.00-7.15 (m, 3H), 7.23 (d, 1H), 7.25-7.32 (m, 1H), 7.41 (s, 1H), 8.25 (d, 1H), 10.96 (s, 1H), 12.11 (br s, 1H).

**Compound 157**

N-(5-fluoropyridin-2-yl)-3-((13S,15R)-13-methyl-17-oxo-7,8,9,11,12,13,14,15,16,17-decahydro-6H-cyclopenta[a]phenanthren-15-yl)propanamide

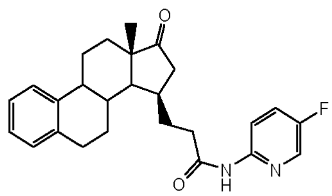

Compound **157** was synthesized in quantitative yield by the Method A1 in THF by using acid **SM-XXVI** and 2-amino-5-fluoropyridine as starting materials in 6 hours reaction time.

$^1$H-NMR (400 MHz, DMSO-$d_6$): 0.98 (s, 3H), 1.30-2.55 (m, 16H), 2.81-2.96 (m, 2H), 7.00-7.15 (m, 3H), 7.25-7.29 (m, 1H), 7.69-7.76 (m, 1H), 8.11-8.17 (m, 1H), 8.31 (d, 1H), 10.62 (s, 1H).

**Compound 158**

N-(5-fluoropyridin-2-yl)-3-((13S,15S,Z)-16-(hydroxymethylene)-13-methyl-17-oxo-7,8,9,11,12,13,14,15,16,17-decahydro-6H-cyclopenta[a]phenanthren-15-yl)propanamide

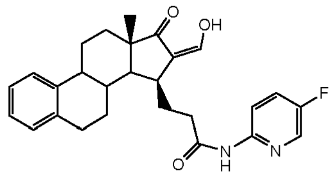

Compound **158** was synthesized in quantitative yield from the compound **157** by the Method B in 3 hours reaction time.

$^1$H-NMR (400 MHz, DMSO-$d_6$): 1.00 (s, 3H), 1.30-2.65 (m, 13H), 2.80-2.98 (m, 3H), 7.00-7.15 (m, 3H), 7.23-7.29 (m, 1H), 7.53 (s, 1H), 7.65-7.69 (m, 1H), 8.10-8.17 (m, 1H), 8.30 (d, 1H), 10.54 (s, 1H), 10.96 (s, 1H).

**Compound 159**

N-(5-fluoropyridin-2-yl)-3-((8aS,12S)-8a-methyl-1,2,6b,7,8,8a,10,12,12a,12b-decahydronaphtho[2',l':4,5]indeno[l,2-c] pyrazol-12-yl)propanamide

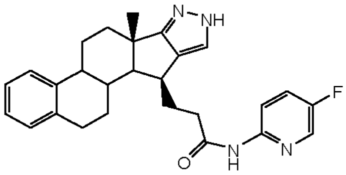

Compound **159** was synthesized in 84% yield from the compound **158** by the Method C in one hour reaction time.

$^1$H-NMR (400 MHz, DMSO-$d_6$): 1.11 (s, 3H), 1.35-2.55 (m, 13H), 2.78-2.99 (m, 3H), 7.00-7.15 (m, 3H), 7.25-7.30 (m, 1H), 7.40 (s, 1H), 7.68-7.76 (m, 1H), 8.12-8.18 (m, 1H), 8.30 (d, 1H), 10.66 (s, 1H), 12.15 (br s, 1H).

**Compound 160**

N-(4-fluoropyridin-2-yl)-3-((13S,15R)-13-methyl-17-oxo-7,8,9,11,12,13,14,15,16,17-decahydro-6H-cyclopenta[a]phenanthren-15-yl)propanamide

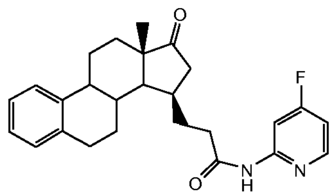

Compound **160** was synthesized in 78% yield by the Method A1 in THF by using acid **SM-XXVI** and 290 mol-% of 2-amino-4-fluoropyridine as starting materials in overnight reaction time.

$^1$H-NMR (400 MHz, DMSO-$d_6$): 0.98 (s, 3H), 1.30-2.47 (m, 16H), 2.81-2.96 (m, 2H), 7.00-7.06 (m, 1H), 7.07-7.16 (m, 3H), 7.25-7.30 (m, 1H), 7.92 (dd, 1H), 8.30-8.37 (m, 1H), 10.82 (s, 1H).

**Compound 161**

N-(4-fluoropyridin-2-yl)-3-((13S,15S,Z)-16-(hydroxymethylene)-13-methyl-17-oxo-7,8,9,11,12,13,14,15,16,17-decahydro-6H-cyclopenta[a]phenanthren-15-yl)propanamide

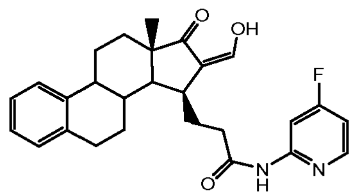

Compound **161** was synthesized in quantitative yield from the compound 160 by the Method B in overnight reaction time and using 1200 mol-% of ethyl formate and 800 mol-% on NaH.

$^1$H-NMR (400 MHz, DMSO-$d_6$): 1.00 (s, 3H), 1.30-2.70 (m, 13H), 2.80-2.99 (m, 3H), 6.98-7.04 (m, 1H), 7.05-7.16 (m, 3H), 7.25-7.30 (m, 1H), 7.53 (s, 1H), 7.91 (dd, 1H), 8.30-8.37 (m, 1H), 10.74 (s, 1H), 10.95 (br s, 1H).

**Compound 162**

N-(4-fluoropyridin-2-yl)-3-((8aS,12S)-8a-methyl-1,2,6b,7,8,8a,10,12,12a,12b-decahydronaphtho[2',1':4,5]indeno[1,2-c]pyrazol-12-yl)propanamide

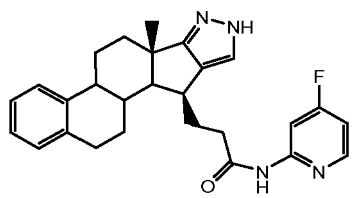

Compound **162** was synthesized in 47% yield from the compound **161** by the Method C in one hour reaction time.

$^1$H-NMR (400 MHz, DMSO-$d_6$): 1.11 (s, 3H), 1.40-2.50 (m, 13H), 2.78-2.99 (m, 3H), 6.98-7.06 (m, 1H), 7.07-7.16 (m, 3H), 7.25-7.30 (m, 1H), 7.39 (s, 1H), 7.93 (dd, 1H), 8.32-8.38 (m, 1H), 10.86 (s, 1H), 12.12 (br s, 1H).

**Compound 163**

N-(4-methoxypyridin-2-yl)-3-((13S,15R)-13-methyl-17-oxo-7,8,9,11,12,13,14,15,16,17-decahydro-6H-cyclopenta[a] phenanthren-15-yl)propanamide

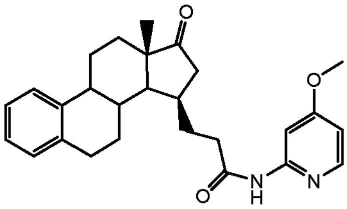

Compound **163** was synthesized in 53% yield by the Method A1 in THF by using acid **SM-XXVI** and 2-amino-4-methoxypyridine as starting materials in 6 hours reaction time.
$^1$H-NMR (400 MHz, DMSO-$d_6$): 0.98 (s, 3H), 1.30-2.47 (m, 16H), 2.81-2.96 (m, 2H), 3.80 (s, 3H), 6.69 (dd, 1H), 7.05-7.16 (m, 3H), 7.25-7.30 (m, 1H), 7.72 (d, 1H), 8.11 (d, 1H), 10.46 (s, 1H).

**Compound 164**

3-((13S,15S,Z)-16-(hydroxymethylene)-13-methyl-17-oxo-7,8,9,11,12,13,14,15,16,17-decahydro-6H-cyclopenta[a] phenanthren-15-yl)-N-(4-methoxypyridin-2-yl) propanamide

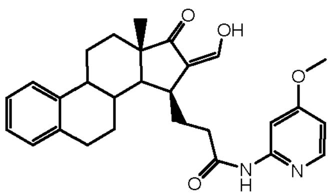

Compound **164** was synthesized in 94% yield from the compound **163** by the Method B in 4 hours reaction time.
$^1$H-NMR (400 MHz, DMSO-$d_6$): 1.00 (s, 3H), 1.30-2.60 (m, 13H), 2.81-3.00 (m, 3H), 3.80 (s, 3H), 6.68 (dd, 1H), 7.05-7.14 (m, 3H), 7.25-7.30 (m, 1H), 7.54 (s, 1H), 7.72 (d, 1H), 8.10 (d, 1H), 10.39 (s, 1H), 10.97 (br s, 1H).

**Compound 165**

N-(4-methoxypyridin-2-yl)-3-((8aS,12S)-8a-methyl-1,2,6b,7,8,8a,10,12,12a,12b-decahydronaphtho[2',1':4,5]indeno[1,2-c]pyrazol-12-yl)propanamide

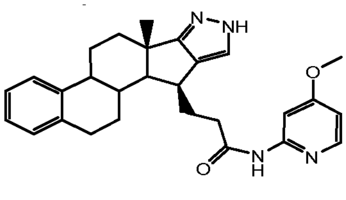

Compound **165** was synthesized in 76% yield from the compound **164** by the Method C in one hour reaction time.
$^1$H-NMR (400 MHz, DMSO-$d_6$): 1.11 (s, 3H), 1.35-2.49 (m, 13H), 2.79-3.00 (m, 3H), 3.80 (s, 3H), 6.68 (dd, 1H), 7.05-7.14 (m, 3H), 7.25-7.30 (m, 1H), 7.39 (s, 1H), 7.73 (d, 1H), 8.12 (d, 1H), 10.49 (s, 1H), 12.12 (br s, 1H).

**Compound 166**

3-((13S,15R)-13-methyl-17-oxo-7,8,9,11,12,13,14,15,16,17-decahydro-6H-cyclopenta[a]phenanthren-15-yl)-N-(4-methylpyridin-2-yl)propanamide

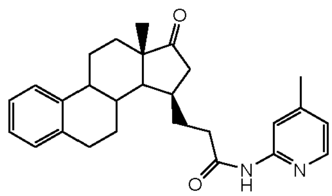

Compound **166** was synthesized in 82% yield by the Method A1 in THF by using acid **SM-XXVI** and 2-amino-4-methylpyridine as starting materials in 6 hours reaction time.

$^1$H-NMR (400 MHz, DMSO-$d_6$): 0.98 (s, 3H), 1.30-2.48 (m, 19H), 2.80-2.95 (m, 2H), 6.92 (d, 1H), 7.02-7.19 (m, 3H), 7.25-7.29 (m, 1H), 7.95 (s, 1H), 8.15 (d, 1H), 10.41 (s, 1H).

**Compound 167**

3-((13S,15S,Z)-16-(hydroxymethylene)-13-methyl-17-oxo-7,8,9,11,12,13,14,15,16,17-decahydro-6H-cyclopenta[a] phenanthren-15-yl)-N-(4-methylpyridin-2-yl) propanamide

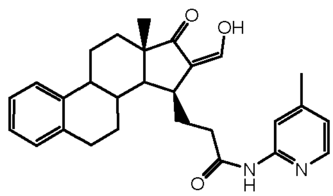

Compound **167** was synthesized in quantitative yield from the compound **166** by the Method B in 3 hours reaction time.

$^1$H-NMR (400 MHz, DMSO-$d_6$): 1.00 (s, 3H), 1.30-2.65 (m, 16H), 2.80-3.05 (m, 3H), 6.91 (d, 1H), 7.02-7.15 (m, 3H), 7.25-7.29 (m, 1H), 7.54 (s, 1H), 7.94 (s, 1H), 8.14 (d, 1H), 10.33 (s, 1H), 10.97 (br s, 1H).

**Compound 168**

3-((8aS,12S)-8a-methyl-1,2,6b,7,8,8a,10,12,12a,12b-decahydronaphtho[2',1':4,5]indeno[1,2-c]pyrazol-12-yl)-N-(4-methylpyridin-2-yl)propanamide

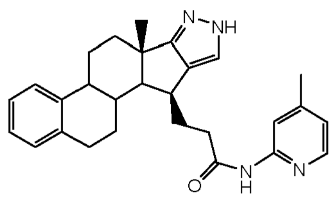

Compound **168** was synthesized in 63% yield from the compound **167** by the Method C in one hour reaction time.

$^1$H-NMR (400 MHz, DMSO-$d_6$): 1.11 (s, 3H), 1.30-2.49 (m, 16H), 2.79-3.03 (m, 3H), 6.92 (d, 1H), 7.05-7.15 (m, 3H), 7.27-7.31 (m, 1H), 7.41 (s, 1H), 7.96 (s, 1H), 8.15 (d, 1H), 10.45 (s, 1H), 12.11 (br s, 1H).

**Compound 169**

3-((13S,15R)-13-methyl-17-oxo-7,8,9,11,12,13,14,15,16,17-decahydro-6H-cyclopenta[a]phenanthren-15-yl)-N-(pyridin-2-yl)propanamide

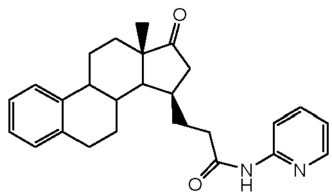

Compound **169** was synthesized in 75% yield by the Method A1 in THF by using acid **SM-XXVI** and 2-aminopyridine as starting materials in overnight reaction time.

$^1$H-NMR (400 MHz, DMSO-$d_6$): 0.99 (s, 3H), 1.30-2.45 (m, 16H), 2.80-2.96 (m, 2H), 7.00-7.15 (m, 4H), 7.21-7.30 (m, 1H), 7.70-7.82 (m, 1H), 8.09 (d, 1H), 8.28-8.33 (m, 1H), 10.50 (s, 1H).

**Compound 170**

3-((13S,15S,Z)-16-(hydroxymethylene)-13-methyl-17-oxo-7,8,9,11,12,13,14,15,16,17-decahydro-6H-cyclopenta[a]phenanthren-15-yl)-N-(pyridin-2-yl)propanamide

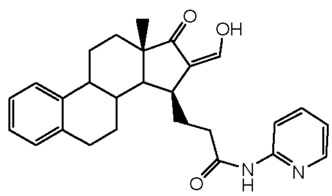

Compound **170** was synthesized in quantitative yield from the compound **169** by the Method B in 4 hours reaction time.

$^1$H-NMR (400 MHz, DMSO-$d_6$): 1.00 (s, 3H), 1.30-2.65 (m, 13H), 2.80-3.05 (m, 3H), 7.00-7.15 (m, 4H), 7.24-7.30 (m, 1H), 7.56 (s, 1H), 7.70-7.80 (m, 1H), 8.08 (d, 1H), 8.28-8.32 (m, 1H), 10.46 (s, 1H), 10.98 (br s, 1H).

**Compound 171**

3-((8aS,12S)-8a-methyl-1,2,6b,7,8,8a,10,12,12a,12b-decahydronaphtho[2',1':4,5]indeno[1,2-c]pyrazol-12-yl)-N-(pyridin-2-yl)propanamide

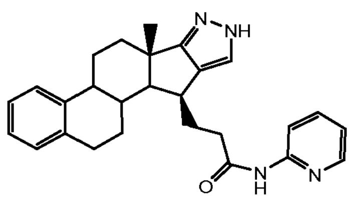

Compound **171** was synthesized in 87% yield from the compound **170** by the Method C in one hour reaction time.

$^1$H-NMR (400 MHz, DMSO-$d_6$): 1.11 (s, 3H), 1.35-2.60 (m, 13H), 2.78-3.02 (m, 3H), 7.03-7.15 (m, 4H), 7.26-7.30 (m, 1H), 7.41 (s, 1H), 7.70-7.80 (m, 1H), 8.10 (d, 1H), 8.28-8.32 (m, 1H), 10.53 (s, 1H), 12.12 (br s, 1H).

**Compound 172**

N-(cyclopropylmethyl)-N-methyl-3-((8aS,12S)-8a-methyl-1,2,6b,7,8,8a,10,12,12a,12b-decahydronaphtho[2',1':4,5]indeno[1,2-c]pyrazol-12-yl)propanamide

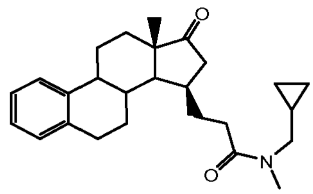

Compound **172** was synthesized in 80% yield by the Method A1 in THF by using acid **SM-XXVI** and 400 mol-% of (cyclopropylmethyl) (methyl)amine, 600 mol-% of pyridine and 400 mol-% of T3P in overnight reaction time at rt and 4 hours at 40 °C.

$^1$H-NMR (400 MHz, $CDCl_3$): 0.21-0.27 (m, 2H), 0.45-0.55 (m, 1H), 0.55-0.65 (m, 1H), 0.87-1.02 (m, 1H), 1.07 (s, 3H), 1.35-2.65 (m, 16H), 2.85-3.05 (m, 2H), 3.01/3.07 (2 x s, 3H, isomers), 3.13-3.36 (m, 2H), 7.00-7.20 (m, 3H), 7.25-7.33 (m, 1H).

**Compound 173**

N-(cyclopropylmethyl)-3-((13S,15S,Z)-16-(hydroxymethylene)-13-methyl-17-oxo-7,8,9,11,12,13,14,15,16,17-decahydro-6H-cyclopenta[a]phenanthren-15-yl)-N-methylpropanamide

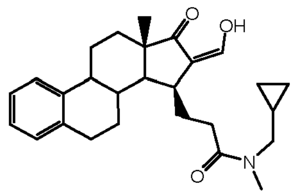

Compound **173** was synthesized in 96% yield from the compound **172** by the Method B in 5 hours reaction time.

$^1$H-NMR (400 MHz, DMSO-$d_6$): 0.17-0.27 (m, 2H), 0.39-0.55 (m, 2H), 0.72-0.90 (m, 1H), 0.97/0.98 (2 x s, 3H, isomers), 1.30-2.70 (m, 13H), 2.80-3.03 (m, 3H), 2.88/3.04 (2 x s, 3H, isomers), 3.05-3.25 (m, 2H), 7.00-7.15 (m, 3H), 7.21-7.30 (m, 1H), 7.49/7.50 (2 x s, 1H, isomers), 11.44 (br s, 1H).

**Compound 174**

N-(cyclopropylmethyl)-N-methyl-3-((8aS,12S)-8a-methyl-1,2,6b,7,8,8a,10,12,12a,12b-decahydronaphtho[2',1':4,5]indeno[1,2-c]pyrazol-12-yl)propanamide

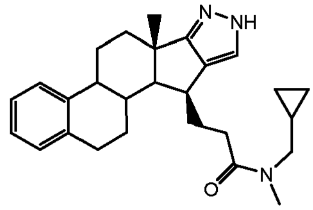

Compound **174** was synthesized in 46% yield from the compound **173** by the Method C in 1.5 hours reaction time.

$^1H$-NMR (400 MHz, DMSO-$d_6$): 0.17-0.27 (m, 2H), 0.35-0.55 (m, 2H), 0.85-1.00 (m, 1H), 1.10 (s, 3H), 1.25-2.49 (m, 13H), 2.80-3.00 (m, 3H), 2.85/3.04 (2 x s, 3H, isomers), 3.05-3.25 (m, 2H), 7.00-7.15 (m, 3H), 7.21-7.30 (m, 1H), 7.38/7.42 (2 xbrs, 1H, isomers), 12.11 (br s, 1H).

**Compound 175**

(13S,15R)-13-methyl-15-(3-oxo-3-(8-oxa-2-azaspiro[4.5]decan-2-yl)propyl)-6,7,8,9,11,12,13,14,15,16-decahydro-17H-cyclopenta[a]phenanthren-17-one

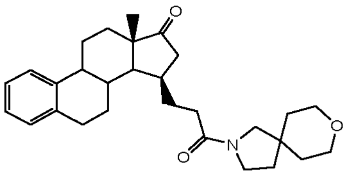

Compound **175** was synthesized in 98% yield by the Method A1 in THF by using acid **SM-XXVI** and 8-oxa-2-aza-spiro(4,5)decane hydrochloride, 800 mol-% of pyridine and 400 mol-% of T3P in overnight reaction time.
$^1H$-NMR (400 MHz, DMSO-$d_6$): 0.97 (s, 3H), 1.30-2.46 (m, 22H), 2.84-2.95 (m, 2H), 3.17-3.25 (m, 1H), 3.29-3.40 (m, 2H), 3.46-3.65 (m, 5H), 7.00-7.15 (m, 3H), 7.24-7.29 (m, 1H).

**Compound 176**

(13S,15S,Z)-16-(hydroxymethylene)-13-methyl-15-(3-oxo-3-(8-oxa-2-azaspiro[4.5]decan-2-yl)propyl)-6,7,8,9,11,12,13,14,15,16-decahydro-17H-cyclopenta[a]phenanthren-17-one

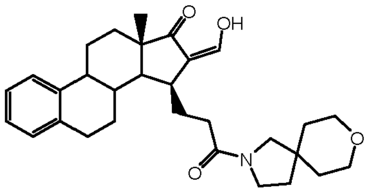

Compound **176** was synthesized in 97% yield from the compound **175** by the Method B in overnight reaction time.
$^1H$-NMR (400 MHz, DMSO-$d_6$): 0.98 (s, 3H), 1.30-2.46 (m, 19H), 2.80-2.99 (m, 3H), 3.17-3.29 (m, 1H), 3.30-3.40 (m, 2H), 3.41-3.69 (m, 5H), 7.00-7.15 (m, 3H), 7.24-7.29 (m, 1H), 7.49 (s, 1H), 11.46 (br s, 1H).

**Compound 177**

3-((8aS,12S)-8a-methyl-1,2,6b,7,8,8a,10,12,12a,12b-decahydronaphtho[2',1':4,5]indeno[1,2-c]pyrazol-12-yl)-1-(8-oxa-2-azaspiro[4.5]decan-2-yl)propan-1-one

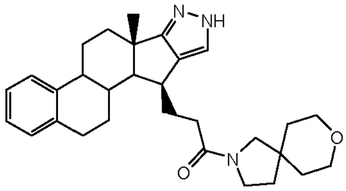

Compound **177** was synthesized in 68% yield from the compound **176** by the Method C in 0.5 hours reaction time.

$^1$H-NMR (400 MHz, DMSO-$d_6$): 1.10 (s, 3H), 1.30-2.46 (m, 19H), 2.80-2.99 (m, 3H), 3.20 (s, 1H), 3.27-3.40 (m, 2H), 3.41-3.69 (m, 5H), 7.00-7.16 (m, 3H), 7.24-7.29 (m, 1H), 7.41/7.45 (2 x br s, 1H), 12.11/12.48 (2 x br s, 1H).

**Compound 178**

(13S,15R)-15-(3-(isoindolin-2-yl)-3-oxopropyl)-13-methyl-6,7,8,9,11,12,13,14,15,16-decahydro-17H-cyclopenta[a]phenanthren-17-one

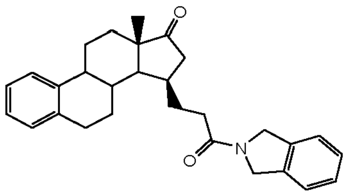

Compound **178** was synthesized in 56% yield by the Method A1 in THF by using acid **SM-XXVI** and isoindoline as starting materials in overnight reaction time. $^1$H-NMR (400 MHz, $CDCl_3$): 1.09 (s, 3H), 1.39-2.60 (m, 16H), 2.85-3.08 (m, 2H), 4.82 (s, 4H), 7.05-7.20 (m, 3H), 7.25-7.34 (m, 5H).

**Compound 179**

(13S,15S,Z)-16-(hydroxymethylene)-15-(3-(isoindolin-2-yl)-3-oxopropyl)-13-methyl-6,7,8,9,11,12,13,14,15,16-decahydro-17H-cyclopenta[a]phenanthren-17-one

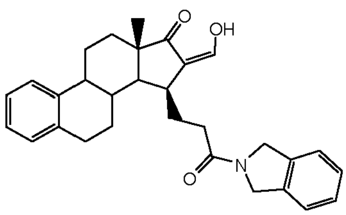

Compound **179** was synthesized in 81% yield after chromatographic purification from the compound **178** by the Method B in overnight reaction time by using 1200 mol-% of ethyl formate and 800 mol-% of NaH.

$^1$H-NMR (400 MHz, DMSO-$d_6$): 1.00 (s, 3H), 1.30-2.66 (m, 13H), 2.79-3.05 (m, 3H), 4.65 (s, 2H), 4.75-4.88 (m, 2H), 7.05-7.15 (m, 3H), 7.25-7.40 (m, 5H). 7.54 (s, 1H), 11.09 (br s, 1H).

**Compound 180**

1-(isoindolin-2-yl)-3-((8aS,12S)-8a-methyl-1,2,6b,7,8,8a,10,12,12a,12b-decahydronaphtho [2',1':4,5]indeno [1,2-c] pyrazol-12-yl)propan-1-one

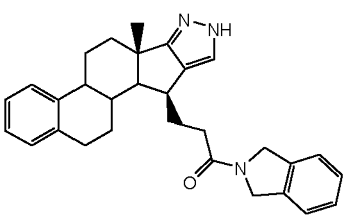

Compound **180** was synthesized in 92% yield from the compound **179** by the Method C in one hour reaction time.

$^1$H-NMR (400 MHz, DMSO-$d_6$): 1.12 (s, 3H), 1.35-2.49 (m, 13H), 2.85-3.01 (m, 3H), 4.64 (s, 2H), 4.80-4.95 (m, 2H), 7.05-7.15 (m, 3H), 7.25-7.40 (m, 5H). 7.47 (s, 1H), 12.12 (br s, 1H).

**Compound 181**

N,N-dimethyl-6-(3-((13S,15R)-13-methyl-17-oxo-7,8,9,11,12,13,14,15,16,17-decahydro-6H-cyclopenta[a]phenanthren-15-yl)propanamido)nicotinamide

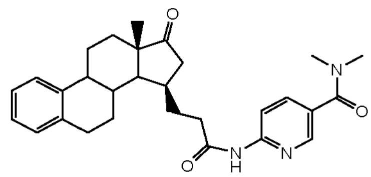

Compound **181** was synthesized in 76% yield by the Method A1 in THF by using acid **SM-XXVI** and 6-amino-N,N-dimethylpyridine-3-carboxamide as starting materials in overnight reaction time.
$^1$H-NMR (400 MHz, DMSO-$d_6$): 0.99 (s, 3H), 1.30-2.56 (m, 16H), 2.79-2.96 (m, 2H), 2.97 (s, 6H), 7.00-7.15 (m, 3H), 7.21-7.30 (m, 1H), 7.85 (dd, 1H), 8.14 (d, 1H), 8.38 (d, 1H), 10.71 (s, 1H).

**Compound 182**

6-(3-((13S,15S,Z)-16-(hydroxymethylene)-13-methyl-17-oxo-7,8,9,11,12,13,14,15,16,17-decahydro-6H-cyclopenta[a]phenanthren-15-yl)propanamido)-N,N-dimethylnicotinamide

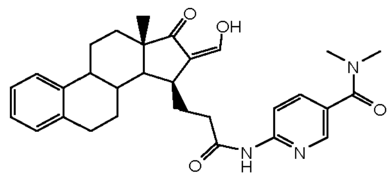

Compound **182** was synthesized in quantitative yield from the compound **181** by the Method B in overnight reaction time by using 1200 mol-% of ethyl formate and 1000 mol-% of NaH.
$^1$H-NMR (400 MHz, DMSO-$d_6$): 1.00 (s, 3H), 1.30-2.66 (m, 13H), 2.79-3.03 (m, 3H), 2.97 (s, 6H), 7.00-7.15 (m, 3H), 7.21-7.30 (m, 1H), 7.54 (s, 1H), 7.84 (dd, 1H), 8.13 (d, 1H), 8.37 (d, 1H), 10.64 (s, 1H), 10.97 (br s, 1H).

**Compound 183**

N,N-dimethyl-6-(3-((8aS,12S)-8a-methyl-1,2,6b,7,8,8a,10,12,12a,12b-decahydronaphtho[2',1':4,5]indeno[1,2-c]pyrazol-12-yl)propanamido)nicotinamide

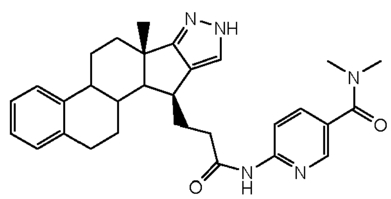

Compound **183** was synthesized in 80% yield from the compound **182** by the Method C in one hour reaction time.
$^1$H-NMR (400 MHz, DMSO-$d_6$): 1.11 (s, 3H), 1.30-2.60 (m, 13H), 2.79-3.03 (m, 3H), 2.98 (s, 6H), 7.00-7.15 (m, 3H), 7.21-7.30 (m, 1H), 7.41 (s, 1H), 7.85 (dd, 1H), 8.15 (d, 1H), 8.39 (d, 1H), 10.76 (s, 1H), 12.14 (br s, 1H).

**Compound 184**

3-((13S,15R)-13-methyl-17-oxo-7,8,9,11,12,13,14,15,16,17-decahydro-6H-cyclopenta[a]phenanthren-15-yl)-N-(1-methyl-1H-pyrazol-3-yl)propanamide

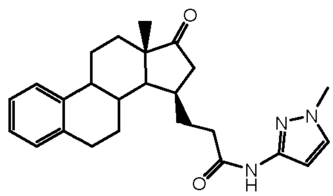

Compound **184** was synthesized in 85% yield by the Method A1 in THF by using acid **SM-XXVI** and 1-methyl-1H-pyrazol-3-amine as starting materials and 400 mol-% of T3P in 2 days reaction time and warming at 40°C for 4 hours.

$^1$H-NMR (400 MHz, DMSO-$d_6$): 0.97 (s, 3H), 1.30-2.47 (m, 16H), 2.79-2.96 (m, 2H), 3.72 (s, 3H), 6.42 (d, 1H), 7.00-7.15 (m, 3H), 7.23-7.30 (m, 1H), 7.51 (d, 1H), 10.36 (s, 1H).

**Compound 185**

3-((13S,15S,Z)-16-(hydroxymethylene)-13-methyl-17-oxo-7,8,9,11,12,13,14,15,16,17-decahydro-6H-cyclopenta[a]phenanthren-15-yl)-N-(1-methyl-1H-pyrazol-3-yl)propanamide

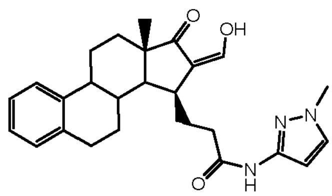

Compound **185** was synthesized in quantitative yield from the compound **184** by the Method B in 5 hours reaction time.

$^1$H-NMR (400 MHz, DMSO-$d_6$): 0.99 (s, 3H), 1.30-2.47 (m, 13H), 2.79-2.96 (m, 3H), 3.72 (s, 3H), 6.42 (d, 1H), 7.00-7.15 (m, 3H), 7.23-7.30 (m, 1H), 7.51 (d, 1H), 7.54 (s, 1H), 10.38 (s, 1H), 11.06 (br s, 1H).

**Compound 186**

3-((8aS,12S)-8a-methyl-1,2,6b,7,8,8a,10,12,12a,12b-decahydronaphtho[2',1':4,5]indeno[l,2-c]pyrazol-12-yl)-N-(1-methyl-1H-pyrazol-3-yl)propanamide

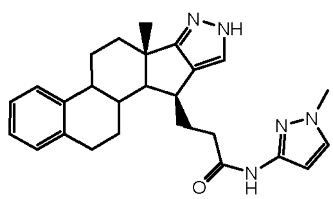

Compound **186** was synthesized in 62% yield from the compound **185** by the Method C in one hour reaction time.

$^1$H-NMR (400 MHz, DMSO-$d_6$): 1.10 (s, 3H), 1.30-2.47 (m, 13H), 2.75-2.96 (m, 3H), 3.72 (s, 3H), 6.43 (d, 1H), 7.00-7.15 (m, 3H), 7.23-7.30 (m, 1H), 7.40 (s, 1H), 7.52 (d, 1H), 10.40 (s, 1H), 12.11 (br s, 1H).

**Compound 187**

3-((13S,15R)-13-methyl-17-oxo-7,8,9,11,12,13,14,15,16,17-decahydro-6H-cyclopenta[a]phenanthren-15-yl)-N-(pyrazin-2-yl)propanamide

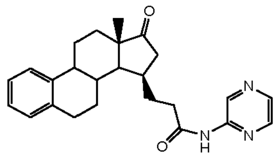

Compound **187** was synthesized in 66% yield by the Method A1 in THF by using acid **SM-XXVI** and aminopyrazine as starting materials in overnight reaction time.

$^1$H-NMR (400 MHz, DMSO-$d_6$): 0.99 (s, 3H), 1.30-2.60 (m, 16H), 2.79-2.97 (m, 2H), 7.00-7.15 (m, 3H), 7.23-7.30 (m, 1H), 8.34 (d, 1H), 8.39 (d, 1H), 9.35 (s, 1H), 10.81 (s, 1H).

**Compound 188**

3-((13S,15S,Z)-16-(hydroxymethylene)-13-methyl-17-oxo-7,8,9,11,12,13,14,15,16,17-decahydro-6H-cyclopenta[a]phenanthren-15-yl)-N-(pyrazin-2-yl)propanamide

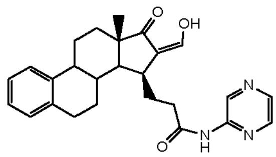

Compound **188** was synthesized in quantitative yield from the compound **187** by the Method B in 4 hours reaction time.

$^1$H-NMR (400 MHz, DMSO-$d_6$): 1.00 (s, 3H), 1.30-2.70 (m, 13H), 2.79-3.05 (m, 3H), 7.00-7.15 (m, 3H), 7.23-7.30 (m, 1H), 7.54 (s, 1H), 8.33 (d, 1H), 8.37 (d, 1H), 9.33 (s, 1H), 10.73 (s, 1H), 10.95 (br s, 1H).

**Compound 189**

3-((8aS,12S)-8a-methyl-1,2,6b,7,8,8a,10,12,12a,12b-decahydronaphtho[2',1':4,5]indeno[1,2-c]pyrazol-12-yl)-N-(pyrazin-2-yl)propanamide

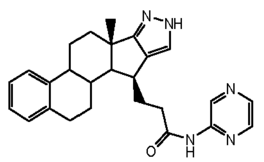

Compound **189** was synthesized in 43% yield after chromatographic purification from the compound **188** by the Method C in one hour reaction time.

$^1$H-NMR (400 MHz, DMSO-$d_6$): 1.12 (s, 3H), 1.39-2.60 (m, 13H), 2.79-3.05 (m, 3H), 7.00-7.15 (m, 3H), 7.23-7.30 (m, 1H), 7.40 (s, 1H), 8.34 (d, 1H), 8.40 (d, 1H), 9.35 (s, 1H), 10.84 (s, 1H), 12.15 (br s, 1H).

**Compound 190**

N-cyclobutyl-3-((13S,15R)-13-methyl-17-oxo-7,8,9,11,12,13,14,15,16,17-decahydro-6H-cyclopenta[a]phenanthren-15-yl)propanamide

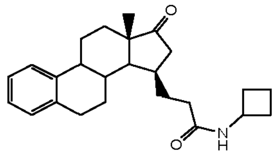

Compound **190** was synthesized in 91% yield by the Method A1 in THF by using acid **SM-XXVI** and cyclobutylamine as starting materials in 5 hours reaction time. $^1$H-NMR (400 MHz, DMSO-$d_6$): 0.95 (s, 3H), 1.30-2.43 (m, 22H), 2.79-2.97 (m, 2H), 4.10-4.25 (m, 1H), 7.00-7.15 (m, 3H), 7.23-7.30 (m, 1H), 8.07 (d, 1H).

**Compound 191**

N-cyclobutyl-3-((13S,15S,Z)-16-(hydroxymethylene)-13-methyl-17-oxo-7,8,9,11,12,13,14,15,16,17-decahydro-6H-cyclopenta[a]phenanthren-15-yl)propanamide

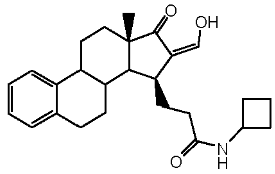

Compound **191** was synthesized in quantitative yield from the compound **190** by the Method B in 4 hours reaction time.

$^1$H-NMR (400 MHz, DMSO-$d_6$): 0.97 (s, 3H), 1.30-2.43 (m, 19H), 2.79-2.97 (m, 3H), 4.10-4.25 (m, 1H), 7.00-7.15 (m, 3H), 7.23-7.30 (m, 1H), 7.53 (s, 1H), 8.39 (d, 1H).

**Compound 192**

N-cyclobutyl-3-((8aS,12S)-8a-methyl-1,2,6b,7,8,8a,10,12,12a,12b-decahydronaphtho[2',1':4,5]indeno[1,2-c]pyrazol-12-yl)propanamide

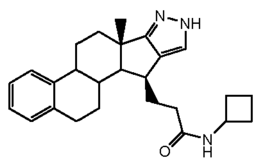

Compound **192** was synthesized in 80% yield from the compound **191** by the Method C in one hour reaction time.

$^1$H-NMR (400 MHz, DMSO-$d_6$): 1.09 (s, 3H), 1.30-2.43 (m, 19H), 2.70-2.98 (m, 3H), 4.10-4.25 (m, 1H), 7.00-7.15 (m, 3H), 7.25-7.30 (m, 1H), 7.37 (s, 1H), 8.10 (d, 1H), 12.12 (br s, 1H).

**Compound 193**

3-((13S,15R)-13-methyl-17-oxo-7,8,9,11,12,13,14,15,16,17-decahydro-6H-cyclo-penta[a]phenanthren-15-yl)-N-(6-methylpyridazin-3-yl)propanamide

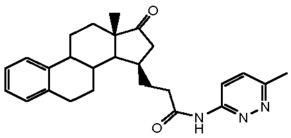

Compound **193** was synthesized in 62% yield by the Method A1 in THF by using acid **SM-XXVI** and 3-amino-6-methylpyridazine as starting materials in 2 days reaction time.

$^1$H-NMR (400 MHz, DMSO-$d_6$): 0.99 (s, 3H), 1.30-2.49 (m, 16H), 2.55 (s, 3H), 2.79-2.97 (m, 2H), 7.00-7.15 (m, 3H), 7.23-7.30 (m, 1H), 7.54 (d, 1H), 8.22 (d, 1H), 11.04 (s, 1H).

**Compound 194**

3-((13S,15S,Z)-16-(hydroxymethylene)-13-methyl-17-oxo-7,8,9,11,12,13,14,15,16,17-decahydro-6H-cyclopenta[a]phenanthren-15-yl)-N-(6-methylpyridazin-3-yl)propanamide

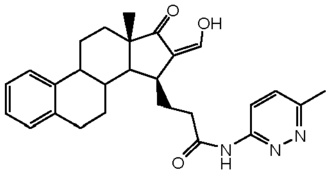

Compound **194** was synthesized in quantitative yield from the compound **193** by the Method B in overnight reaction time and then warming at 30 °C for 5 hours by using 1200 mol-% of ethyl formate and 800 mol-% of NaH.

$^1$H-NMR (400 MHz, DMSO-$d_6$): 1.00 (s, 3H), 1.30-2.70 (m, 13H), 2.55 (s, 3H), 2.80-3.05 (m, 3H), 7.00-7.15 (m, 3H), 7.23-7.30 (m, 1H), 7.52 (d, 1H), 7.54 (s, 1H), 8.21 (d, 1H), 10.93 (br s, 1H).

**Compound 195**

3-((8aS,12S)-8a-methyl-1,2,6b,7,8,8a,10,12,12a,12b-decahydronaphtho[2',1':4,5]indeno[1,2-c]pyrazol-12-yl)-N-(6-methylpyridazin-3-yl)propanamide

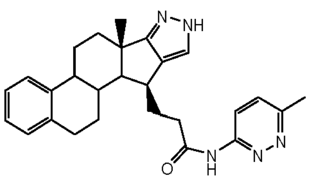

Compound **195** was synthesized in 53% yield from the compound **194** by the Method C in one hour reaction time.

$^1$H-NMR (400 MHz, DMSO-$d_6$): 1.11 (s, 3H), 1.30-2.61 (m, 13H), 2.55 (s, 3H), 2.78-3.05 (m, 3H), 7.00-7.15 (m, 3H), 7.23-7.30 (m, 1H), 7.41 (s, 1H), 7.53 (d, 1H), 8.22 (d, 1H), 11.06 (s, 1H), 12.12 (br s, 1H).

**Compound 196**

3-((13S,15R)-13-methyl-17-oxo-7,8,9,11,12,13,14,15,16,17-decahydro-6H-cyclo-penta[a]phenanthren-15-yl)-N-(5-(4-methylpiperazin-1-yl)pyridin-2-yl)propanamide

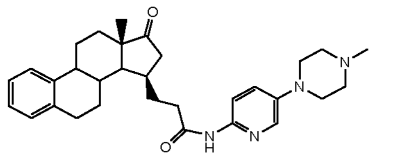

Compound **196** was synthesized in 39% yield by the Method A1 in THF by using acid **SM-XXVI** and 1-methyl-4-(6-aminopyridin-3-yl)piperazine as starting materials in 5 hours reaction time.

$^{1}$H-NMR (400 MHz, DMSO-$d_6$): 0.98 (s, 3H), 1.30-2.49 (m, 16H), 2.21 (s, 3H), 2.40-2.49 (m, 4H), 2.79-2.98 (m, 2H), 3.05-3.15 (m, 4H), 7.00-7.15 (m, 3H), 7.23-7.30 (m, 1H), 7.35-7.41 (m, 1H), 7.90-8.00 (m, 2H), 10.26 (s, 1H).

**Compound 197**

3-((13S,15S,Z)-16-(hydroxymethylene)-13-methyl-17-oxo-7,8,9,11,12,13,14,15,16,17-decahydro-6H-cyclopenta[a]phenanthren-15-yl)-N-(5-(4-methylpiperazin-1-yl)pyridin-2-yl)propanamide

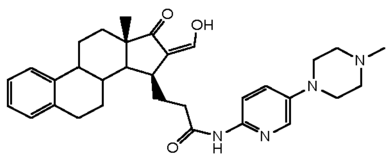

Compound **197** was synthesized in 84% yield from the compound **196** by the Method B in 5 hours reaction time.

$^{1}$H-NMR (400 MHz, DMSO-$d_6$): 0.99 (s, 3H), 1.30-2.49 (m, 13H), 2.24 (s, 3H), 2.43-2.55 (m, 4H), 2.79-3.05 (m, 3H), 3.05-3.15 (m, 4H), 7.00-7.15 (m, 3H), 7.23-7.30 (m, 1H), 7.38 (dd, 1H), 7.59 (s, 1H), 7.93 (d, 1H), 7.98 (d, 1H), 10.31 (s, 1H).

**Compound 198**

3-((8aS,12S)-8a-methyl-1,2,6b,7,8,8a,10,12,12a,12b-decahydronaphtho[2',1':4,5]indeno[1,2-c]pyrazol-12-yl)-N-(5-(4-methylpiperazin-1-yl)pyridin-2-yl)propanamide

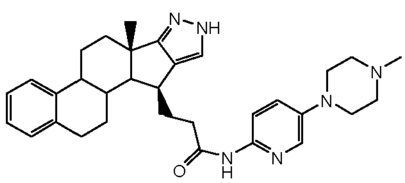

Compound **198** was synthesized in 81% yield from the compound **197** by the Method C in one hour reaction time.

$^{1}$H-NMR (400 MHz, DMSO-$d_6$): 1.10 (s, 3H), 1.30-2.49 (m, 13H), 2.22 (s, 3H), 2.43-2.49 (m, 4H), 2.77-3.05 (m, 3H), 3.05-3.15 (m, 4H), 7.00-7.15 (m, 3H), 7.23-7.30 (m, 1H), 7.38 (dd, 1H), 7.40 (s, 1H), 7.95 (d, 1H), 7.98 (d, 1H), 10.29 (s, 1H), 12.10 (br s, 1H).

**Compound 199**

N-(tert-butyl)-3-((13S,15R)-13-methyl-17-oxo-7,8,9,11,12,13,14,15,16,17-deca-hydro-6H-cyclopenta[a]phenanthren-15-yl)propanamide

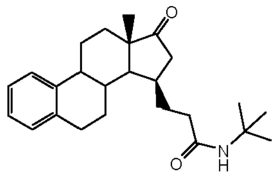

Compound **199** was synthesized in 68% yield by the Method A1 in THF by using acid **SM-XXVI** and *tert*-butylamine as starting materials in 2 days reaction time. $^1$H-NMR (400 MHz, DMSO-$d_6$): 0.96 (s, 3H), 1.24 (s, 9H), 1.30-2.46 (m, 16H), 2.82-2.97 (m, 2H), 7.00-7.15 (m, 3H), 7.24-7.29 (m, 1H), 7.44 (s, 1H).

**Compound 200**

N-(tert-butyl)-3-((13S,15S,Z)-16-(hydroxymethylene)-13-methyl-17-oxo-7,8,9,11,12,13,14,15,16,17-decahydro-6H-cyclopenta[a]phenanthren-15-yl)propanamide

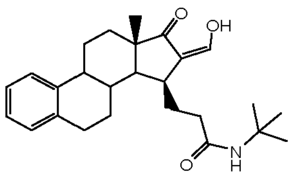

Compound **200** was synthesized in quantitative yield from the compound **199** by the Method B in overnight reaction time by using 1200 mol-% of ethyl formate and 800 mol-% of NaH.

$^1$H-NMR (400 MHz, DMSO-$d_6$): 0.98 (s, 3H), 1.28 (s, 9H), 1.30-2.47 (m, 13H), 2.82-2.97 (m, 3H), 7.01-7.15 (m, 3H), 7.24-7.29 (m, 1H), 7.46 (s, 1H), 7.87 (br s, 1H), 11.88 (br s, 1H).

**Compound 201**

N-(tert-butyl)-3-((8aS,12S)-8a-methyl-1,2,6b,7,8,8a,10,12,12a,12b-decahydronaphtho[2',1':4,5]indeno[1,2-c]pyrazol-12-yl)propanamide

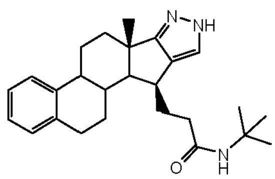

Compound **201** was synthesized in 30% yield after chromatographic purification from the compound **200** by the Method C in one hour reaction time.

$^1$H-NMR (400 MHz, DMSO-$d_6$): 1.09 (s, 3H), 1.25 (s, 9H), 1.13-2.47 (m, 13H), 2.70-2.97 (m, 3H), 7.01-7.16 (m, 3H), 7.24-7.33 (m, 1H), 7.37 (s, 1H), 7.45 (s, 1H), 12.10 (br s, 1H).

**Compound 202**

3-((13S,15R)-4-fluoro-13-methyl-17-oxo-7,8,9,11,12,13,14,15,16,17-decahydro-6H-cyclopenta[a]phenanthren-15-yl)-N-(6-fuoropyridin-2-yl)propanamide

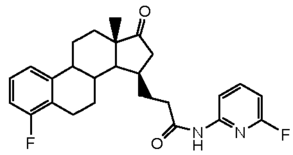

Compound **202** was synthesized in 88% yield by Method A1 in THF by using acid SM-IX and 2-amino-6-fluoropyridine as starting materials in two hours reaction time.

$^1$H-NMR (400 MHz, DMSO-$d_6$): 0.98 (s, 3H), 1.35-1.46 (m, 3H), 1.57-1.77 (m, 4H), 1.93 (m, 1H), 2.16-2.47 (m, 8H), 2.68-2.90 (m, 2H), 6.83 (dd, 1H), 6.97 (dd, 1H), 7.12-7.20 (m, 2H), 7.95 (dd, 1H), 8.01 (d, 1H), 10.69 (s, 1H).

**Compound 203**

3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)-N-(6-fluoropyridin-2-yl)propanamide

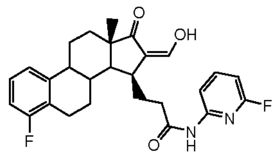

The compound **203** was prepared in 90% yield from the compound **202** by Method B stirring overnight at room temperature.

$^1$H NMR (400 MHz, DMSO-$d_6$): 0.99 (s, 3H), 1.18-2.37 (m, 13H), 2.45-2.96 (m, 4H), 6.81 (dd, 1H), 6.96 (t, 1H), 7.12-7.19 (m, 2H), 7.55 (s, 1H), 7.92-8.02 (m, 2H), 10.64 (s, 1H).

**Compound 204**

3-((8aS,12S)-3-fluoro-8a-methyl-1,2,6b,7,8,8a,10,12,12a,12b-decahydronaphtho[2',1':4,5]indeno[1,2-c]pyrazol-12-yl)-N-(6-fluoropyridin-2-yl)propanamide

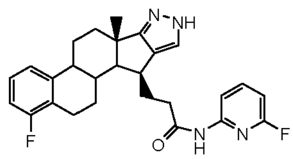

The compound **204** was prepared in 16% yield from the compound **203** by the Method C and purified by crystallization from acetonitrile.

$^1$H NMR (400 MHz, DMSO-$d_6$): 1.11 (s, 3H), 1.41-2.42 (m, 13H), 2.72-2.93 (m, 3H), 6.83 (dd, 1H), 6.98 (t, 1H), 7.16 (m, 2H), 7.40 (s, 1H), 7.94 (m, 1H), 8.02 (d, 1H), 10.72 (s, 1H), 12.13 (br s, 1H).

**Compound 205**

N-cyclohexyl-3-((13S,15R)-4-fluoro-13-methyl-17-oxo-7,8,9,11,12,13,14,15,16,17-decahydro-6H-cyclopenta[a]phenanthren-15-yl)propanamide

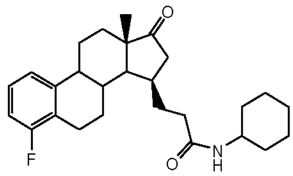

Compound 205 was synthesized in 64% yield by Method A1 using acid SM-IX and cyclohexylamine as starting materials in 2 hours reaction time. The crude product was purified by chromatography.

$^1$H-NMR (400 MHz, DMSO-$d_6$): 0.95 (s, 3H), 1.10-2.41 (m, 26H), 2.67-2.76 (m, 1H), 2.84-2.91 (m, 1H), 3.50-3.53 (m, 1H), 6.94-7.00 (m, 1H), 7.10-7.22 (m, 2H), 7.71 (br d, 1H).

**Compound 206**

N-cyclohexyl-3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)propanamide

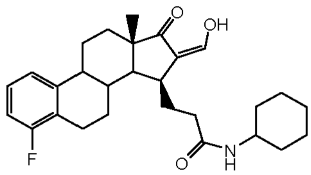

Compound **206** was synthesized in 67% yield from the compound **205** by the Method B in 2 hours reaction time.

$^1$H-NMR (400 MHz, DMSO-$d_6$): 0.97 (s, 3H), 1.09-1.90 (m, 18H), 2.03-2.16 (m, 2H), 2.30-2.45 (m, 3H), 2.65-2.76 (m, 1H), 2.80-2.95 (m, 2H), 3.50-3.65 (m, 1H), 6.90-7.02 (m, 1H), 7.08-7.25 (m, 2H), 7.50 (s, 1H), 8.14 (br s, 1H) 11.84 (br s, 1H).

**Compound 207**

N-cyclohexyl-3-((8aS,12S)-3-fluoro-8a-methyl-1,2,6b,7,8,8a,10,12,12a,12b-decahydronaphtho[2',1':4,5]indeno[1,2-c]pyrazol-12-yl)propanamide

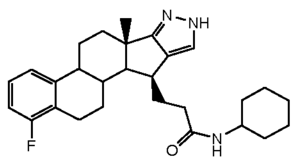

Compound **207** was synthesized in 49% yield from the compound **206** by the Method C in one hour reaction time.

$^1$H-NMR (400 MHz, DMSO-$d_6$): 1.08 (s, 3H), 1.09-1.80 (m, 16H), 1.90-2.00 (m, 1H), 2.00-2.25 (m, 4H), 2.30-2.45 (m, 2H), 2.65-2.80 (m, 2H), 2.82-2.95 (m, 1H), 3.45-3.60 (m, 1H), 6.90-7.02 (m, 1H), 7.08-7.25 (m, 2H), 7.39 (s, 1H), 7.73 (d, 1H) 12.12 (br s, 1H).

**Compound 208**

(13S,15R)-4-fluoro-13-methyl-15-(3-oxo-3-(pyrrolidin-1-yl)propyl)-6,7,8,9,11,12,13,14,15,16-decahydro-17H-cyclopenta[a]phenanthren-17-one

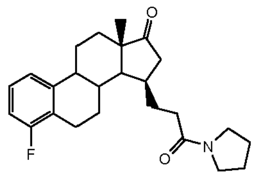

Compound **208** was synthesized in 90% yield by Method A1 using acid SM-IX and pyrrolidine as starting materials in 4 hours reaction time.

$^1$H-NMR (400 MHz, DMSO-$d_6$): 0.97 (s, 3 H), 1.43 - 2.45 (m, 20 H), 2.71 - 2.91 (m, 2 H), 3.39 - 3.47 (m, 4 H), 6.97 (dd, 1H), 7.10-7.20 (m, 2H).

**Compound 209**

(13S,15S,Z)-4-fluoro-16-(hydroxymethylene)-13-methyl-15-(3-oxo-3-(pyrroli-din-1-yl)propyl)-6,7,8,9,11,12,13,14,15,16-decahydro-17H-cyclopenta[a]phenan-thren-17-one

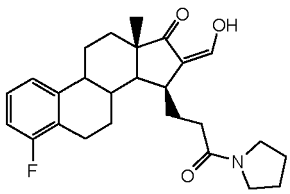

The compound **209** was prepared in 96% yield from the compound **208** by Method B stirring two hours at room temperature.

$^1$H NMR (400 MHz, $CDCl_3$): 1.08 (s, 3H), 1.48-2.99 (m, 19H), 3.16 (m, 1H), 3.43-3.66 (m, 4H), 6.88 (dd, 1H), 7.09 (m, 2H), 7.66 (d, 1H), 12.41 (d, 1H).

**Compound 210**

3-((8aS,12S)-3-fluoro-8a-methyl-1,2,6b,7,8,8a,10,12,12a,12b-decahydronaphtho[2',1':4,5]indeno[1,2-c]pyrazol-12-yl)-1-(pyrrolidin-1-yl)propan-1-one

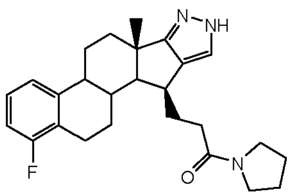

The compound **210** was prepared in 90% yield from the compound **209** by the Method C.

$^1$H NMR (400 MHz, DMSO-$d_6$): 1.10 (s, 3H), 1.36-2.42 (m, 17H), 2.67-2.91 (m, 3H), 3.27-3.47 (m, 4H), 6.98 (dd, 1H), 7.16 (m, 2H), 7.41 (s, 1H), 12.14 (br s, 1H). MS m/z (TOF $ES^+$): 422 (M+1)

**Compound 211**

N-(5-(tert-butyl)isoxazol-3-yl)-3-((13S,15R)-4-fluoro-13-methyl-17-oxo-7,8,9,11,12,13,14,15,16,17-decahydro-6H-cyclopenta[a]phenanthren-15-yl)propanamide

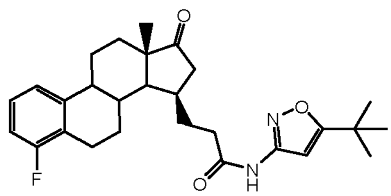

Compound **211** was synthesized in 83% yield by the Method A1 by using acid SM-IX and 3-Amino-5-*tert*-butylisoxazole as starting materials.
$^1H$ NMR (400 MHz, $CDCl_3$): 1.06 (s, 3H), 1.35 (s, 9H), 1.44-1.95 (m, 8H), 2.09- 2.61 (m, 8H), 2.75-3.00 (m, 2H), 6.73 (s, 1H), 6.88 (t, 1H), 7.08 (m, 2H), 9.54 (s, 1H).

**Compound 212**

N-(5-(tert-butyl)isoxazol-3-yl)-3-((13S,15S,Z)-4-fluoro-16-(hydroxymethylene)-13-methyl-17-oxo-7,8,9,11,12,13,14,15,16,17-decahydro-6H-cyclopenta[a]phe-nanthren-15-yl)propanamide

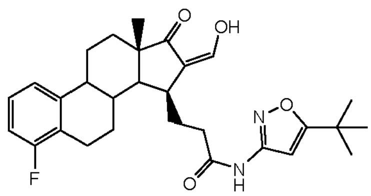

The compound **212** was prepared in 91% yield from the compound **211** by Method B stirring two hours at 0 °C.
$^1H$ NMR (400 MHz, DMSO-$d_6$): 0.99 (s, 3H), 1.28 (s, 9H), 1.38-2.30 (m, 14H), 2.68-2.95 (m, 3H), 6.59 (dd, 1H), 6.97 (t, 1H), 7.14 (m, 2H), 7.56 (s, 1H), 10.92 (s, 1H).

**Compound 213**

N-(5-(tert-butyl)isoxazol-3-yl)-3-((8aS,12S)-3-fluoro-8a-methyl-1,2,6b,7,8,8a,10,12,12a,12b-decahydronaphtho[2',1':4,5]indeno[1,2-c]pyrazol-12-yl)propanamide

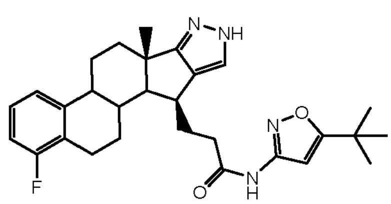

The compound **213** was prepared in 90% yield from the compound **212** by the Method C.
$^1H$ NMR (400 MHz, DMSO-$d_6$): 1.10 (s, 3H), 1.29 (s, 9H), 1.40-2.44 (m, 13H), 2.67-2.93 (m, 3H), 6.61 (s, 1H), 6.98 (t, 1H), 7.16 (m, 2H), 7.39 (s, 1H), 10.97 (s, 1H), 12.13 (br s, 1H). MS m/z (TOF $ES^+$): 491 (M +1)

**Compound 214**

N,N-diethyl-3-((13S,15R)-4-fluoro-13-methyl-17-oxo-7,8,9,11,12,13,14,15,16,17-decahydro-6H-cyclopenta[a]phenanthren-15-yl)propanamide

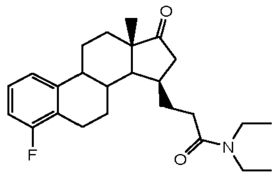

Compound **214** was synthesized in 97% yield by the Method A2 in THF using acid SM-IX and diethylamine as starting materials and 200 mol-% of EDCI and HOBT in 2 hours reaction time.

$^1$H-NMR (200 MHz, DMSO-$d_6$): 0.97 (s, 3H), 1.01 (t, 3H), 1.11 (t, 3H), 1.20-2.47 (m, 16H), 2.60-2.99 (m, 2H), 3.15-3.40 (m, 4H), 6.90-7.06 (m, 1H), 7.08-7.25 (m, 2H).

**Compound 215**

N,N-diethyl-3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)propanamide

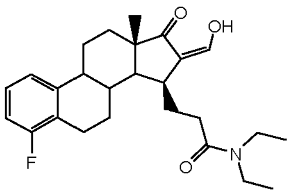

Compound **215** was synthesized in quantitative yield from the compound **214** by the Method B in overnight reaction time.

$^1$H-NMR (400 MHz, DMSO-$d_6$): 0.97 (s, 3H), 1.03 (t, 3H), 1.12 (t, 3H), 1.30-2.45 (m, 13H), 2.55-2.99 (m, 3H), 3.15-4.00 (m, 4H), 6.90-7.02 (m, 1H), 7.08-7.25 (m, 2H), 7.49 (s, 1H), 11.46 (br s, 1H).

**Compound 216**

N,N-diethyl-3-((8aS,12S)-3-fluoro-8a-methyl-1,2,6b,7,8,8a,10,12,12a,12b-decahydronaphtho[2',1':4,5]indeno[1,2-c]pyrazol-12-yl)propanamide

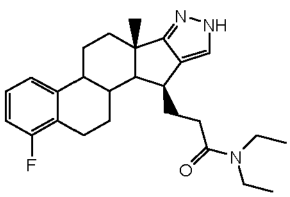

Compound **216** was synthesized in 86% yield from the compound **215** by the Method C in 0.5 hours reaction time.

$^1$H-NMR (400 MHz, DMSO-$d_6$): 1.00 (t, 3H), 1.10 (s, 3H), 1.12 (t, 3H), 1.30-2.45 (m, 13H), 2.69-2.95 (m, 3H), 3.15-3.40 (m, 4H), 6.90-7.02 (m, 1H), 7.08-7.25 (m, 2H), 7.39 (s, 1H), 12.18 (br s, 1H).

**Compound 217**

(13S,15R)-4-fluoro-13-methyl-15-(3-oxo-3-(8-oxa-2-azaspiro[4.5]decan-2-yl)propyl)-6,7,8,9,11,12,13,14,15,16-decahydro-17H-cyclopenta[a]phenanthren-17-one

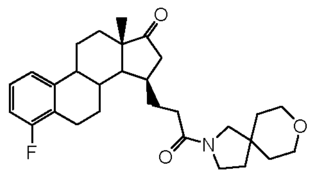

Compound **217** was synthesized in 93% yield by the Method A3 by using acid SM-IX and 8-oxa-2-aza-spiro(4,5)decane hydrochloride as starting materials in 4 hours reaction time.

$^1$H-NMR (400 MHz, DMSO-$d_6$): 0.97 (s, 3H), 1.30-2.45 (m, 22H), 2.65-2.76 (m, 1H), 2.84-2.91 (m, 1H), 3.19-3.22 (m, 1H), 3.29-3.42 (m, 2H), 3.46-3.65 (m, 5H), 6.94-7.00 (m, 1H), 7.10-7.22 (m, 2H).

**Compound 218**

(13S,15S,Z)-4-fluoro-16-(hydroxymethylene)-13-methyl-15-(3-oxo-3-(8-oxa-2-azaspiro [4.5]decan-2-yl)propyl)-6, 7,8,9,11, 12,13,14, 15, 16-decahydro-17H-cyclo-penta[a]phenanthren-17-one

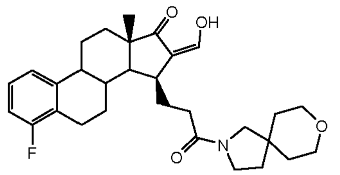

Compound **218** was synthesized in 98% yield from the compound **217** by the Method B in overnight reaction time by using 1000 mol-% of ethyl formate and 600 mol-% of NaH.

$^1$H-NMR (400 MHz, DMSO-$d_6$): 0.97 (s, 3H), 1.30-2.46 (m, 19H), 2.65-2.99 (m, 3H), 3.18-3.66 (m, 8H), 6.94-7.00 (m, 1H), 7.10-7.22 (m, 2H), 7.52 (s, 1H).

**Compound 219**

3-((8aS,12S)-3-fluoro-8a-methyl-1,2,6b,7,8,8a,10,12,12a,12b-decahydronaphtho[2',1':4,5]indeno[1,2-c]pyrazol-12-yl)-1-(8-oxa-2-azaspiro[4.5]decan-2-yl)propan-1-one

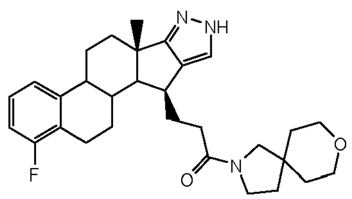

Compound **219** was synthesized in 29% yield from the compound **218** by the Method C in one hour reaction time after chromatographic purification.

$^1$H-NMR (400 MHz, DMSO-$d_6$): 1.09 (s, 3H), 1.30-2.45 (m, 19H), 2.65-2.99 (m, 3H), 3.19-3.65 (m, 8H), 6.94-7.00 (m, 1H), 7.13-7.20 (m, 2H), 7.40-7.44 (m, 1H, isom.), 12.12 (br s, 1H).

**Compound 220**

3-((13S,15R)-4-fluoro-13-methyl-17-oxo-7,8,9,11,12,13,14,15,16,17-decahydro-6H-cyclopenta[a]phenanthren-15-yl)-N,N-dimethylpropanamide

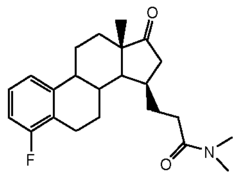

Compound **220** was synthesized in 85% yield by the Method A3 in DCM by using acid SM-IX and dimethylamine hydrochloride as starting materials in 2 hours reaction time.

$^1$H-NMR (200 MHz, DMSO-$d_6$): 0.97 (s, 3H), 1.28-2.40 (m, 16H), 2.62-2.94 (m, 2H), 2.82 (s, 3H), 2.97 (s, 3H), 6.90-7.03 (m, 1H), 7.10-7.25 (m, 2H).

**Compound 221**

3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)-N,N-dimethylpropanamide

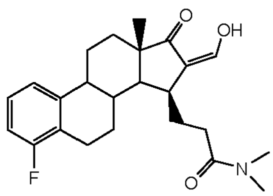

Compound **221** was synthesized in quantitative yield from the compound **220** by the Method B in 4 hours reaction time by using 500 mol-% of ethyl formate and 200 mol-% of NaH.

$^1$H-NMR (200 MHz, DMSO-$d_6$): 0.97 (s, 3H), 1.28-2.48 (m, 13H), 2.62-2.94 (m, 3H), 2.83 (s, 3H), 2.97 (s, 3H), 6.90-7.03 (m, 1H), 7.10-7.25 (m, 2H), 7.57 (br s, 1H).

**Compound 222**

3-((8aS,12S)-3-fluoro-8a-methyl-1,2,6b,7,8,8a,10,12,12a,12b-decahydronaphtho[2',1':4,5]indeno[1,2-c]pyrazol-12-yl)-N,N-dimethylpropanamide

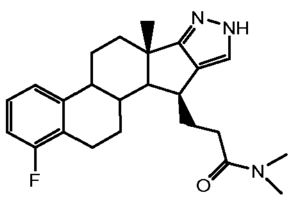

Compound **222** was synthesized in 30% yield after chromatographic purification from the compound **221** by the Method C by refluxing for one hour.

$^1$H-NMR (400 MHz, DMSO-$d_6$): 1.10 (s, 3H), 1.28-2.48 (m, 13H), 2.62-2.94 (m, 3H), 2.81 (s, 3H), 2.99 (s, 3H), 6.92-7.03 (m, 1H), 7.10-7.25 (m, 2H), 7.42 (s, 1H), 12.13 (br s, 1H).

**Compound 223**

3-((13S,15R)-4-fluoro-13-methyl-17-oxo-7,8,9,11,12,13,14,15,16,17-decahydro-6H-cyclopenta[a]phenanthren-15-yl)-N-(tetrahydrofuran-3-yl)propanamide

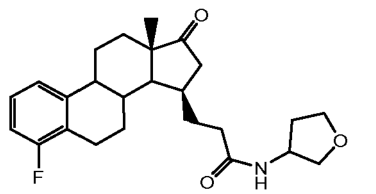

Compound **223** was synthesized in 73% yield by the Method A2 by using acid SM-IX and 3-aminotetrahydrofuran as starting materials in two hours reaction time.

$^1$H NMR (400 MHz, DMSO-$d_6$): 0.95 (s, 3H), 1.34-2.41 (m, 18H), 2.70-2.90 (m, 2H), 344 (m, 1H), 3.64-3.79 (m, 3H), 4.22 (m, 1H), 6.97 (m, 1H), 7.14 (m, 2H), 8.09 (d, 1H).

**Compound 224**

3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)-N-(tetrahydrofuran-3-yl)propanamide

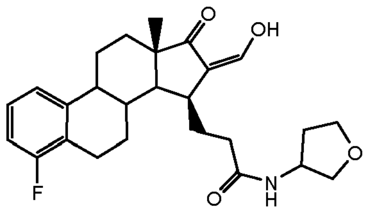

The compound **224** was prepared in 98% yield from the compound **223** by Method B stirring 1.5 hours at room temperature.

$^1$H NMR (400 MHz, DMSO-$d_6$): 0.97 (s, 3H), 1.45-2.36 (m, 15H), 2.69-2.90 (m, 3H), 3.48 (m, 3H), 3.51-3.81 (m, 3H), 6.97 (t, 1H), 7.14 (m, 2H), 7.52 (s, 1H), 8.35 (br s, 1H).

**Compound 225**

3-((8aS,12S)-3-fluoro-8a-methyl-1,2,6b,7,8,8a,10,12,12a,12b-decahydronaphtho[2',1':4,5]indeno[1,2-c]pyrazol-12-yl)-N-(tetrahydrofuran-3-yl)propanamide

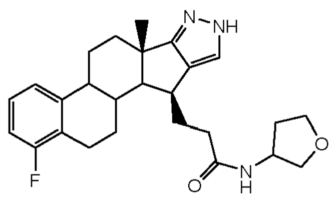

The compound **225** was prepared in 96% yield from the compound **224** by the Method C and purified by chromatography.

$^1$H NMR (400 MHz, DMSO-$d_6$): 1.09 (s, 3H), 1.36-2.41 (m, 15H), 2.75-2.92 (m, 3H), 3.45 (m, 1H), 3.66-3.81

(m, 3H), 4.23 (m, 1H), 6.98 (t, 1H), 7.15 (m, 2H), 7.39 (s, 1H), 8.12 (d, 1H), 12.12 (br s, 1H).

**Compound 226**

3-((13S,15R)-4-fluoro-13-methyl-17-oxo-7,8,9,11,12,13,14,15,16,17-decahydro-6H-cyclopenta[a]phenanthren-15-yl)-N-(6-methylpyridazin-3-yl)propanamide

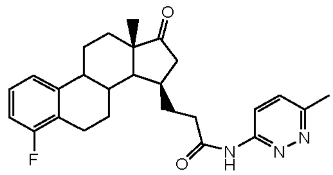

Compound **226** was synthesized in 83% yield by the Method A2 in THF by using 200 mol-% of EDCI and HOBT and acid SM-IX and 3-amino-6-methylpyridazine as starting materials in 4 hours reaction time.

$^{1}$H-NMR (200 MHz, DMSO-$d_6$): 0.98 (s, 3H), 1.20-2.47 (m, 16H), 2.55 (s, 3H), 2.70-2.95 (m, 2H), 6.89-7.06 (m, 1H), 7.08-7.25 (m, 2H), 7.54 (d, 1H), 8.23 (d, 1H), 11.05 (s, 1H).

**Compound 227**

3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)-N-(6-methylpyridazin-3-yl)propanamide

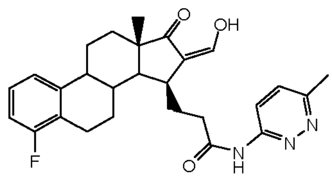

Compound **227** was synthesized in quantitative yield from the compound **226** by the Method B in overnight reaction time.

$^{1}$H-NMR (400 MHz, DMSO-$d_6$): 1.00 (s, 3H), 1.30-2.47 (m, 13H), 2.55 (s, 3H), 2.60-3.05 (m, 3H), 6.90-7.05 (m, 1H), 7.08-7.25 (m, 2H), 7.53 (d, 1H), 7.57 (s, 1H), 8.22 (d, 1H), 11.00 (s, 1H), 11.01 (br s, 1H).

**Compound 228**

3-((8aS,12S)-3-fluoro-8a-methyl-1,2,6b,7,8,8a,10,12,12a,12b-decahydronaphtho[2',1':4,5]indeno[1,2-c]pyrazol-12-yl)-N-(6-methylpyridazin-3-yl)propanamide

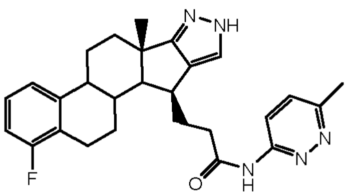

Compound **228** was synthesized in 47% yield from the compound **227** by the Method C in 0.5 hours reaction time.

$^{1}$H-NMR (400 MHz, DMSO-$d_6$): 1.11 (s, 3H), 1.30-2.47 (m, 13H), 2.55 (s, 3H), 2.69-2.95 (m, 3H), 6.90-7.05 (m, 1H), 7.08-7.25 (m, 2H), 7.41 (s, 1H), 7.54 (d, 1H), 8.23 (d, 1H), 11.07 (s, 1H), 12.16 (br s, 1H).

**Compound 229:**

3-((13S,15R)-4-fluoro-13-methyl-17-oxo-7,8,9,11,12,13,14,15,16,17-decahydro-6H-cyclopenta[a]phenanthren-15-yl)-N-(3-fluoropyridin-2-yl)propanamide

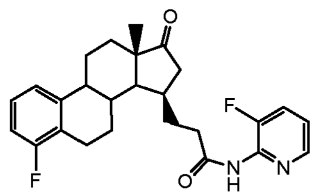

Compound **229** was synthesized in 96% yield by the Method A1 using acid SM-IX and 2-amino-3-fluoropyridine as starting materials in overnight reaction time.
$^1$H-NMR (400 MHz, DMSO-$d_6$): 0.98 (s, 3H), 1.34-1.98 (m, 8H), 2.18- 2.47 (m, 8H), 2.68-2.77 (m, 1H), 2.84-2.90 (m, 1H), 6.97 (m, 1H), 7.10-7.20 (m, 2H), 7.34 (m, 1H), 7.77 (dd, 1H), 8.24 (d, 1H), 10.28 (s, 1H).

**Compound 230**

3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)-N-(3-fluoropyridin-2-yl)propanamide

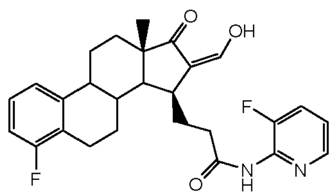

The compound **230** was prepared in 88% yield from the compound **229** by Method B.
$^1$H NMR (400 MHz, DMSO-$d_6$): 0.99 (s, 3H), 1.36-1.83 (m, 7H), 1.94 (m, 1H), 2.20-2.45 (m, 3H), 2.55-2.97 (m, 5H), 6.97 (dd, 1H), 7.14 (m, 2H), 7.35 (m, 1H), 7.55 (s, 1H), 7.75 (m, 1H), 8.23 (d, 1H), 10.24 (s, 1H), 11.01 (br s, 1H).

**Compound 231**

3-((8aS,12S)-3-fluoro-8a-methyl-1,2,6b,7,8,8a,10,12,12a,12b-decahydronaphtho[2',1':4,5]indeno[1,2-c]pyrazol-12-yl)-N-(3-fluoropyridin-2-yl)propanamide

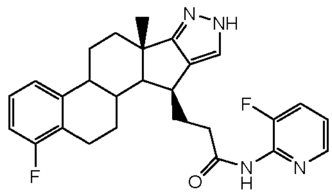

The compound **231** was prepared in 43% yield from the compound **230** by the Method C.
$^1$H NMR (400 MHz, DMSO-$d_6$): 1.11 (s, 3H), 1.41-2.39 (m, 13H), 2.67-2.92 (m, 3H), 6.97 (dd, 1H), 7.16 (m, 2H), 7.34 (m, 1H), 7.44 (s, 1H), 7.76 (m, 1H), 8.25 (d, 1H), 10.32 (s, 1H), 12.15 (br s, 1H).

**Compound 232**

(13S,15R)-4-fluoro-13-methyl-15-(3-morpholino-3-oxopropyl)-6,7,8,9,11,12,13,14,15,16-decahydro-17H-cyclopenta[a]phenanthren-17-one

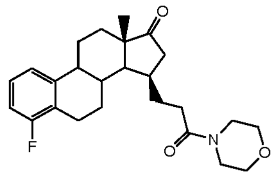

Compound **232** was synthesized in 83% yield by Method A2 in DMF using acid SM-IX and morpholine as starting materials in two hours reaction time.

$^1$H-NMR (200 MHz, DMSO-$d_6$): 0.97 (s, 3H), 1.35-2.37 (m, 15H), 2.76-2.92 (m, 3H), 3.45 (br s, 4H), 3.55 (br s 4H), 6.93-7.02 (m, 1H), 7.16-7.23 (m, 2H).

**Compound 233**

(13S,15S,Z)-4-fluoro-16-(hydroxymethylene)-13-methyl-15-(3-morpholino-3-oxopropyl)-6,7,8,9,11,12,13,14,15,16-decahydro-17H-cyclopenta[a]phenanthren-17-one

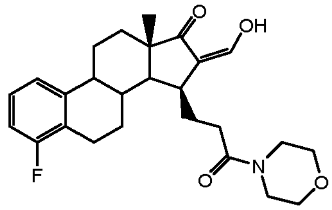

The compound **233** was prepared in quantitative yield from the compound **232** by Method B stirring 2.5 hours at room temperature.

$^1$H NMR (400 MHz, DMSO-$d_6$): 0.97 (s, 3H), 1.38-2.40 (m, 14H), 2.73-3.00 (m, 5H), 3.55 (m, 6H), 6.97 (m, 1H), 7.14 (m, 2H), 7.55 (br s, 1H).

**Compound 234**

3-((8aS,12S)-3-fluoro-8a-methyl-1,2,6b,7,8,8a,10,12,12a,12b-decahydronaphtho[2',1':4,5]indeno[1,2-c]pyrazol-12-yl)-1-morpholinopropan-1-one

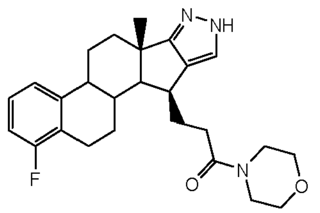

The compound **234** was prepared in 62% yield from the compound **233** by the Method C.

$^1$H NMR (400 MHz, DMSO-$d_6$): 1.09 (s, 3H), 1.34-2.43 (m, 14H), 2.74-2.94 (m, 3H), 3.45-3.56 (m, 7H), 6.98 (m, 1H), 7.17 (m, 2H), 7.42 (s, 1H), 12.15 (br s, 1H). MS m/z (TOF ES$^+$): 438 (M +1)

**Compound 235**

3-((13S,15R)-4-fluoro-13-methyl-17-oxo-7,8,9,11,12,13,14,15,16,17-decahydro-6H-cyclopenta[a]phenanthren-15-yl)-N-(pyridazin-3-yl)propanamide

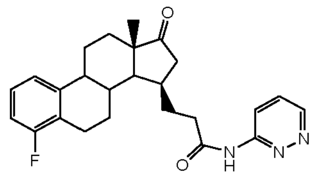

Compound **235** was synthesized in 42% yield by the Method A2 in DMF by using acid SM-IX and 3-aminopyridazine as starting materials in 2 hours reaction time, crystallized from ethanol.

$^1$H-NMR (200 MHz, DMSO-$d_6$): 0.99 (s, 3H), 1.36-2.45 (m, 16H), 2.78-2.91 (m, 2H), 6.92-6.97 (m, 1H), 7.15-7.23 (m, 2H), 7.67 (dd, 1H), 8.33 (d, 1H), 8.95 (d, 1H), 11.14 (s, 1H).

**Compound 236**

3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)-N-(pyridazin-3-yl)propanamide

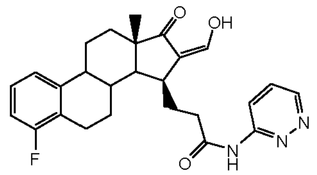

Compound **236** was synthesized in quantitative yield from the compound **235** by the Method B in overnight reaction time.

$^1$H-NMR (400 MHz, DMSO-$d_6$): 0.99 (s, 3H), 1.30-2.47 (m, 13H), 2.57-2.95 (m, 3H), 6.90-7.00 (m, 1H), 7.10-7.20 (m, 2H), 7.64 (dd, 1H), 7.73 (br s, 1H), 8.32 (d, 1H), 8.92 (d, 1H), 11.04 (br s, 1H).

**Compound 237**

3-((8aS,12S)-3-fluoro-8a-methyl-1,2,6b,7,8,8a,10,12,12a,12b-decahydronaphtho[2',1':4,5]indeno[1,2-c]pyrazol-12-yl)-N-(pyridazin-3-yl)propanamide

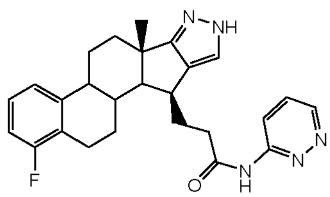

Compound **237** was synthesized in quantitative yield from the compound **236** by the Method C in 0.5 hours reaction time.

$^1$H-NMR (400 MHz, DMSO-$d_6$): 1.11 (s, 3H), 1.30-2.47 (m, 13H), 2.60-2.95 (m, 3H), 6.90-7.05 (m, 1H), 7.10-7.20 (m, 2H), 7.42 (s, 1H), 7.66 (dd, 1H), 8.33 (d, 1H), 8.95 (d, 1H), 11.17 (s, 1H), 12.15 (br s, 1H). MS m/z (TOF ES$^+$): 446 (M+1).

**Compound 238**

3-((13S,15R)-4-fluoro-13-methyl-17-oxo-7,8,9,11,12,13,14,15,16,17-decahydro-6H-cyclopenta[a]phenanthren-15-yl)-N-(4-morpholinopyridin-2-yl)propanamide

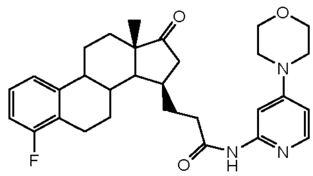

Compound **238** was synthesized in 40% yield by the Method A3 in DMF using acid SM-IX and 4-morpholinopyridin-2-amine as starting materials in overnight reaction time. The crude product was purified by chromatography.
$^{1}$H-NMR (400 MHz, DMSO-$d_6$): 0.97 (s, 3H), 1.34-2.48 (m, 16H), 2.68-2.90 (m, 2H), 3.22 (m, 4H), 3.71 (m, 4H), 6.60 (dd, 1H), 6.97 (m, 1H), 7.14 (m, 2H), 7.67 (s, 1H), 7.94 (d, 1H), 10.22 (s, 1H).

**Compound 239**

3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)-N-(4-morpholinopyridin-2-yl)propanamide

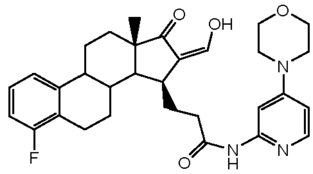

The compound **239** was prepared in 68% yield from the compound **238** by Method B stirring three hours at room temperature. The product was obtained by neutralizing the acidic phase during work-up process.
$^{1}$H NMR (400 MHz, DMSO-$d_6$): 0.99 (s, 3H), 1.18-2.37 (m, 13H), 2.68-2.94 (m, 4H), 3.23 (m, 4H), 3.71 (m, 4H), 6.60 (d, 1H), 6.96 (m, 1H), 7.14 (m, 2H), 7.55 (s, 1H), 7.63 (s, 1H), 7.93 (d, 1H), 10.18 (br s, 1H).

**Compound 240**

3-((8aS,12S)-3-fluoro-8a-methyl-1,2,6b,7,8,8a,10,12,12a,12b-decahydronaphtho[2',1':4,5]indeno[1,2-c]pyrazol-12-yl)-N-(4-morpholinopyridin-2-yl)propanamide

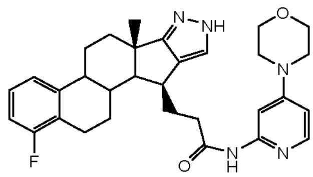

The compound **240** was prepared in 27% yield from the compound **239** by the Method C and purified by heptane trituration.
$^{1}$H NMR (400 MHz, DMSO-$d_6$): 1.10 (s, 3H), 1.42-2.42 (m, 12H), 2.67 (m, 1H), 2.76-2.92 (m, 3H), 3.22 (m, 4H), 3.71 (m, 4H), 6.60 (dd, 1H), 6.98 (m, 1H), 7.16 (m, 2H), 7.39 (s, 1H), 7.67 (s, 1H), 7.95 (d, 1H), 10.25 (s, 1H), 12.12 (br s, 1H).

**Compound 241**

3-((13S,15R)-4-fluoro-13-methyl-17-oxo-7,8,9,11,12,13,14,15,16,17-decahydro-6H-cyclopenta[a]phenanthren-15-yl)-N-(2-hydroxy-2-methylpropyl)-N-methylpropanamide

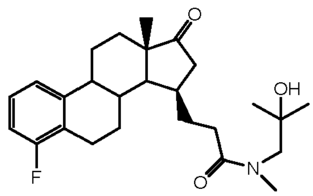

Compound **241** was synthesized in 83% yield as an isomeric mixture 60:40 by the Method A2 in DMF by using acid SM-IX and 2-methyl-1-(methylamino)-2-propanol as starting materials in 2 hours reaction time.

$^1$H-NMR (400 MHz, DMSO-$d_6$): 0.97 (s, 3H), 1.04 (s, 3H), 1.12 (s, 3H), 1.35-1.90 (m, 8H), 2.26-2.41 (m, 8H), 2.68-2.87 (m, 3H), 3.98 (s, 2H), 3.28 (m, 2H), 4.50 (s, 0.6H, isomer), 4.57 (s, 0.4, isomer), 6.97 (m, 1H), 7.15 (m, 2H).

**Compound 242**

3-((13S,15S,Z)-4-fluoro-16-(hydroxymethylene)-13-methyl-17-oxo-7,8,9,11,12,13,14,15,16,17-decahydro-6H-cyclopenta[a]phenanthren-15-yl)-N-(2-hydroxy-2-methylpropyl)-N-methylpropanamide

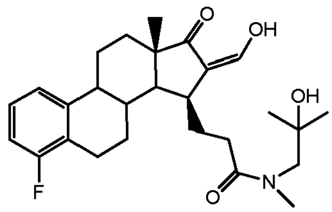

The compound **242** was prepared in 88% yield from the compound **241** by Method B stirring two hours at room temperature.

$^1$H NMR (400 MHz, DMSO-$d_6$): 0.9 (s, 3H), 1.05 (s, 3H), 1.11 (s, 3H), 1.23-2.34 (m, 15H), 2.56-2.91 (m, 5H), 3.08 (s, 2H), 4.51 (br s, 1H), 6.97 (m, 1H), 7.14 (m, 2H), 7.55 (s, 1H).

**Compound 243**

3-((8aS,12S)-3-fluoro-8a-methyl-1,2,6b,7,8,8a,10,12,12a,12b-decahydronaphtho[2',1':4,5]indeno[1,2-c]pyrazol-12-yl)-N-(2-hydroxy-2-methylpropyl)-N-methylpropanamide

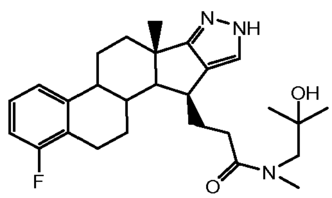

The compound **243** was prepared in 89% yield from the compound **242** by the Method C as an isomeric mixture 60:40.

$^1$H NMR (400 MHz, DMSO-$d_6$): 1.03 (d, 4H), 1.10 (s, 4H), 1.13 (s, 1H), 1.38-2.38 (m, 13H), 2.70-2.99 (m, 4H),

3.10 (s, 2H), 3.28 (m, 2H), 4.50 (s, 0.6H, isomer), 4.59 (s, 0.4H, isomer), 6.98 (dd, 1H), 7.16 (m, 2H), 7.40 (m, 1H), 12.12 (br s, 1H).

**Compound 244**

3-((13S,15R,E)-13-methyl-17-oxo-7,8,9,11,12,13,14,15,16,17-decahydro-6H-cy-clopenta[a]phenanthren-15-yl)-N-(5-methylisoxazol-3-yl)propanamide

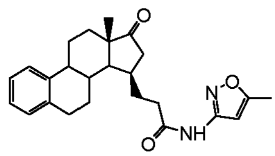

Compound **244** was synthesized in 90% yield by the Method A1 in THF by using acid SM-XXVI and 3-amino-5-methylisoxazole as starting materials in 5 hours reaction time.

$^{1}$H-NMR (400 MHz, DMSO-$d_6$): 0.97 (s, 3H), 1.29-2.49 (m, 16H), 2.36 (s, 3H), 2.80-2.95 (m, 2H), 6.64 (s, 1H), 7.05-7.15 (m, 3H), 7.24-7.28 (m, 1H), 10.88 (s, 1H). MS m/z (TOF ES$^+$): 407 (M+1), 429 (M + Na).

**Compound 245**

3-((13S,15S,Z)-16-(hydroxymethylene)-13-methyl-17-oxo-7,8,9,11,12,13,14,15,16,17-decahydro-6H-cyclopenta[a] phenanthren-15-yl)-N-(5-methylisoxazol-3-yl)propanamide

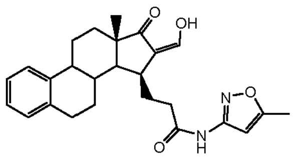

Compound **245** was synthesized in 59% yield from the compound **244** by the Method B in overnight reaction time.

$^{1}$H-NMR (400 MHz, DMSO-$d_6$): 0.99 (s, 3H), 1.34-2.60 (m, 13H), 2.36 (s, 3H), 2.80-2.99 (m, 3H), 6.62 (s, 1H), 7.03-7.15 (m, 3H), 7.23-7.30 (m, 1H), 7.56 (s, 1H), 10.88 (s, 1H), 10.95 (br s, 1H).

**Compound 246**

3-((8aS,12S)-8a-methyl-1,2,6b,7,8,8a,10,12,12a,12b-decahydronaphtho[2',1':4,5]indeno[1,2-c]pyrazol-12-yl)-N-(5-methylisoxazol-3-yl)propanamide

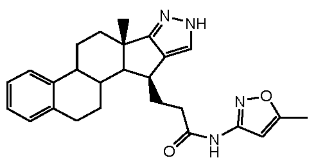

Compound **246** was synthesized in 42% yield after chromatographic purification from the compound **245** by the Method C in 1.5 hours reaction time.

$^{1}$H-NMR (400 MHz, DMSO-$d_6$): 1.10 (s, 3H), 1.34-2.50 (m, 13H), 2.36 (s, 3H), 2.80-2.99 (m, 3H), 6.64 (s, 1H), 7.05-7.15 (m, 3H), 7.25-7.31 (m, 1H), 7.37 (s, 1H), 10.90 (s, 1H), 12.11 (br s, 1H).

**Compound 247**

(13S,15R)-13-methyl-15-(3-morpholino-3-oxopropyl)-6,7,8,9,11,12,13,14,15,16-decahydro-17H-cyclopenta[a]phenanthren-17-one

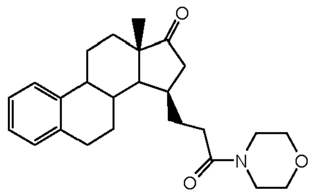

Compound **247** was synthesized in 89% yield by the Method A1 in THF by using acid SM-XXVI and pre-dried morpholino as starting materials in 1.5 hours reaction time.
$^{1}$H-NMR (400 MHz, DMSO-$d_6$): 0.97 (s, 3H), 1.33-2.44 (m, 16H), 2.87 (m, 2H), 3.44 (m, 4H), 3.56 (m, 4H), 7.06-7.14 (m, 3H), 7.27 (m, 1H).

**Compound 248**

(13S,15S,Z)-16-(hydroxymethylene)-13-methyl-15-(3-morpholino-3-oxopropyl)-6,7,8,9,11,12,13,14,15,16-decahydro-17H-cyclopenta[a]phenanthren-17-one

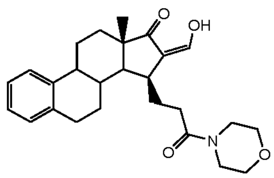

Compound **248** was synthesized in quantitative yield from the compound **247** by the Method B in overnight reaction time.
$^{1}$H-NMR (400 MHz, DMSO-$d_6$): 0.97 (s, 3H), 1.35-2.47 (m, 13H), 2.91 (m, 3H), 3.44 (m, 4H), 3.54 (m, 4H), 7.10 (m, 3H), 7.26 (d, 1H), 7.50 (s, 1H), 11.18 (br s, 1H).

**Compound 249**

3-((8aS,12S)-8a-methyl-1,2,6b,7,8,8a,10,12,12a,12b-decahydronaphtho[2',1':4,5]indeno[1,2-c]pyrazol-12-yl)-1-morpholinopropan-1-one

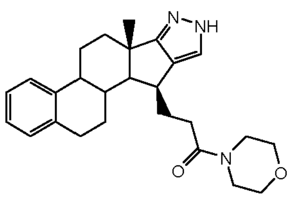

Compound **249** was synthesized in 66% yield from the compound **248** by the Method C in 30 minutes reaction time.
$^{1}$H-NMR (400 MHz, DMSO-$d_6$): 1.10 (s, 3H), 1.41-2.43 (m, 13H), 2.89 (s, 3H), 3.43-3.58 (m, 8H), 7.10 (m, 3H), 7.29 (m, 1H), 7.41 (s, 1H), 12.11 (br s, 1H).

**Compound 250**

3-((13S,15R)-13-methyl-17-oxo-7,8,9,11,12,13,14,15,16,17-decahydro-6H-cyclo-penta[a]phenanthren-15-yl)-N-(5-morpholinopyridin-2-yl)propanamide

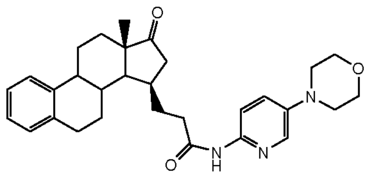

Compound **250** was synthesized in 89% yield by the Method A1 in THF by using acid SM-XXVI and 5-morpholinopyridin-2-amine as starting materials in overnight reaction time.
$^1$H-NMR (400 MHz, DMSO-$d_6$): 0.98 (s, 3H), 1.30-2.48 (m, 16H), 2.80-2.95 (m, 2H), 3.06-3.11 (m, 4H), 3.70-3.78 (m, 4H), 7.03-7.15 (m, 3H), 7.25-7.29 (m, 1H), 7.40 (dd, 1H), 7.96 (d, 1H), 8.00 (d, 1H), 10.28 (s, 1H).

**Compound 251**

3-((13S,15S,Z)-16-(hydroxymethylene)-13-methyl-17-oxo-7,8,9,11,12,13,14,15,16,17-decahydro-6H-cyclopenta[a]phenanthren-15-yl)-N-(5-morpholinopyridin-2-yl)propanamide

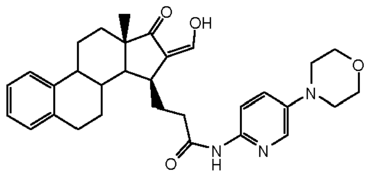

Compound **251** was synthesized in 83% yield from the compound **250** by the Method B in overnight reaction time.
$^1$H-NMR (400 MHz, DMSO-$d_6$): 0.99 (s, 3H), 1.34-2.60 (m, 13H), 2.80-2.95 (m, 3H), 3.06-3.11 (m, 4H), 3.70-3.78 (m, 4H), 7.03-7.15 (m, 3H), 7.25-7.29 (m, 1H), 7.40 (dd, 1H), 7.54 (s, 1H), 7.95 (d, 1H), 8.00 (d, 1H), 10.24 (s, 1H), 11.02 (br s, 1H).

**Compound 252**

3-((8aS,12S)-8a-methyl-1,2,6b,7,8,8a,10,12,12a,12b-decahydronaphtho[2',1':4,5]indeno[1,2-c]pyrazol-12-yl)-N-(5-morpholinopyridin-2-yl)propanamide

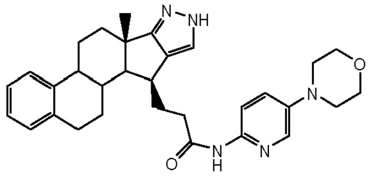

Compound **252** was synthesized in 73% yield from the compound **251** by the Method C in one hour reaction time.
$^1$H-NMR (400 MHz, DMSO-$d_6$): 1.11 (s, 3H), 1.34-2.50 (m, 13H), 2.78-2.95 (m, 3H), 3.06-3.11 (m, 4H), 3.70-3.78 (m, 4H), 7.03-7.15 (m, 3H), 7.25-7.29 (m, 1H), 7.39 (dd, 1H), 7.40 (s, 1H), 7.95 (d, 1H), 8.00 (d, 1H), 10.30 (s, 1H), 12.09 (br s, 1H).

**Compound 253**

3-((13S,15R)-13-methyl-17-oxo-7,8,9,11,12,13,14,15,16,17-decahydro-6H-cyclo-penta[a]phenanthren-15-yl)-N-(pyridazin-3-yl)propanamide

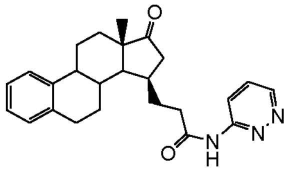

Compound **253** was synthesized in 43% yield by the Method A1 in THF by using acid SM-XXVI and 3-aminopyridazine as starting materials in overnight reaction time.
$^1$H-NMR (400 MHz, DMSO-$d_6$): 0.99 (s, 3H), 1.30-2.62 (m, 16H), 2.80-2.96 (m, 2H), 7.05-7.15 (m, 3H), 7.25-7.29 (m, 1H), 7.66 (dd, 1H), 8.32 (d, 1H), 8.95 (d, 1H), 11.13 (s, 1H).

**Compound 254**

3-((13S,15S,Z)-16-(hydroxymethylene)-13-methyl-17-oxo-7,8,9,11,12,13,14,15,16,17-decahydro-6H-cyclopenta[a]phenanthren-15-yl)-N-(pyridazin-3-yl)propanamide

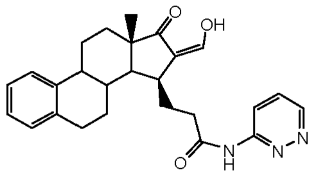

Compound **254** was synthesized in quantitative yield from the compound 253 by the Method B in 6 hours reaction time.
$^1$H-NMR (400 MHz, DMSO-$d_6$): 1.00 (s, 3H), 1.34-2.70 (m, 13H), 2.80-2.99 (m, 3H), 7.03-7.15 (m, 3H), 7.23-7.30 (m, 1H), 7.56 (s, 1H), 7.65 (dd, 1H), 8.31 (d, 1H), 8.93 (d, 1H), 10.95 (br s, 1H), 11.07 (s, 1H).

**Compound 255**

3-((8aS,12S)-8a-methyl-1,2,6b,7,8,8a,10,12,12a,12b-decahydronaphtho[2',1':4,5]indeno[1,2-c]pyrazol-12-yl)-N-(pyridazin-3-yl)propanamide

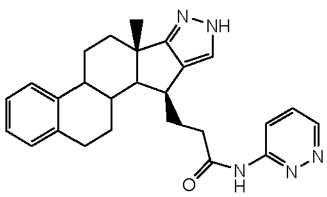

Compound **255** was synthesized in 57% yield from the compound **254** by the Method C in one hour reaction time.
$^1$H-NMR (400 MHz, DMSO-$d_6$): 1.12 (s, 3H), 1.38-2.63 (m, 13H), 2.80-2.99 (m, 3H), 7.03-7.15 (m, 3H), 7.23-7.30 (m, 1H), 7.41 (s, 1H), 7.66 (dd, 1H), 8.33 (d, 1H), 8.95 (d, 1H), 11.16 (s, 1H), 12.13 (br s, 1H).

**Compound 256**

N-(6-fluoropyridin-2-yl)-3-((13S,15R)-13-methyl-17-oxo-7,8,9,11,12,13,14,15,16,17-decahydro-6H-cyclopenta[a]phenanthren-15-yl)propanamide

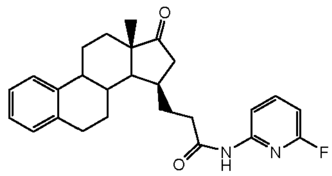

Compound **256** was synthesized in 92% yield by the Method A1 in THF by using acid SM-XXVI and 2-amino-6-fluoropyridine as starting materials in 4 hours reaction time.
$^1$H-NMR (400 MHz, DMSO-$d_6$): 0.98 (s, 3H), 1.30-2.47 (m, 16H), 2.80-2.95 (m, 2H), 6.83 (dd, 1H), 7.05-7.15 (m, 3H), 7.26-7.28 (m, 1H), 7.91-7.97 (m, 1H), 8.00-8.03 (m, 1H), 10.69 (s, 1H).

**Compound 257**

N-(6-fluoropyridin-2-yl)-3-((13S,15S,Z)-16-(hydroxymethylene)-13-methyl-17-oxo-7,8,9,11,12,13,14,15,16,17-decahydro-6H-cyclopenta[a]phenanthren-15-yl)propanamide

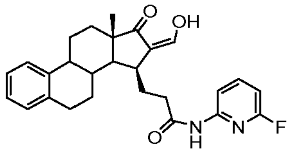

Compound **257** was synthesized in 46% yield after chromatographic purification from the compound **256** by the Method B in 2 hours reaction time.
$^1$H-NMR (400 MHz, DMSO-$d_6$): 1.00 (s, 3H), 1.34-2.60 (m, 13H), 2.80-2.99 (m, 3H), 6.81 (dd, 1H), 7.03-7.15 (m, 3H), 7.23-7.30 (m, 1H), 7.58 (s, 1H), 7.88-7.97 (m, 1H), 7.98-8.03 (m, 1H), 10.69 (s, 1H), 10.96 (br s, 1H).

**Compound 258**

N-(6-fluoropyridin-2-yl)-3-((8aS,12S)-8a-methyl-1,2,6b,7,8,8a,10,12,12a,12b-decahydronaphtho[2',1':4,5]indeno[1,2-c]pyrazol-12-yl)propanamide

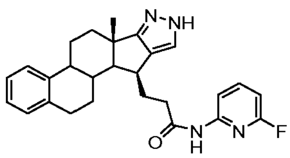

Compound **258** was synthesized in 29% yield after chromatographic purification from the compound **257** by the Method C in 1.5 hours reaction time.
$^1$H-NMR (400 MHz, DMSO-$d_6$): 1.11 (s, 3H), 1.36-2.49 (m, 13H), 2.75-2.99 (m, 3H), 6.83 (dd, 1H), 7.03-7.19 (m, 3H), 7.25-7.33 (m, 1H), 7.39 (s, 1H), 7.90-7.99 (m, 1H), 8.00-8.04 (m, 1H), 10.72 (s, 1H), 12.12 (br s, 1H).

**Compound 259**

N-(3,5-difluoropyridin-2-yl)-3-((13S,15R)-13-methyl-17-oxo-7,8,9,11,12,13,14,15,16,17-decahydro-6H-cyclopenta[a]phenanthren-15-yl)propanamide

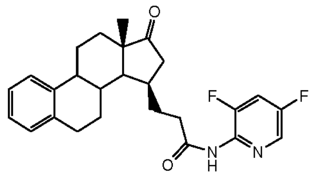

Compound **259** was synthesized in 90% yield by the Method A1 in THF by using acid SM-XXVI and 2-amino-3,5-difluoropyridine as starting materials in overnight reaction time.

$^1H$-NMR (400 MHz, DMSO-$d_6$): 0.98 (s, 3H), 1.30-2.47 (m, 16H), 2.80-2.94 (m, 2H), 7.05-7.15 (m, 3H), 7.26-7.28 (m, 1H), 7.98-8.03 (m, 1H), 8.34-8.35 (m, 1H), 10.31 (s, 1H).

**Compound 260**

N-(3,5-difluoropyridin-2-yl)-3-((13S,15S,Z)-16-(hydroxymethylene)-13-methyl-17-oxo-7,8,9,11,12,13,14,15,16,17-decahydro-6H-cyclopenta[a]phenanthren-15-yl)propanamide

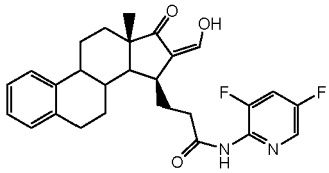

Compound **260** was synthesized in quantitative yield from the compound **259** by the Method B in 4 hours reaction time.

$^1H$-NMR (400 MHz, DMSO-$d_6$): 0.99 (s, 3H), 1.30-2.62 (m, 13H), 2.80-2.99 (m, 3H), 7.03-7.15 (m, 3H), 7.23-7.30 (m, 1H), 7.55 (s, 1H), 7.95-8.04 (m, 1H), 8.32 (d, 1H), 10.26 (s, 1H), 10.98 (br s, 1H).

**Compound 261**

N-(3,5-difluoropyridin-2-yl)-3-((8aS,12S)-8a-methyl-1,2,6b,7,8,8a,10,12,12a,12b-decahydronaphtho [2',1 ':4,5] indeno [1,2-c] pyrazol-12-yl)propanamide

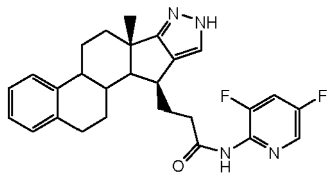

Compound **261** was synthesized in 66% yield from the compound **260** by the Method C in one hour reaction time.

$^1H$-NMR (400 MHz, DMSO-$d_6$): 1.11 (s, 3H), 1.36-2.50 (m, 13H), 2.80-2.99 (m, 3H), 7.03-7.15 (m, 3H), 7.25-7.30 (m, 1H), 7.44 (s, 1H), 7.95-8.05 (m, 1H), 8.34 (d, 1H), 10.35 (s, 1H), 12.14 (br s, 1H).

**Compound 262**

N-(3-fluoropyridin-2-yl)-3-((13S,15R)-13-methyl-17-oxo-7,8,9,11,12,13,14,15,16,17-decahydro-6H-cyclopenta[a]phenanthren-15-yl)propanamide

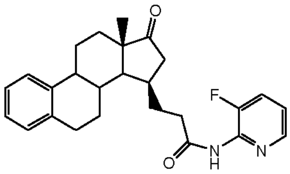

Compound **262** was synthesized in 88% yield by the Method A1 in THF by using acid SM-XXVI and 2-amino-3-fluoropyridine as starting materials in overnight reaction time.

$^1$H-NMR (400 MHz, DMSO-$d_6$): 0.99 (s, 3H), 1.30-2.47 (m, 16H), 2.81-2.96 (m, 2H), 7.05-7.16 (m, 3H), 7.26-7.28 (m, 1H), 7.30-7.37 (m, 1H), 7.73-7.79 (m, 1H), 8.23-8.25 (m, 1H), 10.27 (s, 1H).

**Compound 263**

N-(3-fluoropyridin-2-yl)-3-((13S,15S,Z)-16-(hydroxymethylene)-13-methyl-17-oxo-7,8,9,11,12,13,14,15,16,17-decahydro-6H-cyclopenta[a]phenanthren-15-yl)propanamide

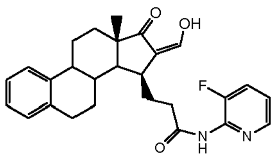

Compound **263** was synthesized in quantitative yield from the compound **262** by the Method B in 5 hours reaction time.

$^1$H-NMR (400 MHz, DMSO-$d_6$): 1.00 (s, 3H), 1.34-2.65 (m, 13H), 2.80-2.99 (m, 3H), 7.03-7.15 (m, 3H), 7.20-7.30 (m, 1H), 7.31-7.36 (m, 1H), 7.57 (s, 1H), 7.70-7.78 (m, 1H), 8.20-8.24 (m, 1H), 10.30 (s, 1H), 11.01 (br s, 1H).

**Compound 264**

N-(3-fluoropyridin-2-yl)-3-((8aS,12S)-8a-methyl-1,2,6b,7,8,8a,10,12,12a,12b-decahydronaphtho[2',1':4,5]indeno[1,2-c]pyrazol-12-yl)propanamide

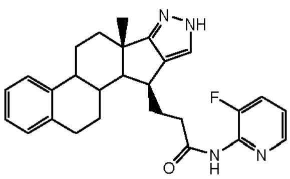

Compound **264** was synthesized in 40% yield from the compound **263** by the Method C in one hour reaction time.

$^1$H-NMR (400 MHz, DMSO-$d_6$): 1.11 (s, 3H), 1.39-2.50 (m, 13H), 2.80-2.99 (m, 3H), 7.03-7.15 (m, 3H), 7.25-7.30 (m, 1H), 7.31-7.36 (m, 1H), 7.45 (s, 1H), 7.73-7.79 (m, 1H), 8.22-8.26 (m, 1H), 10.32 (s, 1H), 12.13 (br s, 1H).

**Compound 265**

3-((13S,15R)-3-chloro-13-methyl-17-oxo-7,8,9,11,12,13,14,15,16,17-decahydro-6H-cyclopenta[a]phenanthren-15-yl)-N-(3-fluoropyridin-2-yl)propanamide

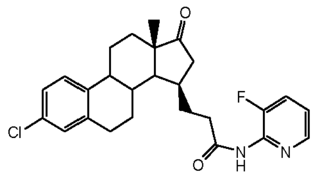

Compound **265** was synthesized in 81% yield by the Method A1 in THF by using acid SM-XVII and 2-amino-3-fluoropyridine as starting materials in overnight reaction time.
$^1$H-NMR (400 MHz, DMSO-$d_6$): 0.98 (s, 3H), 1.30-2.47 (m, 16H), 2.81-2.94 (m, 2H), 7.15-7.16 (m, 2H), 7.28-7.30 (m, 1H), 7.32-7.36 (m, 1H), 7.73-7.79 (m, 1H), 8.23-8.25 (d, 1H), 10.27 (s, 1H).

**Compound 266**

3-((13S,15S,Z)-3-chloro-16-(hydroxymethylene)-13-methyl-17-oxo-7,8,9,11,12,13,14,15,16,17-decahydro-6H-cyclopenta[a]phenanthren-15-yl)-N-(3-fluoropyridin-2-yl)propanamide

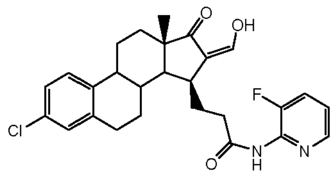

Compound **266** was synthesized in quantitative yield from the compound **265** by the Method B in 4 hours reaction time.
$^1$H-NMR (400 MHz, DMSO-$d_6$): 0.99 (s, 3H), 1.34-2.60 (m, 13H), 2.80-2.99 (m, 3H), 7.10-7.23 (m, 2H), 7.25-7.35 (m, 2H), 7.59 (s, 1H), 7.70-7.79 (m, 1H), 8.20-8.25 (m, 1H), 10.34 (br s, 1H), 11.01 (br s, 1H).

**Compound 267**

3-((8aS,12S)-4-chloro-8a-methyl-1,2,6b,7,8,8a,10,12,12a,12b-decahydronaphtho[2',1':4,5]indeno[1,2-c]pyrazol-12-yl)-N-(3-fluoropyridin-2-yl)propanamide

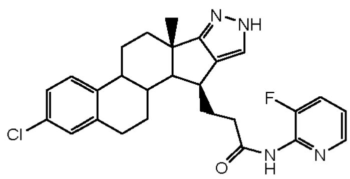

Compound **267** was synthesized in 58% yield from the compound **266** by the Method C in one hour reaction time.
$^1$H-NMR (400 MHz, DMSO-$d_6$): 1.11 (s, 3H), 1.34-2.50 (m, 13H), 2.80-2.99 (m, 3H), 7.11-7.22 (m, 2H), 7.25-7.37 (m, 2H), 7.44 (s, 1H), 7.70-7.79 (m, 1H), 8.23-8.26 (m, 1H), 10.32 (s, 1H), 12.13 (br s, 1H).

PHARMACOLOGICAL TESTS

The following tests are provided to demonstrate the present invention in illustrative way and should not be considered as limiting in the scope of invention. Further, the concentrations of the compound in the assays are exemplary and should not be taken as limiting. A person skilled in the art may define pharmaceutically relevant concentrations with method known in the art.

**Inhibition of 17β-hydroxysteroid dehydrogenase type 1 enzyme**

**17β-HSD1 production and isolation:** Recombinant baculovirus was generated by the "Bac to Bac Expression System" (Invitrogen). Recombinant bacmid was transfected to Sd9 insect cells using "Cellfectin Reagent" (Invitrogen). 60 h later cells were harvested; the microsomal fraction was isolated as described by Puranen, T.J., Poutanen, M.H., Peltoketo, H.E., Vihko, P.T. and Vihko, R.K. (1994) Site-directed mutagenesis of the putative active site of human 17 β-hydroxysteroid dehydrogenase type 1. Biochem. J. 304: 289-293. Aliquots were stored frozen until determination of enzymatic activity.

**Assay - Inhibition of recombinant human 17β-HSD1:** Recombinant protein (1 μg/ml) was incubated in 20 mM KH2PO4 pH 7.4 with 30 nM estrone (including 800 000 cpm/ml of $^{3}$H-estrone) and 1 mM NADPH for 30 min at RT, in the presence of the potential inhibitor at concentrations 1 μM or 0.1 μM. Inhibitor stock solutions were prepared in DMSO. Final concentration of DMSO was adjusted to 1% in all samples. The enzyme reaction was stopped by addition of 10% trichloroacetic acid (final concentration). Samples were centrifuged in a microtiter plate at 4000 rpm for 10 min. Supernatants were applied to reverse phase HPLC on a Waters Symmetry C18 column, equipped with a Waters Sentry Guard column. Isocratic HPLC runs were performed at RT at a flow rate of 1 ml/min in acetonitrile:water 48:52 as running solvent. Radioactivity was monitored in the eluate by a Scintillation Analyzer. Total radioactivity for estrone and estradiol were determined in each sample and percent conversion of estrone to estradiol was calculated according to the following formula:

% conversion = 100 x

{(cpm estradiol in sample with inhibitor) /

[(cpm estrone in sample with inhibitor) + (cpm estradiol in sample with inhibitor)]}

/

{[(cpm estradiol in sample without inhibitor) /

[(cpm estrone in sample without inhibitor) + (cpm estradiol in sample without inhibitor)]}.

Percent inhibition was calculated followingly: % inhibition = 100 -% conversion.

The values % inhibition were determined for exemplified compounds and the results are summarized in Table 2.

**Inhibition of the 17β-hydroxysteroid dehydrogenase type 2 enzyme**

**17β-HSD2 production and isolation:** Similarly to 17β-HSD1 the Recombinant baculovirus was generated by the "Bac to Bac Expression System" (Invitrogen). Recombinant bacmid was transfected to Sd9 insect cells using "Cellfectin Reagent" (Invitrogen). 60 h later cells were harvested and supernatant were fractionated by the following protocol:

- cells were dissolved into 40 ml of A-buffer (40 mM TRIS, pH8.0, 20% glycerol, 20 μM NAD, 0.4 mM PMSF, 150 mM NaCl, 0.5% dodecyl-(3-maltoside + protease inhibitor cocktail)
- cells were sonicated
- lysate was incubated on ice for 15 min

- lysate was centrifuged 5000 rpm 15 min, + 4°C
- centrifugation of the supernatant 180 000 g 30 min, + 4°C
- pellet was dissolved into 8 ml of A-buffer
- not resuspended material was removed by centrifugation 5000 rpm 15 min, + 4°C
- the clear supernatant was divided into 100 μl aliquots and were stored frozen until determination of enzymatic activity.

The amount of 17β-HSD2 was analysed by immunoblotting and total protein concentration of each extract batch was determined.

**Assay - Inhibition of recombinant human 17β-HSD2:** Recombinant protein (4 μg/ml) was incubated in 20 mM KH2PO4 pH 8.5 with 50 nM estradiol (including 800 000 cpm/ml of $^3$H-estradiol) and 1 mM NADH for 30 min at RT, in the presence of the potential inhibitor at concentrations 1 μM or 0.1 μM. Inhibitor stock solutions were prepared in DMSO. Final concentration of DMSO was adjusted to 1% in all samples. The enzyme reaction was stopped by addition of 10% trichloroacetic acid (final concentration). Samples were centrifuged in a microtiter plate at 4000 rpm for 10 min. Supernatants were applied to reverse phase HPLC on a Waters Symmetry C18 column, equipped with a Waters Sentry Guard column. Isocratic HPLC runs were performed at RT at a flow rate of1 ml/min in acetonitrile:water 48:52 as running solvent. Radioactivity was monitored in the eluate by a Scintillation Analyzer. Total radioactivity for estrone and estradiol were determined in each sample and percent conversion of estradiol to estrone was calculated according to the following formula:

$$\% \text{ conversion} = 100 \text{ x } \{(\text{cpm estrone in sample with inhibitor}) / [(\text{cpm estradiol in sample with inhibitor}) + (\text{cpm estrone in sample with inhibitor})]\} / \{[(\text{cpm estrone in sample without inhibitor}) / [(\text{cpm estradiol in sample without inhibitor}) + (\text{cpm estrone in sample without inhibitor})]\}.$$

Percent inhibition was calculated followingly: % inhibition = 100 -% conversion.

The values % inhibition were determined for exemplified compounds and the results are summarized in Table 2.

**Inhibition of the estrone to estradiol conversion in a rabbit tissue homogenate**

The assay is based on an enzymatic reaction where HSD1 enzyme that is expressed in rabbit placenta tissue converts its natural substance estrone (E1) to estradiol (E2) in the presence of a co-factor β-NADPH.

**Homogenization of rabbit placenta tissue:** Weight a piece of the frozen tissue into a Precellys ck28 bead tube. Add buffer solution (20 mM $KH_2PO_4$ with 1mM EDTA, pH 7,4) in 1:2 ratio (e.g. 300 mg of tissue : 600 μl reaction buffer solution). Insert the bead tubes to homogenizer and homogenize 2 x 30 s. 6000 rpm. Centrifugated 5 min., 2600 rpm at +4°C and collect supernatant. Aliquots of homogenate are stored in -80°C.

**Assay - Inhibition of E1 to E2 conversion in rabbit placenta tissue:** The reaction takes place in a buffer solution (20 mM $KH_2PO_4$ with 1mM EDTA, pH 7,4), including appropriate amount of rabbit placenta homogenate, co-factor (1 mM β-NADPH), Substrate (30 nM estrone), labelled substrate as tracer (5 nM [$^3$H]-estrone), and the potential inhibitor at concentrations 1 μM or 0.1 μM. Inhibitor stock solutions were prepared in DMSO. Final concentration of DMSO was adjusted to 1% in all samples. During a 30-minute incubation part of the estrone is converted to estradiol. The reaction is stopped by lowering the pH to 1 with 10% trichloro acetic acid (TCA). The substrate and conversion products are analyzed by HPLC and a Scintillation counter analyzer. Total radioactivity for estrone and estradiol were determined in each sample and percent conversion of estrone to estradiol was calculated according to the following formula:

$$\% \text{ conversion} = 100 \text{ x } \{(\text{cpm estradiol in sample with inhibitor}) / [(\text{cpm estrone in sample with inhibitor}) + (\text{cpm estradiol in sample with inhibitor})]\} / \{(\text{cpm estradiol in sample without inhibitor}) / [(\text{cpm estrone in sample without inhibitor}) + (\text{cpm estradiol in sample without inhibitor})]\}.$$

Percent inhibition was calculated followingly: % inhibition = 100 - % conversion. The values % inhibition were determined for exemplified compounds and the results are summarized in Table 2.

**Metabolic Stability Assay**

Compound stock solutions of the invention were prepared in DMSO. Final concentration of DMSO was adjusted to 1% in all samples. The in vitro metabolic stability of the compounds of the invention was determined for exemplified compounds using human hepatocyte incubations; study compounds at concentration of 1 μM were incubated 0, 10, 20, 40 and 60 min at 37°C. Samples were collected at all time points and compounds were detected by LC-MS/MS analysis. The percent compound remaining is calculated by comparing the peak area of the parent compound at each time point to time zero. In vitro metabolic stability was determined as half life (T1/2), which was determined by regression analysis of the percent parent disappearance vs. time curve. The results are summarized in Table 2.

PHARMACOLOGICAL TEST RESULTS

**Table 2**

| Compound | HSD1_ inhibition % @100 nM | HSD2_ inhibition % @ 1 μM | Human hepatocytes MetStab T1/2 min | Rabbit placenta inhibition % @100 nM |
|---|---|---|---|---|
| 3 | 98 | 9 | | 54 |
| 6 | 93 | 8 | | 86 |
| 9 | 82 | 3 | 61 | 26 |
| 12 | 92 | 5 | | 46 |
| 15 | 96 | 6 | 43 | 64 |
| 18 | 91 | 1 | 41 | 44 |
| 21 | 97 | 4 | 40 | 68 |
| 24 | 83 | 1 | 33 | 27 |
| 27 | 96 | 2 | | 55 |
| 30 | 95 | 3 | 117 | 53 |
| 33 | 95 | 8 | 107 | 68 |
| 36 | 100 | 8 | 37 | 55 |
| 39 | 91 | 9 | 67 | 69 |
| 42 | 91 | 34 | 47 | 82 |
| 45 | 90 | 3 | 35 | |
| 48 | 91 | 5 | 34 | 57 |
| 51 | 90 | 1 | 31 | 59 |

(continued)

| Compound | HSD1_ inhibition % @100 nM | HSD2_ inhibition % @ 1 μM | Human hepatocytes MetStab T1/2 min | Rabbit placenta inhibition % @100 nM |
|---|---|---|---|---|
| 54 | 87 | 8 | 68 | 52 |
| 57 | 92 | 16 | 58 | 48 |
| 60 | 93 | 9 | | 16 |
| 63 | 96 | 2 | | 62 |
| 66 | 74 | 3 | | 39 |
| 69 | 93 | 1 | | 10 |
| 72 | 93 | 2 | | |
| 75 | 71 | 2 | | 15 |
| 78 | 87 | 3 | | 39 |
| 81 | 81 | 3 | | 31 |
| 84 | 87 | 2 | 148 | 27 |
| 87 | 87 | 6 | 127 | 16 |
| 90 | 70 | 3 | 77 | 30 |
| 93 | 79 | 4 | 66 | 31 |
| 96 | 74 | 5 | 75 | 42 |
| 99 | 84 | 4 | 97 | 10 |
| 102 | 80 | 8 | | 19 |
| 105 | 82 | 9 | | 28 |
| 108 | 56 | -2 | | 45 |
| 111 | 48 | 1 | | 55 |
| 114 | 53 | -1 | | 43 |
| 117 | 73 | 2 | | |
| 120 | 67 | 4 | | 34 |
| 123 | 87 | 8 | 155 | 17 |
| 126 | 89 | 12 | 147 | 37 |
| 129 | 84 | 8 | | 28 |
| 135 | 86 | 2 | | 31 |
| 138 | 94 | 7 | 51 | 18 |
| 141 | 93 | -1 | 45 | 35 |
| 147 | 86 | 11 | 9 | |
| 153 | 85 | 4 | | 10 |
| 156 | 81 | -3 | 93 | 56 |
| 159 | 90 | 3 | 77 | 23 |
| 162 | 91 | 9 | 83 | 44 |
| 165 | 85 | -2 | 44 | 34 |
| 168 | 90 | 5 | 68 | 58 |
| 171 | 89 | -1 | 73 | 27 |

(continued)

| Compound | HSD1_ inhibition % @100 nM | HSD2_ inhibition % @ 1 μM | Human hepatocytes MetStab T1/2 min | Rabbit placenta inhibition % @100 nM |
|---|---|---|---|---|
| 174 | 88 | 10 | | 14 |
| 177 | 89 | 4 | | 16 |
| 180 | 92 | 12 | | 14 |
| 183 | 91 | 10 | | 50 |
| 186 | 81 | 1 | | 11 |
| 189 | 90 | 9 | 30 | 46 |
| 192 | 86 | 2 | | 14 |
| 195 | 70 | 10 | | 61 |
| 198 | 88 | 18 | | 77 |
| 201 | 87 | 4 | 13 | |
| 204 | 96 | 1 | | 63 |
| 207 | 95 | 4 | 18 | 14 |
| 210 | 94 | 0 | 11 | 19 |
| 213 | 93 | 10 | | 25 |
| 216 | 93 | 6 | 8 | 10 |
| 219 | 91 | 3 | 7 | 14 |
| 222 | 91 | 0 | 12 | 11 |
| 225 | 88 | -2 | | 10 |
| 228 | 87 | 3 | | 67 |
| 231 | 86 | 4 | 29 | 35 |
| 234 | 85 | 0 | 26 | |
| 237 | 84 | 1 | | 58 |
| 240 | 84 | 2 | | 35 |
| 243 | 80 | 5 | 15 | |
| 246 | 80 | 15 | | 19 |
| 249 | 59 | 7 | | |
| 252 | 90 | 7 | | 46 |
| 255 | 66 | 0 | | 46 |
| 258 | 90 | 2 | | 43 |
| 261 | 50 | 9 | | |
| 264 | 54 | 4 | | |

UTILITY OF THE INVENTION

Compounds of the invention show selective inhibitory potential of the 17β-HSD1 enzyme and little or no inhibitory activity to the 17β-HSD2 enzyme and therefore, may be useful for the treatment of a steroid hormone dependent disease or disorder, in particular for treatment and prevention of several diseases and conditions that include, but are not limited to, breast cancer, prostate carcinoma, ovarian cancer, uterine cancer, endometrial cancer, endometrial hyperplasia, endometriosis, uterine fibroids, adenomyosis, polycystic ovarian syndrome, dysmenorrhea, menorrhagia, metrorrhagia, contraception, prostadynia, benign prostatic hyperplasia, urinary dysfunction, lower urinary tract symptoms, chronic

prostatitis/chronic pelvic pain syndrome (CP/CPPS), systemic lupus erythematosus (SLE), multiple sclerosis, obesity, rheumatoid arthritis, chronic obstructive pulmonary disease (COPD), lung cancer, colon cancer, tissue wounds, skin wrinkles and cataracts.

Further, compounds of the present invention may be useful for the treatment of diseases and disorders associated with increased levels of estradiol and which may be prevented, treated, and/or ameliorated by an inhibitor of 17β-HSD1 enzyme.

"Treatment or prevention" as used herein includes prophylaxis, or prevention of, as well as lowering the individual's risk of falling ill with the named disorder or condition, or alleviation, amelioration, elimination, or cure of the said disorder once it has been established.

Compounds of the present invention may be administered in an effective amount within the dosage range of about 0.1 μg/kg to about 300 mg/kg, preferably between 1.0 μg/kg to 10 mg/kg body weight. Compounds of the present invention may be administered in a single daily dose, or the total daily dosage may be administered in divided doses of two, three or four times daily.

"An effective amount" refers to an amount of a compound that confers a therapeutic effect on the treated subject. The therapeutic effect may be objective (i.e. measurable by some test or marker) or subjective (i.e. subject gives an indication of or feels an effect). Such treatment need not necessarily completely ameliorate the condition of disease. Further, such treatment or prevention can be used in conjunction with other traditional treatments for reducing the condition known to those skilled in the art.

Compounds of the invention are most preferably used alone or in combination i.e. administered simultaneously, separately or sequentially with other active ingredients. Compounds of the invention may be administered by various routes, for example, oral, parenteral, subcutaneous, intravenous, intraarticular, intrathecal, intramuscular, intraperitoneal, and by intradermal injections, and via transdermal, rectal, buccal, oromucosal, nasal, ocular routes and via inhalation and via implant.

Compounds may be formulated into a suitable composition; suitable administration forms include, for example, solutions, dispersions, suspensions, powders, capsules, tablet, pills, controlled release capsules, controlled release tablets and controlled release pills. In addition to the pharmacologically active compounds, the pharmaceutical compositions of the compounds can contain suitable pharmaceutically acceptable carriers comprising excipients and auxiliaries that facilitate processing of the active compounds into preparations that can be used pharmaceutically.

Furthermore, compounds of formula (I) can be used as synthesis intermediates for the preparation of other compounds, in particular of other pharmaceutically active ingredients, which are obtainable from compounds of formula (I), for example by introduction of substituents or modification of functional groups. The invention and its embodiments are not limited to the examples described above but may vary within the scope of the claims.

## Claims

1. A compound of formula (I)

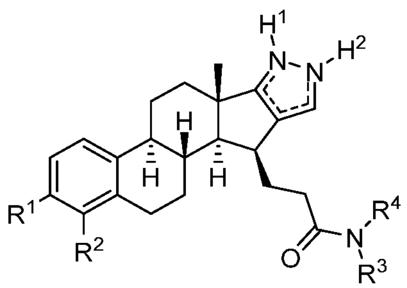

(I)

wherein

R1 and R2 are each independently selected from the group consisting of H and halogen;

(i) R3 is selected from the group consisting of H and C1-4 alkyl and

R4 is selected from the group consisting of

- phenyl, 6 membered unsaturated, or aromatic heterocycle comprising 1 to 3 heteroatom(s) independently

selected from the group consisting of nitrogen, sulfur, and oxygen, and being optionally substituted with one to five substituent(s) independently selected from the group consisting of halogen, CN, C1-4-alkyl, C1-3-(per)haloalkyl, OH, oxo, C1-3-alkoxy, morpholino, $C(O)N(C1\text{-}3\text{-alkyl})_2$ and a 6 membered saturated heterocycle with 1 to 3 heteroatom(s) selected from the group consisting of nitrogen, oxygen and sulfur, optionally substituted with C1-4-alkyl; and
- 6 membered saturated heterocycle comprising 1 to 3 heteroatom(s) independently selected from the group consisting of nitrogen, oxygen and sulfur and being optionally substituted with one to three substituent(s) independently selected from the group consisting of halogen, CN, C1-4-alkyl, C1-3-(per)haloalkyl, OH, oxo, and C1-3-alkoxy, or two adjacent substituents may form a 5 or 6 membered saturated fused ring;

provided that only one of hydrogens $H^1$ and $H^2$ is present at the same time, and the position of the double bonds in the pyrazole ring to which the hydrogens $H^1$ and $H^2$ are attached is determined based on which of the hydrogen $H^1$ and $H^2$ is present
or a pharmaceutically acceptable salt thereof.

**2.** The compound according to claim 1, wherein the compound has formula (Ia)

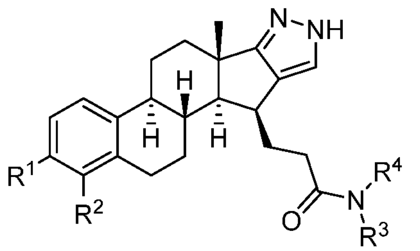

(Ia)

in which formula (Ia) R1, R2, R3, and R4 are as defined in claim 1,
or a pharmaceutically acceptable salt thereof.

**3.** The compound according to claim 1 or 2, wherein

- R1 is selected from the group consisting of H, F and Cl,
- R2 is selected from the group consisting of H, F and Cl, and
- R3 and R4 are as defined in claim 1,

or a pharmaceutically acceptable salt thereof.

**4.** The compound according to any of the claims 1 to 3, wherein

- R1 is selected from the group consisting of H, F and Cl,
- R2 is H or F, and
- R3 and R4 are as defined in claim 1,

or a pharmaceutically acceptable salt thereof.

**5.** The compound according to any of the claims 1 to 4, wherein

- R1 is H
- R2 is F, and
- R3 and R4 are as defined in claim 1,

or a pharmaceutically acceptable salt thereof.

**6.** The compound according to any of the claims 1 to 5, wherein

- R3 is H,
- R4 is a 6 membered unsaturated or aromatic heterocycle with 1 to 3 heteroatoms selected from the group consisting of nitrogen, oxygen and sulfur, optionally substituted with one or two substituent(s) selected from the

group consisting of CN, C1-4-alkyl, C1-3-alkoxy, halogen and $C(O)N(C1\text{-}3\text{-alkyl})_2$ or
- R4 is

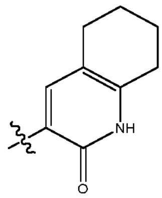

or a pharmaceutically acceptable salt thereof.

**7.** The compound according to any of the claims 1 to 3, wherein

- R1 is H, Cl or F,
- R2 is H, Cl or F,
- R3 is H,
- R4 is a 6 membered aromatic heterocycle with 1 to 3 heteroatoms selected from the group consisting of nitrogen, oxygen and sulfur, optionally substituted with one or two substituent(s) selected from the group consisting of CN, C1-4-alkyl, C1-3-alkoxy, halogen and $C(O)N(C1\text{-}3\text{-alkyl})_2$, or
- R4 is

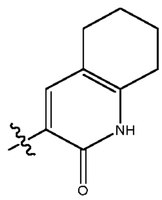

or a pharmaceutically acceptable salt thereof.

**8.** The compound according to any of the claims 1 to 4 or 7, wherein

- R1 is H or F,
- R2 is H or F,
- R3 is H,
- R4 is a 6 membered aromatic heterocycle with 1 to 3 heteroatoms selected from the group consisting of nitrogen, oxygen and sulfur, optionally substituted with one or two substituent(s) selected from the group consisting of CN, C1-4-alkyl, C1-3-alkoxy, halogen and $C(O)N(C1\text{-}3\text{-alkyl})_2$, or
- R4 is

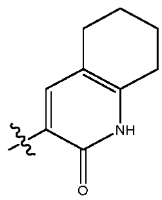

or a pharmaceutically acceptable salt thereof.

**9.** The compound according to any of the claims 1 to 4 or claims 7 to 8, wherein

- R1 is H,
- R2 is H or F,
- R3 is H,

- R4 is a 6 membered aromatic heterocycle with 1 to 3 heteroatoms selected from the group consisting of nitrogen, oxygen and sulfur, optionally substituted with one or two substituent(s) selected from the group consisting of CN, C1-4-alkyl, C1-3-alkoxy, halogen and C(O)N(C1-3-alkyl)$_2$,

or a pharmaceutically acceptable salt thereof.

**10.** The compound according to any of the claims 1 to 6 or claims 7 to 8, wherein

- R1 is H,
- R2 is F,
- R3 is H,
- R4 is a 6 membered aromatic heterocycle with 1 to 3 heteroatoms selected from the group consisting of nitrogen, oxygen and sulfur, optionally substituted with one or two substituent(s) selected from the group consisting of CN, methyl, methoxy, F and C(O)N(methyl)$_2$, or
- R4 is

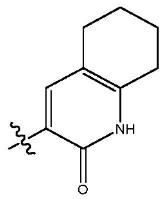

or a pharmaceutically acceptable salt thereof.

**11.** The compound according to any of the claims 1 to 10, wherein the compound is selected from the group consisting of:

-3-((SaS, 125) -3- fluoro-Sa -methyl-1,2,6b, 7,S,8a, 10, 12, 12a, 12b-decahydronaphtho [2’,1’:4,5] indeno [1,2-c]pyrazol-12-yl)-*N*-(6-methoxypyridazin-3-yl)-propanamide,
-*N*-(5-Cyanopyridin-2-yl)-3-((8a5’,125")-3-fluoro-8a-methyl-1,2,6b,7,8,8a,10,12,12a,12b-decahydronaphtho[2’,1’:4,5]indeno[1,2-c]pyrazol-12-yl)propanamide,
-3-((8a*S*,12*S*)-3-fluoro-Sa-methyl-1,2,6b,7,S,8a,10,12,12a,12b-decahydronaphtho[2’,1’:4,5]indeno[1,2-c] pyrazol-12-yl)-*N*-(4-fluoropyridin-2-yl)propanamide,
-3-((8a*S*,12*S*)-3-fluoro-8a-methyl-1,2,6b,7,8,8a,10,12,12a,12b-decahydronaphtho [2’,1’:4,5] indeno [1,2-c] pyrazol-12-yl)-*N*-(5-methoxypyridin-2-yl)propanamide,
-3-((SaS, 12S) -3-fluoro-Sa-methyl-1,2,6b, 7,S,Sa, 10, 12, 12a, 12b-decahydronaphtho[2’,1’:4,5]indeno[1,2-c] pyrazol-12-yl)-N-(4-methylpyridin-2-yl)propanamide,
-3-((8aS,12S)-3-fluoro-8a-methyl-1,2,6b,7,8,8a,10,12,12a,12b-decahydronaphtho[2’,1’:4,5]indeno[1,2-c]pyrazol-12-yl)-N-(2-oxo-1,2,5,6,7,8-hexahydroquinolin-3-yl)propanamide,
-6-(3-((8aS,12S)-3-fluoro-8a-methyl-1,2,6b,7,8,8a,10,12,12a,12b-decahydronaphtho[2’,1’:4,5]indeno[1,2-c] pyrazol-12-yl)propanamido)-N,N-dimethylnicotinamide,
-N-(6-methoxypyridazin-3-yl)-3-((8aS,12S)-8a-methyl-1,2,6b,7,8,8a,10,12,12a,12b-decahydronaphtho[2’,1’:4,5]indeno[1,2-c]pyrazol-12-yl)propanamide,
- N,N -dimethyl-6-(3-((SaS, 12S) -Sa-methyl-1,2,6b, 7,S,Sa, 10, 12, 12a, 12b-decahydronaphtho [2’, 1’:4,S] indeno [1,2-c] pyrazol- 12 -yl) propanamido) nicotinamide,
-N-(5-fluoropyridin-2-yl)-3-((8aS,12S)-8a-methyl-1,2,6b,7,8,8a,10,12,12a,12b-decahydronaphtho[2’,1’:4,5]indeno[1,2-c]pyrazol-12-yl)propanamide, and
-N-(4-fluoropyridin-2-yl)-3-((8aS,12S)-8a-methyl-1,2,6b,7,8,8a,10,12,12a,12b-decahydronaphtho[2’,1’:4,5]indeno[1,2-c]pyrazol-12-yl)propanamide,

or a pharmaceutically acceptable salt thereof.

**12.** A method for the preparation of a compound of formula (I) as defined in any of claims 1 to 11, comprising reacting a compound of formula (II)

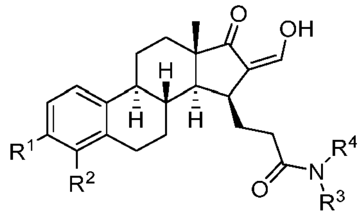

(II)

in which formula (II) R1, R2, R3 and R4 are as defined in claim 1, with hydrazine hydrate to obtain a compound of formula (I); and optionally converting the compound of formula (I) to a pharmaceutically acceptable salt thereof.

**13.** A compound of formula (II)

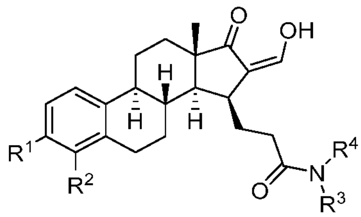

(II)

in which formula (II) R1, R2, R3 and R4 are as defined in claim 1.

**14.** A compound according to any of the claims 1 to 11, for use as a medicament.

**15.** A compound according to any of the claims 1 to 11, for use in treatment or prevention of a disease selected from a group consisting of breast cancer, prostate carcinoma, ovarian cancer, uterine cancer, endometrial cancer, endometrial hyperplasia, endometriosis, uterine fibroids, adenomyosis, polycystic ovarian syndrome, dysmenorrhea, menorrhagia, metrorrhagia, contraception, prostadynia, benign prostatic hyperplasia, urinary dysfunction, lower urinary tract symptoms, chronic prostatitis/chronic pelvic pain syndrome (CP/CPPS), systemic lupus erythematosus (SLE), multiple sclerosis, obesity, rheumatoid arthritis, chronic obstructive pulmonary disease (COPD), lung cancer, colon cancer, tissue wounds, skin wrinkles and cataracts.

**16.** A compound according to any of the claims 1 to 11, for use in treatment of a disease selected from a group consisting of breast cancer, prostate carcinoma, ovarian cancer, uterine cancer, endometrial cancer, endometrial hyperplasia, endometriosis, uterine fibroids, adenomyosis, polycystic ovarian syndrome, dysmenorrhea, menorrhagia, metrorrhagia, contraception, prostadynia, benign prostatic hyperplasia, urinary dysfunction, lower urinary tract symptoms, chronic prostatitis/chronic pelvic pain syndrome (CP/CPPS), systemic lupus erythematosus (SLE), multiple sclerosis, obesity, rheumatoid arthritis, chronic obstructive pulmonary disease (COPD), lung cancer, colon cancer, tissue wounds, skin wrinkles and cataracts.

**17.** A pharmaceutical composition comprising an effective amount of one or more compound(s) according to any of claims 1 to 11, together with one or more pharmaceutically acceptable excipient(s).

**18.** The pharmaceutical composition according to claim 17, in combination with one or more other active ingredient(s).

**Patentansprüche**

**1.** Verbindung mit der Formel (I)

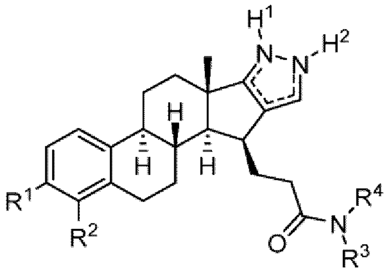

(I)

wobei

R1 und R2 jeweils unabhängig ausgewählt sind aus der Gruppe, die besteht aus H und Halogen;

(i) R3 ausgewählt ist aus der Gruppe, die besteht aus H und C1-4-Alkyl, und

R4 ausgewählt ist aus der Gruppe, die besteht aus

- Phenyl, 6-gliedrigem ungesättigtem oder aromatischem Heterocyclus, der 1 bis 3 Heteroatom/e umfasst, die unabhängig ausgewählt sind aus der Gruppe, die besteht aus Stickstoff, Schwefel und Sauerstoff, und wahlweise substituiert mit einem bis fünf Substituent/en, die unabhängig ausgewählt sind aus der Gruppe, die besteht aus Halogen, CN, C1-4-Alkyl, C1-3-(Per)Haloalkyl, OH, Oxo, C1-3-Alkoxy, Morpholino, C(O)N(C1-3-Alkyl)$_2$ und einem 6-gliedrigen gesättigten Heterocyclus mit 1 bis 3 Heteroatom/en, die ausgewählt sind aus der Gruppe, die besteht aus Stickstoff, Sauerstoff und Schwefel, wahlweise substituiert mit C1-4-Alkyl; und
- 6-gliedrigem gesättigtem Heterocyclus, der 1 bis 3 Heteroatom/e umfasst, die unabhängig ausgewählt sind aus der Gruppe, die besteht aus Stickstoff, Sauerstoff und Schwefel, und wahlweise substituiert mit einem bis drei Substituenten, die unabhängig ausgewählt sind aus der Gruppe, die besteht aus Halogen, CN, C1-4-Alkyl, C1-3-(Per)Haloalkyl, OH, Oxo und C1-3-Alkoxy, oder zwei benachbarte Substituenten einen 5- oder 6-gliedrigen gesättigten kondensierten Ring bilden können;

unter der Maßgabe, dass nur einer der Wasserstoffe $H^1$ und $H^2$ gleichzeitig vorhanden ist und die Position der Doppelbindungen in dem Pyrazolring, mit dem die Wasserstoffe $H^1$ und $H^2$ gebunden sind, basierend darauf bestimmt wird, welcher von dem Wasserstoff $H^1$ und $H^2$ vorhanden ist,
oder ein pharmazeutisch annehmbares Salz davon.

**2.** Verbindung nach Anspruch 1, wobei die Verbindung die Formel (Ia) aufweist

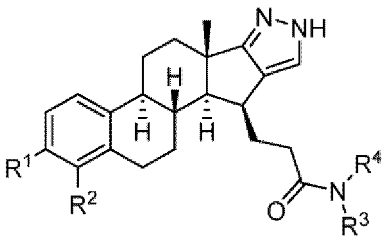

(Ia)

wobei in der Formel (Ia) R1, R2, R3 und R4 sind wie in Anspruch 1 definiert,
oder ein pharmazeutisch annehmbares Salz davon.

**3.** Verbindung nach Anspruch 1 oder 2, wobei

- R1 ausgewählt ist aus der Gruppe, die besteht aus H, F und Cl,
- R2 ausgewählt ist aus der Gruppe, die besteht aus H, F und Cl, und
- R3 und R4 sind wie in Anspruch 1 definiert,

oder ein pharmazeutisch annehmbares Salz davon.

**4.** Verbindung nach einem der Ansprüche 1 bis 3, wobei

- R1 ausgewählt ist aus der Gruppe, die besteht aus H, F und Cl,
- R2 H oder F ist, und
- R3 und R4 sind wie in Anspruch 1 definiert,

oder ein pharmazeutisch annehmbares Salz davon.

**5.** Verbindung nach einem der Ansprüche 1 bis 4, wobei

- R1 H ist
- R2 F ist, und
- R3 und R4 sind wie in Anspruch 1 definiert,

oder ein pharmazeutisch annehmbares Salz davon.

**6.** Verbindung nach einem der Ansprüche 1 bis 5, wobei

- R3 H ist,
- R4 ein 6-gliediger ungesättigter oder aromatischer Heterozyklus mit 1 bis 3 Heteroatomen ist, die ausgewählt sind aus der Gruppe, die besteht aus Stickstoff, Sauerstoff und Schwefel, wahlweise substituiert mit einem oder zwei Substituenten, die ausgewählt sind aus der Gruppe, die besteht aus CN, C1-4-Alkyl, C1-3-Alkoxy, Halogen und C(O)N(C1-3-Alkyl)$_2$ oder
- R4 ist:

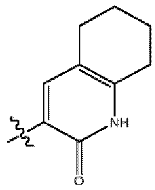

oder ein pharmazeutisch annehmbares Salz davon.

**7.** Verbindung nach einem der Ansprüche 1 bis 3, wobei

- R1 H, Cl oder F ist,
- R2 H, Cl oder F ist,
- R3 H ist,
- R4 ein 6-gliediger aromatischer Heterozyklus mit 1 bis 3 Heteroatomen ist, die ausgewählt sind aus der Gruppe, die besteht aus Stickstoff, Sauerstoff und Schwefel, wahlweise substituiert mit einem oder zwei Substituenten, die ausgewählt sind aus der Gruppe, die besteht aus CN, C1-4-Alkyl, C1-3-Alkoxy, Halogen und C(O)N(C1-3-Alkyl)$_2$, oder
- R4 ist:

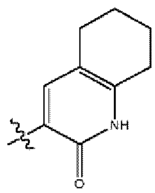

oder ein pharmazeutisch annehmbares Salz davon.

**8.** Verbindung nach einem der Ansprüche 1 bis 4 oder 7, wobei

- R1 H oder F ist,
- R2 H oder F ist,
- R3 H ist,
- R4 ein 6-gliediger aromatischer Heterozyklus mit 1 bis 3 Heteroatomen ist, die ausgewählt sind aus der Gruppe, die besteht aus Stickstoff, Sauerstoff und Schwefel, wahlweise substituiert mit einem oder zwei Substituenten, die ausgewählt sind aus der Gruppe, die besteht aus CN, C1-4-Alkyl, C1-3-Alkoxy, Halogen und C(O)N(C1-3-Alkyl)$_2$ oder
- R4 ist:

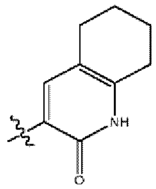

oder ein pharmazeutisch annehmbares Salz davon.

**9.** Verbindung nach einem der Ansprüche 1 bis 4 oder der Ansprüche 7 bis 8, wobei

- R1 H ist,
- R2 H oder F ist,
- R3 H ist,
- R4 ein 6-gliediger aromatischer Heterozyklus mit 1 bis 3 Heteroatomen ist, die ausgewählt sind aus der Gruppe, die besteht aus Stickstoff, Sauerstoff und Schwefel, wahlweise substituiert mit einem oder zwei Substituenten, die ausgewählt sind aus der Gruppe, die besteht aus CN, C1-4-Alkyl, C1-3-Alkoxy, Halogen und C(O)N(C1-3-Alkyl)$_2$,

oder ein pharmazeutisch annehmbares Salz davon.

**10.** Verbindung nach einem der Ansprüche 1 bis 6 oder der Ansprüche 7 bis 8, wobei

- R1 H ist,
- R2 F ist,
- R3 H ist,
- R4 ein 6-gliediger aromatischer Heterozyklus mit 1 bis 3 Heteroatomen ist, die ausgewählt sind aus der Gruppe, die besteht aus Stickstoff, Sauerstoff und Schwefel, wahlweise substituiert mit einem oder zwei Substituenten, die ausgewählt sind aus der Gruppe, die besteht aus CN, Methyl, Methoxy und C(O)N(Methyl)$_2$, oder
- R4 ist:

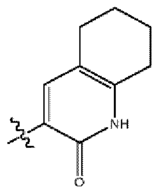

oder ein pharmazeutisch annehmbares Salz davon.

**11.** Verbindung nach einem der Ansprüche 1 bis 10, wobei die Verbindung ausgewählt ist aus der Gruppe, die besteht aus:

-3-((8aS,12S)-3-Fluor-8a-methyl-1,2,6b,7,8,8a,10,12,12a,12b-decahydronaphtho[2',1':4,5]indeno[1,2-c]pyrazol-12-yl)-N-(6-methoxypyridazin-3-yl)-propanamid,
-N-(5-Cyanopyridin-2-yl)-3-((8a5,12S)-3-fluor-8a-methyl-1,2,6b,7,8,8a,10,12,12a,12b-decahydronaphtho[21,11 :4,5]indeno[1,2-c]pyrazol-12-yl)propanamid,

-3-((8aS,12S)-3-Fluor-8a-methyl-1,2,6b,7,8,8a,10,12,12a,12b-decahydronaphtho[2',1':4,5]indeno[1,2-c]pyrazol-12-yl)-N-(4-fluorpyridin-2-yl)propanamid,
-3-((8aS,12S)-3-Fluor-8a-methyl-1,2,6b,7,8,8a,10,12,12a,12b-decahydronaphtho[2',1':4,5]indeno[1,2-c]pyrazol-12-yl)-N-(5-methoxypyridin-2-yl)propanamid,
-3-((8aS,12S)-3-Fluor-8a-methyl-1,2,6b,7,8,8a,10,12,12a,12b-decahydronaphtho[2',1':4,5]indeno[1,2-c]pyrazol-12-yl)-N-(4-methylpyridin-2-yl)pro-panamid,
-3-((8aS,12S)-3-Fluor-8a-methyl-1,2,6b,7,8,8a,10,12,12a,12b-decahydronaphtho[2',1':4,5]indeno[1,2-c]pyrazol-12-yl)-N-(2-oxo-1,2,5,6,7,8-hexahy-droquinolin-3-yl)propanamid,
-6-(3-((8aS,12S)-3-Fluor-8a-methyl-1,2,6b,7,8,8a,10,12,12a,12b-decahydronaphtho[2',1':4,5]indeno[1,2-c]pyrazol-12-yl)propanamido)-N,N-dimethyl-nicotinamid,
-N-(6-Methoxypyridazin-3-yl)-3-((8aS,12S)-8a-methyl-1,2,6b,7,8,8a,10,12,12a,12b-decahydronaphtho[2',1':4,5]indeno[1,2-c]pyrazol-12-yl)propanamid,
-N,N-Dimethyl-6-(3-((8aS,12S)-8a-methyl-1,2,6b,7,8,8a,10,12,12a,12b-decahydronaphtho[2',1':4,5]indeno[1,2-c]pyrazol-12-yl)propanamido)nicotinamid,
-N-(5-Fluorpyridin-2-yl)-3-((8aS,12S)-8a-methyl-1,2,6b,7,8,8a,10,12,12a,12b-decahydronaphtho[2',1':4,5]indeno[1,2-c]pyrazol-12-yl)propanamid, und
-N-(4-Fluorpyridin-2-yl)-3-((8aS,12S)-8a-methyl-1,2,6b,7,8,8a,10,12,12a,12b-decahydronaphtho[2',1':4,5]indeno[1,2-c]pyrazol-12-yl)propanamid,

oder ein pharmazeutisch annehmbares Salz davon.

**12.** Verfahren zur Herstellung einer Verbindung mit der Formel (I) nach einem der Ansprüche 1 bis 11, welches das Reagieren einer Verbindung mit der Formel (II),

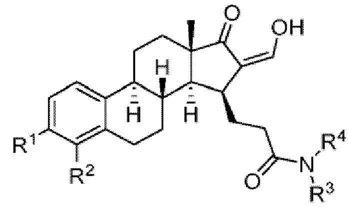
(II)

wobei in der Formel (II) R1, R2, R3 und R4 sind wie in Anspruch 1 definiert,
mit Hydrazinhydrat, um eine Verbindung mit der Formel (I) zu erhalten; und wahlweise das Umsetzen der Verbindung mit der Formel (I) zu einem pharmazeutisch annehmbaren Salz davon umfasst.

**13.** Verbindung mit der Formel (II)

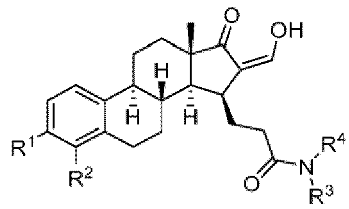
(II)

wobei in der Formel (II) R1, R2, R3 und R4 sind wie in Anspruch 1 definiert.

**14.** Verbindung nach einem der Ansprüche 1 bis 11 zur Verwendung als ein Arzneimittel.

**15.** Verbindung nach einem der Ansprüche 1 bis 11 zur Verwendung bei der Behandlung oder Prävention einer Krankheit, die ausgewählt ist aus einer Gruppe, die besteht aus Brustkrebs, Prostatakarzinom, Eierstockkrebs, Gebärmutterkrebs, Endometriumkarzinom, Endometriumhyperplasie, Endometriose, Uterusmyomen, Adenomyose, polyzystischem Ovarialsyndrom, Dysmenorrhoe, Menorrhagie, Metrorrhagie, Empfängnisverhütung, Prostatodynie, benigner Prostatahyperplasie, Harnwegsfunktionsstörungen, Symptomen der unteren Harnwege, chronischer Prostatitis/chronischem Beckenschmerzsyndrom (CP/CPPS), systemischem Lupus erythematodes (SLE), multipler Sklerose, Adipositas, rheumatoider Arthritis, chronischobstruktiver Lungenerkrankung (COPD), Lungenkrebs, Darmkrebs, Gewebewunden, Hautfalten und Katarakten.

16. Verbindung nach einem der Ansprüche 1 bis 11 zur Verwendung bei der Behandlung einer Krankheit, die ausgewählt ist aus einer Gruppe, die besteht aus Brustkrebs, Prostatakarzinom, Eierstockkrebs, Gebärmutterkrebs, Endometriumkarzinom, Endometriumhyperplasie, Endometriose, Uterusmyomen, Adenomyose, polyzystischem Ovarialsyndrom, Dysmenorrhoe, Menorrhagie, Metrorrhagie, Empfängnisverhütung, Prostatodynie, benigner Prostatahyperplasie, Harnwegsfunktionsstörungen, Symptomen der unteren Harnwege, chronischer Prostatitis/chronischem Beckenschmerzsyndrom (CP/CPPS), systemischem Lupus erythematodes (SLE), multipler Sklerose, Adipositas, rheumatoider Arthritis, chronischobstruktiver Lungenerkrankung (COPD), Lungenkrebs, Darmkrebs, Gewebewunden, Hautfalten und Katarakten.

17. Pharmazeutische Zusammensetzung, die eine wirksame Menge von einer oder mehreren Verbindungen nach einem der Ansprüche 1 bis 11, zusammen mit einem oder mehreren pharmazeutisch annehmbaren Hilfsstoffen umfasst.

18. Pharmazeutische Zusammensetzung nach Anspruch 17 in Kombination mit einem oder mehreren anderen Wirkstoffen.

## Revendications

1. Composé de formule (I)

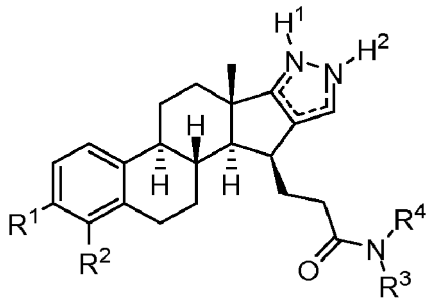

(I)

dans laquelle

chacun de R1 et R2 est indépendamment choisi dans le groupe constitué par H et un halogène ;

(i) R3 est choisi dans le groupe constitué par H et alkyle en C1 à C4 et

R4 est choisi dans le groupe constitué par

- un phényle, un hétérocycle à 6 chaînons insaturé ou aromatique comprenant 1 à 3 hétéroatomes indépendamment choisis dans le groupe constitué par l'azote, le soufre et l'oxygène, et éventuellement substitué par un à cinq substituants indépendamment choisis dans le groupe constitué par un halogène, CN, alkyle en C1 à C4, (per)halogénoalkyle en C1 à C3, OH, oxo, alcoxy en C1 à C3, morpholino, C(O)N(alkyle en C1 à C3)$_2$ et un hétérocycle à 6 chaînons saturé ayant 1 à 3 hétéroatomes choisis dans le groupe constitué par l'azote, l'oxygène et le soufre, éventuellement substitué par alkyle en C1 à C4 ; et
- un hétérocycle à 6 chaînons saturé comprenant 1 à 3 hétéroatomes indépendamment choisis dans le groupe constitué par l'azote, l'oxygène et le soufre et éventuellement substitué par un à trois substituants indépendamment choisis dans le groupe constitué par un halogène, CN, alkyle en C1 à C4, (per)halogénoalkyle en C1 à C3, OH, oxo, et alcoxy en C1 à C3, ou bien deux substituants adjacents peuvent former un cycle à 5 ou 6 chaînons saturé condensé ;

sous réserve qu'un seul des hydrogènes $H^1$ et $H^2$ soit présent en même temps, et que la position des doubles liaisons dans le cycle pyrazole auquel les hydrogènes $H^1$ et $H^2$ sont attachés soit déterminée sur la base duquel parmi les hydrogènes $H^1$ et $H^2$ est présent,
ou un sel pharmaceutiquement acceptable de celui-ci.

2. Composé selon la revendication 1, lequel composé est de formule (Ia)

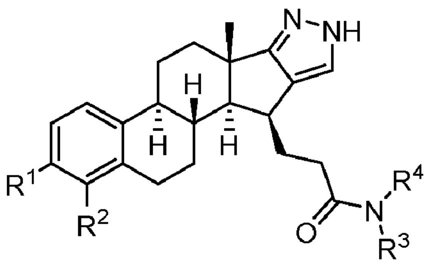

(Ia)

formule (Ia) dans laquelle R1, R2, R3 et R4 sont tels que définis dans la revendication 1, ou un sel pharmaceutiquement acceptable de celui-ci.

**3.** Composé selon la revendication 1 ou 2, dans lequel

- R1 est choisi dans le groupe constitué par H, F et Cl,
- R2 est choisi dans le groupe constitué par H, F et Cl, et
- R3 et R4 sont tels que définis dans la revendication 1,

ou un sel pharmaceutiquement acceptable de celui-ci.

**4.** Composé selon l'une quelconque des revendications 1 à 3, dans lequel

- R1 est choisi dans le groupe constitué par H, F et Cl,
- R2 est H ou F, et
- R3 et R4 sont tels que définis dans la revendication 1,

ou un sel pharmaceutiquement acceptable de celui-ci.

**5.** Composé selon l'une quelconque des revendications 1 à 4, dans lequel

- R1 est H,
- R2 est F, et
- R3 et R4 sont tels que définis dans la revendication 1,

ou un sel pharmaceutiquement acceptable de celui-ci.

**6.** Composé selon l'une quelconque des revendications 1 à 5, dans lequel

- R3 est H,
- R4 est un hétérocycle à 6 chaînons insaturé ou aromatique ayant 1 à 3 hétéroatomes choisis dans le groupe constitué par l'azote, l'oxygène et le soufre, éventuellement substitué par un ou deux substituants choisis dans le groupe constitué par un halogène, CN, alkyle en C1 à C4, alcoxy en C1 à C3 et C(O)N(alkyle en C1 à C3)$_2$ ou
- R4 est

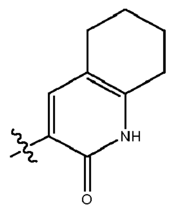

ou un sel pharmaceutiquement acceptable de celui-ci.

**7.** Composé selon l'une quelconque des revendications 1 à 3, dans lequel

- R1 est H, Cl ou F,

- R2 est H, Cl ou F,
- R3 est H,
- R4 est un hétérocycle à 6 chaînons aromatique ayant 1 à 3 hétéroatomes choisis dans le groupe constitué par l'azote, l'oxygène et le soufre, éventuellement substitué par un ou deux substituants choisis dans le groupe constitué par un halogène, CN, alkyle en C1 à C4, alcoxy en C1 à C3 et C(O)N(alkyle en C1 à C3)$_2$ ou
- R4 est

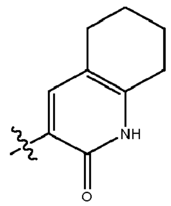

ou un sel pharmaceutiquement acceptable de celui-ci.

**8.** Composé selon l'une quelconque des revendications 1 à 4 et 7, dans lequel

- R1 est H ou F,
- R2 est H ou F,
- R3 est H,
- R4 est un hétérocycle à 6 chaînons aromatique ayant 1 à 3 hétéroatomes choisis dans le groupe constitué par l'azote, l'oxygène et le soufre, éventuellement substitué par un ou deux substituants choisis dans le groupe constitué par un halogène, CN, alkyle en C1 à C4, alcoxy en C1 à C3 et C(O)N(alkyle en C1 à C3)$_2$ ou
- R4 est

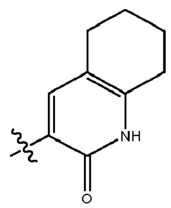

ou un sel pharmaceutiquement acceptable de celui-ci.

**9.** Composé selon l'une quelconque des revendications 1 à 4, 7 et 8, dans lequel

- R1 est H,
- R2 est H ou F,
- R3 est H,
- R4 est un hétérocycle à 6 chaînons aromatique ayant 1 à 3 hétéroatomes choisis dans le groupe constitué par l'azote, l'oxygène et le soufre, éventuellement substitué par un ou deux substituants choisis dans le groupe constitué par un halogène, CN, alkyle en C1 à C4, alcoxy en C1 à C3 et C(O)N(alkyle en C1 à C3)$_2$,

ou un sel pharmaceutiquement acceptable de celui-ci.

**10.** Composé selon l'une quelconque des revendications 1 à 6, 7 et 8, dans lequel

- R1 est H,
- R2 est F,
- R3 est H,
- R4 est un hétérocycle à 6 chaînons aromatique ayant 1 à 3 hétéroatomes choisis dans le groupe constitué par l'azote, l'oxygène et le soufre, éventuellement substitué par un ou deux substituants choisis dans le groupe constitué par CN, méthyle, méthoxy, F et C(O)N(méthyle)$_2$, ou
- R4 est

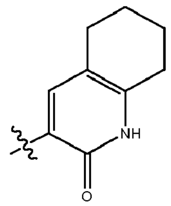

ou un sel pharmaceutiquement acceptable de celui-ci.

**11.** Composé selon l'une quelconque des revendications 1 à 10, lequel composé est choisi dans le groupe constitué par les suivants :

- 3-((8aS,12S)-3-fluoro-8a-méthyl-1,2,6b,7,8,8a,10,12,12a,12b-décahydronaphto[2',1':4,5]indéno[1,2-c]pyrazol-12-yl)-N-(6-méthoxypyridazin-3-yl)-propanamide,
- N-(5-cyanopyridin-2-yl)-3-((8aS,12S)-3-fluoro-8a-méthyl-1,2,6b,7,8,8a,10,12,12a,12b-décahydronaphto[2',1':4,5]indéno[1,2-c]pyrazol-12-yl)propanamide,
- 3-((8aS,12S)-3-fluoro-8a-méthyl-1,2,6b,7,8,8a,10,12,12a,12b-décahydronaphto[2',1' : 4,5]indéno[1,2-c]pyrazol-12-yl)-N-(4-fluoropyridin-2-yl)propanamide,
- 3-((8aS,12S)-3-fluoro-8a-méthyl-1,2,6b,7,8,8a,10,12,12a,12b-décahydronaphto[2',1':4,5]indéno[1,2-c]pyrazol-12-yl)-N-(5-méthoxypyridin-2-yl)propanamide,
- 3-((8aS,12S)-3-fluoro-8a-méthyl-1,2,6b,7,8,8a,10,12,12a,12b-décahydronaphto[2',1':4,5]indéno[1,2-c]pyrazol-12-yl)-N-(4-méthylpyridin-2-yl)propanamide,
- 3-((8aS,12S)-3-fluoro-8a-méthyl-1,2,6b,7,8,8a,10,12,12a,12b-décahydronaphto[2',1':4,5]indéno[1,2-c]pyrazol-12-yl)-N-(2-oxo-1,2,5,6,7,8-hexahydroquinolin-3-yl)propanamide,
- 6-(3-((8aS,12S)-3-fluoro-8a-méthyl-1,2,6b,7,8,8a,10,12,12a,12b-décahydronaphto[2',1':4,5]indéno[1,2-c]pyrazol-12-yl)propanamido)-N,N-diméthylnicotinamide,
- N-(6-méthoxypyridazin-3-yl)-3-((8aS,12S)-8a-méthyl-1,2,6b,7,8,8a,10,12,12a,12b-décahydronaphto[2',1':4,5]indéno[1,2-c]pyrazol-12-yl)propanamide,
- N,N-diméthyl-6-(3-(8aS,12S)-8a-méthyl-1,2,6b,7,8,8a,10,12,12a,12b-décahydronaphto[2',1':4,5]indéno[1,2-c]pyrazol-12-yl)propanamido)nicotinamide,
- N-(5-fluoropyridin-2-yl)-3-((8aS,12S)-8a-méthyl-1,2,6b,7,8,8a,10,12,12a,12b-décahydronaphto[2',1':4,5]indéno[1,2-c]pyrazol-12-yl)propanamide, et
- N-(4-fluoropyridin-2-yl)-3-((8aS,12S)-8a-méthyl-1,2,6b,7,8,8a,10,12,12a,12b-décahydronaphto[2',1':4,5]indéno[1,2-c]pyrazol-12-yl)propanamide,

ou un sel pharmaceutiquement acceptable de celui-ci.

**12.** Procédé pour la préparation d'un composé de formule (I) tel que défini dans l'une quelconque des revendications 1 à 11,
comprenant la réaction d'un composé de formule (II)

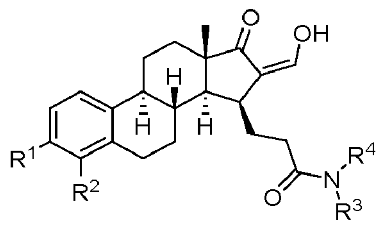

(II)

formule (II) dans laquelle R1, R2, R3 et R4 sont tels que définis dans la revendication 1,
avec de l'hydrate d'hydrazine pour que soit obtenu un composé de formule (I) ; et éventuellement la conversion du composé de formule (I) en un sel pharmaceutiquement acceptable de celui-ci.

**13.** Composé de formule (II)

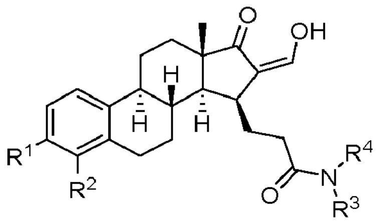

(II)

formule (II) dans laquelle R1, R2, R3 et R4 sont tels que définis dans la revendication 1.

**14.** Composé selon l'une quelconque des revendications 1 à 11, pour une utilisation en tant que médicament.

**15.** Composé selon l'une quelconque des revendications 1 à 11, pour une utilisation dans le traitement ou la prévention d'une maladie choisie dans le groupe constitué par un cancer du sein, un carcinome de la prostate, un cancer ovarien, un cancer utérin, un cancer endométrial, une hyperplasie endométriale, une endométriose, des fibroïdes utérins, une adénomyose, un syndrome des ovaires polykystiques, une dysménorrhée, une ménorrhagie, une métrorrhagie, une contraception, une prostatodynie, une hyperplasie prostatique bénigne, un dysfonctionnement urinaire, des symptômes des voies urinaires inférieures, une prostatite chronique/syndrome douloureux pelvien chronique (CP/CPPS), un lupus érythémateux disséminé (LED), une sclérose en plaques, une obésité, une polyarthrite rhumatoïde, une broncho-pneumopathie chronique obstructive (BPCO), un cancer pulmonaire, un cancer du côlon, des lésions tissulaires, des rides cutanées et des cataractes.

**16.** Composé selon l'une quelconque des revendications 1 à 11, pour une utilisation dans le traitement d'une maladie choisie dans le groupe constitué par un cancer du sein, un carcinome de la prostate, un cancer ovarien, un cancer utérin, un cancer endométrial, une hyperplasie endométriale, une endométriose, des fibroïdes utérins, une adénomyose, un syndrome des ovaires polykystiques, une dysménorrhée, une ménorrhagie, une métrorrhagie, une contraception, une prostatodynie, une hyperplasie prostatique bénigne, un dysfonctionnement urinaire, des symptômes des voies urinaires inférieures, une prostatite chronique/syndrome douloureux pelvien chronique (CP/CPPS), un lupus érythémateux disséminé (LED), une sclérose en plaques, une obésité, une polyarthrite rhumatoïde, une broncho-pneumopathie chronique obstructive (BPCO), un cancer pulmonaire, un cancer du côlon, des lésions tissulaires, des rides cutanées et des cataractes.

**17.** Composition pharmaceutique comprenant une quantité efficace d'un ou plusieurs composés de l'une quelconque des revendications 1 à 11, conjointement avec un ou plusieurs excipients pharmaceutiquement acceptables.

**18.** Composition pharmaceutique selon la revendication 17, en combinaison avec un ou plusieurs autres principes actifs.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 200142181 A
- WO 2003022835 A
- WO 2003033487 A
- WO 2004046111 A
- WO 2004060488 A
- WO 2004110459 A
- WO 2005032527 A
- WO 2005084295 A
- WO 2004085457 A
- WO 2006003012 A
- WO 2006003013 A
- WO 2004085345 A
- WO 2006027347 A
- WO 2005047303 A
- WO 2008034796 A
- WO 9946279 A
- WO 2014207309 A
- WO 2014207310 A
- WO 2014207311 A
- WO 2008124922 A, Labrie
- WO 2006125800 A
- WO 2008065100 A
- WO 2010059943 A
- WO 2004089971 A, Labrie, Fernand


### Non-patent literature cited in the description


- **POIRIER D.** *Curr Med Chem,* 2003, vol. 10, 453-77
- **POIRIER D.** *Expert Opin. Ther. Patents,* 2010, vol. 20 (9), 1123-1145
- **HORWITZ et al.** *J. Med. Chem.,* 1986, vol. 29 (5), 692-698
- **KOBAYASHI.** *Tetrahedron,* 2015, vol. 71 (35), 5918-5924
- **MESSINGER et al.** *Mol Cell Endocrinol.,* 2009, vol. 301, 216-224
- **FURUYA et al.** *JACS,* 2009, vol. 13 (15), 1662
- **PAN et al.** *Organic Letters,* 2011, vol. 13 (18), 4974-4976
- **PURANEN, T.J. ; POUTANEN, M.H. ; PELTOKETO, H.E. ; VIHKO, P.T. ; VIHKO, R.K.** Site-directed mutagenesis of the putative active site of human 17 β-hydroxysteroid dehydrogenase type 1. *Biochem. J.,* 1994, vol. 304, 289-293